# EUROPEAN PATENT APPLICATION

(11) **EP 3 967 687 A1**
(43) Date of publication of application: **16.03.2022**
(21) Application number: 20805531.9
(22) Date of filing: 09.05.2020
(51) Int. Cl.: C07D 401/12, C07D 401/10, C07D 405/14, A61K 31/4418, A61P 35/00

(54) **SUBSTITUTED PHENYLPROPENYLPYRIDINE DERIVATIVE, AND PREPARATION METHOD THEREFOR AND MEDICAL USE THEREOF**

(30) Priority: 10.05.2019 CN 201910388895
(71) Applicant: Shanghai Haiyan Pharmaceutical Technology Co., Ltd, Shanghai 201203 (CN); Yangtze River Pharmaceutical Group Co., Ltd., Taizhou, Jiangsu 225321 (CN)
(72) Inventor: ZHU, Weixing, Shanghai 201203 (CN); GUAN, Huiping, Shanghai 201203 (CN); CHEN, Yonggang, Shanghai 201203 (CN); LI, Shaobo, Shanghai 201203 (CN); HAO, Xuhui, Shanghai 201203 (CN); LIU, Qiang, Shanghai 201203 (CN); QIN, Cong, Shanghai 201203 (CN); ZHU, Jie, Shanghai 201203 (CN); ZHANG, Qing, Shanghai 201203 (CN); HUANG, Dong, Shanghai 201203 (CN); LIU, Jing, Shanghai 201203 (CN)
(74) Representative: Dekker, Henrike Cornelie Christine
(86) International application number: PCT/CN2020/089481
(87) International publication number: WO 2020/228649

(57) **Abstract**

Disclosed are a substituted phenylpropenylpyridine derivative, a preparation method therefor and the use thereof as a PD-1/PD-L1 inhibitor. In particular, disclosed are a compound of formula (I) or a pharmaceutically acceptable salt, stereoisomer, solvate, or prodrug thereof, a preparation method therefor and the use thereof. The definition of each group in the formula is detailed in the description and claims.

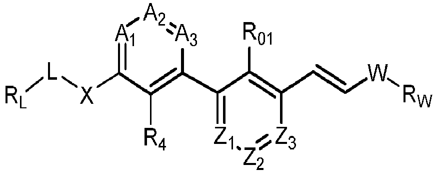

## Description

### TECHNICAL FIELD

The present disclosure belongs to the field of medical technology. In particular, the present disclosure particularly relates to substituted phenylpropenyl pyridine derivatives and their preparation method, use as a PD-1/PD-L1 immunomodulator, and pharmaceutical compositions prepared therefrom.

### BACKGROUND OF THE INVENTION

Programmed cell death protein- 1 (PD-1) is a member of the CD28 superfamily that delivers negative signals upon interaction with its two ligands, PD-L1 or PD-L2. PD-1 and its ligands are broadly expressed and exert a wider range of immunoregulatory roles in the activation of T cells and tolerance to T cells compared with other CD28 members. PD-1 and its ligands are involved in attenuating infectious immunity and tumor immunity, and facilitating chronic infection and tumor progression. The biological significance of PD- 1 and its ligand suggests the therapeutic potential of manipulation of PD- 1 pathway against various human diseases (Ariel Pedoeem et al., Curr Top Microbiol Immunol. (2011); 350:17-37).

T-cell activation and dysfunction relies on direct and modulated receptors. Based on their functional outcome, co-signaling molecules can be divided as co-stimulators and co-inhibitors, which positively and negatively control the priming, growth, differentiation and functional maturation of a T-cell response (Li Shi, et al., Journal of Hematology & Oncology 2013, 6:74).

Therapeutic antibodies that block the programmed cell death protein-1 (PD-1) immune checkpoint pathway prevent T-cell down regulation and promote immune responses against cancer. Several PD-1 pathway inhibitors have shown robust activity in various phases of clinical trials (RD Harvey, Clinical Pharmacology & Therapeutics (2014); 96 2, 214-223).

Programmed cell death protein-1 (PD-1) is a co-receptor that is expressed predominantly by T cells. The binding of PD-1 to its ligands, PD-L1 or PD-L2, is vital for the physiological regulation of the immune system. A major functional role of the PD-1 signaling pathway is the inhibition of self-reactive T cells, which serve to protect against autoimmune diseases. Elimination of the PD-1 pathway can therefore result in the breakdown of immune tolerance that can ultimately lead to the development of pathogenic autoimmunity. Conversely, tumor cells can at times co-opt the PD-1 pathway to escape from immunosurveillance mechanisms. Therefore, blockade of the PD-1 pathway has become an attractive target in cancer therapy. Current approaches include six agents that are either PD-1 and PD-L1 targeted neutralizing antibodies or fusion proteins. More than forty clinical trials are underway to better define the role of PD-1 blockade in variety of tumor types (Hyun-Tak Jin et al., Clinical Immunology (Amsterdam, Netherlands) (2014), 153(1), 145-152).

PD-1 or PD-L1 inhibitory antibodies or fusion proteins are reported in international applications.

Further, peptides or peptidomimetic compounds, which are capable of suppressing and/or inhibiting the programmed cell death protein-1 (PD-1) signaling pathway, are reported in international applications.

Still there is a need for more potent, better and/or selective immune modulators of PD-1 pathway. The present disclosure provides 1,3,4-diazole and 1,3,4-thiadiazole compounds which are capable of suppressing and/or inhibiting the programmed cell death protein-1 (PD-1) signaling pathway.

### SUMMARY OF THE INVENTION

The purpose of the present disclosure is to provide compounds with novel structures that can be used as PD-1/PD-L1 inhibitors.

In the first aspect, the present disclosure provides a compound represented by formula (I) or a stereoisomer thereof, or a pharmaceutically acceptable salt, solvate or prodrug thereof: 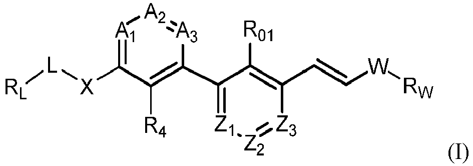 wherein,
L, W are each independently C₆₋₁₀ aryl, C₅₋₁₄ heteroaryl or 3- to 16-membered saturated or unsaturated heterocycle; the C₆₋₁₀ aryl, C₅₋₁₄ heteroaryl, 3- to 16-membered saturated or unsaturated heterocycle are unsubstituted or substituted with 1, 2 or 3 substituents independently selected from Rp;
A₁ is N or CR₁; A₂ is N or CR₂; A₃ is N or CR₃; A₁, A₂, A₃ are not N at the same time;
Z₁ is N or CR₀₂; Z₂ is N or CR₀₃; Z₃ is N or CR₀₄; Z₁, Z₂, Z₃ are not N at the same time;
R₁, R₂, R₃, R₀₂, R₀₃, R₀₄ are each independently hydrogen or -X₁-(CR₁₁R₁₂)ₙ₁-X₂-(CR₂₁R₂₂)ₙ₂-R_{A};
X is a bond, O, S, NR₅, -C(O)NR₅-, -NR₅C(O)-, -SO₂NR₅-, -NR₅SO₂-, -NR₅C(O)NR₅-, -NR₅C(O)O-, -CH=CH-, -C≡C-, -(CR₆R₇)_{q}-, -(CR₆R₇)_{q}-O-, -O-(CR₆R₇)_{q}-, C₃₋₆ cycloalkyl or 3- to 6-membered saturated monoheterocycle; the C₃₋₆ cycloalkyl, 3- to 6-membered saturated monoheterocycle are optionally substituted with 1, 2 or 3 groups independently selected from halogen, C₁₋₃ alkyl, C₁₋₃ alkoxy, halogenated C₁₋₃ alkyl, halogenated C₁₋₃ alkoxy;
X₁, X₂ are each independently a bond, O, S, NR₈, -C(O)-, -C(O)NR₈-, -NR₈C(O)-, -SO₂NR₈-, -NR₈SO₂-,-NR₈C(O)NR₈-, -NR₈C(O)O-, -CH=CH-, -C≡C-, -(CR₉R₁₀)ᵣ-, -(CR₉R₁₀)ᵣ-O-, -O-(CR₉R₁₀)ᵣ-, C₃₋₆ cycloalkyl or 3- to 6-membered saturated monoheterocycle; the C₃₋₆ cycloalkyl, 3- to 6-membered saturated monoheterocycle are optionally substituted with 1, 2 or 3 groups independently selected from halogen, C₁₋₃ alkyl, C₁₋₃ alkoxy, halogenated C₁₋₃ alkyl, halogenated C₁₋₃ alkoxy;
n1, n2 are each independently 0, 1, 2, 3, or 4;
q, r are each independently 1, 2, 3 or 4;
R₅, R₆, R₇, R₈, R₉, R₁₀ are each independently hydrogen, cyano, acetyl, hydroxyl, carboxyl, nitro, halogen (preferably F or Cl), substituted or unsubstituted C₁₋₁₀ alkyl (preferably C₁₋₆ alkyl, more preferably C₁₋₃ alkyl), substituted or unsubstituted C₁₋₁₀ alkoxy (preferably C₁₋₆ alkoxy, more preferably C₁₋₃ alkoxy), halogenated C₁₋₁₀ alkyl (preferably halogenated C₁₋₆ alkyl, more preferably halogenated C₁₋₃ alkyl), halogenated C₁₋₁₀ alkoxy (preferably halogenated C₁₋₆ alkoxy, more preferably halogenated C₁₋₃ alkoxy), halogenated C₁₋₁₀ alkylthio (preferably halogenated C₁₋₆ alkylthio, more preferably halogenated C₁₋₃ alkylthio), NRₐ₀R_{b0}, substituted or unsubstituted C₃₋₁₀ cycloalkyl, substituted or unsubstituted C₆₋₁₀ aryl, substituted or unsubstituted C₅₋₁₄ heteroaryl, substituted or unsubstituted 3- to 16-membered saturated or unsaturated heterocycle; wherein Rₐ₀, R_{b0} are each independently hydrogen, C₁₋₃ alkyl or halogenated C₁₋₃ alkyl;
R₄, R₀₁, R₁₁, R₁₂, R₂₁, R₂₂ are each independently hydrogen, cyano, acetyl, hydroxyl, carboxyl, nitro, halogen (preferably F or Cl), substituted or unsubstituted C₁₋₁₀ alkyl (preferably C₁₋₆ alkyl, more preferably C₁₋₃ alkyl), substituted or unsubstituted C₁₋₁₀ alkoxy (preferably C₁₋₆ alkoxy, more preferably C₁₋₃ alkoxy), halogenated C₁₋₁₀ alkyl (preferably halogenated C₁₋₆ alkyl, more preferably halogenated C₁₋₃ alkyl), halogenated C₁₋₁₀ alkoxy (preferably halogenated C₁₋₆ alkoxy, more preferably halogenated C₁₋₃ alkoxy), halogenated C₁₋₁₀ alkylthio (preferably halogenated C₁₋₆ alkylthio, more preferably halogenated C₁₋₃ alkylthio), NRₐ₀R_{b0}, substituted or unsubstituted C₃₋₁₀ cycloalkyl, substituted or unsubstituted C₆₋₁₀ aryl, substituted or unsubstituted C₅₋₁₄ heteroaryl, substituted or unsubstituted 3- to 16-membered saturated or unsaturated heterocycle; wherein Rₐ₀, R_{b0} are each independently hydrogen, C₁₋₃ alkyl or halogenated C₁₋₃ alkyl;
the "substituted" means that 1, 2, 3, 4 or 5 hydrogen atoms in the group are each independently replaced by R_{P};
R_{A}, R_{P} are each independently cyano, nitro, halogen (preferably F or Cl), C₁₋₁₀ alkyl (preferably C₁₋₆ alkyl, more preferably C₁₋₃ alkyl), C₃₋₁₀ cycloalkyl (preferably C₃₋₆ cycloalkyl), C₂₋₁₀ alkenyl (preferably C₂₋₆ alkenyl, more preferably C₂₋₄ alkenyl), C₂₋₁₀ alkynyl (preferably C₂₋₆ alkynyl, more preferably C₂₋₄ alkynyl), C₆₋₁₀ aryl(preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle, -OR^{a}, -SR^{a}, -C(O)R^{b}, -OC(O)R^{b}, -S(O)R^{b}, -S(O)₂R^{b}, -NR^{d}C(O)R^{b}, -NR^{d}S(O)R^{b}, -NR^{d}S(O)₂R^{b}, -NHOR^{C}, -C(O)OR^{C}, -NR^{d}C(O)OR^{C}, -C(O)NR^{d}R^{e}, -OC(O)NR^{d}R^{e}, -NR^{d}R^{e}, -NR^{d}C(O)NR^{d}R^{e}, -C(=NR^{d})R^{f}, -C(=NR^{d})NR^{d}R^{e}, -NR^{d}C(=NR^{d})NR^{d}R^{e}, -S(O)NR^{d}R^{e} or -S(O)zNR^{d}R^{e}; wherein the C₁₋₁₀ alkyl, C₃₋₁₀ cycloalkyl, C₂₋₁₀ alkenyl, C₂₋₁₀ alkynyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle are unsubstituted or substituted with 1, 2, 3, 4 or 5 groups independently selected from -X₃-(CR₃₁R₃₂)ₘ₁-X₄-(CR₄₁R₄₂)ₘ₂-R_{B};
R_{L} is hydrogen, cyano, nitro, halogen (preferably F or Cl), halogenated C₁₋₁₀ alkylthio (preferably halogenated C₁₋₆ alkylthio, more preferably halogenated C₁₋₃ alkylthio), C₁₋₁₀ alkyl (preferably C₁₋₆ alkyl, more preferably C₁₋₃ alkyl), C₃₋₁₀ cycloalkyl (preferably C₃₋₆ cycloalkyl), C₂₋₁₀ alkenyl (preferably C₂₋₆ alkenyl, more preferably C₂₋₄ alkenyl), C₂₋₁₀ alkynyl (preferably C₂₋₆ alkynyl, more preferably C₂₋₄ alkynyl), C₆₋₁₀ aryl(preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle, -C₁₋₄ alkyl-NR^{g}R^{h}, -halogenated C₁₋₄ alkyl- NR^{g}R^{h}, -C₃₋₆ cycloalkyl-NR^{g}R^{h}, -C₂₋₄ alkenyl-NR^{g}R^{h}, -C₂₋₄ alkynyl-NR^{g}R^{h}, -NR^{g}R^{h}, -NHNR^{g}R^{h}, -C(O)NR^{g}R^{h}, -NHC(O)NR^{g}R^{h}, -S(O)₂NR^{g}R^{h}, -OR^{a}, -SR^{b}, -C(O)R^{b}, -OC(O)R^{b}, -NR^{d}C(O)R^{b}, -NR^{d}S(O)R^{b}, -NR^{d}S(O)₂R^{b}, -S(O)R^{b}, -S(O)₂R^{b}, -NHOR^{c}, -C(O)OR^{C}, -NR^{d}C(O)OR^{C}, -OC(O)NR^{d}R^{e}, -C(=NR^{d})R^{f}, -C(=NR^{d})NR^{d}R^{e}, -NR^{d}C(=NR^{d})NR^{d}R^{e} or -S(O)NR^{d}R^{e}; wherein the C₁₋₁₀ alkyl, C₃₋₁₀ cycloalkyl, C₂₋₁₀ alkenyl, C₂₋₁₀ alkynyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl-3- to 16-membered saturated or unsaturated heterocycle are unsubstituted or substituted with 1, 2, 3, 4 or 5 groups independently selected from - X₃-(CR₃₁R₃₂)ₘ₁-X₄-(CR₄₁R₄₂)ₘ₂-R_{B};
wherein R^{a}, R^{b}, R^{c}, R^{d}, R^{e}, R^{f} are each independently hydrogen, halogen (preferably F or Cl), cyano, nitro, amino, acetyl, hydroxyl, -C₁₋₄ alkyl-carboxyl, -NHC₁₋₄ alkyl, -N(C₁₋₄ alkyl)₂, -S(O)₂NRₐ₀R_{b0}, C₁₋₁₀ alkyl (preferably C₁₋₆ alkyl, more preferably C₁₋₃ alkyl), C₃₋₁₀ cycloalkyl (preferably C₃₋₆ cycloalkyl), C₁₋₁₀ alkoxy (preferably C₁₋₆ alkoxy, more preferably C₁₋₃ alkoxy), C₂₋₁₀ alkenyl (preferably C₂₋₆ alkenyl, more preferably C₂₋₄ alkenyl), C₂₋₁₀ alkynyl (preferably C₂₋₆ alkynyl, more preferably C₂₋₄ alkynyl), C₆₋₁₀ aryl(preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle; wherein Rₐ₀, R_{b0} are each independently hydrogen, C₁₋₃ alkyl or halogenated C₁₋₃ alkyl; the C₁₋₁₀ alkyl, C₃₋₁₀ cycloalkyl, C₁₋₁₀ alkoxy, C₂₋₁₀ alkenyl, C₂₋₁₀ alkynyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle are unsubstituted or substituted with 1, 2, 3, 4 or 5 groups independently selected from -X₃-(CR₃₁R₃₂)ₘ₁-X₄-(CR₄₁R₄₂)ₘ₂-R_{B};
R^{g}, R^{h} are each independently hydrogen, C₁₋₆ alkyl(preferably C₁₋₃ alkyl), halogenated C₁₋₆ alkyl(preferably halogenated C₁₋₃ alkyl), C₃₋₆ cycloalkyl, C₆₋₁₀ aryl(preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -C(O)R^{g1}, -C(O)OR^{g1}, -C(O)NR^{g1}R^{g2} or -SO₂NR^{g1}R^{g2}, wherein the C₁₋₆ alkyl, halogenated C₁₋₆ alkyl, C₃₋₆ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 Rⁱ¹;
or R^{g}, R^{h} and the nitrogen atom attached thereto together form a 4- to 11-membered saturated or unsaturated heterocycle, wherein the 4- to 11-membered saturated or unsaturated heterocycle is optionally substituted with 1, 2 or 3 Rⁱ²;
R^{g1} and R^{g2} are each independently hydrogen, C₁₋₆ alkyl(preferably C₁₋₃ alkyl), C₂₋₆ alkenyl (preferably C₂₋₄ alkenyl), C₂₋₆ alkynyl (preferably C₂₋₄ alkynyl), C₁₋₆ alkoxy(preferably C₁₋₃ alkoxy), halogenated C₁₋₆ alkyl(preferably halogenated C₁₋₃ alkyl), halogenated C₁₋₆ alkoxy(preferably halogenated C₁₋₃ alkoxy), C₃₋₁₀ cycloalkyl (preferably C₃₋₆ cycloalkyl), C₆₋₁₀ aryl(preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 Rⁱ³;
Rⁱ¹, Rⁱ², Rⁱ³ are each independently cyano, halogen, C₁₋₆ alkyl(preferably C₁₋₃ alkyl), C₂₋₆ alkenyl (preferably C₂₋₄ alkenyl), C₂₋₆ alkynyl (preferably C₂₋₄ alkynyl), C₁₋₆ alkoxy(preferably C₁₋₃ alkoxy), halogenated C₁₋₆ alkyl(preferably halogenated C₁₋₃ alkyl), halogenated C₁₋₆ alkoxy(preferably halogenated C₁₋₃ alkoxy), C₃₋₁₀ cycloalkyl (preferably C₃₋₆ cycloalkyl), C₆₋₁₀ aryl(preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -OR^{j1}, -SR^{j1}, , -NHOR^{j1}, -C(O)R^{j1}, -C(O)NR^{j1}R^{j2}, -C(O)OR ^{j1}, -OC(O)R^{j1}, -OC(O)NR^{j1}R^{j2}, -NR^{j1}R^{j2}, -NR^{j1}C(O)R^{j2}, -NR^{j1}C(O)OR^{j2}, -NR^{j1}C(O)NR^{j2}R^{j3}, -C(=NR^{j1})R^{j2}, -C(=NR^{j1})NR^{j2}R^{j3}, -NR^{j1}C(=NR^{j2})NR^{j3}R^{j4}, -NR^{j1}S(O)R^{j2}, -NR^{j1}S(O)₂R^{j2}, -S(O)R^{j1}, -S(O)NR^{j1}R^{j2}, -S(O)₂R^{j1} or -S(O)₂NR^{j2}R^{j3}; wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 R^{k1};
R^{j1}, R^{j2}, R^{j3}, R^{j4} are each independently hydrogen, C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy or halogenated C₁₋₆ alkoxy, wherein the C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy, halogenated C₁₋₆ alkoxy are optionally substituted with 1, 2 or 3 R^{k1};
X₃, X₄ are each independently a bond, O, S, NR₁₃, -C(O)NR₁₃-, -NR₁₃C(O)-, -SO₂NR₁₃-, -NR₁₃SO₂-, -NR₁₃C(O)NR₁₃-, -NR₁₃C(O)O-, -CH=CH-, -C=C-, -(CR₁₄R₁₅)ₚ-, -(CR₁₄R₁₅)ₚ-O-, -O-(CR₁₄R₁₅)ₚ-, C₃₋₆ cycloalkyl or 3- to 6-membered saturated monoheterocycle; the C₃₋₆ cycloalkyl, 3- to 6-membered saturated monoheterocycle are optionally substituted with 1, 2 or 3 groups independently selected from halogen, C₁₋₃ alkyl, C₁₋₃ alkoxy, halogenated C₁₋₃ alkyl, halogenated C₁₋₃ alkoxy;
m1, m2 are each independently 0, 1, 2, 3 or 4;
p is 1, 2, 3 or 4;
R₁₃, R₁₄, R₁₅ are each independently hydrogen or C₁₋₁₀ alkyl (preferably C₁₋₆ alkyl, more preferably C₁₋₃ alkyl); the C₁₋₁₀ alkyl is unsubstituted or substituted with 1, 2 or 3 substituents independently selected from the following group: cyano, acetyl, hydroxyl, carboxyl, nitro, halogen (preferably F or Cl), C₁₋₁₀ alkyl (preferably C₁₋₆ alkyl, more preferably C₁₋₃ alkyl), C₁₋₁₀ alkoxy (preferably C₁₋₆ alkoxy, more preferably C₁₋₃ alkoxy), halogenated C₁₋₁₀ alkyl (preferably halogenated C₁₋₆ alkyl, more preferably halogenated C₁₋₃ alkyl), halogenated C₁₋₁₀ alkoxy (preferably halogenated C₁₋₆ alkoxy, more preferably halogenated C₁₋₃ alkoxy), halogenated C₁₋₁₀ alkylthio (preferably halogenated C₁₋₆ alkylthio, more preferably halogenated C₁₋₃ alkylthio), NRₐ₀R_{b0}, C₃₋₁₀ cycloalkyl, C₆₋₁₀ aryl, C₅₋₁₄ heteroaryl, 3- to 16-membered saturated or unsaturated heterocycle; wherein the C₁₋₁₀ alkyl, C₁₋₁₀ alkoxy, C₆₋₁₀ aryl, C₅₋₁₄ heteroaryl, C₃₋₁₀ cycloalkyl, 3- to 16-membered saturated or unsaturated heterocycle are unsubstituted or substituted with 1, 2 or 3 substituents each independently selected from acetyl, hydroxyl, cyano, carboxyl, nitro, halogen, C₁₋₆ alkyl, halogenated C₁₋₆ alkyl, C₁₋₆ alkoxy, halogenated C₁₋₆ alkoxy, C₃₋₆ cycloalkyl, NRₐ₀R_{b0}, -CONRₐ₀R_{b0}, -SO₂NRₐ₀R_{b0}; Rₐ₀, R_{b0} are each independently hydrogen, C₁₋₃ alkyl or halogenated C₁₋₃ alkyl;
R₃₁, R₃₂, R₄₁, R₄₂ are each independently hydrogen, cyano, acetyl, hydroxyl, carboxyl, nitro, halogen (preferably F or Cl), C₁₋₁₀ alkyl (preferably C₁₋₆ alkyl, more preferably C₁₋₃ alkyl), C₁₋₁₀ alkoxy (preferably C₁₋₆ alkoxy, more preferably C₁₋₃ alkoxy), halogenated C₁₋₁₀ alkyl (preferably halogenated C₁₋₆ alkyl, more preferably halogenated C₁₋₃ alkyl), halogenated C₁₋₁₀ alkoxy (preferably halogenated C₁₋₆ alkoxy, more preferably halogenated C₁₋₃ alkoxy), halogenated C₁₋₁₀ alkylthio (preferably halogenated C₁₋₆ alkylthio, more preferably halogenated C₁₋₃ alkylthio), NRₐ₀R_{b0}, C₃₋₁₀ cycloalkyl, C₆₋₁₀ aryl, C₅₋₁₄ heteroaryl, 3- to 16-membered saturated or unsaturated heterocycle; the C₁₋₁₀ alkyl, C₁₋₁₀ alkoxy, C₆₋₁₀ aryl, C₅₋₁₄ heteroaryl, C₃₋₁₀ cycloalkyl, 3- to 16-membered saturated or unsaturated heterocycle are unsubstituted or substituted with 1, 2 or 3 substituents each independently selected from acetyl, hydroxyl, cyano, carboxyl, nitro, halogen, C₁₋₆ alkyl, halogenated C₁₋₆ alkyl, C₁₋₆ alkoxy, halogenated C₁₋₆ alkoxy, C₃₋₆ cycloalkyl, NRₐ₀R_{b0}, -CONRₐ₀R_{b0}, -SO₂NRₐ₀R_{b0}; Rₐ₀, R_{b0} are each independently hydrogen, C₁₋₃ alkyl or halogenated C₁₋₃ alkyl;
R_{B} is cyano, acetyl, hydroxyl, carboxyl, nitro, halogen (preferably F or Cl), C₁₋₁₀ alkyl (preferably C₁₋₆ alkyl, more preferably C₁₋₃ alkyl), C₁₋₁₀ alkoxy (preferably C₁₋₆ alkoxy, more preferably C₁₋₃ alkoxy), halogenated C₁₋₁₀ alkyl (preferably halogenated C₁₋₆ alkyl, more preferably halogenated C₁₋₃ alkyl), halogenated C₁₋₁₀ alkoxy (preferably halogenated C₁₋₆ alkoxy, more preferably halogenated C₁₋₃ alkoxy), halogenated C₁₋₁₀ alkylthio (preferably halogenated C₁₋₆ alkylthio, more preferably halogenated C₁₋₃ alkylthio), NRₐ₀R_{b0}, -CONRₐ₀R_{b0}, -SO₂NRₐ₀R_{b0}, -SO₂C₁₋₁₀ alkyl (preferably -SO₂C₁₋₆ alkyl, more preferably -SO₂C₁₋₃ alkyl), C₆₋₁₀ aryl(preferably phenyl), C₅₋₁₄ heteroaryl, C₃₋₁₀ cycloalkyl, 3- to 16-membered saturated or unsaturated heterocycle, wherein the C₆₋₁₀ aryl, C₅₋₁₄ heteroaryl, C₃₋₁₀ cycloalkyl, 3- to 16-membered saturated or unsaturated heterocycle are unsubstituted or substituted with 1, 2 or 3 substituents each independently selected from acetyl, hydroxyl, cyano, carboxyl, nitro, halogen, C₁₋₆ alkyl, halogenated C₁₋₆ alkyl, C₁₋₆ alkoxy, halogenated C₁₋₆ alkoxy, C₃₋₆ cycloalkyl, NRₐ₀R_{b0}, -CONRₐ₀R_{b0}, -SO₂NRₐ₀R_{b0}; Rₐ₀, R_{b0} are each independently hydrogen or C₁₋₃ alkyl, halogenated C₁₋₃ alkyl.
R_{W} is hydrogen, cyano, halogen, hydroxyl, carboxyl, amino, C₁₋₄ alkyl, C₁₋₄ alkoxy, halogenated C₁₋₄ alkyl, halogenated C₁₋₄ alkoxy, -NH-C₁₋₄ alkyl, -N(C₁₋₄ alkyl)₂, or -(CR^{m1}R^{m2})ᵥ-NR^{m3}R^{m4}; wherein the C₁₋₄ alkyl, C₁₋₄ alkoxy are optionally substituted with 1 or 2 cyano, halogen, hydroxyl, carboxyl, amino, C₁₋₄ alkyl, C₁₋₄ alkoxy, halogenated C₁₋₄ alkyl, halogenated C₁₋₄ alkoxy, -NH C₁₋₄ alkyl or -N (C₁₋₄ alkyl)₂;
v is 0, 1, 2 or 3;
R^{m1}, R^{m2} are each independently hydrogen, cyano, halogen, hydroxyl, carboxyl, amino, C₁₋₄ alkyl, C₁₋₄ alkoxy, halogenated C₁₋₄ alkyl, halogenated C₁₋₄ alkoxy, -NH-C₁₋₄ alkyl, -N(C₁₋₄ alkyl)₂, C₃₋₆ cycloalkyl, phenyl, 5- or 6-membered monocyclic heteroaryl or 4- to 6-membered saturated or unsaturated heterocycle, wherein the C₁₋₄ alkyl, C₁₋₄ alkoxy, halogenated C₁₋₄ alkyl, halogenated C₁₋₄ alkoxy, -NH-C₁₋₄ alkyl, -N(C₁₋₄ alkyl)₂, C₃₋₆ cycloalkyl, phenyl, 5- or 6-membered monocyclic heteroaryl, 4- to 6-membered saturated or unsaturated heterocycle are optionally substituted with 1, 2 or 3 R^{k2};
or R^{m1}, R^{m2} and the carbon atom attached thereto together form a 3-, 4-, 5- or 6-membered cycloalkyl ring or 4-, 5-, 6- or 7-membered saturated or unsaturated heterocycle, wherein the cycloalkyl ring, saturated or unsaturated heterocycle are optionally substituted with 1 or 2 R^{k2};
or R^{m1} and R^{m3} are connected to form a 4-, 5-, 6- or 7-membered saturated or unsaturated heterocycle, wherein the saturated or unsaturated heterocycle contains 1 or 2 heteroatoms independently selected from N, O and S, the saturated or unsaturated heterocycle is optionally substituted with 1 or 2 R^{k2};
R^{m3}, R^{m4} are each independently hydrogen, C₁₋₆ alkyl(preferably C₁₋₃ alkyl), halogenated C₁₋₆ alkyl(preferably halogenated C₁₋₃ alkyl), C₃₋₆ cycloalkyl, C₆₋₁₀ aryl(preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -C(O)Rⁿ¹, -C(O)ORⁿ¹, -C(O)NRⁿ¹Rⁿ², SO₂NRⁿ¹Rⁿ², wherein the C₁₋₆ alkyl, halogenated C₁₋₆ alkyl, C₃₋₆ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl-3- to 16-membered saturated or unsaturated heterocycle are optionally substituted with 1, 2 or 3 R^{o1};
or R^{m3}, R^{m4} and the nitrogen atom attached thereto together form a 4-, 5-, 6-, 7-, 8-, 9-, 10- or 11-membered saturated or unsaturated heterocycle, wherein the saturated or unsaturated heterocycle is optionally substituted with 1, 2 or 3 R^{o2};
Rⁿ¹, Rⁿ² are each independently hydrogen, C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl-3- to 16-membered saturated or unsaturated heterocycle are optionally substituted with 1, 2 or 3 R^{o3};
R^{o1}, R^{o2}, R^{o3} are each independently cyano, halogen, C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl-3- to 16-membered saturated or unsaturated heterocycle, -OR^{p1}, -SR^{p1}, , -NHOR^{p1}, -C(O)R^{p1}, -C(O)NR^{p1}R^{p2}, -C(O)OR^{p1}, -OC(O)R^{p1}, -OC(O)NR^{p1}R^{p2}, -NR^{p1}R^{p2}, -NR^{p1}C(O)R^{p2}, -NR^{p1}C(O)OR^{p2}, -NR^{p1}C(O)NR^{p2}R^{p3}, -C(=NR^{p1})R^{p2}, -C(=NR^{p1})NR^{p2}R^{p3}, -NR^{p1}C(=NR^{p2})NR^{p3}R^{p4}, -NR^{p1}S(O)R^{p2}, -NR^{p1}S(O)₂R^{p2}, -S(O)R^{p1}, -S(O)NR^{p1}R^{p2}, -S(O)₂R^{p1} or -S(O)₂NR^{p2}R^{p3}; wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 R^{k2};
R^{p1}, R^{p2}, R^{p3}, R^{p4} are each independently hydrogen, C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy or halogenated C₁₋₆ alkoxy, wherein the C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy, halogenated C₁₋₆ alkoxy are optionally substituted with 1, 2 or 3 R^{k2};
R^{k1}, R^{k2} are each independently cyano, halogen, hydroxyl, carboxyl, amino, C₁₋₆ alkyl, -NH-C₁₋₆ alkyl, -N(C₁₋₆ alkyl)₂, C₁₋₆ alkoxy, halogenated C₁₋₆ alkyl, halogenated C₁₋₆ alkoxy, C₃₋₆ cycloalkyl, phenyl, 5- or 6-membered monocyclic heteroaryl or 4- to 6-membered saturated or unsaturated heterocycle, wherein the C₁₋₆ alkyl, -NH-C₁₋₆ alkyl, -N(C₁₋₆ alkyl)₂, C₁₋₆ alkoxy, halogenated C₁₋₆ alkyl, halogenated C₁₋₆ alkoxy, C₃₋₆ cycloalkyl, phenyl, 5- or 6-membered monocyclic heteroaryl, 4- to 6-membered saturated or unsaturated heterocycle are optionally substituted with 1, 2 or 3 groups independently selected from cyano, halogen, hydroxyl, carboxyl, amino, C₁₋₄ alkyl, C₁₋₄ alkoxy, halogenated C₁₋₄ alkyl, halogenated C₁₋₄ alkoxy, C₃₋₆ cycloalkyl, phenyl, 5- or 6-membered monocyclic heteroaryl, 4- to 6-membered saturated or unsaturated heterocycle.

In another preferred example, L, W are each independently phenyl, 5- or 6-membered monocyclic heteroaryl, 8- to 10-membered bicyclic heteroaryl, 11- to 14-membered tricyclic heteroaryl, 3- to 6-membered saturated or unsaturated monoheterocycle, 7- to 11-membered monospiro heterocycle, 10- to 16-membered bispiro heterocycle, 7- to 10-membered bicyclic fused heterocycle, 7- to 10-membered bridged heterocycle, or are the structures shown in formula A, formula B, formula C or formula D: 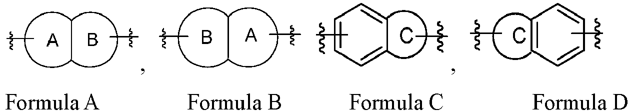 wherein ring A is 5- or 6-membered monocyclic heteroaryl ring, ring B , ring C are each independently 5-to 6-membered saturated or unsaturated monoheterocycle or 5- to 6-membered saturated or unsaturated monocyclic ring; the phenyl, 5- or 6-membered monocyclic heteroaryl, 8- to 10-membered bicyclic heteroaryl, 11- to 14-membered tricyclic heteroaryl, 3- to 6-membered saturated or unsaturated monoheterocycle, 7- to 11-membered monospiro heterocycle, 10- to 16-membered bispiro heterocycle, 7- to 10-membered bicyclic fused heterocycle, 7- to 10-membered bridged heterocycle, the structures shown in formula A, formula B, formula C, and formula D are unsubstituted or substituted with 1, 2 or 3 substituents independently selected from Rp.

In another preferred example, the C₆₋₁₀ aryl in L and W is selected from phenyl, 2,3-dihydro-1H-indene or 1,2,3,4-tetrahydronaphthalene.

In another preferred example, the C₆₋₁₀ aryl in L and W is selected from

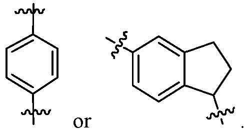

In another preferred example, the 5- or 6-membered monocyclic heteroaryl in L and W is selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine. In another preferred example, the 5- or 6-membered monocyclic heteroaryl in L and W is pyridine.

In another preferred example, the 5- or 6-membered monocyclic heteroaryl in L and W is selected from:

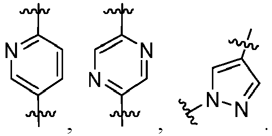

In another preferred example, the 5- or 6-membered monocyclic heteroaryl in L and W is selected from:

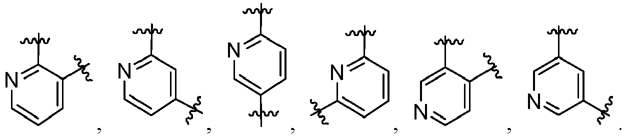

In another preferred example, the 8- to 10-membered bicyclic heteroaryl in L and W is a 9- or 10-membered bicyclic heteroaryl formed by fusing a benzene ring with a 5- or 6-membered monocyclic heteroaryl, or is an 8- to 10-membered bicyclic heteroaryl formed by fusing a 5- or 6-membered monocyclic heteroaryl with a 5- or 6-membered monocyclic heteroaryl; wherein the 5- or 6-membered monocyclic heteroaryl is selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In another preferred example, the 5- or 6-membered monocyclic heteroaryl forming an 8- to 10-membered bicyclic heteroaryl or an 11- to 14-membered tricyclic heteroaryl is selected from the following structures: 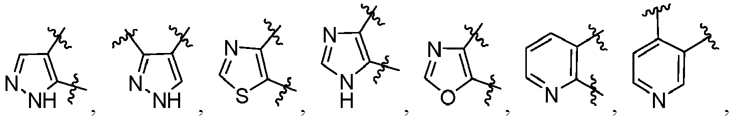 wherein the attached two carbon atoms represented by " 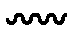 " are adjacent pairs of carbon atoms shared when fused with other rings.

In another preferred example, the 5- or 6-membered monocyclic heteroaryl forming an 8- to 10-membered bicyclic heteroaryl ring is: 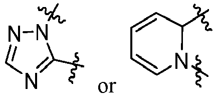 wherein the attached two carbon atoms represented by " 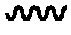 " are adjacent pairs of carbon atoms shared when fused with other rings.

In another preferred example, the ring A in formula A and formula B is selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In another preferred example, the ring A in formula A and formula B is selected from the following structures: 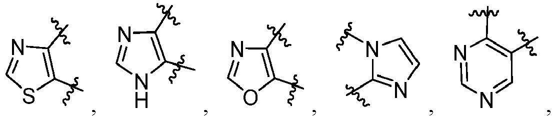 wherein the attached two ring atoms represented by 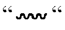 are adjacent pairs of atoms shared when fused with other rings.

In another preferred example, the ring A in formula A and formula B is selected from the following structures: 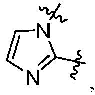 wherein the attached two ring atoms represented by 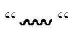 are adjacent pairs of atoms shared when fused with other rings.

In another preferred example, when the ring B in formula A and formula B , and the ring C in formula C and formula D are 5- to 6-membered saturated or unsaturated monoheterocycle, they are each independently selected from: oxazolidine, pyrrolidine-2-one, pyrrolidine-2,5-dione, 1,3-dioxolane, dihydrofuran-2(3H)-one, dihydrofuran-2,5-dione, piperidine-2-one, piperidine-2,6-dione, tetrahydro-2H-pyran-2-one, imidazolidine, tetrahydrofuran, tetrahydrothiophene, tetrahydropyrrole, 1,3-dioxolane-2-one, oxazolidine-2-one, imidazolidine-2-one, piperidine, piperazine, piperazine-2-one, morpholine, morpholine-3-one, morpholine-2-one, thiomorpholine-3-one-1,1-dioxide, thiomorpholine, thiomorpholine-1,1-dioxide, tetrahydropyran.

In another preferred example, when the ring B in formula A and formula B , and the ring C in formula C and formula D are 5- to 6-membered saturated or unsaturated monocyclic ring, they are each independently selected from: cyclopentyl, cyclopentenyl, cyclohexyl, cyclohexenyl, cyclohexadienyl, cyclopentanone, cyclopentane-1,3-dione, cyclohexanone, cyclohexane-1,3-dione.

In another preferred example, the 8- to 10-membered bicyclic heteroaryl ring in L and W is selected from:

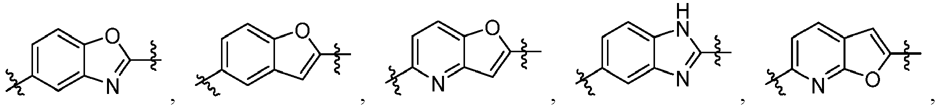

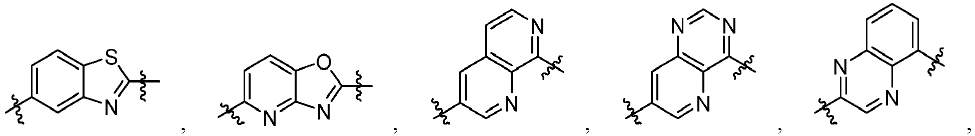

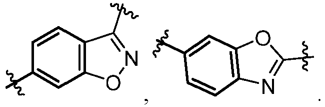

In another preferred example, the 8- to 10-membered bicyclic heteroaryl ring in L and W is selected from:

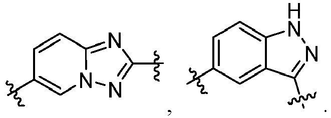

In another preferred example, the formula A, formula B, formula C, and formula D in L and W are selected from the following structures:

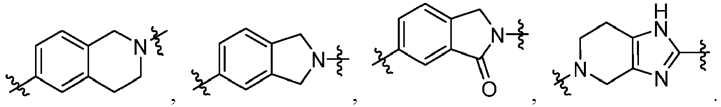

In another preferred example, the formula A, formula B, formula C, and formula D in L and W are selected from the following structures:

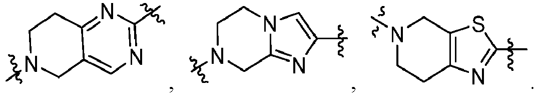

In another preferred example, L and W are each independently selected from the following rings: 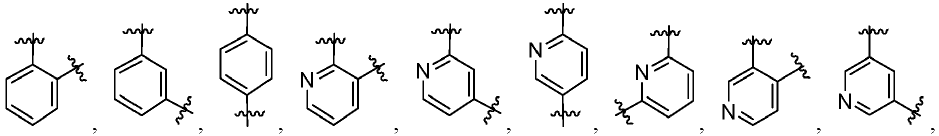 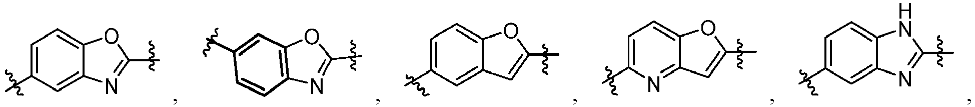 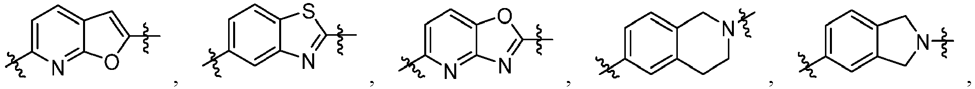 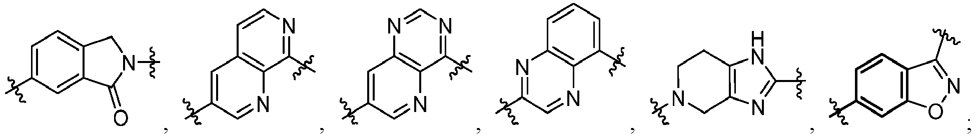 the ring is unsubstituted or substituted with 1, 2 or 3 substituents independently selected from Rp.

In another preferred example, L and W are each independently selected from the following rings: 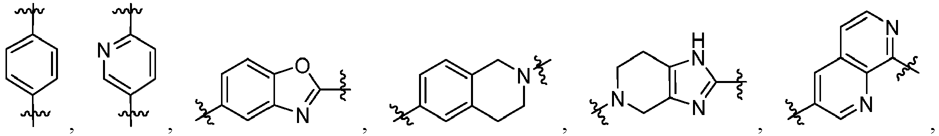 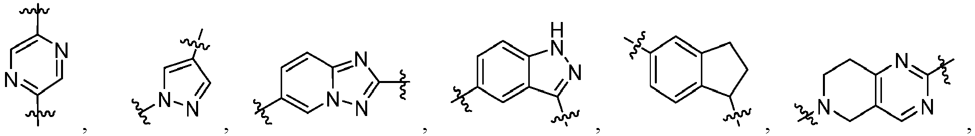 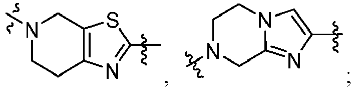 the ring is unsubstituted or substituted with 1, 2 or 3 substituents independently selected from Rp.

In another preferred example, R_{L} is hydrogen or -Y₀-NR^{g}R^{h}; wherein Y₀ is a bond, NH, C₁₋₄ alkyl, halogenated C₁₋₄ alkyl, C₃₋₆ cycloalkyl, -NHC(O) -, -SO₂-, -C(O)-, -CH=CH- or -C≡C-; R^{g}, R^{h} are each independently hydrogen, C₁₋₆ alkyl(preferably C₁₋₃ alkyl), halogenated C₁₋₆ alkyl(preferably halogenated C₁₋₃ alkyl), C₃₋₆ cycloalkyl, C₆₋₁₀ aryl(preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -C(O)R^{g1},-C(O)OR^{g1}, -C(O)NR^{g1}R^{g2} or -SO₂NR^{g1}R^{g2}, wherein the C₁₋₆ alkyl, halogenated C₁₋₆ alkyl, C₃₋₆ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 Rⁱ¹; or R^{g}, R^{h} and the nitrogen atom attached thereto together form a 4- to 11-membered saturated or unsaturated heterocycle, wherein the 4- to 11-membered saturated or unsaturated heterocycle is optionally substituted with 1, 2 or 3 Rⁱ²; R^{g1} and R^{g2} are each independently hydrogen, C₁₋₆ alkyl(preferably C₁₋₃ alkyl), C₂₋₆ alkenyl (preferably C₂₋₄ alkenyl), C₂₋₆ alkynyl (preferably C₂₋₄ alkynyl), C₁₋₆ alkoxy(preferably C₁₋₃ alkoxy), halogenated C₁₋₆ alkyl(preferably halogenated C₁₋₃ alkyl), halogenated C₁₋₆ alkoxy(preferably halogenated C₁₋₃ alkoxy), C₃₋₁₀ cycloalkyl (preferably C₃₋₆ cycloalkyl), C₆₋₁₀ aryl(preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 Rⁱ³; Rⁱ¹, Rⁱ², Rⁱ³ are each independently cyano, halogen, C₁₋₆ alkyl(preferably C₁₋₃ alkyl), C₂₋₆ alkenyl (preferably C₂₋₄ alkenyl), C₂₋₆ alkynyl (preferably C₂₋₄ alkynyl), C₁₋₆ alkoxy(preferably C₁₋₃ alkoxy), halogenated C₁₋₆ alkyl(preferably halogenated C₁₋₃ alkyl), halogenated C₁₋₆ alkoxy(preferably halogenated C₁₋₃ alkoxy), C₃₋₁₀ cycloalkyl (preferably C₃₋₆ cycloalkyl), C₆₋₁₀ aryl (preferably phenyl), C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -OR^{j1}, -SR^{j1}, , -NHOR^{j1}, -C(O)R^{j1}, -C(O)NR^{j1}R^{j2}, -C(O)OR ^{j1}, -OC(O)R^{j1}, -OC(O)NR^{j1}R^{j2}, -NR^{j1}R^{j2}, -NR^{j1}C(O)R^{j2}, -NR^{j1}C(O)OR^{j2}, -NR^{j1}C(O)NR^{j2}R^{j3}, -C(=NR^{j1})R^{j2}, -C(=NR^{j1})NR^{j2}R^{j3}, -NR^{j1}C(=NR^{j2})NR^{j3}R^{j4}, -NR^{j1}S(O)R^{j2}, -NR^{j1}S(O)₂R^{j2}, -S(O)R^{j1}, -S(O)NR^{j1}R^{j2}, -S(O)₂R^{j1} or -S(O)₂NR^{j2}R^{j3}; wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 R^{k1}; R^{j1}, R^{j2}, R^{j3}, R^{j4} are each independently hydrogen, C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy or halogenated C₁₋₆ alkoxy, wherein the C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy, halogenated C₁₋₆ alkoxy are optionally substituted with 1, 2 or 3 R^{k1}; R^{k1} is cyano, halogen, hydroxyl, carboxyl, amino, C₁₋₆ alkyl, -NH-C₁₋₆ alkyl, -N(C₁₋₆ alkyl)₂, C₁₋₆ alkoxy, halogenated C₁₋₆ alkyl, halogenated C₁₋₆ alkoxy, C₃₋₆ cycloalkyl, phenyl, 5- or 6-membered monocyclic heteroaryl or 4- to 6-membered saturated or unsaturated heterocycle, wherein the C₁₋₆ alkyl, -NH-C₁₋₆ alkyl, -N(C₁₋₆ alkyl)₂, C₁₋₆ alkoxy, halogenated C₁₋₆ alkyl, halogenated C₁₋₆ alkoxy, C₃₋₆ cycloalkyl, phenyl, 5- or 6-membered monocyclic heteroaryl, 4- to 6-membered saturated or unsaturated heterocycle are optionally substituted with 1, 2 or 3 groups independently selected from cyano, halogen, hydroxyl, carboxyl, amino, C₁₋₄ alkyl, C₁₋₄ alkoxy, halogenated C₁₋₄ alkyl, halogenated C₁₋₄ alkoxy, C₃₋₆ cycloalkyl, phenyl, 5- or 6-membered monocyclic heteroaryl, 4- to 6-membered saturated or unsaturated heterocycle.

In another preferred example, Y₀ is a bond, NH, CH₂, CH₂CH₂, halogenated C₁₋₃ alkyl, C₃₋₆ cycloalkyl, -NHC(O) -, -SO₂-, -C(O)-, -CH=CH- or -C≡C-; the C₃₋₆ cycloalkyl is cyclopropyl, cyclobutyl, cyclopentyl or cyclohexyl. In another preferred example, Y₀ is a bond or CH₂.

In another preferred example, R^{g}, R^{h} are each independently hydrogen, C₁₋₃ alkyl, halogenated C₁₋₃ alkyl, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, -C(O)R^{g1},-C(O)OR^{g1}, -C(O)NR^{g1}R^{g2} or -SO₂NR^{g1}R^{g2}, wherein the C₁₋₃ alkyl, halogenated C₁₋₃ alkyl, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 Rⁱ¹; or R^{g}, R^{h} and the nitrogen atom attached thereto together form a 4- to 6-membered saturated monoheterocycle, wherein the 4- to 6-membered saturated monoheterocycle are optionally substituted with 1, 2 or 3 Rⁱ²; R^{g1} and R^{g2} are each independently hydrogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 Rⁱ³; Rⁱ¹, Rⁱ², Rⁱ³ are each independently cyano, halogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, -OR^{j1}, -SR^{j1}, , -NHOR^{j1}, -C(O)R^{j1}, -C(O)NR^{j1}R^{j2}, -C(O)OR ^{j1}, -OC(O)R^{j1}, -OC(O)NR^{j1}R^{j2}, -NR^{j1}R^{j2}, -NR^{j1}C(O)R^{j2}, -NR^{j1}C(O)OR^{j2}, -NR^{j1}C(O)NR^{j2}R^{j3}, -C(=NR^{j1})R^{j2}, -C(=NR^{j1})NR^{j2}R^{j3}, -NR^{j1}C(=NR^{j2})NR^{j3}R^{j4}, -NR^{j1}S(O)R^{j2}, -NRh^{j1}S(O)₂R^{j2}, -S(O)R^{j1}, -S(O)NR^{j1}R^{j2}, -S(O)₂R^{j1} or -S(O)₂NR^{j2}R^{j3}; wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 R^{k1}; R^{j1}, R^{j2}, R^{j3}, R^{j4} are each independently hydrogen, C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy or halogenated C₁₋₃ alkoxy, wherein the C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy, halogenated C₁₋₃ alkoxy are optionally substituted with 1, 2 or 3 R^{k1}; R^{k1} is cyano, halogen, hydroxyl, carboxyl, amino, C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle, wherein the C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle are optionally substituted with 1, 2 or 3 groups independently selected from cyano, halogen, hydroxyl, carboxyl, amino, C₁₋₃ alkyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 4- to 6-membered saturated monoheterocycle.

In another preferred example, the -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle in R^{g}, R^{h}, R^{g1}, R^{g2}, R^{j1}, Rⁱ² and Rⁱ³ are -CH₂-phenyl, -CH₂-5- or 6-membered monocyclic heteroaryl, -CH₂-C₃₋₆ cycloalkyl, -CH₂-3- to 6-membered saturated monoheterocycle.

In another preferred example, R_{L} is -Y₀-NR^{g}R^{h}; wherein Y₀ is a bond, NH, CH₂, CH₂CH₂, halogenated C₁₋₃ alkyl, C₃₋₆ cycloalkyl, -NHC(O)-, -SO₂-, -C(O)-, -CH=CH- or -C≡C-; wherein the C₃₋₆ cycloalkyl is cyclopropyl, cyclobutyl, cyclopentyl or cyclohexyl; R^{g}, R^{h} and the nitrogen atom attached thereto together form a 4- to 6-membered saturated monoheterocycle, wherein the 4- to 6-membered saturated monoheterocycle are optionally substituted with 1, 2 or 3 Rⁱ²; Rⁱ² is cyano, halogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -CH₂-phenyl, -CH₂-5- or 6-membered monocyclic heteroaryl, -CH₂-C₃₋₆ cycloalkyl, -CH₂-3- to 6-membered saturated monoheterocycle, -OR^{j1}, -SR^{j1}, , -NHOR^{j1}, -C(O)R^{j1}, -C(O)NR^{j1}R^{j2}, -C(O)OR ^{j1}, -OC(O)R^{j1}, -OC(O)NR^{j1}R^{j2}, -NR^{j1}R^{j2}, _NR^{j1}C(O)R^{j2}, -NR^{j1}C(O)OR^{j2}, -NR^{j1}C(O)NR^{j2}R^{j3}, -C(=NR^{j1})R^{j2}, -C(=NR^{j1})NR^{j2}R^{j3}, -NR^{j1}C(=NR^{j2})NR^{j3}R^{j4}, -NR^{j1}S(O)R^{j2}, -NR^{j1}S(O)₂R^{j2}, -S(O)R^{j1}, -S(O)NR^{j1}R^{j2}, -S(O)₂R^{j1} or -S(O)₂NR^{j2}R^{j3}; wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -CH₂-phenyl, -CH₂-5- or 6-membered monocyclic heteroaryl, -CH₂-C₃₋₆ cycloalkyl, -CH₂-3- to 6-membered saturated monoheterocycle are optionally substituted with 1, 2 or 3 R^{k1}; R^{j1}, R^{j2}, R^{j3}, R^{j4} are each independently hydrogen, C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy or halogenated C₁₋₃ alkoxy, wherein the C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy, halogenated C₁₋₃ alkoxy are optionally substituted with 1, 2 or 3 R^{k1}; R^{k1} is cyano, halogen, hydroxyl, carboxyl, amino, C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle, wherein the C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle are optionally substituted with 1, 2 or 3 groups independently selected from cyano, halogen, hydroxyl, carboxyl, amino, C₁₋₃ alkyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 4- to 6-membered saturated monoheterocycle.

In another preferred example, the 4- to 6-membered saturated monoheterocycle together formed by R^{g}, R^{h} and the nitrogen atom attached thereto is selected from: azetidine, tetrahydropyrrole, piperidine, piperazine, morpholine, thiomorpholine, thiomorpholine-1,1-dioxide.

In another preferred example, the 5- or 6-membered monocyclic heteroaryl in R^{g}, R^{h}, R^{g1}, R^{g2}, R^{j1}, Rⁱ², Rⁱ³, R^{j1}, R^{j2}, R^{j3}, R^{j4}, R^{k1} is each independently selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine.

In another preferred example, the 3- to 6-membered saturated monoheterocycle in R^{g}, R^{h}, R^{g1}, R^{g2} , R^{j1}, Rⁱ², Rⁱ³ is each independently selected from: aziridine, ethylene oxide, azetidine, oxetane, tetrahydrofuran, tetrahydrothiophene, tetrahydropyrrole, oxazolidine, dioxolane, piperidine, piperazine, morpholine, dioxane, thiomorpholine, thiomorpholine-1,1-dioxide, tetrahydropyran.

In another preferred example, the 4- to 6-membered saturated monoheterocycle in R^{j1}, R^{j2}, R^{j3}, R^{j4}, R^{k1}is each independently selected from: azetidine, oxetane, tetrahydrofuran, tetrahydrothiophene, tetrahydropyrrole, oxazolidine, dioxolane, piperidine, piperazine, morpholine, dioxane, thiomorpholine, thiomorpholine-1,1-dioxide, tetrahydropyran.

In another preferred example, the C₃₋₆ cycloalkyl in R^{g}, R^{h}, R^{g1}, R^{g2}, R^{j1}, Rⁱ², Rⁱ³, R^{j1}, R^{j2}, R^{j3}, R^{j4}, R^{k1} is each independently selected from: cyclopropyl, cyclobutyl, cyclopentyl, cyclohexyl.

In another preferred example, R_{L} is hydrogen or -CH₂-NR^{g}R^{h}; wherein R^{g}, R^{h} are each independently hydrogen, C₁₋₃ alkyl; or R^{g}, R^{h} and the nitrogen atom attached thereto together form a 4-, 5- or 6-membered saturated monoheterocycle(preferably azetidine, tetrahydropyrrole, piperidine); wherein the C₁₋₃ alkyl, 4-, 5- or 6-membered saturated monoheterocycle are unsubstituted or optionally substituted with 1 or 2 substituents independently selected from C₁₋₃ alkyl, hydroxyl, carboxyl, -OAc, -COOC₁₋₃ alkyl.

In another preferred example, -NR^{g}R^{h} is selected from:

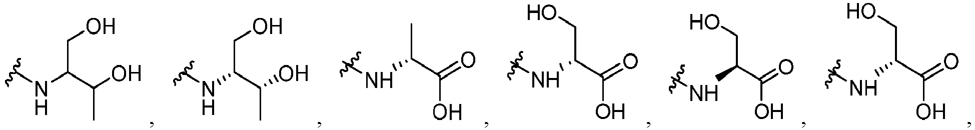

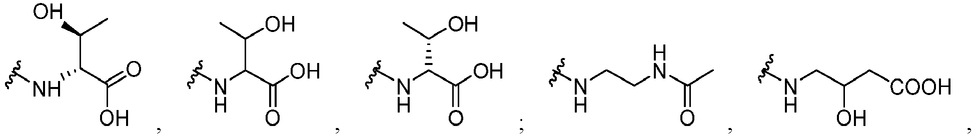

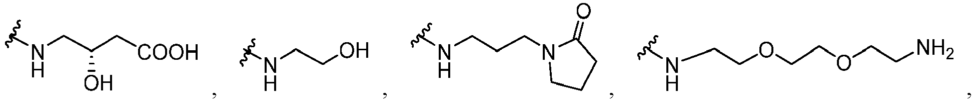

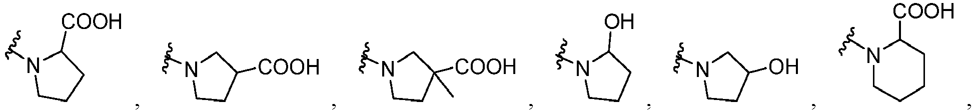

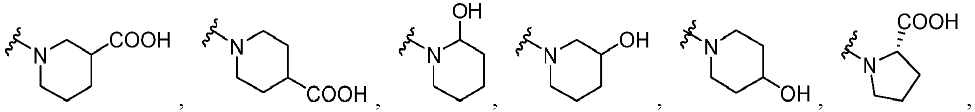

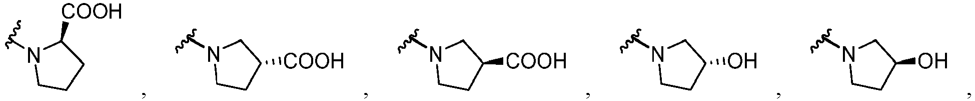

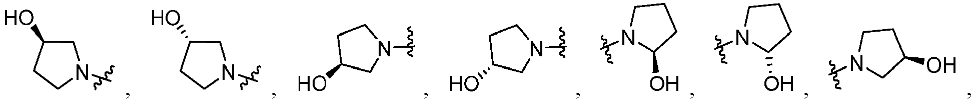

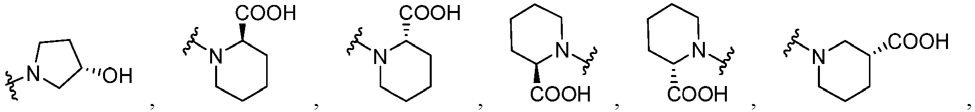

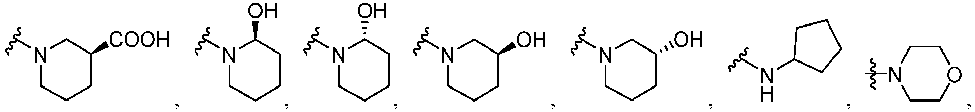

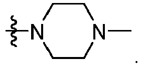

In another preferred example, -NR^{g}R^{h} is selected from:

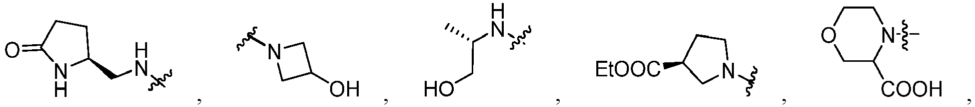

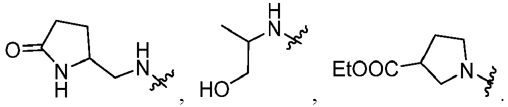

In another preferred example, R_{W} is hydrogen, cyano, halogen, hydroxyl, carboxyl, amino, C₁₋₃ alkyl, C₁₋₃ alkoxy, halogenated C₁₋₃ alkyl, halogenated C₁₋₃ alkoxy, -NH-C₁₋₃ alkyl, -N(C₁₋₃ alkyl)₂, or -(CR^{m1}R^{m2})ᵥ-NR^{m3}R^{m4}; wherein the C₁₋₃ alkyl, C₁₋₃ alkoxy are optionally substituted with 1 or 2 cyano, halogen, hydroxyl, carboxyl, amino, C₁₋₃ alkyl, C₁₋₃ alkoxy, halogenated C₁₋₃ alkyl, halogenated C₁₋₃ alkoxy, -NH C₁₋₃ alkyl or -N (C₁₋₃ alkyl)₂; v is 0, 1, 2 or 3; R^{m1}, R^{m2} are each independently hydrogen, cyano, halogen, hydroxyl, carboxyl, amino, C₁₋₃ alkyl, C₁₋₃ alkoxy, halogenated C₁₋₃ alkyl, halogenated C₁₋₃ alkoxy, -NH-C₁₋₃ alkyl, -N(C₁₋₃ alkyl)₂, C₃₋₆ cycloalkyl, phenyl, 5- or 6-membered monocyclic heteroaryl or 4- to 6-membered saturated monoheterocycle, wherein the C₁₋₃ alkyl, C₁₋₃ alkoxy, halogenated C₁₋₃ alkyl, halogenated C₁₋₃ alkoxy, -NH-C₁₋₃ alkyl, -N(C₁₋₃ alkyl)₂, C₃₋₆ cycloalkyl, phenyl, 5- or 6-membered monocyclic heteroaryl, 4- to 6-membered saturated monoheterocycle are optionally substituted with 1, 2 or 3 R^{k2}; or R^{m1}, R^{m2} and the carbon atom attached thereto together form a 3-, 4-, 5- or 6-membered cycloalkyl ring or 4-, 5- or 6-membered saturated monoheterocycle, wherein the cycloalkyl ring, saturated monoheterocycle are optionally substituted with 1 or 2 R^{k2}; or R^{m1} and R^{m3} are connected to form a 4-, 5-, 6- or 7-membered saturated monoheterocycle, wherein the saturated monoheterocycle contains 1 or 2 heteroatoms independently selected from N, O or S, the saturated monoheterocycle are optionally substituted with 1 or 2 R^{k2}; R^{m3}, R^{m4} are each independently hydrogen, C₁₋₃ alkyl, halogenated C₁₋₃ alkyl, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, -C(O)Rⁿ¹, -C(O)ORⁿ¹, -C(O)NRⁿ¹Rⁿ², SO₂NRⁿ¹Rⁿ², wherein the C₁₋₃ alkyl, halogenated C₁₋₃ alkyl, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 R^{o1}; or R^{m3}, R^{m4} and the nitrogen atom attached thereto together form a 4-, 5-, 6-, 7-, 8-, 9-, 10- or 11-membered saturated or unsaturated heterocycle, wherein the saturated or unsaturated heterocycle is optionally substituted with 1, 2 or 3 R^{o2}; Rⁿ¹, Rⁿ² are each independently hydrogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 R^{o3}; R^{o1}, R^{o2}, R^{o3} are each independently cyano, halogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, -OR^{p1}, -SR^{p1}, , -NHOR^{p1}, -C(O)R^{p1}, -C(O)NR^{p1}R^{p2}, -C(O)OR ^{p1}, -OC(O)R^{p1}, -OC(O)NR^{p1}R^{p2}, -NR^{p1}R^{p2}, -NR^{p1}C(O)R^{p2}, -NR^{p1}C(O)OR^{p2}, -NR^{p1}C(O)NR^{p2}R^{p3}, -C(=NR^{p1})R^{p2}, -C(=NR^{p1})NR^{p2}R^{p3}, -NR^{p1}C(=NR^{p2})NR^{p3}R^{p4}, -NR^{p1}S(O)R^{p2}, -NR^{p1}S(O)₂R^{p2}, -S(O)R^{p1}, -S(O)NR^{p1}R^{p2}, -S(O)₂R^{p1} or -S(O)₂NR^{p2}R^{p3}; wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 R^{k2}; R^{p1}, R^{p2}, R^{p3}, R^{p4} are each independently hydrogen, C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy or halogenated C₁₋₃ alkoxy, wherein the C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy, halogenated C₁₋₃ alkoxy are optionally substituted with 1, 2 or 3 R^{k2}; R^{k2}is cyano, halogen, hydroxyl, carboxyl, amino, C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle, wherein the C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle are optionally substituted with 1, 2 or 3 groups independently selected from cyano, halogen, hydroxyl, carboxyl, amino, C₁₋₃ alkyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 4- to 6-membered saturated monoheterocycle.

In another preferred example, the 3-, 4-, 5- or 6-membered cycloalkyl ring together formed by R^{m1}, R^{m2} and the carbon atom attached thereto is selected from: cyclopropyl, cyclobutyl, cyclopentyl, cyclohexyl.

In another preferred example, the 4-, 5- or 6-membered saturated monoheterocycle together formed by R^{m1}, R^{m2} and the carbon atom attached thereto is selected from: azetidine, oxetane, tetrahydrofuran, tetrahydrothiophene, tetrahydropyrrole, oxazolidine, dioxolane, piperidine, piperazine, morpholine, dioxane, thiomorpholine, thiomorpholine-1,1-dioxide, tetrahydropyran.

In another preferred example, the 4-, 5-, 6- or 7-membered saturated monoheterocycle formed by connecting R^{m1} and R^{m3} is selected from: azetidine, tetrahydropyrrole, oxazolidine, piperidine, piperazine, morpholine, thiomorpholine, thiomorpholine-1,1-dioxide.

In another preferred example, the 4-, 5-, 6-, 7-, 8-, 9-, 10- or 11-membered saturated or unsaturated heterocycle together formed by R^{m3}, R^{m4} and the nitrogen atom attached thereto is 3- to 6-membered saturated monoheterocycle, 7- to 11-membered monospiro heterocycle, 10- to 16-membered bispiro heterocycle or 7- to 10-membered bicyclic fused heterocycle.

In another preferred example, the 3- to 6-membered saturated monoheterocycle is selected from: azetidine, tetrahydropyrrole, oxazolidine, piperidine, piperazine, morpholine, thiomorpholine, thiomorpholine-1,1-dioxide.

In another preferred example, the 7- to 11-membered monospiro heterocycle is a spiro heterocycle that shares one spiro atom and contains 1 or 2 heteroatoms selected from nitrogen and oxygen.

In another preferred example, the 7- to 11-membered monospiro heterocycle is selected from the following group:

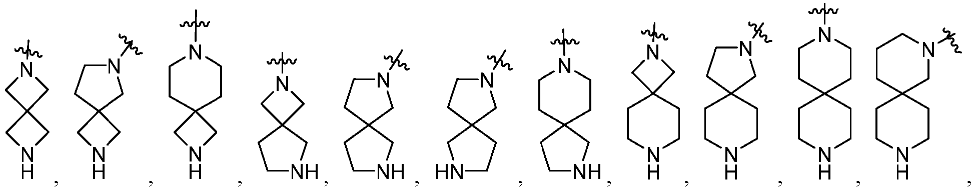

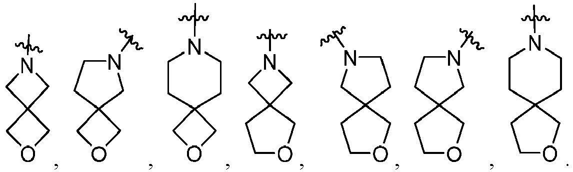

In another preferred example, the 10- to 16-membered bispiro heterocycle is a spiro heterocycle that shares two spiro atoms and contains 1 or 2 heteroatoms selected from nitrogen and oxygen.

In another preferred example, R_{W} is hydrogen or -(CR^{m1}R^{m2})ᵥ-NR^{m3}R^{m4}; wherein v is 0, 1, 2 or 3; R^{m1}, R^{m2} are each independently hydrogen, cyano, halogen, hydroxyl, carboxyl, amino, C₁₋₃ alkyl, C₁₋₃ alkoxy, halogenated C₁₋₃ alkyl, halogenated C₁₋₃ alkoxy, -NH-C₁₋₃ alkyl, -N(C₁₋₃ alkyl)₂; R^{m3}, R^{m4} and the nitrogen atom attached thereto together form a 4-, 5- or 6-membered saturated monoheterocycle, wherein the saturated monoheterocycle are optionally substituted with 1, 2 or 3 R^{o2}; R^{o2} is cyano, halogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, -OR^{p1}, -SR^{p1}, , -NHOR^{p1}, -C(O)R^{p1}, -C(O)NR^{p1}R^{p2}, -C(O)OR^{p1}, -OC(O)R^{p1}, -OC(O)NR^{p1}R^{p2}, -NR^{p1}R^{p2}, -NR^{p1}C(O)R^{p2}, -NR^{p1}C(O)OR^{p2}, -NR^{p1}C(O)NR^{p2}R^{p3}, -C(=NR^{p1})R^{p2}, -C(=NR^{p1})NR^{p2}R^{p3}, -NR^{p1}C(=NR^{p2})NR^{p3}R^{p4}, -NR^{p1}S(O)R^{p2}, -NR^{p1}S(O)₂R^{p2}, -S(O)R^{p1}, -S(O)NR^{p1}R^{p2}, -S(O)₂R^{p1} or -S(O)₂NR^{p2}R^{p3}; wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 R^{k2}; R^{p1}, R^{p2}, R^{p3}, R^{p4} are each independently hydrogen, C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy or halogenated C₁₋₃ alkoxy, wherein the C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy, halogenated C₁₋₃ alkoxy are optionally substituted with 1, 2 or 3 R^{k2}; R^{k2} is cyano, halogen, hydroxyl, carboxyl, amino, C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle, wherein the C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle are optionally substituted with 1, 2 or 3 groups independently selected from cyano, halogen, hydroxyl, carboxyl, amino, C₁₋₃ alkyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 4- to 6-membered saturated monoheterocycle.

In another preferred example, the -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle in R^{m3}, R^{m4}, Rⁿ¹, Rⁿ², R^{o1}, R^{o2}, R^{o3} is -CH₂-phenyl, -CH₂-5- or 6-membered monocyclic heteroaryl, -CH₂-C₃₋₆ cycloalkyl, -CH₂-3- to 6-membered saturated monoheterocycle.

In another preferred example, the 5- or 6-membered monocyclic heteroaryl in R^{m1}, R^{m2}, R^{m3}, R^{m4}, Rⁿ¹, Rⁿ², R^{o1}, R^{o2}, R^{o3}, R^{p1}, R^{p2}, R^{p3}, R^{p4}, R^{k2} is each independently selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine.

In another preferred example, the C₃₋₆ cycloalkyl in R^{m1}, R^{m2}, R^{m3}, R^{m4}, Rⁿ¹, Rⁿ², R^{o1}, R^{o2}, R^{o3}, R^{p1}, R^{p2}, R^{p3}, R^{p4}, R^{k2} is each independently selected from: cyclopropyl, cyclobutyl, cyclopentyl, cyclohexyl.

In another preferred example, the 4- to 6-membered saturated monoheterocycle in R^{m1}, R^{m2}, R^{p1}, R^{p2}, R^{p3}, R^{p4}, R^{k2} is each independently selected from: azetidine, oxetane, tetrahydrofuran, tetrahydrothiophene, tetrahydropyrrole, oxazolidine, dioxolane, piperidine, piperazine, morpholine, dioxane, thiomorpholine, thiomorpholine-1,1-dioxide, tetrahydropyran.

In another preferred example, the 3- to 6-membered saturated monoheterocycle in R^{m3}, R^{m4}, Rⁿ¹, Rⁿ², R^{o1}, R^{o2}, R^{o3} is each independently selected from: aziridine, ethylene oxide, azetidine, oxetane, tetrahydrofuran, tetrahydrothiophene, tetrahydropyrrole, oxazolidine, dioxolane, piperidine, piperazine, morpholine, dioxane, thiomorpholine, thiomorpholine-1,1-dioxide, tetrahydropyran.

In another preferred example, R_{W} is hydrogen or -CH₂-NR^{m3}R^{m4}; wherein R^{m3}, R^{m4} are each independently hydrogen, C₁₋₃ alkyl; or R^{m3}, R^{m4} and the nitrogen atom attached thereto together form a 4-, 5- or 6-membered saturated monoheterocycle(preferably azetidine, tetrahydropyrrole, piperidine); wherein the C₁₋₃ alkyl, 4-, 5- or 6-membered saturated monoheterocycle are unsubstituted or optionally substituted with 1 or 2 substituents independently selected from C₁₋₃ alkyl, hydroxyl, carboxyl, -OAc, -COOC₁₋₃ alkyl.

In another preferred example, -NR^{m3}R^{m4} is selected from:

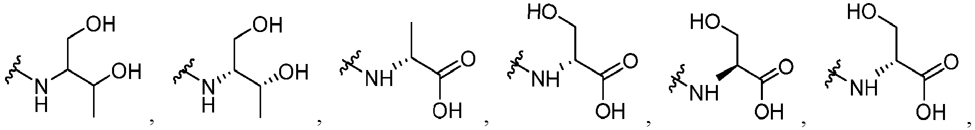

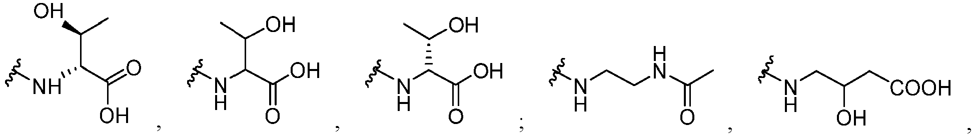

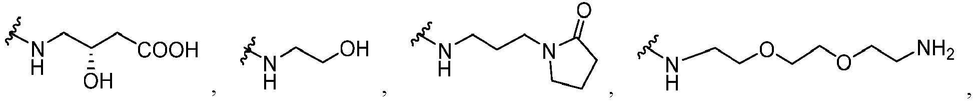

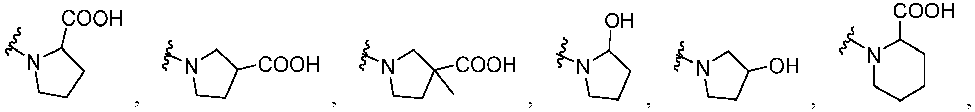

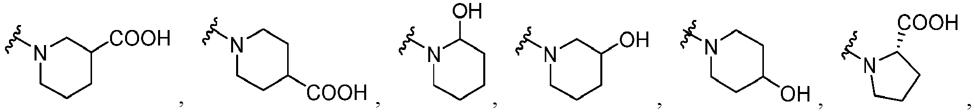

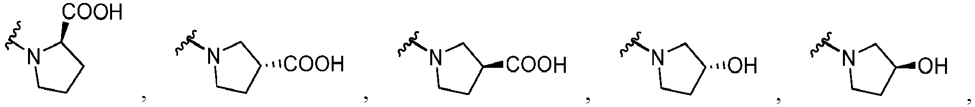

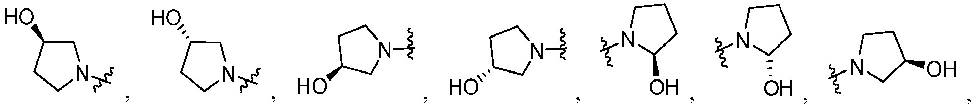

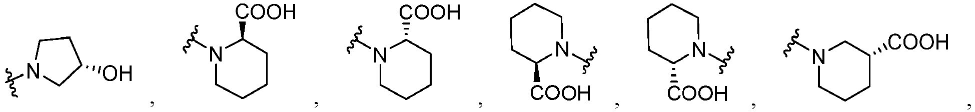

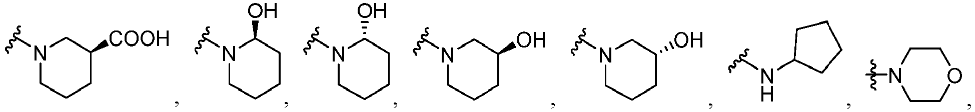

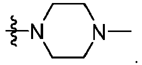

In another preferred example, -NR^{m3}R^{m4} is selected from:

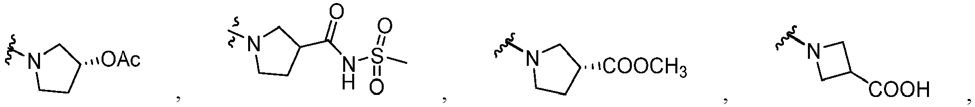

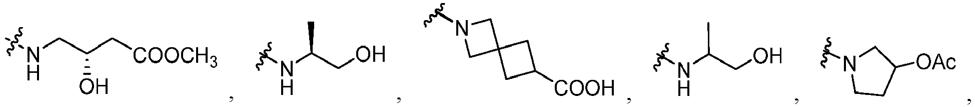

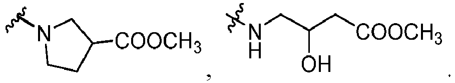

In another preferred example, X is a bond, O, NR₅, -C(O)NR₅- or -NR₅C(O)-; R₅ is hydrogen, methyl, ethyl, n-propyl or isopropyl.

In another preferred example, X is a bond, NH, -C(O)NH- or - NHC(O)-.

In another preferred example, A₁ is CR₁; A₂ is CR₂; A₃ is CR₃. In another preferred example, A₁ is N; A₂ is CR₂; A₃ is CR₃. In another preferred example, A₁ is CR₁; A₂ is CR₂; A₃ is N. In another preferred example, A₁ is CR₁; A₂ is N; A₃ is CR₃. In another preferred example, Z₁ is N; Z₂ is CR₀₃; Z₃ is CR₀₄. In another preferred example, Z₁ is CR₀₂; Z₂ is CR₀₃; Z₃ is N. In another preferred example, Z₁ is CR₀₂; Z₂ is N; Z₃ is CR₀₄. In another preferred example, Z₁ is CR₀₂; Z₂ is CR₀₃; Z₃ is CR₀₄. In another preferred example, A₁ is CR₁; A₂ is CR₂; A₃ is CR_{3;} Z₁ is CR₀₂; Z₂ is CR₀₃; Z₃ is CR₀₄. In another preferred example, A₁ is CR₁; A₂ is CR₂; A₃ is CR_{3;} Z₁ is CR₀₂; Z₂ is CR₀₃; Z₃ is N. In another preferred example, A₁ is CR₁; A₂ is CR₂; A₃ is CR₃; Z₁ is N; Z₂ is CR₀₃; Z₃ is CR₀₄.

In another preferred example, R_{A}, R_{P} are each independently cyano, nitro, halogen, halogenated C₁₋₃ alkylthio, C₁₋₃ alkyl, C₃₋₆ cycloalkyl, C₂₋₄ alkenyl, C₂₋₄ alkynyl, phenyl, 5- or 6-membered monocyclic heteroaryl, 3- to 6-membered saturated monoheterocycle, -CH₂-phenyl, -CH₂-5- or 6-membered monocyclic heteroaryl, -CH₂-C₃₋₆ cycloalkyl, -CH₂-3- to 6-membered saturated monoheterocycle, -OR^{a}, -SR^{a}, -C(O)R^{b}, -OC(O)R^{b}, -S(O)R^{b}, -S(O)₂R^{b}, -NR^{d}C(O)R^{b}, -NR^{d}S(O)R^{b}, -NR^{d}S(O)₂R^{b}, -NHOR^{c}, -C(O)OR^{c}, -NR^{d}C(O)OR^{c}, -C(O)NR^{d}R^{e}, -OC(O)NR^{d}R^{e}, -NR^{d}R^{e}, -NR^{d}C(O)NR^{d}R^{e}, -C(=NR^{d})R^{f}, -C(=NR^{d})NR^{d}R^{e}, -NR^{d}C(=NR^{d})NR^{d}R^{e}, -S(O)NR^{d}R^{e} or -S(O)₂NR^{d}R^{e}; wherein the C₁₋₃ alkyl, C₃₋₆ cycloalkyl, C₂₋₄ alkenyl, C₂₋₄ alkynyl, phenyl, 5- or 6-membered monocyclic heteroaryl, 3- to 6-membered saturated monoheterocycle, -CH₂-phenyl, -CH₂-5- or 6-membered monocyclic heteroaryl, -CH₂-C₃₋₆ cycloalkyl, -CH₂-3- to 6-membered saturated monoheterocycle are unsubstituted or substituted with 1, 2, 3, 4 or 5 groups independently selected from -X₃-(CR₃₁R₃₂)ₘ₁-X₄-(CR₄₁R₄₂)ₘ₂-R_{B}; wherein R^{a}, R^{b}, R^{c}, R^{d}, R^{e}, R^{f} are each independently hydrogen, halogen, halogenated C₁₋₃ alkylthio, cyano, nitro, amino, acetyl, hydroxyl, -CH₂-carboxyl, -NHC₁₋₃ alkyl, -N(C₁₋₃ alkyl)₂, -S(O)₂NRₐ₀R_{b0}, C₁₋₃ alkyl, C₃₋₆ cycloalkyl, C₁₋₃ alkoxy, C₂₋₄ alkenyl, C₂₋₄ alkynyl, phenyl, 5- or 6-membered monocyclic heteroaryl, 3- to 6-membered saturated monoheterocycle, -CH₂-phenyl, -CH₂-5- or 6-membered monocyclic heteroaryl, -CH₂-C₃₋₆ cycloalkyl, -CH₂-3- to 6-membered saturated monoheterocycle; wherein Rₐ₀, R_{b0} are each independently hydrogen, C₁₋₃ alkyl or halogenated C₁₋₃ alkyl; the C₁₋₃ alkyl, C₃₋₆ cycloalkyl, C₁₋₃ alkoxy, C₂₋₄ alkenyl, C₂₋₄ alkynyl, phenyl, 5- or 6-membered monocyclic heteroaryl, 3- to 6-membered saturated monoheterocycle, -CH₂-phenyl, -CH₂-5- or 6-membered monocyclic heteroaryl, -CH₂-C₃₋₆ cycloalkyl, -CH₂-3- to 6-membered saturated monoheterocycle are unsubstituted or substituted with 1, 2, 3, 4 or 5 groups independently selected from -X₃-(CR₃₁R₃₂)ₘ₁-X₄-(CR₄₁R₄₂)ₘ₂-R_{B}; X₃, X₄ are each independently a bond, O, S, NR₁₃, -C(O)NR₁₃-, -NR₁₃C(O)-, -SO₂NR₁₃-, -NR₁₃SO₂-, -NR₁₃C(O)NR₁₃-, -NR₁₃C(O)O-, -CH=CH-, -C≡C-, -(CR₁₄R₁₅)ₚ-, -(CR₁₄R₁₅)ₚ-O-, -O-(CR₁₄R₁₅)ₚ-, C₃₋₆ cycloalkyl or 3- to 6-membered saturated monoheterocycle; the C₃₋₆ cycloalkyl, 3- to 6-membered saturated monoheterocycle are optionally substituted with 1, 2 or 3 groups independently selected from halogen, C₁₋₃ alkyl, C₁₋₃ alkoxy, halogenated C₁₋₃ alkyl, halogenated C₁₋₃ alkoxy; m1, m2 are each independently 0, 1, 2, 3 or 4; p is 1, 2, 3 or 4; R₁₃, R₁₄, R₁₅ are each independently hydrogen or C₁₋₃ alkyl; the C₁₋₃ alkyl is unsubstituted or substituted with 1, 2 or 3 substituents independently selected from the following group: cyano, acetyl, hydroxyl, carboxyl, nitro, halogen, C₁₋₃ alkyl, C₁₋₃ alkoxy, halogenated C₁₋₃ alkyl, halogenated C₁₋₃ alkoxy, halogenated C₁₋₃ alkylthio, NRₐ₀R_{b0}, C₃₋₆ cycloalkyl, phenyl, 5- or 6-membered monocyclic heteroaryl, 3- to 6-membered saturated monoheterocycle; wherein the C₁₋₃ alkyl, C₁₋₃ alkoxy, phenyl, 5- or 6-membered monocyclic heteroaryl, C₃₋₆ cycloalkyl, 3- to 6-membered saturated monoheterocycle are unsubstituted or substituted with 1, 2 or 3 substituents each independently selected from acetyl, hydroxyl, cyano, carboxyl, nitro, halogen, C₁₋₆ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, -SO₂NRₐ₀R_{b0}; Rₐ₀, R_{b0} are each independently hydrogen, C₁₋₃ alkyl or halogenated C₁₋₃ alkyl; R₃₁, R₃₂, R₄₁, R₄₂ are each independently hydrogen, cyano, acetyl, hydroxyl, carboxyl, nitro, halogen, C₁₋₃ alkyl, C₁₋₃ alkoxy, halogenated C₁₋₃ alkyl, halogenated C₁₋₃ alkoxy, halogenated C₁₋₃ alkylthio, NRₐ₀R_{b0}, C₃₋₆ cycloalkyl, phenyl, 5- or 6-membered monocyclic heteroaryl, 3- to 6-membered saturated monoheterocycle; the C₁₋₃ alkyl, C₁₋₃ alkoxy, phenyl, 5- or 6-membered monocyclic heteroaryl, C₃₋₆ cycloalkyl, 3- to 6-membered saturated monoheterocycle are unsubstituted or substituted with 1, 2 or 3 substituents each independently selected from acetyl, hydroxyl, cyano, carboxyl, nitro, halogen, C₁₋₃ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, -SO₂NRₐ₀R_{b0}; Rₐ₀, R_{b0} are each independently hydrogen, C₁₋₃ alkyl or halogenated C₁₋₃ alkyl; R_{B} is cyano, acetyl, hydroxyl, carboxyl, nitro, halogen, C₁₋₃ alkyl, C₁₋₃ alkoxy, halogenated C₁₋₃ alkyl, halogenated C₁₋₃ alkoxy, halogenated C₁₋₃ alkylthio, NRₐ₀R_{b0}, -CONRₐ₀R_{b0}, -SO₂NRₐ₀R_{b0}, -SO₂C₁₋₃ alkyl, phenyl, 5- or 6-membered monocyclic heteroaryl, C₃₋₆ cycloalkyl, 3- to 6-membered saturated monoheterocycle, wherein the phenyl, 5- or 6-membered monocyclic heteroaryl, C₃₋₆ cycloalkyl, 3- to 6-membered saturated monoheterocycle are unsubstituted or substituted with 1, 2 or 3 substituents each independently selected from acetyl, hydroxyl, cyano, carboxyl, nitro, halogen, C₁₋₃ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, -SO₂NRₐ₀R_{b0}; Rₐ₀, R_{b0} are each independently hydrogen or C₁₋₃ alkyl, halogenated C₁₋₃ alkyl.

In another preferred example, R_{A}, R_{P} are each independently hydrogen, cyano, acetyl, hydroxyl, carboxyl, nitro, halogen, C₁₋₃ alkyl, C₁₋₃ alkoxy, halogenated C₁₋₃ alkyl, halogenated C₁₋₃ alkoxy, halogenated C₁₋₃ alkylthio, NRₐ₀R_{b0}, -CONRₐ₀R_{b0}, -SO₂NRₐ₀R_{b0}, -SO₂C₁₋₃ alkyl, phenyl, 5- or 6-membered monocyclic heteroaryl, C₃₋₆ cycloalkyl, 3- to 6-membered saturated monoheterocycle, wherein the phenyl, 5- or 6-membered monocyclic heteroaryl, C₃₋₆ cycloalkyl, 3- to 6-membered saturated monoheterocycle are unsubstituted or substituted with 1, 2 or 3 substituents each independently selected from acetyl, hydroxyl, cyano, carboxyl, nitro, halogen, C₁₋₃ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, -SO₂NRₐ₀R_{b0}; wherein Rₐ₀, R_{b0} are each independently hydrogen, C₁₋₃ alkyl or halogenated C₁₋₃ alkyl.

In another preferred example, R_{P} is cyano, acetyl, halogen(preferably Cl, F), C₁₋₃ alkyl(preferably methyl, ethyl, isopropyl), C₁₋₃ alkoxy(preferably methoxy), halogenated C₁₋₃ alkyl(preferably trifluoromethyl), NRₐ₀R_{b0}, -CONRₐ₀R_{b0}, C₃₋₆ cycloalkyl(preferably cyclopropyl, cyclohexyl), 3- to 6-membered saturated monoheterocycle(preferably tetrahydropyran), -CH₂-3- to 6-membered saturated or unsaturated heterocycle; wherein the acetyl, C₁₋₃ alkyl, C₁₋₃ alkoxy, C₃₋₆ cycloalkyl, 3- to 6-membered saturated monoheterocycle, -CH₂-3- to 6-membered saturated or unsaturated heterocycle are unsubstituted or substituted with 1 or 2 substituents each independently selected from hydroxyl, cyano, carboxyl, NRₐ₀R_{b0}; wherein Rₐ₀, R_{b0} are each independently hydrogen, C₁₋₃ alkyl(preferably methyl).

In another preferred example, the 5- or 6-membered monocyclic heteroaryl in R_{A}, Rp, R^{a}, R^{b}, R^{c}, R^{d}, R^{e}, R^{f}, R₁₃, R₁₄, R₁₅, R₃₁, R₃₂, R₄₁, R₄₂, R_{B} is each independently selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine. In another preferred example, the 3-to 6-membered saturated monoheterocycle in R_{A}, Rp, R^{a}, R^{b}, R^{c}, R^{d}, R^{e}, R^{f}, X₃, X₄, R₁₃, R₁₄, R₁₅, R₃₁, R₃₂, R₄₁, R₄₂, R_{B} is each independently selected from: aziridine, ethylene oxide, azetidine, oxetane, tetrahydrofuran, tetrahydrothiophene, tetrahydropyrrole, oxazolidine, dioxolane, piperidine, piperazine, morpholine, dioxane, thiomorpholine, thiomorpholine-1,1-dioxide, tetrahydropyran. In another preferred example, the C₃₋₆ cycloalkyl in R_{A}, Rp, R^{a}, R^{b}, R^{c}, R^{d}, R^{e}, R^{f}, X₃, X₄, R₁₃, R₁₄, R₁₅, R₃₁, R₃₂, R₄₁, R₄₂, R_{B} is each independently selected from: cyclopropyl, cyclobutyl, cyclopentyl, cyclohexyl.

In another preferred example, R₁, R₂, R₃, R₄ are each independently hydrogen, hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl. In another preferred example, R₁, R₂, R₃ are hydrogen; R₄ is hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, R₀₁, R₀₂, R₀₃ are each independently hydrogen, hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl. In another preferred example, R₀₂, R₀₃ are hydrogen; R₀₁ is hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, R₁, R₂, R₃, R₀₂, R₀₃ are hydrogen; R₀₁, R₄ are each independently F, Cl, cyano, C₁₋₃ alkyl, C₁₋₃ alkoxy, halogenated C₁₋₃ alkyl or halogenated C₁₋₃ alkoxy. In another preferred example, R₁, R₂, R₃, R₀₂, R₀₃, R₀₄ are each independently hydrogen.

In another preferred example, R₄, R₀₁ are each independently halogen (preferably F or Cl), cyano, C₁₋₃ alkyl(preferably methyl) or halogenated C₁₋₃ alkyl(preferably trifluoromethyl). In another preferred example, the C₆₋₁₀ aryl in L, W, R₄, R₅, R₆, R₇, R₈, R₉, R₁₀, R₀₁, R₁₁, R₁₂, R₂₁, R₂₂, R_{W}, R_{L}, R_{A}, R_{P}, R^{a}, R^{b}, R^{c}, R^{d}, R^{e}, R^{f}, R₁₃, R₁₄, R₁₅, R₃₁, R₃₂, R₄₁, R₄₂, R_{B}, and the C₆₋₁₀ aryl in the-C₁₋₄ alkyl-( C₆₋₁₀ aryl or C₅₋₁₄ heteroaryl) and the -(C₆₋₁₀ aryl or C₅₋₁₄ heteroaryl)-C₁₋₄ alkyl described in R_{W}, R_{L}, R_{A}, R_{P}, R^{a}, R^{b}, R^{c}, R^{d}, R^{e}, R^{f} are each independently phenyl, naphthyl, or is 9- or 10-membered bicyclic aryl formed by fusing a benzene ring and a 5- or 6-membered monocyclic alkyl; wherein the 5- or 6-membered monocyclic oalkyl is selected from: cyclopentyl, cyclopentenyl, cyclohexyl, cyclohexenyl, cyclohexadienyl, cyclopentanone, cyclopentane-1,3-dione, cyclohexanone, cyclohexane-1,3-dione.

In another preferred example, the C₅₋₁₄ heteroaryl in L, W, R₄, R₅, R₆, R₇, R₈, R₉, R₁₀, R₀₁, R₁₁, R₁₂, R₂₁, R₂₂, R_{W}, R_{L}, R_{A}, R_{P}, R^{a}, R^{b}, R^{c}, R^{d}, R^{e}, R^{f}, R₁₃, R₁₄, R₁₅, R₃₁, R₃₂, R₄₁, R₄₂, R_{B}, and the C₅₋₁₄ heteroaryl in -C₁₋₄ alkyl-( C₆₋₁₀ aryl or C₅₋₁₄ heteroaryl) and -(C₆₋₁₀ aryl or C₅₋₁₄ heteroaryl)-C₁₋₄ alkyl described in R_{W}, R_{L}, R_{A}, R_{P}, R^{a}, R^{b}, R^{c}, R^{d}, R^{e}, R^{f} are each independently 5- to 6-membered monocyclic heteroaryl, 8- to 10-membered bicyclic heteroaryl or 11- to 14-membered tricyclic heteroaryl. In another preferred example, the 3- to 16-membered saturated or unsaturated heterocycle in L, W, R₄, R₅, R₆, R₇, R₈, R₉, R₁₀, R₀₁, R₁₁, R₁₂, R₂₁, R₂₂, R_{W}, R_{L}, R_{A}, R_{P}, R^{a}, R^{b}, R^{c}, R^{d}, R^{e}, R^{f}, R₁₃, R₁₄, R₁₅, R₃₁, R₃₂, R₄₁, R₄₂, R_{B}, and the 3- to 16-membered saturated or unsaturated heterocycle in -C₁₋₄ alkyl- 3- to 16-membered saturated or unsaturated heterocycle and 3- to 16-membered saturated or unsaturated heterocycle-C₁₋₄ alkyl described in R_{W}, R_{L}, R_{A}, R_{P}, R^{a}, R^{b}, R^{c}, R^{d}, R^{e}, R^{f} are each independently 3- to 6-membered saturated or unsaturated monoheterocycle, 7- to 11-membered monospiro heterocycle, 10- to 16-membered bispiro heterocycle, 7- to 10-membered bicyclic fused heterocycle or 7- to 10-membered bridged heterocycle. In another preferred example, the 3- to 6-membered saturated or unsaturated monoheterocycle is selected from: aziridine, ethylene oxide, azetidine, azetidine-2-one, oxetane, oxetane-2-one, oxazolidine, pyrrolidine-2-one, pyrrolidine-2,5-dione, 1,3-dioxolane, dihydrofuran-2(3H)-one, dihydrofuran-2,5-dione, piperidine-2-one, piperidine-2,6-dione, tetrahydro-2H-pyran-2-one, imidazolidine, tetrahydrofuran, tetrahydrothiophene, tetrahydropyrrole, 1,3-dioxolane-2-one, oxazolidine-2-one, imidazolidine-2-one, piperidine, piperazine, piperazine-2-one, morpholine, morpholine-3-one, morpholine-2-one, thiomorpholine-3-one-1,1-dioxide, thiomorpholine, thiomorpholine-1,1-dioxide, tetrahydropyran.

In another preferred example, the C₃₋₁₀ cycloalkyl in R₅, R₆, R₇, Rs, R₉, Rio, R₄, R₀₁, R₁₁, R₁₂, R₂₁, R₂₂, R_{W}, R_{L}, R_{A}, R_{P}, R₁₃, R₁₄, R₁₅, R₃₁, R₃₂, R₄₁, R₄₂, R_{B}, and the C₃₋₁₀ cycloalkyl in -C₁₋₄ alkyl-C₃₋₁₀ cycloalkyl and -C₃₋₁₀ cycloalkyl-C₁₋₄ alkyl described in R_{W}, R_{L}, R_{A}, R_{P}, R^{a}, R^{b}, R^{c}, R^{d}, R^{e}, R^{f} are each independently cyclopropyl, cyclobutyl, cyclopentyl, cyclopentenyl, cyclohexyl, cyclohexenyl, cyclohexadienyl, cycloheptyl, cycloheptatrienyl, cyclooctyl or 6- to 10-membered spiro ring.

In another preferred example, the C₃₋₆ cycloalkyl in X, X₁, X₂, X₃, X₄ are each independently cyclopropyl, cyclobutyl, cyclopentyl or cyclohexyl. In another preferred example, the 3- to 6-membered saturated monoheterocycle in X, X₁, X₂, X₃, X₄ are each independently aziridine, ethylene oxide, azetidine, oxetane, tetrahydrofuran, tetrahydrothiophene, tetrahydropyrrole, oxazolidine, dioxolane, piperidine, piperazine, morpholine, dioxane, thiomorpholine, thiomorpholine-1,1-dioxide, tetrahydropyran. In another preferred example, X₁, X₂ are each independently a bond, O, S, NH, -C(O)NH-, -NHC(O)-, -SO₂NH-, -NHSO₂-, -C(O)-, -C(O)O-, -CH=CH- or -C≡C-.

In another preferred example, R₁₁, R₁₂, R₂₁, R₂₂ are each independently hydrogen, halogen, methyl, methoxy, CF₃, OCF₃, SCF₃, CN, NO₂, NH₂, acetyl, hydroxyl, carboxyl, substituted or unsubstituted C₁₋₃ alkyl, substituted or unsubstituted C₁₋₃ alkoxy, substituted or unsubstituted C₃₋₆ cycloalkyl, substituted or unsubstituted phenyl, substituted or unsubstituted 5- to 6-membered monocyclic heteroaryl, substituted or unsubstituted 3- to 6-membered saturated monoheterocycle, substituted or unsubstituted 7- to 11-membered monospiro heterocycle, substituted or unsubstituted 10- to 16-membered bispiro heterocycle, substituted or unsubstituted 7- to 10-membered bicyclic fused heterocycle or substituted or unsubstituted 7- to 10-membered bridged heterocycle; the "substituted" means that 1, 2, 3, 4 or 5 hydrogen atoms in the group are each independentlyubstituted by a substituent selected from the following group: acetyl, hydroxyl, cyano, carboxyl, nitro, halogen, C₁₋₃ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, -SO₂NRₐ₀R_{b0}; wherein Rₐ₀, R_{b0} are each independently hydrogen, C₁₋₃ alkyl or halogenated C₁₋₃ alkyl.

In another preferred example, R₄, R₀₁ are each independently hydrogen, cyano, acetyl, hydroxyl, carboxyl, nitro, halogen, substituted or unsubstituted C₁₋₃ alkyl, substituted or unsubstituted C₁₋₃ alkoxy, halogenated C₁₋₃ alkyl, halogenated C₁₋₃ alkoxy, halogenated C₁₋₃ alkylthio, NRₐ₀R_{b0}, substituted or unsubstituted C₃₋₆ cycloalkyl, substituted or unsubstituted phenyl, substituted or unsubstituted 5- to 6-membered monocyclic heteroaryl, substituted or unsubstituted 3- to 6-membered saturated monoheterocycle, substituted or unsubstituted 7- to 11-membered monospiro heterocycle, substituted or unsubstituted 10- to 16-membered bispiro heterocycle, substituted or unsubstituted 7- to 10-membered bicyclic fused heterocycle or substituted or unsubstituted 7- to 10-membered bridged heterocycle; the "substituted" means that 1, 2, 3, 4 or 5 hydrogen atoms in the group are each independently substituted by a substituent selected from the following group: acetyl, hydroxyl, cyano, carboxyl, nitro, halogen, C₁₋₃ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, -SO₂NRₐ₀R_{b0}; wherein Rₐ₀, R_{b0} are each independently hydrogen, C₁₋₃ alkyl or halogenated C₁₋₃ alkyl.

In another preferred example, R₄, R₀₁ are each independently hydrogen, halogen, methyl, methoxy, CF₃, OCF₃, SCF₃, CN, NO₂, NH₂, acetyl, hydroxyl, carboxyl, substituted or unsubstituted C₁₋₃ alkyl, substituted or unsubstituted C₁₋₃ alkoxy, substituted or unsubstituted C₃₋₆ cycloalkyl, substituted or unsubstituted phenyl, substituted or unsubstituted 5- to 6-membered monocyclic heteroaryl, substituted or unsubstituted 3- to 6-membered saturated monoheterocycle, substituted or unsubstituted 7- to 11-membered monospiro heterocycle, substituted or unsubstituted 10- to 16-membered bispiro heterocycle, substituted or unsubstituted 7- to 10-membered bicyclic fused heterocycle or substituted or unsubstituted 7- to 10-membered bridged heterocycle; the "substituted" means that 1, 2, 3, 4 or 5 hydrogen atoms in the group are each independently substituted by a substituent selected from the following group: acetyl, hydroxyl, cyano, carboxyl, nitro, halogen, C₁₋₃ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, -SO₂NRₐ₀R_{b0}; wherein Rₐ₀, R_{b0} are each independently hydrogen, C₁₋₃ alkyl or halogenated C₁₋₃ alkyl.

In another preferred example, L is substituted or unsubstituted phenyl, substituted or unsubstituted 9- to 10-membered bicyclic aryl formed by fusing a phenyl with a 5- to 6-membered saturated or unsaturated monocyclic ring, substituted or unsubstituted 5- or 6-membered monocyclic heteroaryl, substituted or unsubstituted 8- to 10-membered bicyclic heteroaryl, substituted or unsubstituted 9- to 10-membered heteroaryl formed by fusing a 5- or 6-membered monocyclic heteroaryl with a 5- to 6-membered saturated or unsaturated monoheterocycle; the "substituted" means that 1, 2 or 3 hydrogen atoms in the group are each independently substituted by a substituent selected from Rₚ. In another preferred example, W is substituted or unsubstituted phenyl or pyridyl; the "substituted" means that 1, 2 or 3 hydrogen atoms in the group are each independently substituted by a substituent selected from Rₚ.

In another preferred example, L, W are each independently substituted or unsubstituted phenyl or pyridyl; the "substituted" means that 1, 2 or 3 hydrogen atoms in the group are each independently substituted by -X₁-(CR₁₁R1₂)ₙ₁-X₂-(CR₂₁R₂₂)ₙ₂-R_{A}; wherein R_{A} is cyano, acetyl, hydroxyl, carboxyl, nitro, halogen, C₁₋₃ alkyl, C₁₋₃ alkoxy, halogenated C₁₋₃ alkyl, halogenated C₁₋₃ alkoxy, halogenated C₁₋₃ alkylthio, NRₐ₀R_{b0}, -CONRₐ₀R_{b0}, -SO₂NRₐ₀R_{b0}, -SO₂C₁₋₃ alkyl, phenyl, 5- or 6-membered monocyclic heteroaryl, C₃₋₆ cycloalkyl, 3- to 6-membered saturated monoheterocycle, wherein the phenyl, 5- or 6-membered monocyclic heteroaryl, C₃₋₆ cycloalkyl, 3- to 6-membered saturated monoheterocycle are unsubstituted or substituted with 1, 2 or 3 substituents each independently selected from acetyl, hydroxyl, cyano, carboxyl, nitro, halogen, C₁₋₃ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, -SO₂NRₐ₀R_{b0}; Rₐ₀, R_{b0}, R₁₁, R₁₂, R₂₁, R₂₂ are each independently hydrogen or C₁₋₃ alkyl; X₁, X₂ are each independently a bond, O, S, NH, -C(O)NH-, -SO₂NH-, -C(O)-, -C(O)O-, -CH=CH-, or -C≡C-; n1, n2 are each independently 0, 1, 2, 3, or 4.

In another preferred example, L, W are each independently substituted or unsubstituted phenyl or pyridyl; the "substituted" means that 1, 2 or 3 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, L, W are each independently substituted or unsubstituted 8- to 10-membered bicyclic heteroaryl ring; the "substituted" means that 1, 2 or 3 hydrogen atoms in the group are each independently substituted with -X₁-(CR₁₁R₁₂)ₙ₁-X₂-(CR₂₁R₂₂)ₙ₂-R_{A}; wherein R_{A} is cyano, acetyl, hydroxyl, carboxyl, nitro, halogen, C₁₋₃ alkyl, C₁₋₃ alkoxy, halogenated C₁₋₃ alkyl, halogenated C₁₋₃ alkoxy, halogenated C₁₋₃ alkylthio, NRₐ₀R_{b0}, -CONRₐ₀R_{b0}, -SO₂NRₐ₀R_{b0}, -SO₂C₁₋₃ alkyl, phenyl, 5- or 6-membered monocyclic heteroaryl, C₃₋₆ cycloalkyl, 3- to 6-membered saturated monoheterocycle, wherein the phenyl, 5- or 6-membered monocyclic heteroaryl, C₃₋₆ cycloalkyl, 3- to 6-membered saturated monoheterocycle are unsubstituted or substituted with 1, 2 or 3 substituents each independently selected from acetyl, hydroxyl, cyano, carboxyl, nitro, halogen, C₁₋₃ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, -SO₂NRₐ₀R_{b0}; Rₐ₀, R_{b0}, R₁₁, R₁₂, R₂₁, R₂₂ are each independently hydrogen or C₁₋₃ alkyl; X₁, X₂ are each independently a bond, O, S, NH, -C(O)NH-, -SO₂NH-, -C(O)-, -C(O)O-, -CH=CH-, or -C≡C-; n1, n2 are each independently 0, 1, 2, 3, or 4.

In another preferred example, L, W are each independently substituted or unsubstituted 8- to 10-membered bicyclic heteroaryl ring; the "substituted" means that 1, 2 or 3 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, L is substituted or unsubstituted phenyl or pyridyl; W is substituted or unsubstituted 8- to 10-membered bicyclic heteroaryl ring; the "substituted" means that 1, 2 or 3 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, L is substituted or unsubstituted 8- to 10-membered bicyclic heteroaryl ring ; W is substituted or unsubstituted phenyl or pyridyl; the "substituted" means that 1, 2 or 3 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, X is a bond; L is substituted or unsubstituted 8- to 10-membered bicyclic heteroaryl ring; the "substituted" means that 1 or 2 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl(preferably methyl), halogenated C₁₋₃ alkyl(preferably trifluoromethyl), C₁₋₃ alkoxy(preferably methoxy), halogenated C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, X is a bond; Lis selected from the following ring: 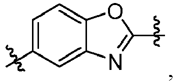 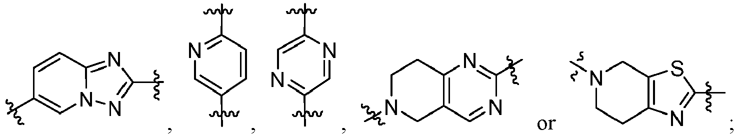 the ring is unsubstituted or 1 or 2 hydrogen atoms on the ring are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl(preferably methyl), halogenated C₁₋₃ alkyl(preferably trifluoromethyl), C₁₋₃ alkoxy(preferably methoxy), halogenated C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, X is NH; L is substituted or unsubstituted 8- to 10-membered bicyclic heteroaryl ring ; the "substituted" means that 1 or 2 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl(preferably methyl), halogenated C₁₋₃ alkyl(preferably trifluoromethyl), C₁₋₃ alkoxy(preferably methoxy), halogenated C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, X is NH; L is selected from the following ring: 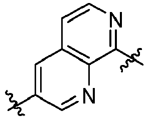 or 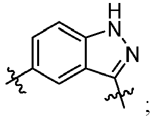 the ring is unsubstituted or 1 or 2 hydrogen atoms on the ring are substituted with substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl(preferably methyl), halogenated C₁₋₃ alkyl(preferably trifluoromethyl), C₁₋₃ alkoxy(preferably methoxy), halogenated C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; wherein Rₐ₀, R_{b0} are each independently hydrogen or C₁₋₃ alkyl.

In another preferred example, the 5- to 6-membered saturated or unsaturated monoheterocycle forming a fused bicyclic ring is selected from the following structures: 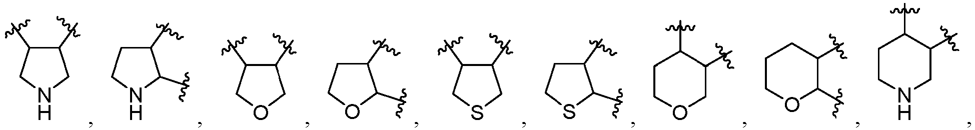 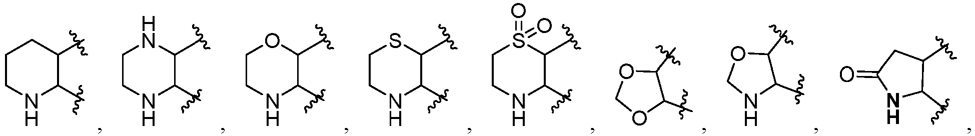 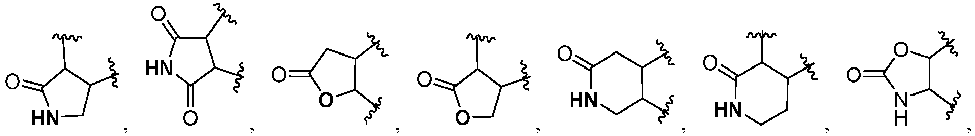 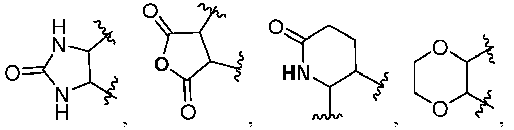 wherein the attached two carbon atoms represented by 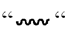 are adjacent pairs of carbon atoms shared when fused with other rings.

In another preferred example, the 5- to 6-membered saturated or unsaturated monoheterocycle forming a fused bicyclic ring is: 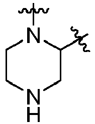 or wherein the attached two carbon atoms represented by 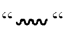 are adjacent pairs of carbon atoms shared when fused with other rings.

In another preferred example, the 8- to 10-membered bicyclic heteroaryl ring in L, W is selected from: benzoxazole, benzoisoxazole, benzimidazole, benzothiazole, benzisothiazole, benzotriazole, benzofuran, benzothiophene, indole, indazole, isoindole, quinoline, isoquinoline, quinazoline, quinoxaline, cinnoline, pyridoxazole, pyridoisoxazole, pyridoimidazole, pyridothiazole, pyridoisothiazole, pyridotriazole, pyridofuran, pyridothiophene, pyridopyrrole, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine.

In another preferred example, the compound is selected from Table A.

In another preferred example, the compounds of Table A comprise:

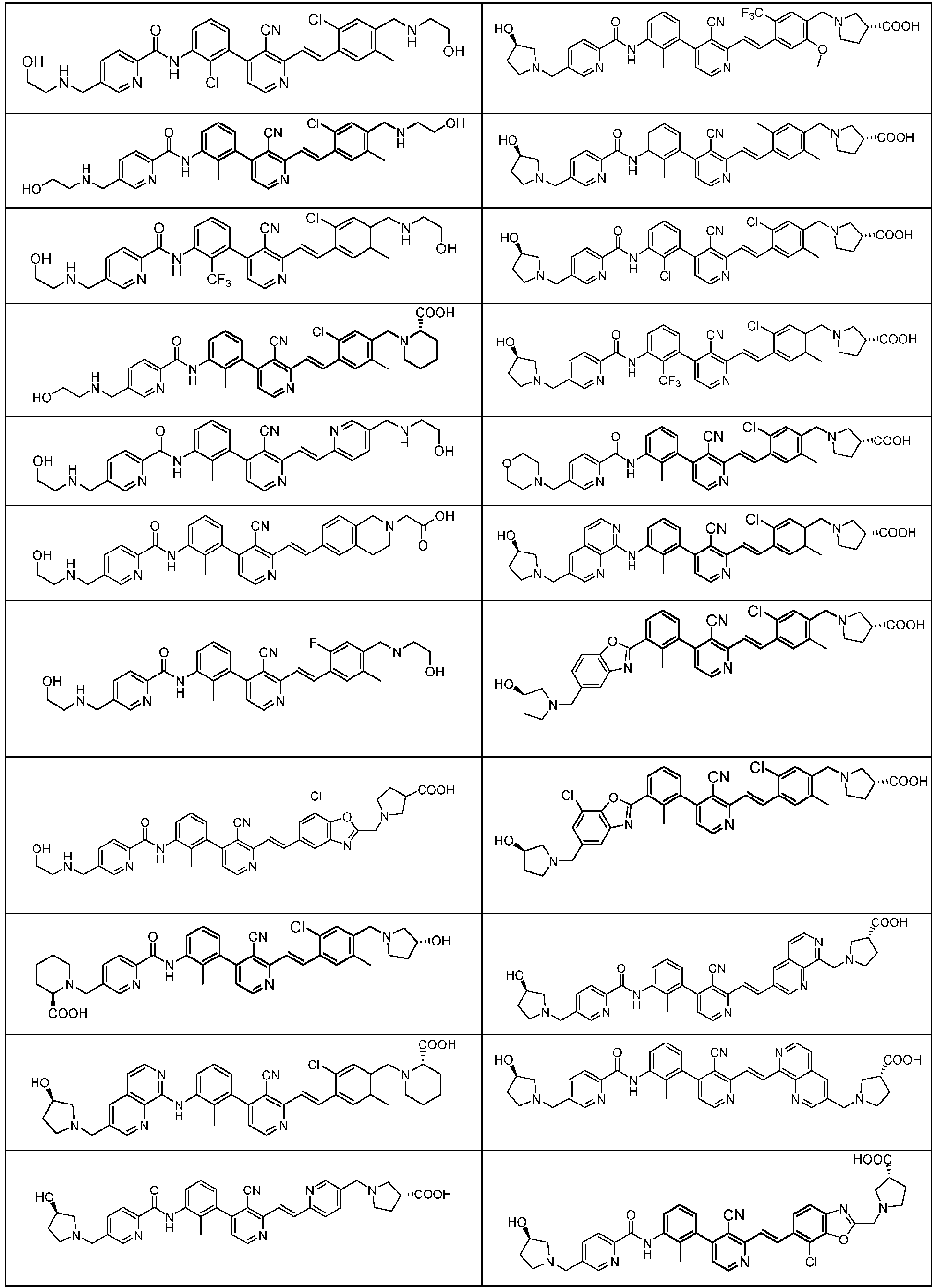

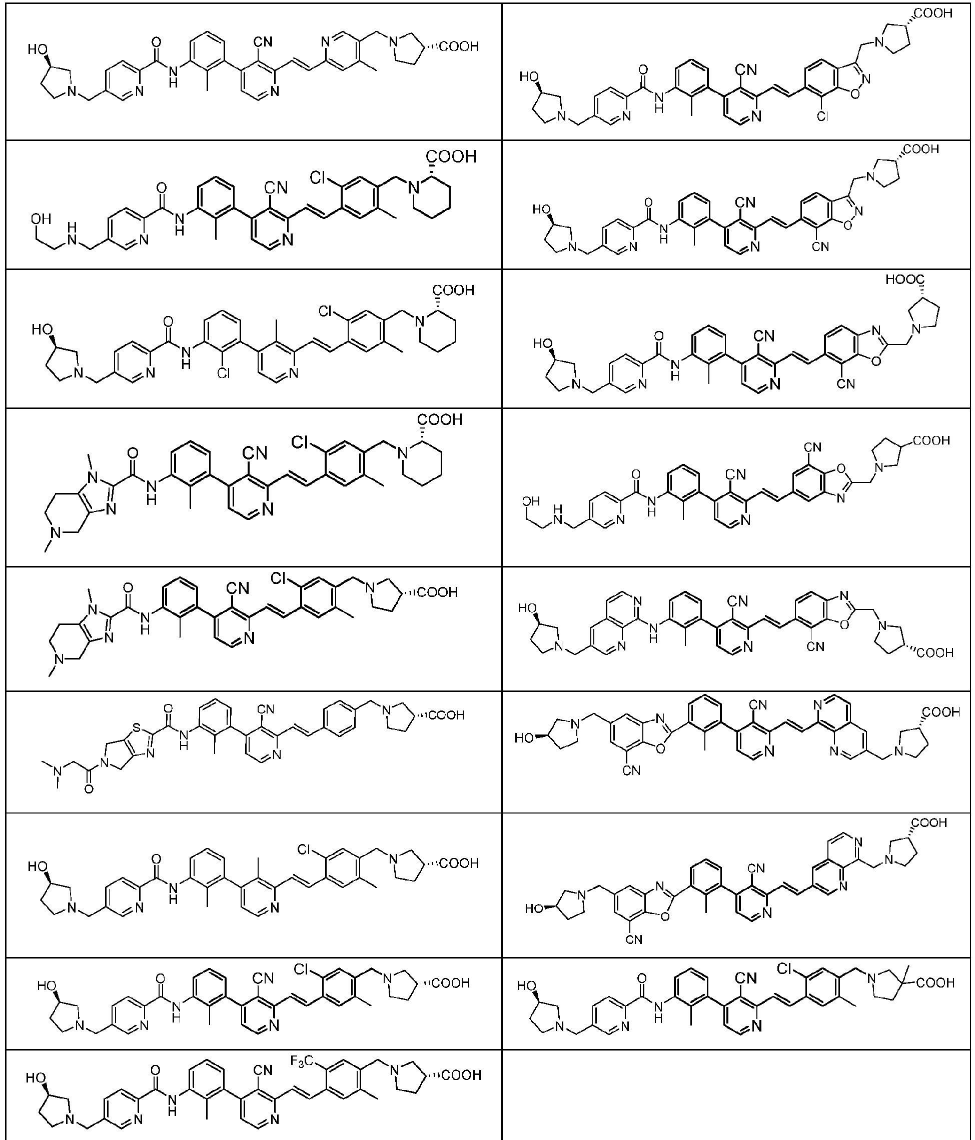

In another preferred example, the compound is selected from Table B.

In another preferred example, the compounds of Table B comprise:

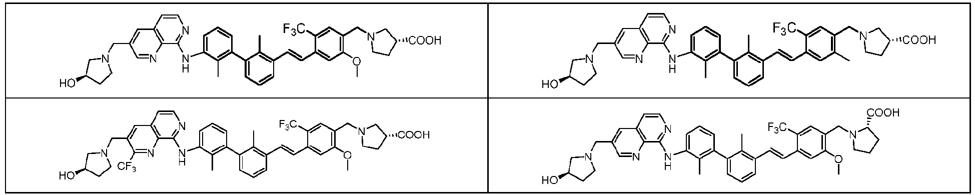

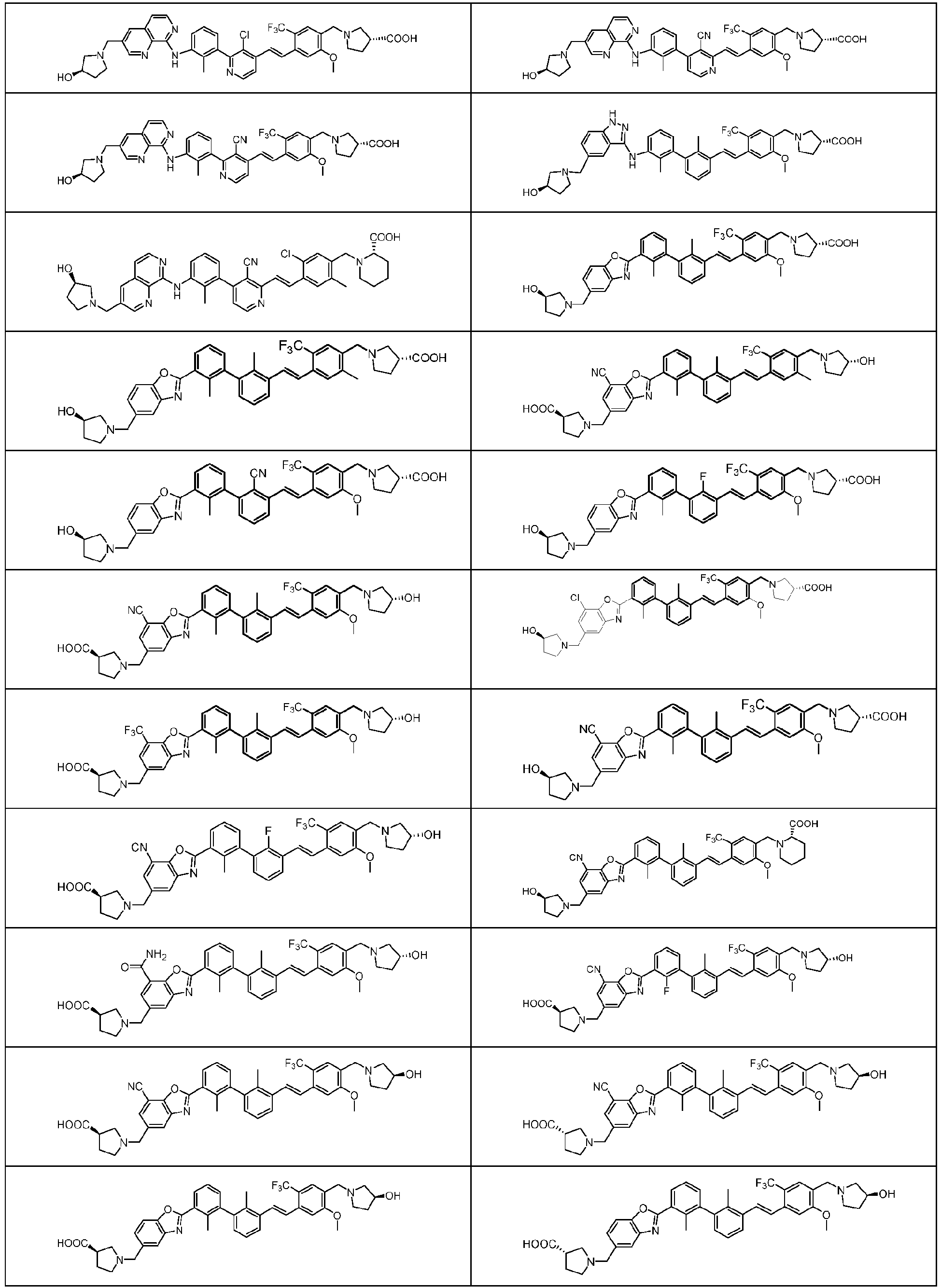

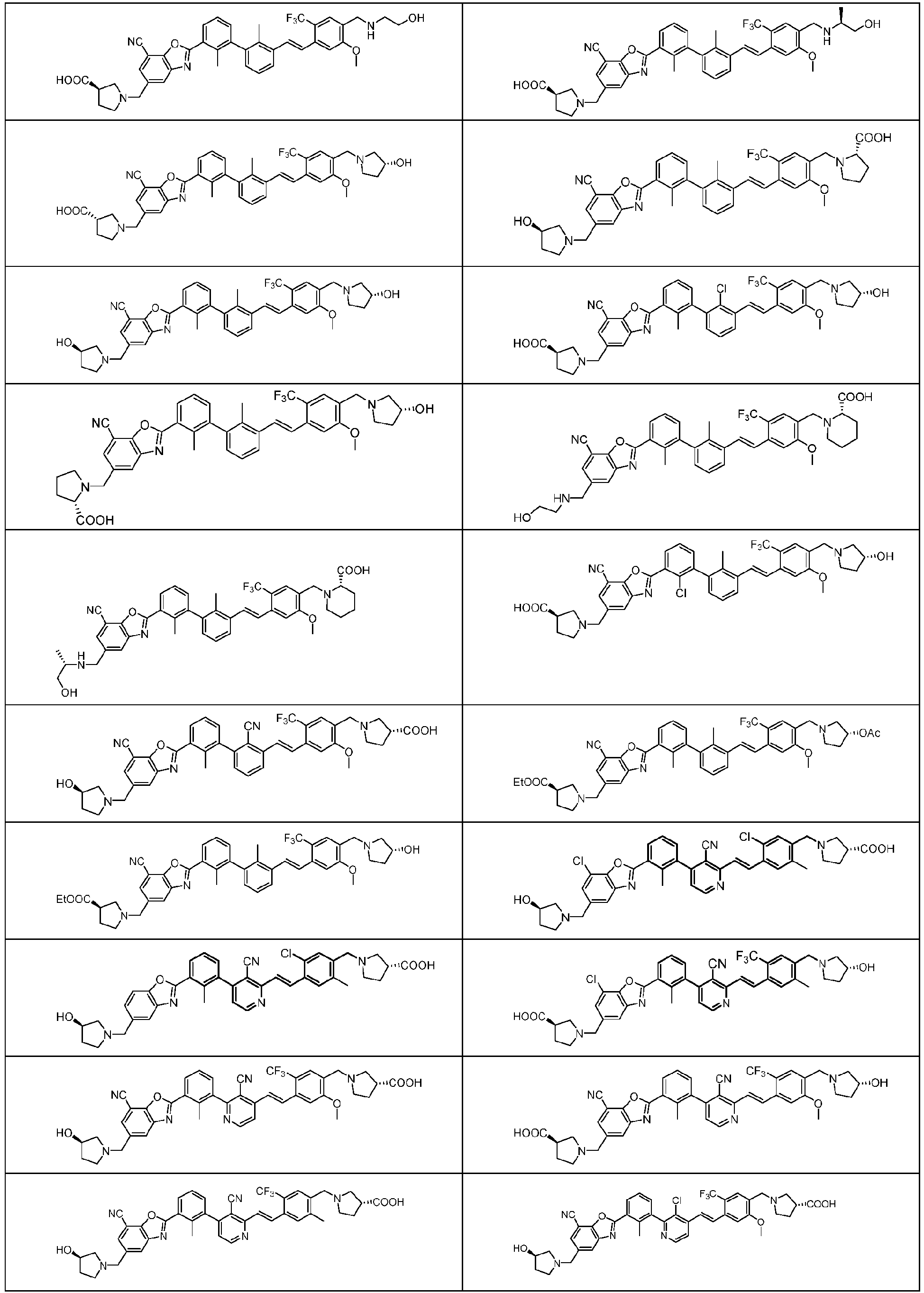

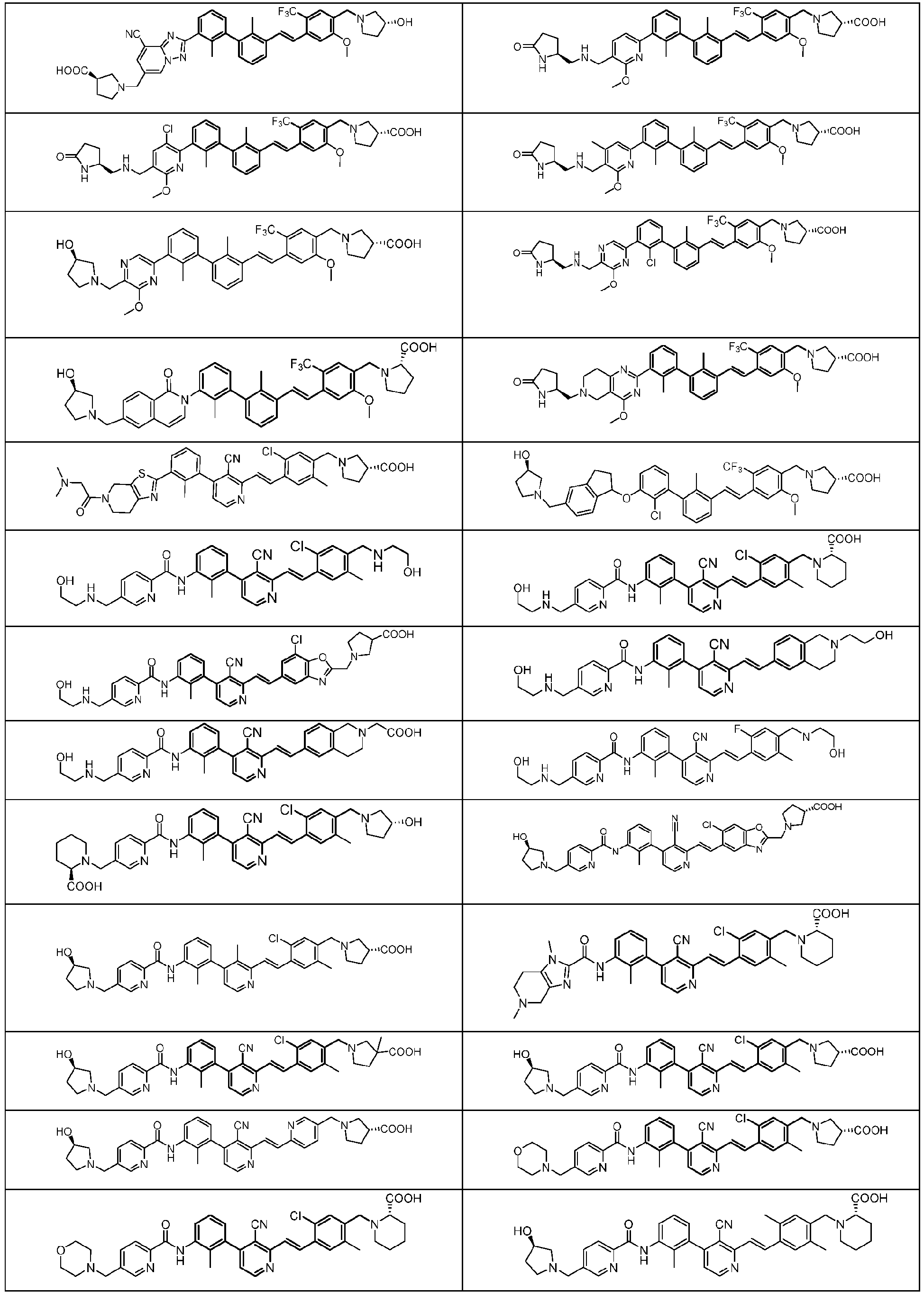

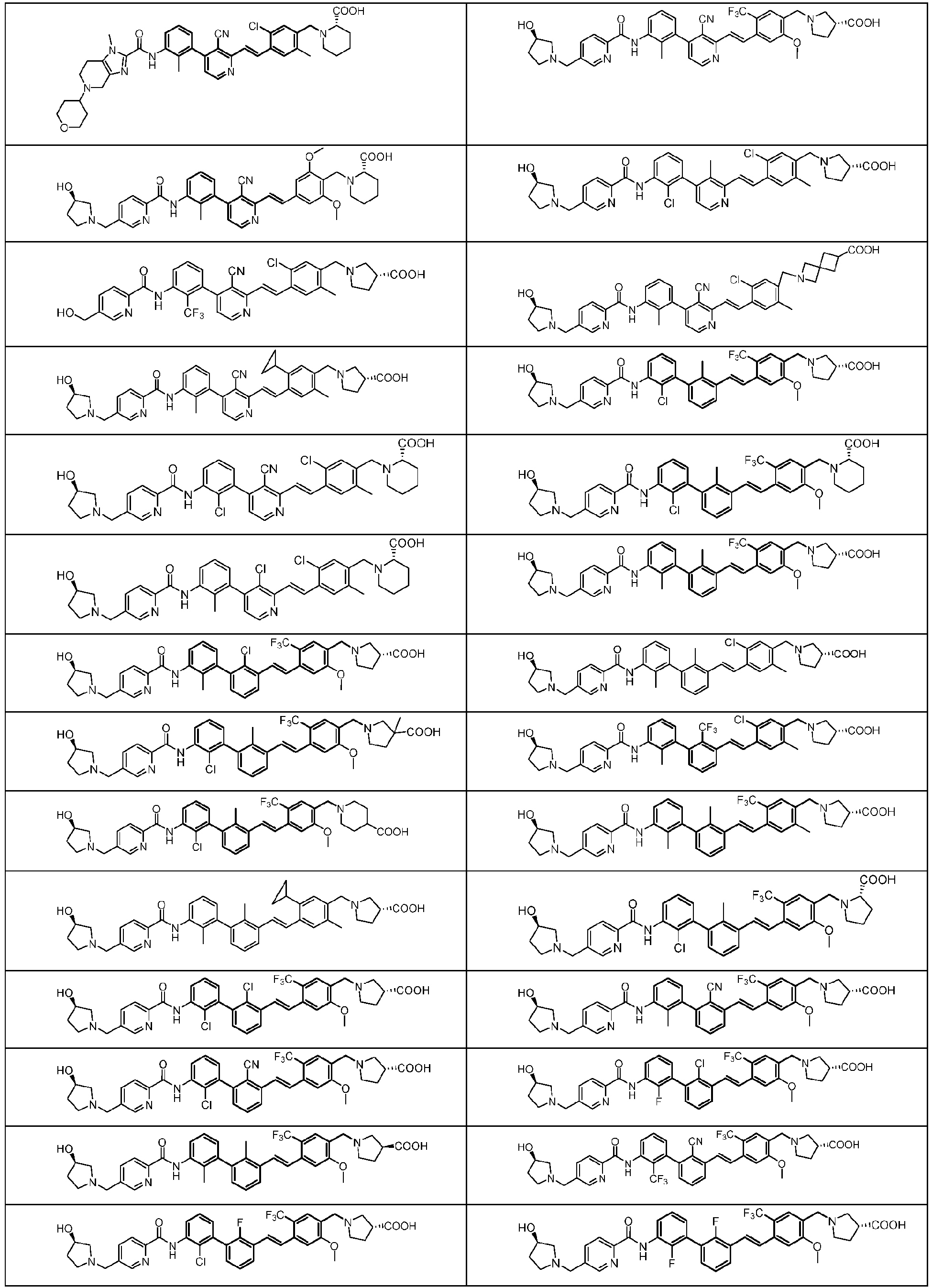

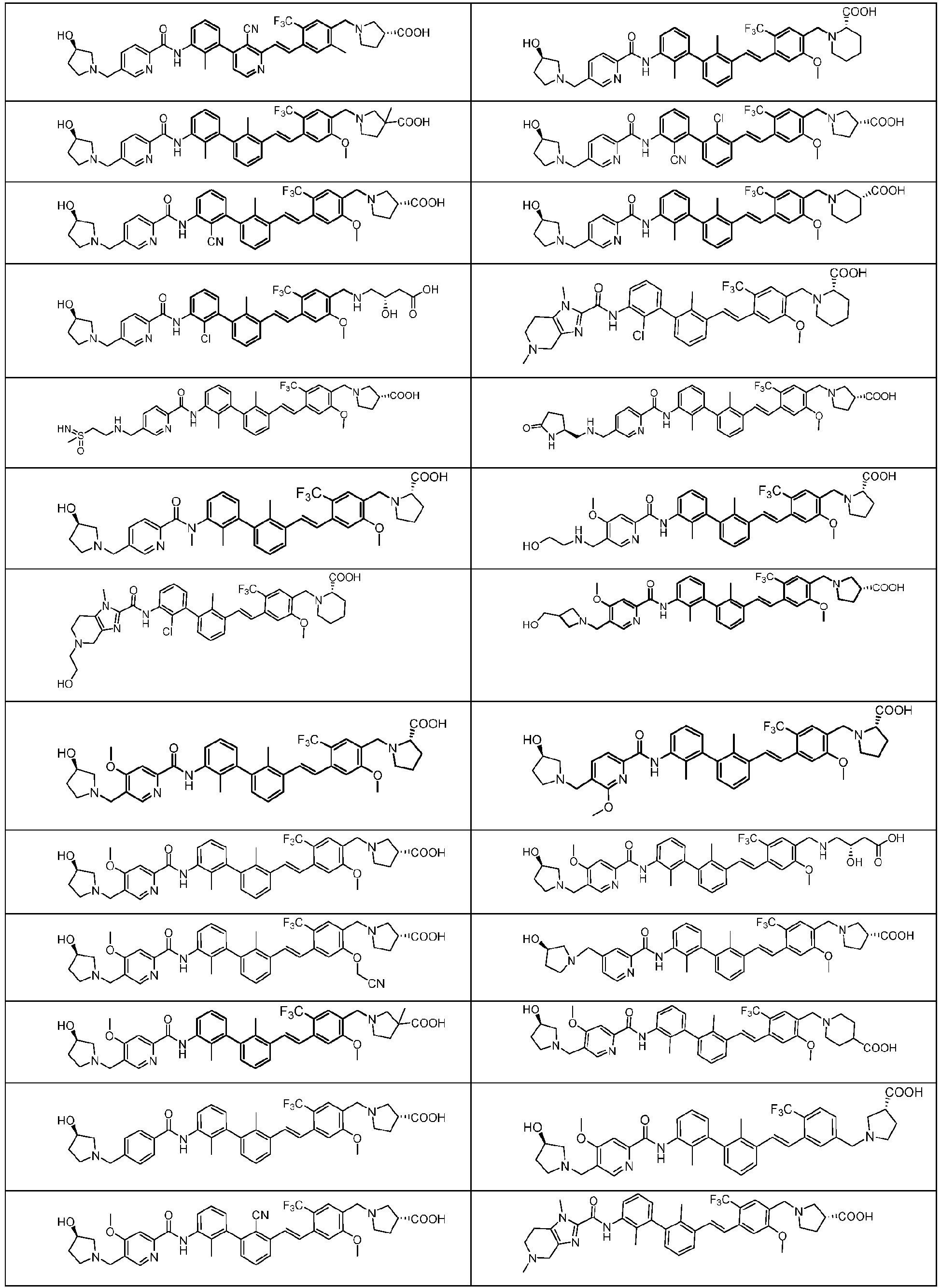

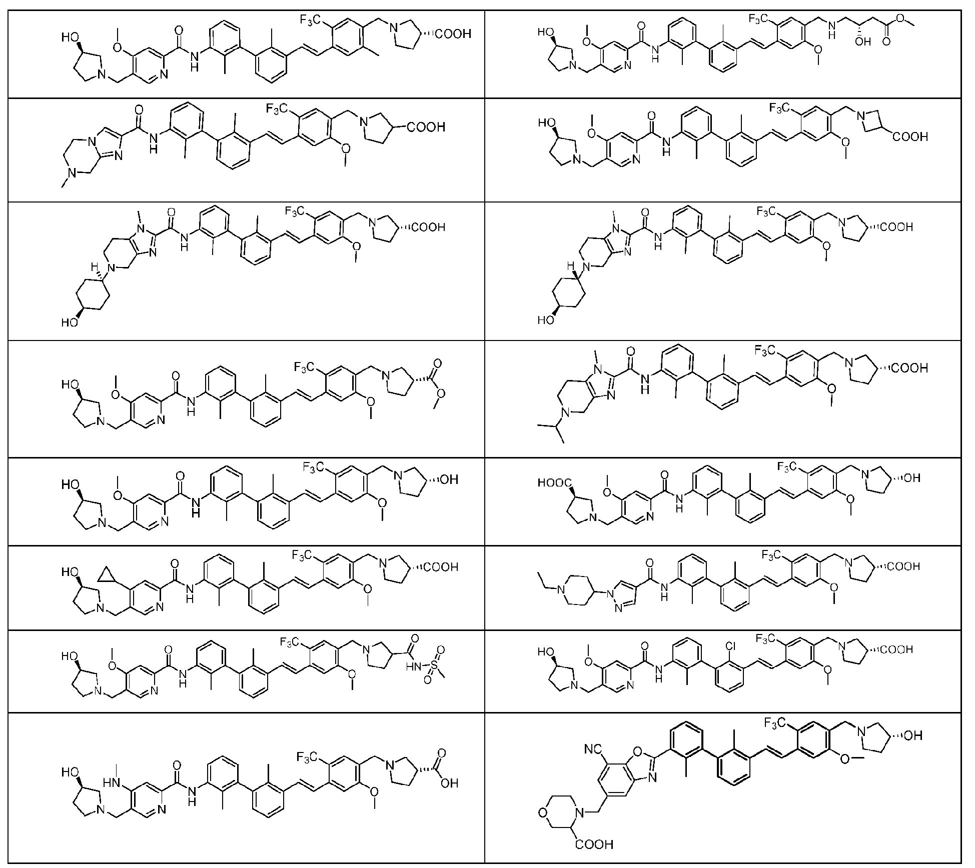

Although the above table lists the structures of the preferred compounds of the present disclosure, it should be understood that when the cyclohexyl has a set of para-substituted substituents, the two carbon atoms on the cyclohexyl that is connected to the set of para-substituted substituents are not chiral centers, the chemical bond notation in form 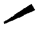 and 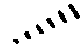 only means that the two chemical bonds connected to the set of para-substituted substituents are in trans or cis structure relative to the cyclohexyl group, therefore, the compounds represented by exchanging these two chemical bonds 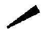 and 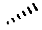 with each other also fall within the protection scope of the present disclosure.

In another preferred example, the R_{L}, L, X, A₁, A₂, A₃, Z₁, Z₂, Z₃, R₄, R₀₁, W, R_{W} are each independently a corresponding group in each specific compound in the examples. In another preferred example, the compound of formula (I) is selected from the specific compounds noted in the examples. In another preferred example, the compound of formula (I) is selected from compounds prepared in the examples of this application.

In the second aspect, the present disclosure provides a pharmaceutical composition comprising the compound according to the first aspect of the present disclosure or a stereoisomer thereof, or a pharmaceutically acceptable salt, solvate or prodrug thereof; and a pharmaceutically acceptable carrier.

In the third aspect, the present disclosure provides a pharmaceutical composition comprising the compound according to the first aspect of the present disclosure or a stereoisomer thereof, or a pharmaceutically acceptable salt, solvate or prodrug thereof; and at least one other agent, wherein the other agent is an anticancer agent, a chemotherapeutic agent, or an antiproliferative compound.

In the fourth aspect, the present disclosure provides use of the compound according to the first aspect of the present disclosure or a stereoisomer thereof, or a pharmaceutically acceptable salt, solvate or prodrug thereof, or the pharmaceutical composition according to the second and third aspects of the present disclosure in the preparation of a drug for treating a cancer or infectious disease.

In another preferred example, the cancer is selected from: bone cancer, cancer of the head or neck, pancreatic cancer, skin cancer, cutaneous or intraocular malignant melanoma, uterine cancer, ovarian cancer, rectal cancer, cancer of the anal region, stomach cancer, testicular cancer, uterine cancer, fallopian tube cancer, endometrial cancer, cervical cancer, vaginal cancer, vulval cancer, Hodgkin's Disease, non-Hodgkin's lymphoma, esophagus cancer, small intestine cancer, cancer of the endocrine system, thyroid gland cancer, parathyroid gland cancer, adrenal gland cancer, soft tissue sarcoma, urethral cancer, penis cancer, chronic or acute leukemias including acute myeloid leukemia, chronic myeloid leukemia, acute lymphocytic leukemia, chronic lymphocytic leukemia, childhood solid tumours, lymphocytic lymphoma, bladder cancer, kidney or ureter cancer, renal pelvis cancer, central nervous system (CNS) neoplasm, primary CNS lymphoma, tumour angiogenesis, spinal axis tumour, brain stem glioma, pituitary adenoma, Kaposi's sarcoma, epidermoid cancer, squamous cell cancer, T-cell lymphoma, environmentally induced cancers including those induced by asbestos, and combinations of said cancers.

In another preferred example, the infectious disease is a bacterial infectious disease, a viral infectious disease or a fungal infectious disease.

In the fifth aspect, the present disclosure provides a method for modulating the immune response mediated by the PD-1 signaling pathway in a subject, comprising administering to the subject a therapeutically effective amount of the compound according to the first aspect of the present disclosure or a stereoisomer thereof, or a pharmaceutically acceptable salt, solvate or prodrug thereof, or the pharmaceutical composition according to the second or third aspect of the present disclosure.

In the sixth aspect, the present disclosure provides a method for inhibiting the growth and/or migration of tumor cells in a subject, comprising administering to the subject a therapeutically effective amount of the compound according to the first aspect of the present disclosure or a stereoisomer thereof, or a pharmaceutically acceptable salt, solvate or prodrug thereof, or the pharmaceutical composition according to the second or third aspect of the present disclosure.

In another preferred example, the tumor cell is from a cancer selected from: breast cancer, colon cancer, lung cancer, melanoma, prostate cancer and renal cancer.

In another preferred example, the tumor cell is from a cancer selected from the list consisting of bone cancer, cancer of the head or neck, pancreatic cancer, skin cancer, cutaneous or intraocular malignant melanoma, uterine cancer, ovarian cancer, rectal cancer, cancer of the anal region, stomach cancer, testicular cancer, uterine cancer, fallopian tube cancer, endometrial cancer, cervical cancer, vaginal cancer, vulval cancer, Hodgkin's Disease, non-Hodgkin's lymphoma, esophagus cancer, small intestine cancer, cancer of the endocrine system, thyroid gland cancer, parathyroid gland cancer, adrenal gland cancer, soft tissue sarcoma, urethral cancer, penis cancer, chronic or acute leukemias including acute myeloid leukemia, chronic myeloid leukemia, acute lymphocytic leukemia, chronic lymphocytic leukemia, childhood solid tumours, lymphocytic lymphoma, bladder cancer, kidney or ureter cancer, renal pelvis cancer, central nervous system (CNS) neoplasm, primary CNS lymphoma, tumour angiogenesis, spinal axis tumour, brain stem glioma, pituitary adenoma, Kaposi's sarcoma, epidermoid cancer, squamous cell cancer, T-cell lymphoma, environmentally induced cancers including those induced by asbestos, and combinations of said cancers.

In the seventh aspect, the present disclosure provides a method for treating an infectious disease in a subject, comprising administering to the subject a therapeutically effective amount of the compound according to the first aspect of the present disclosure or a stereoisomer thereof, or a pharmaceutically acceptable salt, solvate or prodrug thereof, or the pharmaceutical composition according to the second or third aspect of the present disclosure.

In the eighth aspect, the present disclosure provides a method for treating a bacterial, viral and fungal infection in a subject, comprising administering to the subject a therapeutically effective amount of the compound according to the first aspect of the present disclosure or a stereoisomer thereof, or a pharmaceutically acceptable salt, solvate or prodrug thereof, or the pharmaceutical composition according to the secondor third aspect of the present disclosure.

In another preferred example, the infectious disease includes but not limited to HIV, Influenza, Herpes, Giardia, Malaria, Leishmania, the pathogenic infection by the virus Hepatitis(A, B, & C), herpes virus (e.g., VZV, HSV-I, HAV-6, HSV-II, and CMV, Epstein Barr virus), adenovirus, influenza virus, flaviviruses, echovirus, rhinovirus, coxsackie virus, cornovirus, respiratory syncytial virus, mumps virus, rotavirus, measles virus, rubella
virus, parvovirus, vaccinia virus, HTLV virus, dengue virus, papillomavirus, molluscum virus, poliovirus, rabies virus, JC virus and arboviral encephalitis virus, pathogenic infection by the bacteria chlamydia, rickettsial bacteria, mycobacteria, staphylococci, streptococci, pneumonococci, meningococci and conococci, klebsiella, proteus, serratia, pseudomonas, E. coli, legionella, diphtheria, salmonella, bacilli, cholera, tetanus, botulism, anthrax, plague, leptospirosis, and Lyme's disease bacteria, pathogenic infection by the fungi Candida (albicans, krusei, glabrata, tropicalis, etc.), Cryptococcus neoformans, Aspergillus (fumigatus, niger, etc.), Genus Mucorales (mucor, absidia, rhizophus), Sporothrix schenkii, Blastomyces dermatitidis, Paracoccidioides brasiliensis, Coccidioides immitis and Histoplasma capsulatum, and pathogenic infection by the parasites Entamoeba histolytica, Balantidium coli, Naegleriafowleri, Acanthamoeba sp., Giardia lambia, Cryptosporidium sp., Pneumocystis carinii, Plasmodium vivax, Babesia microti, Trypanosoma brucei, Trypanosoma cruzi, Leishmania donovani, Toxoplasma gondi, Nippostrongylus brasiliensis.

It should be understood that each of the above technical features of the present disclosure and each technical feature specifically described below (such as in Examples) can be combined with each other within the scope of the present disclosure so as to constitute new or preferred technical solutions which need not be specified again herein.

### DETAILED DESCRIPTION OF THE DISCLOSURE

The inventors have conducted extensive and intensive studies and have unexpectedly found that such immunomodulators have high inhibitory activity especially against PPI-HTRF and other enzymes. Therefore, this series of compounds are expected to be developed as drugs for the treatment of tumors. On this basis, the inventor completed the present disclosure.

### DEFINITION OF TERMS

As used herein, "alkyl" refers to a straight or branched saturated aliphatic hydrocarbyl. C₁₋₁₀ alkyl is an alkyl containing 1 to 10 carbon atoms, preferably C₁₋₈ alkyl, more preferably C₁₋₆ alkyl, more preferably C₁₋₃ alkyl whose definition is similar. Non-limiting examples of the alkyl include methyl, ethyl, n-propyl, isopropyl, n-butyl, isobutyl, t-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched isomers thereof, etc..

As used herein, "heteroalkyl" by itself or in combination with another term means a stable linear or branched hydrocarbon atomic group or a combination thereof, and consists of a certain number of carbon atoms and at least one heteroatom. "C₁₋₁₀ heteroalkyl" means an alkyl group containing 1 to 10 carbon atoms, in which the carbon atoms are replaced by 1, 2 or 3 heteroatoms selected from O, N and S, wherein nitrogen and sulfur atoms are optionally oxidized, and nitrogen heteroatoms are optionally quaternized. The heteroatom can be located in any internal position of the heteroalkyl group, including the position where the alkyl group is attached to the rest of the molecule. However, the terms "alkoxy", "alkylamino" and "alkylthio" (or thioalkoxy) are customary expressions and refer to those alkyl groups connected to the rest of the molecule through an oxygen atom, amino or sulfur atom, respectively. Examples include but are not limited to-CH₂-CH₂-O-CH₃, -CH₂-NH-CH₂-CH₃, -CH₂-CH₂-N(CH₃)-CH₃, -CH₂-S-CH₂-CH₃, -CH₂-CH₂-S(O)-CH₃, -CH₂-CH₂-S(O)₂-CH₃, -CH₂-CH₂-O-CH₂-CH₂-O-CH₂-CH₃, -CH₂-CH₂-O-CH₂-NH-CH₂-CH₃. At most two heteroatoms can be continuous, for example-CH₂-NH-O-CH₃.

As used herein, "cycloalkyl" and "cycloalkyl ring" can be used interchangeably and refer to a saturated or partially unsaturated monocyclic or polycyclic cyclic hydrocarbon group. "C₃₋₁₀ cycloalkyl" refers to a monocyclic or bicyclic cyclic hydrocarbon group containing 3 to 10 carbon atoms, and "C₃₋₈ monocycloalkyl" refers to a saturated or unsaturated monocyclic cyclic hydrocarbon group containing 3 to 8 carbon atoms, preferably contains 3 to 6 carbon atoms. In the present disclosure, "C₃₋₆ cycloalkyl" and "C₃₋₆ monocycloalkyl" can be used interchangeably, and both refer to a monocyclic cyclic hydrocarbon group. When "C₃₋₁₀ cycloalkyl" is bicyclic, it refers to a 6- to 10-membered spiro ring. The ring carbon atoms of the cycloalkyl group may be optionally substituted with 1, 2, or 3 oxo groups to form a cyclic ketone structure. Non-limiting examples of C₃₋₈ monocycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, cyclobutanone, cyclobutane-1,2-dione, cyclopentanone, cyclopentane-1,3-dione, cyclohexanone, cyclohexane-1,3-dione, etc., preferably cyclopropyl, cyclopentyl, and cyclohexenyl.

As used herein, "spiro" refers to a polycyclic group in which two single sings share one carbon atom (spiro atom), wherein these polycyclic groups may contain one or more double bonds, but none of the rings has a completely conjugated π electron system. According to the number of rings therein, the spiros are divided into bicyclic spiros or polycyclic spiros, preferably 6- to 10-membered bicyclic spiro ring. And 4 membered /5 membered, 5 membered /5 membered, or 5 membered /6 membered bicyclic spiros are more preferable. For example:

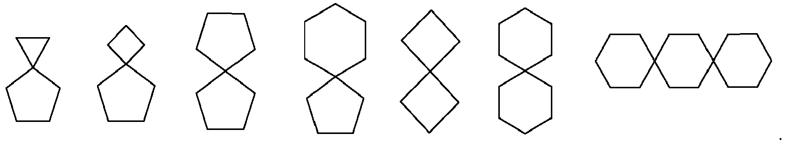

As used herein, "C₂₋₁₀ alkenyl" refers to a linear or branched unsaturated aliphatic hydrocarbon group having carbon-carbon double bonds (C=C) which has 2-10 (preferably 2-6) carbon atoms, for example, vinyl, propenyl, isopropenyl, n-butenyl, isobutenyl, pentenyl, hexenyl and the like.

As used herein, "C₂₋₁₀ alkynyl" refers to a linear or branched unsaturated aliphatic hydrocarbon group having carbon-carbon triple bonds which has 2-10 (preferably 2-6) carbon atoms, for example, ethynyl, propynyl, n-butynyl, isobutynyl, pentynyl, hexynyl and the like.

As used herein, "heterocycle" and "heterocyclic group" can be used interchangeably and refer to a saturated or partially unsaturated monocyclic or polycyclic cyclic hydrocarbon group, which contains 3 to 20 ring atoms, of which one or more (preferably 1, 2 , 3 or 4) ring atoms are heteroatoms selected from nitrogen, oxygen or S(O)ₘ (wherein m is an integer of 0 to 2), but do not include the ring part of -OO-, -OS- or -SS-, the remaining ring atoms are carbon atoms. The nitrogen atom can be substituted or unsubstituted (ie N or NR, R is hydrogen or other substituents already defined herein). The ring carbon atoms on the heterocycle may be optionally substituted with 1, 2 or 3 oxo groups to form a cyclic ketone, cyclic lactone or cyclic lactam structure. The heterocycle preferably contains 3 to 16 ring atoms, more preferably contains 3 to 10 ring atoms, wherein 1, 2, 3, or 4 ring atoms are heteroatoms.

As used herein, "3- to 16-membered saturated or unsaturated heterocycle" refers to a heterocycle containing 3 to 16 ring atoms, comprising monoheterocycle, spiro heterocycle, fused heterocycle and bridged heterocycle. As used herein, "3- to 6-membered saturated or unsaturated monoheterocycle" refers to a monoheterocycle containing 3 to 6 ring atoms, preferably 5 to 6 ring atoms, wherein 1 to 2 ring atoms are heteroatoms. As used herein, "4- to 7-membered saturated or unsaturated heterocycle" refers to a monoheterocycle containing 4 to 7 ring atoms, preferably 4 to 6 ring atoms, wherein 1 to 2 ring atoms are heteroatoms. Non-limiting examples of saturated or unsaturated heterocycles include: aziridine, ethylene oxide, azetidine, azetidine-2-one, oxetane, oxetane-2-one, oxazolidine, pyrrolidine-2-one, pyrrolidine-2,5-dione, 1,3-dioxolane, dihydrofuran-2(3H)-one, dihydrofuran-2,5-dione, piperidine-2-one, piperidine-2,6-dione, tetrahydro-2H-pyran-2-one, imidazolidine, tetrahydrofuran, tetrahydrothiophene, tetrahydropyrrole, 1,3-dioxolane-2-one, oxazolidine-2-one, imidazolidine-2-one, piperidine, piperazine, piperazine-2-one, morpholine, morpholine-3-one, morpholine-2-one, thiomorpholine-3-one-1,1-dioxide, thiomorpholine, thiomorpholine-1,1-dioxide, tetrahydropyran and the like. The above saturated or unsaturated heterocycle may optionally be fused with the cycloalkyl, heterocycle, aryl or heteroaryl group of the present disclosure to form a fused polycyclic ring.

As used herein, the term "7- to 11-membered monospiro heterocycle" refers to a spiro heterocycle that shares one spiro atom and contains 1 or 2 heteroatoms selected from nitrogen and oxygen. Non-limiting examples include:

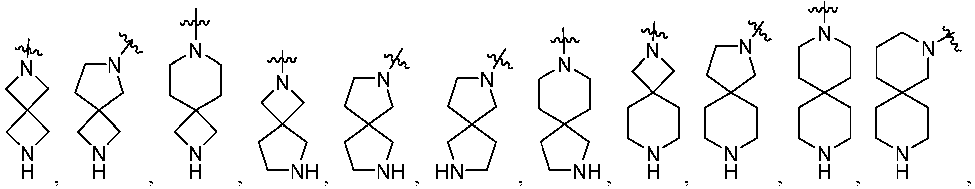

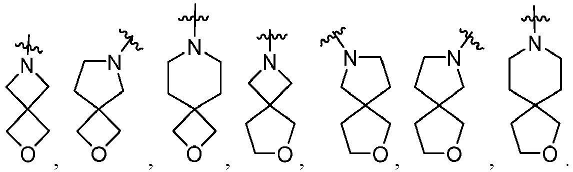

As used herein, the term "10- to 16-membered bispiro heterocycle" refers to a spiro heterocycle that shares two spiro atoms and contains 1 or 2 heteroatoms selected from nitrogen and oxygen.

As used herein, the term "7- to 10-membered bicyclic fused heterocycle" refers to a bicyclic fused heterocycle formed by fusing 4- to 6-membered saturated or unsaturated monoheterocycle with 4- to 6-membered saturated or unsaturated monoheterocycle, or a bicyclic fused heterocycle formed by fusing 4- to 6-membered saturated or unsaturated monoheterocycle with 4- to 6-membered monocycloalkyl, wherein saturated or unsaturated monoheterocycle and monocycloalkyl are as defined herein.

As used herein, the term "heteroaryl ring " and "heteroaryl" can be used interchangeably and refer to monocyclic or fused polycyclic (that is, rings sharing adjacent carbon atoms or a pair of heteroatoms) groups containing 1 to 4 heteroatoms, which have 5 to 14 ring atoms, preferably 5 to 10 ring atoms, more preferably 5, 6 , 8, 9, or 10 ring atoms, wherein the heteroatoms are selected from the group consisting of oxygen, sulfur and nitrogen, and the nitrogen and sulfur atoms can be optionally oxidized, and nitrogen atom can be optionally quaternized. The heteroaryl group has 6, 10 or 14 π electrons shared in the ring system. At least one ring in the ring system is aromatic. In the present disclosure, C₅₋₁₄ heteroaryl include 5- or 6-membered monocyclic heteroaryl, 8- to 10-membered bicyclic heteroaryl, 11- to 14-membered tricyclic heteroaryl, the heteroaryl formed by fusing 5- or 6-membered monocyclic heteroaryl ring with 5- to 6-membered saturated or unsaturated monoheterocycle, the heteroaryl formed by fusing 5- or 6-membered monocyclic heteroaryl ring with 5- to 6-membered saturated or unsaturated monocyclic ring, the heteroaryl formed by fusing benzene ring with 5- to 6-membered saturated or unsaturated monoheterocycle, wherein saturated or unsaturated monoheterocycle, saturated or unsaturated monocyclic ring are as defined herein. As used herein, "5- to 6-membered monocyclic heteroaryl" refers to a monocyclic heteroaryl ring containing 5 to 6 ring atoms, examples include (but not limited to): thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine, etc. As used herein, "8- to 10-membered bicyclic heteroaryl" refers to a 9- or 10-membered bicyclic heteroaryl formed by fusing a benzene ring with a 5- or 6-membered monocyclic heteroaryl, an 8- to 10-membered bicyclic heteroaryl formed by fusing a 5- or 6-membered monocyclic heteroaryl with a 5- or 6-membered monocyclic heteroaryl; wherein the 5- or 6-membered monocyclic heteroaryl is selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In some embodiments of the disclosure, Non-limiting examples of the heteroaryl formed by fusing a 5- to 6-membered saturated or unsaturated monoheterocycle with a 5- or 6-membered monocyclic heteroaryl include:

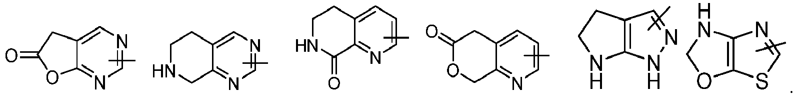

As used herein, "C₆₋₁₀ aryl" refers to an all-carbon monocyclic or fused polycyclic (that is, rings that share adjacent carbon atoms pairs) group with a conjugated π-electron system, and is an aryl group containing 6 to 10 carbon atoms, such as phenyl and naphthyl, or is an 7- to 10-membered bicyclic aryl (preferably a 9- or 10-membered bicyclic aryl formed by fusing a benzene ring and a 5- or 6-membered monocyclic cycloalkyl) formed by fusiing a benzene ring with a 3- to 6-membered monocycloalkyl.

As used herein, "C₁₋₁₀ alkoxy" refers to -O-(C₁₋₁₀ alkyl), wherein alkyl is defined as above. C₁₋₈ alkoxy is preferred, and C₁₋₆ alkoxy is more preferred, and C₁₋₃ alkoxy is more preferred. Non-limiting examples include methoxy, ethoxy, propoxy, isopropoxy, butoxy, tert-butoxy, isobutoxy, pentoxy and the like.

As used herein, "C₁₋₁₀ alkylthio" refers to -S-(C₁₋₁₀ alkyl), wherein alkyl is defined as above. C₁₋₈ alkylthio is preferred, C₁₋₆ alkylthio is more preferred, and C₁₋₃ alkylthio is more preferred. Non-limiting examples include methylthio, ethylthio, propylthio, isopropylthio, butylthio, tert-butylthio, isobutylthio, pentylthio and the like.

As used herein, "C₃₋₈ cycloalkoxy" refers to -O-(C₃₋₈ monocycloalkyl), wherein cycloalkyl is defined as above. C₃₋₆ cycloalkoxy is preferred. Non-limiting examples include cyclopropoxy, cyclobutoxy, cyclopentyloxy, cyclohexyloxy and the like.

As used herein, "a bond" means that the two groups connected by it are connected by a covalent bond. As used herein, "halogen" refers to fluorine, chlorine, bromine or iodine. As used herein, "halogenated" means that one or more (e.g., 1, 2, 3, 4, or 5) hydrogen atoms in a group are substituted by halogen atoms. For example, "halogenated C₁₋₁₀ alkyl" means that the alkyl is substituted with one or more (e.g., 1, 2, 3, 4, or 5) halogen atoms, wherein alkyl is defined as above. Halogenated C₁₋₆ alkyl is preferred, and halogenated C₁₋₃ alkyl is more preferred. Examples of halogenated C₁₋₈ alkyl include, but are not limited to, monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, trifluoroethyl, and the like. For example, "halogenated C₁₋₁₀ alkoxy" means that the alkoxy is substituted with one or more (e.g., 1, 2, 3, 4, or 5) halogen atoms, wherein alkoxy is defined as above. Halogenated C₁₋₆ alkoxy is preferred, and halogenated C₁₋₃ alkoxy is more preferred. Examples include, but are not limited to, trifluoromethoxy, trifluoroethoxy, monofluoromethoxy, monofluoroethoxy, difluoromethoxy, difluoroethoxy, and the like. For example, "halogenated C₁₋₁₀ alkylthio" means that the alkylthio is substituted with one or more (e.g., 1, 2, 3, 4, or 5) halogen atoms, wherein alkylthio is defined as above. Halogenated C₁₋₆ alkylthio is preferred, and halogenated C₁₋₃ alkylthio is more preferred. Examples include, but are not limited to, trifluoromethylthio, trifluoroethylthio, monofluoromethylthio, monofluoroethylthio, difluoromethylthio, difluoroethylthio, and the like. For example, "halogenated C₃₋₈ monocycloalkyl" means that the cycloalkyl is substituted with one or more (e.g., 1, 2, 3, 4, or 5) halogen atoms, wherein cycloalkyl is defined as above. Halogenated C₃₋₆ cycloalkyl is preferred. Examples include, but are not limited to, trifluorocyclopropyl, monofluorocyclopropyl, monofluorocyclohexyl, difluorocyclopropyl, difluorocyclohexyl, and the like.

As used herein, "amino" refers to NH₂, "cyano" refers to CN, "nitro" refers to NO₂, "benzyl" refers to -CH₂-phenyl, "oxo" refers to =O, "carboxyl" refers to -C(O)OH, -COOH or -CO₂H, "acetyl" refers to -C(O)CH₃, "hydroxymethyl" refers to -CH₂OH, "hydroxyethyl" refers to -CH₂CH₂OH or -CH(OH)CH₃, "cyanomethyl" refers to -CH₂CN, "cyanoethyl" refers to -CH₂CH₂CN, "hydroxyl" refers to -OH, "thiol" refers to SH, the structure of "cyclopropylidene" is: 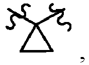 "acetylamino" refers to -NH-C(O)CH₃, "pyrrolidone" refers to

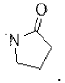

As used herein, "substituted" means that one or more hydrogen atoms in a group, preferably 1 to 5 hydrogen atoms, are each independently substituted by the corresponding number of substituents, more preferably, 1 to 3 hydrogen atoms are each independently substituted by the corresponding number of substituents. It is obvious that substituents are only in their possible chemical positions, and those skilled in the art can, without any undue effort, determine (by experiment or theory) that it is possible or impossible. For example, an amino or hydroxyl group with free hydrogen may be unstable when combined with a carbon atom having an unsaturated (such as olefinic) bond.

The various substituent groups described herein above can themselves be substituted with the groups described herein.

The "pharmaceutically acceptable salt" includes pharmaceutically acceptable acid addition salts and pharmaceutically acceptable base addition salts. "Pharmaceutically acceptable acid addition salt" means a salt formed with an inorganic or organic acid, with retaining the bioavailability of the free base without any other side effects. "Pharmaceutically acceptable base addition salts", includes but not limited to salts of inorganic bases such as sodium, potassium, calcium and magnesium salts, and the like, and salts of organic bases such as ammonium salts, triethylamine salts, lysine salts, arginine salts and the like.

As mentioned herein, "solvate" refers to a complex of the compound of the present disclosure with a solvent. They either react in a solvent or precipitate or crystallize from the solvent. For example, a complex formed with water is referred to as a "hydrate". Solvates of the compound of formula (I) are within the scope of the present disclosure. The compounds of formula (I) of the present disclosure may contain one or more chiral centers and exist in different optically active forms. When the compound contains one chiral center, the compound contains enantiomers. The present disclosure includes the two isomers and mixtures of the two isomers, such as racemic mixtures. Enantiomers can be separated by methods known in the art, such as crystallization, chiral chromatography and the like. When the compound of formula (I) contains more than one chiral center, diastereomers may be present. The present disclosure includes separated optically pure specific isomers as well as mixtures of diastereomers. Diastereomers can be separated by methods known in the art, such as crystallization and preparative chromatography.

The present disclosure includes prodrugs of the above compounds. Prodrugs include those in which known amino protecting groups or carboxy protecting groups can be hydrolyzed under physiological conditions or released via an enzymatic reaction to give the parent compound. Specific prodrug preparation methods can be referred to (Saulnier, M.G.; Frennesson, D.B.; Deshpande, M.S.; Hansel, S.B. and Vysa, D.M. Bioorg. Med. Chem Lett. 1994, 4, 1985-1990; and Greenwald, R.B.; Choe, YH.; Conover, C.D.; Shum, K.; Wu, D.; Royzen, MJ Med. Chem. 2000, 43, 475.).

In general, the compound of the present disclosure, or a pharmaceutically acceptable salt thereof, or a solvate thereof, or a stereoisomer, or prodrug thereof, can be administered in suitable dosage forms with one or more pharmaceutically acceptable carriers. These dosage forms are suitable for oral, rectal, topical, intraoral, and other parenteral administration (e.g., subcutaneous, intramuscular, intravenous, etc.). For example, dosage forms suitable for oral administration include capsules, tablets, granules, syrups, and the like. The compound of the present disclosure contained in these preparations may be a solid powder or granule, a solution or suspension in an aqueous or non-aqueous liquid, a water-in-oil or oil-in-water emulsion, or the like. The above dosage forms can be prepared from the active compound with one or more carriers or excipients via conventional pharmaceutical methods. The above carriers need to be compatible with the active compound or other excipients. For solid preparations, commonly used non-toxic carriers include, but not limited to, mannitol, lactose, starch, magnesium stearate, cellulose, glucose, sucrose, and the like. Carriers for liquid preparations include water, physiological saline, glucose aqueous solution, ethylene glycol, polyethylene glycol, and the like. The active compound can form a solution or suspension with the above carriers.

The composition of the present disclosure is formulated, quantified, and administered in a manner consistent with medical practices. The "therapeutically effective amount" of a given compound will be determined by factors such as the particular condition to be treated, the individual to be treated, the cause of the condition, the target of the drug, the mode of administration and the like.

As used herein, "therapeutically effective amount" refers to an amount of the compound of the present disclosure that will elicit the biological or medical response, for example, reduction or inhibition of an enzyme or a protein activity, or amelioration of a symptom, alleviation of a condition, slow or delay disease progression, or prevention of a disease, etc, in an individual.

The therapeutically effective amount of the compound of the present disclosure, or a pharmaceutically acceptable salt thereof, or a solvate thereof, or a stereoisomer thereof contained in the pharmaceutical composition of the present disclosure is preferably 0.1 mg-5 g/kg (body weight).

As used herein, "pharmaceutically acceptable carrier" refers to non-toxic, inert, solid or semi-solid substance or a liquid filler, a diluent, an encapsulating material or an auxiliary formulation or any type of excipient that is compatible with a patient which is preferably a mammal and more preferably a human. It is suitable for delivering active agent to a target without terminating the activity of the agent.

As used herein, "patient" refers to an animal, preferably a mammal, and more preferably a human being. The term "mammal" refers to a warm-blooded vertebrate mammal, including, for example, cat, dog, rabbit, bear, fox, wolf, monkey, deer, rat, pig and human.

As used herein, "treating/treatment" refers to alleviating, delaying, attenuating the progress of, preventing, or maintaining an existing disease or disorder (eg, cancer). The treating/treatment also includes curing one or more symptoms of the disease or disorder, preventing its development or reducing it to some extent.

### PREPARATION METHOD

The experimental methods which do not specify the specific conditions in the following examples are generally carried out according to conventional conditions such as those described in Sambrook et al., Molecular Cloning: Laboratory Manual (New York: Cold Spring Harbor Laboratory Press, 1989), or conditions as recommended by the manufacturers. Unless otherwise defined, the terms used herein have the same meaning as those familiar to those skilled in the art. In addition, any method and material similar or equivalent to the recorded content can be applied in the present disclosure.

The compound represented by formula (I) can be prepared by the method described in the examples and similar methods, or by combining the examples described in this specification or by combining with known methods. The preparation of the compound of the embodiment of the present disclosure is not limited to the preparation method described in the present disclosure.

The main advantages of the present disclosure compared with the prior art are that:
A series of novel substituted phenylpropenyl pyridine derivatives are provided, which, as immunomodulators, have high inhibitory activity for PD-1/PD-L1, and can be used as drugs for the treatment of tumors.

The present disclosure will be further illustrated below with reference to the specific examples. It should be understood that these examples are only to illustrate the disclosure but not to limit the disclosure of the disclosure. The experimental methods without specific conditions in the following embodiments are generally carried out according to conventional conditions, or in accordance with the conditions recommended by the manufacturer. Unless indicated otherwise, parts and percentage are calculated by weight. Unless otherwise defined, terms used herein are of the same meanings that are familiar to those skilled in the art. In addition, any methods and materials similar or equivalent to those described herein can be applied to the present disclosure.

As used herein, DMB refers to 2,4-dimethoxybenzyl, THF refers to tetrahydrofuran, EA refers to ethyl acetate, PE refers to petroleum ether, Ac₂O refers to acetic anhydride, NBS refers to N-bromosuccinimide, DCM refers to dichloromethane, DME refers to dimethyl ether, AIBN refers to azobisisobutyronitrile, Pd(dppf)Cl₂ refers to [1,1'-bis(diphenylphosphino)ferrocene]dichloropalladium(II), Pd(DPPF)Cl₂·CH₂Cl₂ refers to [1,1'-bis(diphenylphosphino)ferrocene]dichloropalladium(II) dichloromethane complex, TFA refers to trifluoroacetic acid, TBSCl refers to tert butyl dimethylchlorosilane, NCS is N-chlorosuccinimide, DHP refers to dihydropyran, LiAlH₄ refers to lithium aluminum hydride, PMB refers to p-methoxybenzyl, LiHMDS refers to lithium bis(trimethylsilyl)amide, Pd₂(dba)₃ refers to tris(dibenzylideneacetone)dipalladium, RuPhos refers to 2-dicyclohexylphosphorus-2',6'-diisopropoxy-1,1'-biphenyl, DMAP refers to 4-dimethylaminopyridine, THP refers to tetrahydropyran, n-BuLi refers to n-Butyllithium, TMsOTf refers to trimethylsilyl trifluoromethanesulfonate, TEBAC refers to benzyltriethylammonium chloride, HATU refers to 2-(7-azabenzotriazol-1-yl)-N,N,N',N'-tetramethyluronium hexafluorophosphate, DMF refers to dimethylformamide, DMAC is N,N-dimethylacetamide, DMSO refers to dimethyl sulfoxide, DIEA is N,N-diisopropyl ethylamine, TEA refers to triethylamine, BINAP refers to (2R,3S)-2,2'-bis(diphenylphosphino)-1,1'-binaphthyl, DDQ refers to 2,3-dichloro-5,6-dicyanobenzoquinone, CDI is N,N'-carbonyldiimidazole, DMP refers to dimethyl phthalate, ACN refers to acetonitrile, NMP is N-methylpyrrolidone, Sphos-Pd-G2 catalyst (CAS number: 1375325-64-6). As used herein, room temperature refers to about 20-25°C.

### Preparation of intermediate 3b

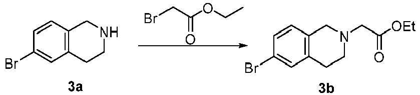

6-bromo-1,2,3,4-tetrahydroisoquinoline 3a (2.11g, 10.0 mmol) was dissolved in 40 ml THF, potassium carbonate (2.70g, 20.0 mmol), ethyl bromoacetate (1.90g, 10.0 mmol) were add. The reaction solution was stirred and reacted at 140°C for 16 hours, concentrated under reduced pressure, and the residue was purified by column chromatography (eluent: petroleum ether/ethyl acetate = 31%) to obtain the compound ethyl 2-(6-bromo-3,4- dihydroisoquinolin-2(1H)-yl)acetate **3b** (400 mg, pale yellow oil) with a yield of 26%.MS m/z (ESI):298.1 [M+1].

Intermediate **63a** 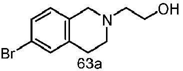 can be obtained by referring to the preparation method of intermediate 3b, except that ethyl bromoacetate is replaced by 2-bromoethanol.

Intermediate **87b** 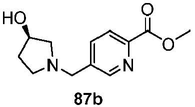 can be obtained by referring to the preparation method of intermediate 59b, except that methyl 2-bromo-4-bromomethyl benzoate is replaced by methyl 5-bromomethylpyridinecarboxylate.

### Preparation of intermediate 22a

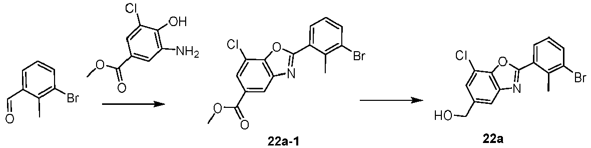

Step 1: Compound 3-bromo-2-methylbenzaldehyde (0.98 g, 4.92 mmol) was dissolved in 30 mL of ethanol, and methyl 3-amino-5-chloro-4-hydroxybenzoate (1.04 g, 5.16 mmol) was added. After the reaction was reacted for 1 hour under the protection of argon at room temperature, the reaction solution was concentrated under reduced pressure, and the residue was dissolved in 30 mL of anhydrous DCM, and dichlorodicyanobenzoquinone (1.12 g, 4.92 mmol) was added and then stirred at room temperature for 1 hour. 10 mL of saturated sodium bicarbonate solution was added to quench the reaction. 60 mL DCM was added to the reaction solution, washed with saturated sodium bicarbonate solution (10 mL×2), the organic phase was dried over anhydrous sodium sulfate, filtered, the filtrate was concentrated under reduced pressure, and the residue was purified by silica gel column chromatography (eluent: petroleum ether : ethyl acetate = 80: 20) to get the compound methyl 2-(3-bromo-2-methylphenyl)-7-chlorobenzo[d]oxazol-5-carboxylate (1.4 g, yellow solid) with a yield of 75%. MS m/z (ESI):379.9, [M+1].

Step 2: Methyl 2-(3-bromo-2-methylphenyl)-7-chlorobenzo[d]oxazol-5-carboxylate (395 mg, 1.04 mmol) was dissolved in 10 mL of anhydrous DCM. Diisobutyl aluminum hydride (1 mol/L toluene solution, 2.08 mmol, 2.08 mL) was added dropwise under the protection of nitrogen at -78 °C. After stirring for half an hour at -78 °C, the reaction continued at 0 °C for 1 hour. The reaction was quenched by adding 0.5 mL of water, and then 0.5 mL of 2mol/L sodium hydroxide aqueous solution was added and stirred for 5 minutes, 0.5 mL of water was added again, and the mixture was stirred for 5 minutes. Then sodium sulfate was added for solid drying and stirred continuouslyfor 10 minutes. The reaction solution was filtered through celite, the filtrate was concentrated under reduced pressure, and the residue was purified by silica gel column chromatography (eluent: petroleum ether: ethyl acetate = 50: 50) to obtain the compound (2-(3-bromo-2-methylphenyl)-7-chlorobenzo[d]oxazol-5-yl) methanol 22a (238 mg, white solid) with a yield of 65%. MS m/z (ESI):351.9, [M+1].

Intermediate **16a** 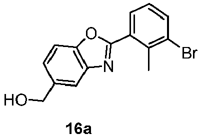 can be obtained by referring to the preparation method of intermediate 22a, except that methyl 3-amino-5-chloro-4-hydroxybenzoate is replaced by methyl 3-amino-4-hydroxybenzoate.

### Preparation of intermediate 18a

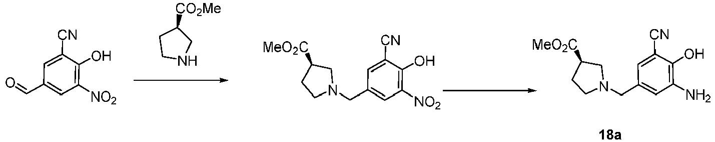

Step 1: 5-formyl-2-hydroxy-3-nitrobenzonitrile(1.5g, 7.8 mmol) was dissolved in 10 ml DMF, methyl (R)- pyrrolidine-3-carboxylate hydrochloride (1.2g, 9.4 mmol) was added, the mixture was heated to 60°C and reacted for 1 hour, and then sodium cyanoborohydride (1.2g, 18.8 mmol) was added. After the addition, the reaction continued at 60°C for 1 hour. The solid was removed by filtration and the solvent was removed under reduced pressure. The residue was purified by silica gel column chromatography (DCM: methanol = 90: 10) to obtain methyl (R)-1-(3-cyano-4-hydroxy-5-nitrobenzyl)pyrrolidine-3-carboxylate (1.5g, yellow solid) with a yield of 65%. MS m/z (ESI): 306.1 [M+1].

Step 2: Methyl (R)-1-(3-cyano-4-hydroxy-5-nitrobenzyl)pyrrolidine-3-carboxylate(1.4 g, 4.6 mmol) was dissolved in 15 ml of 1,4-dioxane and 3ml of water, palladium-carbon (140mg) was added, and the mixture was reacted overnight under protection of hydrogen at room temperature, filtered to remove palladium-carbon. The solvent was removed under reduced pressure to obtain methyl (R)-1-(3-amino-5-cyano-4-hydroxybenzyl) pyrrolidine-3-carboxylate 18a (750 mg, brown solid) with a yield of 62%. MS m/z (ESI): 276.0[M+1].

Intermediate **23a** can be obtained by referring to the preparation method of intermediate 18a, except that 5-formyl-2-hydroxy-3-nitrobenzonitrile is replaced by 4-hydroxy-3-nitro-5-trifluoromethyl benzaldehyde. Intermediate **30a** can be obtained by referring to the preparation method of intermediate 18a, except that methyl (R)-pyrrolidine-3-carboxylate hydrochloride is replaced by methyl (S)- pyrrolidine-3-carboxylate hydrochloride. Intermediate **31a** can be obtained by referring to the preparation method of intermediate 18a, except that compound 5-formyl-2-hydroxy-3-nitrobenzonitrile is replaced by compound 4-hydroxy-3-nitrobenzaldehyde. Intermediate **32a** can be obtained by referring to the preparation method of intermediate 18a, except that 5-formyl-2-hydroxy-3-nitrobenzonitrile is replaced by 4-hydroxy-3-nitrobenzaldehyde, and methyl (R)- pyrrolidine-3-carboxylate hydrochloride is replaced by methyl (S)- pyrrolidine-3-carboxylate hydrochloride. Intermediate **39a** can be obtained by referring to the preparation method of intermediate 18a, except that methyl (R)- pyrrolidine-3-carboxylate hydrochloride in step 1 is replaced by methyl L-prolinate hydrochloride. Intermediate **44a** can be obtained by referring to the preparation method of intermediate 18a, except that methyl (R)-pyrrolidine-3-carboxylate hydrochloride is replaced by ethyl (R)- pyrrolidine-3-carboxylate hydrochloride. Intermediate **48a** can be obtained by referring to the preparation method of intermediate 18a, except that compound 5-formyl-2-hydroxy-3-nitrobenzonitrile is replaced by compound 3-chloro-4-hydroxy-5-nitrobenzaldehyde.

| Intermediate structure | Intermediate structure | Intermediate structure | Intermediate structure |
|---|---|---|---|
| 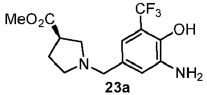 | 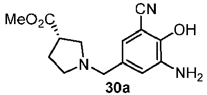 | 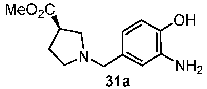 | 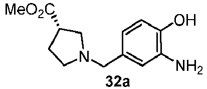 |
| 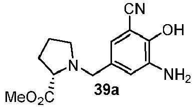 | 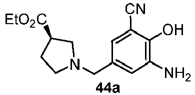 | 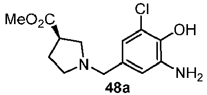 | |

### Preparation of intermediate 18b

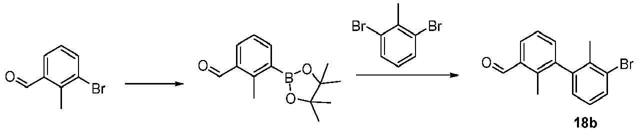

Step 1: 3-bromo-2-methylbenzaldehyde (4.0g, 20.2 mmol) was dissolved in 20ml 1,4-dioxane, bis(pinacolato)diboron (7.7g, 30.3mmol), and [1,1'-bis(diphenylphosphino)ferrocene]dichloropalladium(II) (1.64g, 2.02 mmol) and potassium acetate (3.9g, 40.4mmol) were added in sequence. The reaction solution was heated to 100°C under the protection of argon and reacted for 3 hours, and filtered to remove the solid, the solvent was removed under reduced pressure, and the residue was purified by silica gel column chromatography (petroleum ether: ethyl acetate = 5: 1) to obtain 2-methyl-3-(4,4,5,5-tetramethyl-1,3,2-dioxaborolan-2-yl)benzaldehyde (4.5mg, yellow solid) with a yield of 90%. MS m/z (ESI): 247.1[M+1].

Step 2: 2-methyl-3-(4,4,5,5-tetramethyl-1,3,2-dioxaborolan-2-yl)benzaldehyde (3.5g, 14.3 mmol) was dissolved in 20ml 1,4-dioxane, and then 2,6-dibromotoluene(4.2g, 17.1mmol), [1,1-bis(diphenylphosphino)ferrocene]dichloropalladium(II) (1.17g, 1.43mmol), potassium carbonate (3.9g, 28.6mmol) and 6ml water were added in sequence. The reaction solution was heated to 100°C under the protection of argon and reacted for 3 hours, and filtered to remove the solid, the solvent was removed under reduced pressure, and the residue was purified by silica gel column chromatography (eluent: petroleum ether: ethyl acetate = 3: 1) to obtain 3'-bromo-2,2'-dimethyl-[1,1-biphenyl]-3-carbaldehyde **18b** (2.2g, yellow solid) with a yield of 53.5%. MS m/z (ESI): 288.9[M+1].

Intermediate **25a** can be obtained by referring to the preparation method of intermediate 18b, except that 2,6-dibromotoluene is replaced by 1,3-dibromo-2-fluorobenzene. Intermediate **28a** can be obtained by referring to the preparation method of intermediate 18b, except that 3-bromo-2-methylbenzaldehyde is replaced by 3-bromo-2-fluorobenzaldehyde. Intermediate **38a** can be obtained by referring to the preparation method of intermediate 18b, except that 2,6-dibromotoluene is replaced by 1,3-dibromo-2-chlorobenzene. Intermediate **42a** can be obtained by referring to the preparation method of intermediate 18b, except that 3-bromo-2-methylbenzaldehyde is replaced by compound 3-bromo-2-chlorobenzaldehyde. Intermediate **48b** can be obtained by referring to the preparation method of intermediate 18b, except that 2,6-dibromotoluene is replaced by compound 2,4-dibromonicotinonitrile.

| Intermediate structure | Intermediate structure | Intermediate structure |
|---|---|---|
| 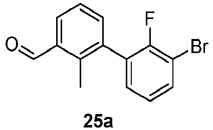 | 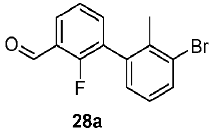 | 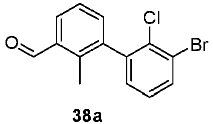 |
| 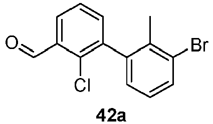 | 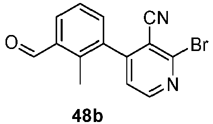 | |

Intermediate **59a** can be obtained by referring to the preparation method of intermediate Id, except that 2,4-dibromonicotinonitrile is replaced by 1-bromo-2-methyl-3-iodobenzene. Intermediate **65a** can be obtained by referring to the preparation method of intermediate Id, except that 2,4-dibromonicotinonitrile is replaced by 2-bromo-4-iodo-3-methylpyridine. Intermediate **76a** can be obtained by referring to the preparation method of intermediate Id, except that 3-bromo-2-methylaniline is replaced by 3-bromo-2-chloroaniline, and 2,4-dibromonicotinonitrile is replaced by 2-bromo-4-iodo-3-methylpyridine. Intermediate **77a** can be obtained by referring to the preparation method of intermediate Id, except that 3-bromo-2-methylaniline is replaced by 3-bromo-2-(trifluoromethyl)aniline. Intermediate **81a** can be obtained by referring to the preparation method of intermediate Id, except that 3-bromo-2-methylaniline is replaced by 3-bromo-2-chloroaniline Intermediate **83a** can be obtained by referring to the preparation method of intermediate Id, except that 2,4-dibromonicotinonitrile is replaced by 2-bromo-4-iodo-3-chloropyridine. Intermediate **85a** can be obtained by referring to the preparation method of intermediate Id, except that 2,4-dibromonicotinonitrile is replaced by 1-bromo-2-chloro-3-iodobenzene. Intermediate **87a** can be obtained by referring to the preparation method of intermediate Id, except that 3-bromo-2-methylaniline is replaced by 3-bromo-2-chloroaniline, and 2,4-dibromonicotinonitrile is replaced by 1-bromo-2-methyl-3-iodobenzene. Intermediate **88a** can be obtained by referring to the preparation method of intermediate Id, except that 2,4-dibromonicotinonitrile is replaced by 1-bromo-2-trifluoromethyl-3-iodobenzene. Intermediate **93a** can be obtained by referring to the preparation method of intermediate Id, except that 3-bromo-2-methylaniline is replaced by 3-bromo-2-chloroaniline, and 2,4-dibromonicotinonitrile is replaced by 1-bromo-2-chloro-3-iodobenzene. Intermediate **94a** can be obtained by referring to the preparation method of intermediate Id, except that 2,4-dibromonicotinonitrile is replaced by 2-bromo-6-iodobenzonitrile. Intermediate **95a** can be obtained by referring to the preparation method of intermediate Id, except that 3-bromo-2-methylaniline is replaced by 3-bromo-2-chloroaniline, and 2,4-dibromonicotinonitrile is replaced by 2-bromo-6-iodobenzonitrile. Intermediate **96a** can be obtained by referring to the preparation method of intermediate Id, except that 3-bromo-2-methylaniline is replaced by 3-bromo-2-fluoroaniline, and 2,4-dibromonicotinonitrile is replaced by 1-bromo-3-iodo-2-chlorobenzene. Intermediate **98a** can be obtained by referring to the preparation method of intermediate Id, except that 3-bromo-2-methylaniline is replaced by 3-bromo-2-trifluoromethylaniline, and 2,4-dibromonicotinonitrile is replaced by 1-bromo-3-iodo-2-benzonitrile. Intermediate **99a** can be obtained by referring to the preparation method of intermediate Id, except that 3-bromo-2-methylaniline is replaced by 3-bromo-2-chloroaniline, and 2,4-dibromonicotinonitrile is replaced by 1-bromo-3-iodo-2-fluorobenzene. Intermediate **100a** can be obtained by referring to the preparation method of intermediate Id, except that 3-bromo-2-methylaniline is replaced by 3-bromo-2-fluoroaniline, and 2,4-dibromonicotinonitrile is replaced by 1-bromo-3-iodo-2-fluorobenzene. Intermediate **104a** can be obtained by referring to the preparation method of intermediate Id, except that 3-bromo-2-methylaniline is replaced by 3-bromo-2-cyanoaniline, and 2,4-dibromonicotinonitrile is replaced by 1-bromo-3-iodo-2-chlorobenzene. Intermediate **105a** can be obtained by referring to the preparation method of intermediate Id, except that 3-bromo-2-methylaniline is replaced by 3-bromo-2-cyanoaniline, and 2,4-dibromonicotinonitrile is replaced by 1-bromo-3-iodo-2-toluene. Intermediate **111a** can be obtained by referring to the preparation method of intermediate Id, except that 3-bromo-N,2-dimethylaniline is replaced by 3-bromo-2-cyanoaniline, and 2,4-dibromonicotinonitrile is replaced by 1-bromo-3-iodo-2-toluene.

| Intermediate structure | Intermediate structure | Intermediate structure | Intermediate structure |
|---|---|---|---|
| 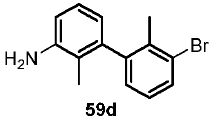 | 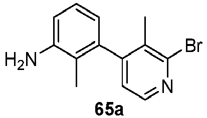 | 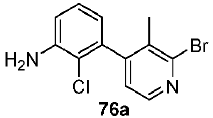 | 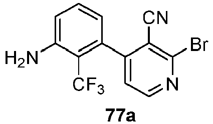 |
| 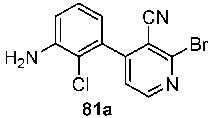 | 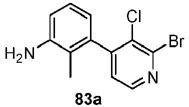 | 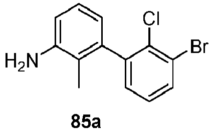 | 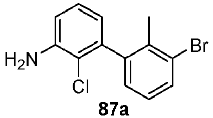 |
| 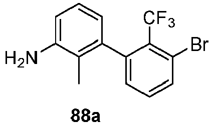 | 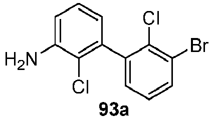 | 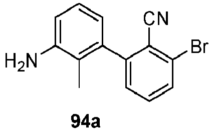 | 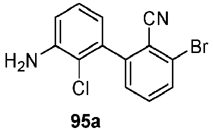 |
| 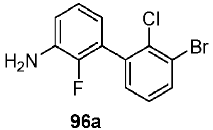 | 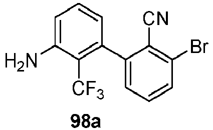 | 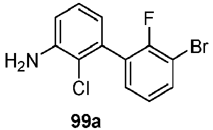 | 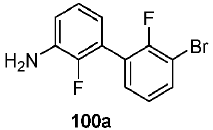 |
| 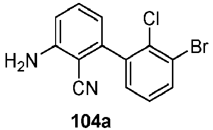 | 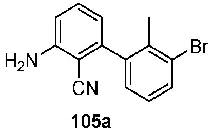 | 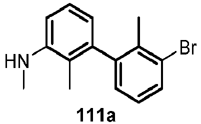 | |

### Preparation of intermediate 10j

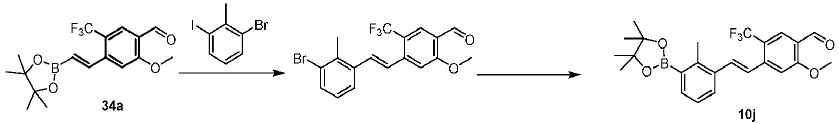

Step 1: Compound **34a** (10.0 mmol) was dissolved in 45 mL 1,4-dioxane and 15 mL water, and 1-bromo-3-iodo-2-toluene (10.0 mmol), [1,1'-bis(diphenylphosphino)ferrocene|dichloropalladium(II) dichloromethane complex (733 mg, 1.0 mmol) and sodium carbonate (1.0 g, 10.0 mmol) were added. The reaction solution was stirred and reacted for 3 hours at 100°C under the protection of argon, and filtered, and the filtrate was concentrated under reduced pressure. The residue was purified by silica gel column chromatography with 10%~75% ethyl acetate in petroleum ether as an eluent to obtain (E)-4-(3-bromo-2-methylstyryl)-2-methoxy-5-(trifluoromethyl)benzaldehyde.

Step 2: (E)-4-(3-bromo-2-methylstyryl)-2-methoxy-5-(trifluoromethyl)benzaldehyde (2.53 mmol) was dissolved in 20 mL 1,4-dioxane, and bis(pinacolato)diboron( 962 mg, 3.79 mmol), potassium acetate(743 mg, 7.58 mmol), [1,1'-bis(diphenylphosphino)ferrocene]dichloropalladium(II) dichloromethane complex(207 mg, 0.25 mmol) were added in sequence. The reaction solution was replaced with nitrogen three times and heated to 100 °C, stirred and reacted for 20 hours, and then cooled to room temperature. The reaction solution was filtered through celite, the filtrate was concentrated under reduced pressure, and the residue was purified by silica gel column chromatography with 10%~75% ethyl acetate in petroleum ether as an eluent to obtain the compound **10j.**

Intermediate 17a can be obtained by referring to the preparation method of intermediate **10j**, except that compound **34a** is replaced by compound **18d.** Intermediate 19a can be obtained by referring to the preparation method of intermediate **10j,** except that 1-bromo-3-iodo-2-methylbenzene is replaced by 2-bromo-6-iodobenzonitrile. Intermediate 20a can be obtained by referring to the preparation method of intermediate **10j,** except that 1-bromo-3-iodo-2-methylbenzene is replaced by 1-bromo-2-fluoro-3-iodobenzene. Intermediate **46a** can be obtained by referring to the preparation method of intermediate **10j,** except that 1-bromo-3-iodo-2-methylbenzene is replaced by 2,4-dibromonicotinonitrile, and compound 34a is replaced by compound 46b. Intermediate **49a** can be obtained by referring to the preparation method of intermediate **10j,** except that 1-bromo-3-iodo-2-methylbenzene is replaced by 2, 4-dibromo-3-pyridinecarbonitrile. Intermediate **51a** can be obtained by referring to the preparation method of intermediate **10j,** except that 1-bromo-3-iodo-2-methylbenzene is replaced by 2,4-dibromonicotinonitrile, and compound 34a is replaced by compound 18d. Intermediate **52a** can be obtained by referring to the preparation method of intermediate **10j,** except that 1-bromo-3-iodo-2-methylbenzene is replaced by 2-bromo-3-chloro-4-iodopyridine.

| Intermediate structure | Intermediate structure | Intermediate structure |
|---|---|---|
| 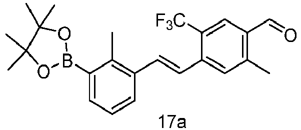 | 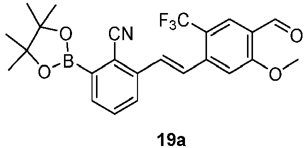 | 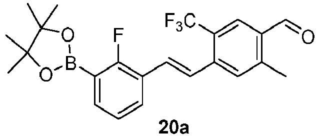 |
| 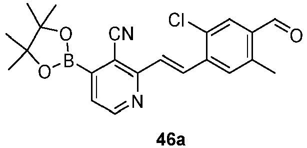 | 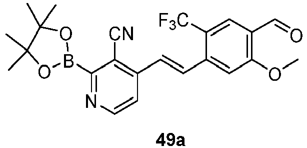 | 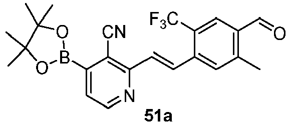 |
| 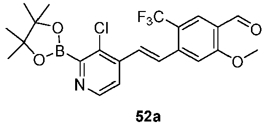 | | |

### Preparation of intermediate 55a

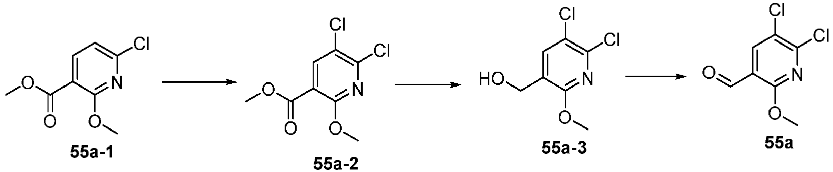

Step 1: Compound **55a-1** (800mg, 2.98mmol) was dissolved in 8ml of concentrated hydrochloric acid, and sodium hypochlorite (8ml) was added at 0°C. The reaction solution was heated to 100°C, stirred and reacted for 3 hours and then cooled to room temperature. The reaction solution was quenched with saturated sodium bicarbonate, extracted with ethyl acetate (30^{∗}3ml), dried over anhydrous sodium sulfate, and concentrated under reduced pressure to obtain compound **55a-2** (700mg, white solid) with a yield of 74.5% The compound **55a-2** was used directly in the next reaction. MS m/z (ESI): 236.0[M+1].

Step 2: Compound **55a-2** (700mg, 4.0mmol) was dissolved in 20ml of anhydrous tetrahydrofuran, and lithium aluminum hydride (339.0mg, 8.94mmol) was added at 0°C. The reaction solution was stirred and reacted at 0°C for 1 hour. The reaction solution was quenched with saturated sodium bicarbonate, extracted with ethyl acetate (30^{∗}3ml), dried over anhydrous sodium sulfate, concentrated under reduced pressure. The residue was purified by silica gel column chromatography (eluent: petroleum ether: ethyl acetate = 90: 10) to obtain compound **55a-3** (350 mg, white solid) with a yield of 56.8%. MS m/z (ESI): 208.0[M+1].

Step 3: Compound **55a-3** (350 mg, 1.69 mmol) was dissolved in 20 ml of DCM, and Dess-Martin oxidant (1.4g, 3.38 mmol) was added at room temperature. The reaction solution was reacted for 2 hours, filtered to remove the solid. The filtrate was concentrated, and the residue was purified by silica gel column chromatography (petroleum ether: ethyl acetate = 60: 40) to obtain compound **55a** (210 mg, yellow solid) with a yield of 66.0%. MS m/z (ESI): 206.0[M+1].

### Preparation of intermediate 119c

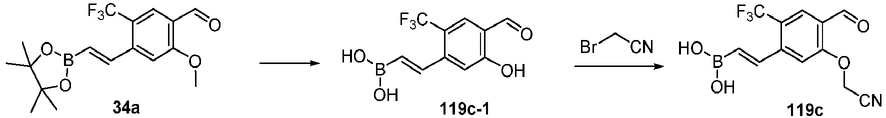

Step 1: Compound **34a** (500 mg, 1.4 mmol) and boron tribromide (1.0M, 7ml) were dissolved in 5ml anhydrous DCM, and the reaction solution was stirred at room temperature for 3.0 hours. The reaction solution was quenched with 3.0ml saturated sodium bicarbonate solution, extracted with ethyl acetate ( 30^{∗}3ml), dried over anhydrous sodium sulfate, and concentrated under reduced pressure. The residue was purified by silica gel column chromatography (eluent: DCM: methanol = 90: 10) to obtain compound **119c-1** (150 mg, yellow solid) with a yield of 41.1%. MS m/z (ESI): 261.0[M+1].

Step 2: Compound **119c-1** (150 mg, 0.58 mmol) and bromoacetonitrile (137 mg, 1.16 mmol) were dissolved in 5 ml anhydrous DMF, and potassium carbonate (160 mg, 1.16 mmol) was added at room temperature. The reaction solution was stirred at room temperature for 1.0 hour. The reaction solution was quenched with 3.0ml water, extracted with ethyl acetate (30^{∗}3ml), dried over anhydrous sodium sulfate, concentrated under reduced pressure. The residue was purified by silica gel column chromatography (eluent: DCM: methanol = 90: 10) to obtain compound **119c** (90 mg, yellow solid) with a yield of 51.9%. MS m/z (ESI): 300.1[M+1].

### Preparation of intermediate 137i

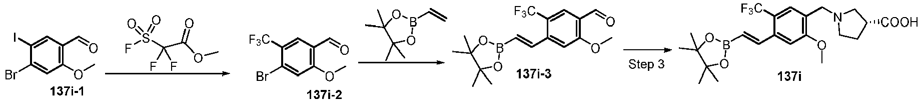

Step 1: Compound **137i-1** (20 mmol), methyl 2,2-difluoro-2-(fluorosulfonyl)acetate (19.2g, 100 mmol) and cuprous iodide (1.9g, 10 mmol) were added to 60 ml of NMP, the reaction solution was heated to 80 °C and reacted overnight under argon protection, the reactants were concentrated, and the residue was purified by silica gel column chromatography with 5%~35% ethyl acetate in petroleum ether as an eluent to obtain compound **137i-2,** which was directly used in the next step.

Step 2: Compound 137i-2 (0.035 mol) was dissolved in 20 ml of toluene, vinyl borate pinacol ester (10.8g, 0.07mol), bis(dibenzylideneacetone)palladium (1.0g, 0.0035 mol), tri-tert-butyl phosphine tetrafluoroborate (1.0g, 0.0035 mol) and DIEA (7.08g, 0.07mmol) were added in sequence, the reaction solution was heated to 95 °C in a sealed tube reactor under the protection of argon, and reacted for 3.0 hours, filtered to remove the solids, and the residue was purified by silica gel column chromatography with 0~35% ethyl acetate in petroleum ether as an eluent to obtain compound **137i-3**.

Step 3: Compound **137i-3** (0.5 mmol) and (R)-pyrrolidine-3-carboxylic acid (0.5 mol) were dissolved in 10 mL methanol, sodium cyanoborohydride (31 mg, 0.5 mmol) was added, and the reaction solution was stirred and reacted under reflux for 2 hours, concentrated under reduced pressure. The residue was purified by silica gel column chromatography with 5%-75% ethyl acetate in petroleum ether as an eluent to obtain the product **137i.**

### Preparation of intermediate 139a

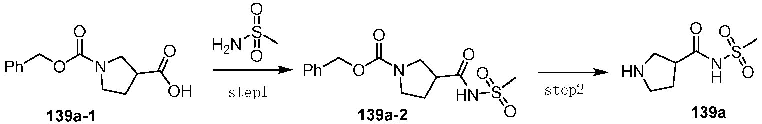

Step 1: Compound 139a-1 (500 mg, 2.0 mmol) was dissolved in 10 ml of DMF, CDI (973 mg, 6.0 mmol) was added at room temperature, and the reaction solution was stirred and reacted at 65 °C for 1 hour. After the system was cooled to 0 °C, a solution of NaH (240 mg, 10.0 mmol) and methanesulfonamide (1.14 g, 12.0 mmol) in 5 ml DMF was slowly added to the reaction solution, and then continued to stir at room temperature for 3 hours. The reaction solution was quenched with water, extracted with ethyl acetate. The organic phase was washed with saturated brine, dried over anhydrous sodium sulfate, filtered to remove solid, the filtrate was concentrated, and the residue was purify by silica gel column chromatography (eluent: DCM: methanol = 95: 5) to obtain compound 139a-2 (600 mg, yellow solid) with a yield of 92.0%. MS m/z (ESI): 327.1 [M+1].

Step 2: Compound 139a-2 (326 mg, 1.0 mmol) was dissolved in 10 ml methanol, and Pd/C (33 mg, 10% wt) was added at room temperature. The reaction solution was stirred at room temperature for 3 hours under a hydrogen atmosphere. The reaction solution was filtered, and after the filter cake was washed with methanol and water in sequence, the filtrate was concentrated to obtain compound 139a (189.1 mg, white oily liquid) with a yield of 98.0%. MS m/z (ESI): 193.1 [M+1].

### Preparation of intermediate 34a

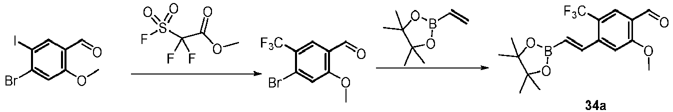

Step 1: 4-bromo-5-iodo-2-methoxybenzaldehyde (20 mmol), methyl 2,2-difluoro-2-(fluorosulfonyl)acetate (19.2g, 100 mmol) and cuprous iodide (1.9g, 10 mmol) were added to 60 mL of NMP, the reaction solution was heated to 80 °C and reacted under argon protection overnight, the reactants were concentrated, and the residue was purified by silica gel column chromatography with 5%~35% ethyl acetate in petroleum ether as an eluent to obtain 4-bromo-2-methoxy-5-trifluoromethylbenzaldehyde.

Step 2: 4-bromo-2-methoxy-5-(trifluoromethyl)benzaldehyde (10g, 0.035 mol) was dissolved in 20ml of toluene, vinyl borate pinacol ester (10.8g, 0.07mol), bis(dibenzylideneacetone)palladium(1.0g, 0.0035 mol), tri-tert-butyl phosphine tetrafluoroborate (1.0g, 0.0035 mol) and DIEA (7.08g, 0.07mmol) were added in sequence, the reaction solution was heated to 95 °C in a sealed tube reactor under the protection of argon, and reacted for 3.0 hours, filtered to remove the solid, and the residue was purified by silica gel column chromatography (eluent: petroleum ether: ethyl acetate = 95:5) to obtain compound 34a (8.0 g, yellow solid) with a yield of 63.2%. MS m/z (ESI): 357.1[M+1],

Intermediate 18d can be obtained by referring to the preparation method of intermediate **34a,** except that 4-bromo-5-iodo-2-methoxybenzaldehyde is replaced by 4-bromo-5-iodo-2-methylbenzaldehyde. Intermediate **46b** can be obtained by referring to the preparation method of intermediate **34a,** except that 4-bromo-2-methoxy-5-(trifluoromethyl)benzaldehyde is replaced by 4-bromo-5-chloro-2-methylbenzaldehyde. Intermediate **91a** can be obtained by referring to the preparation method of intermediate **34a,** except that 4-bromo-2-methoxy-5-(trifluoromethyl)benzaldehyde is replaced by 4-bromo-5-cyclopropyl-2-methylbenzaldehyde. Intermediate **124a** can be obtained by referring to the preparation method of intermediate **34a,** except that 4-bromo-5-iodo-2-methoxybenzaldehyde is replaced by 3-bromo-4-iodobenzaldehyde.

| Intermediate structure | Intermediate structure | Intermediate structure | Intermediate structure |
|---|---|---|---|
| 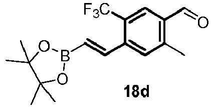 | 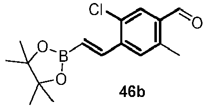 | 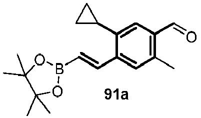 | 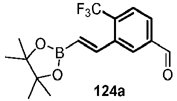 |

### Preparation of intermediate 109f

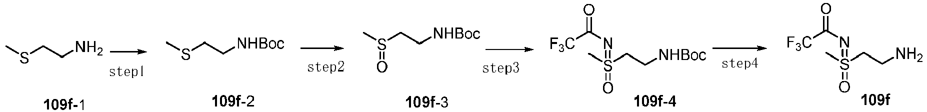

Step 1: Compound **109f -1** (2.5 g, 19.7 mmol) was dissolved in 30 mL DCM, and TEA (5.0 g, 49.3 mmol) and (Boc)₂O (4.7 g, 21.7 mmol) were added in sequence, the reaction solution was stirred and reacted at room temperature overnight, and 0.5M HCl was added to the reaction solution, extracted with DCM three times. The organic phases were combined, and concentrated under reduced pressure to obtain compound **109f-2** (3.8g, yellow oil) with a yield of 100%. MS m/z (ESI): 192.0 [M+1].

Step 2: Compound **109f -2** (2.0 g, 10.5 mmol) was dissolved in 50 mL DCM and 50 mL H₂O, and NaIO₄ (2.24 g, 10.5 mmol) was added, the reaction solution was stirred overnight at room temperature, filtered, and the filtrate was extracted three times with DCM. The organic phases were combined and concentrated under reduced pressure to obtain compound **109f -3** (3.8g, yellow oil) with a yield of 100%. MS m/z (ESI): 208.1 [M+1].

Step 3: Compound **109f -3** (1.0 g, 4.83 mmol) was dissolved in 80 mL DCM, MgO (773 mg, 19.3 mmol), F₃CCONH₂ (1.09 g, 9.66 mmol), Rh₂(OAc)₄ (854 mg, 1.93 mmol) ) and PhI(OAc)₂ (2.33 g, 7.25 mmol) were added in sequence, the reaction solution was stirred and reacted at room temperature overnight, filtered and concentrated under reduced pressure, and the residue was purified by silica gel column chromatography (eluent: petroleum ether: ethyl acetate = 2: 1) to obtain compound **109f -4** (750mg, yellow solid) with a yield of 50%. MS m/z (ESI): 319.1 [M+1].

Step 4: Compound **109f-4** (50 mg, 0.0761 mmol) was dissolved in 5 mL DCM, 0.5 mL TFA was added, the reaction solution was stirred and reacted at room temperature for 2 hours, and concentrated under reduced pressure to obtain compound **109f** (80 mg, yellow oil). MS m/z (ESI): 219.0 [M+1].

### Preparation of intermediate 79c

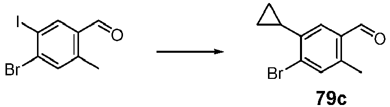

Step 1: 4-bromo-5-iodo-2-methylbenzaldehyde (0.65g, 2.0 mmol) and cyclopropylboronic acid (0.12g, 2.0 mmol) were dissolved in 20 ml dioxane and 6 ml water, and then potassium carbonate (0.28g, 2.0 mmol) and Pd(dppf)Cl₂ (150g, 0.2 mmol) were added in sequence, the reaction solution was stirred and reacted under the protection of nitrogen at 100°C for 4 hours, concentrated under reduced pressure to obtain compound 4-bromo-5- cyclopropyl-2-methylbenzaldehyde 79c (50 mg, pale yellow oil) with a yield of 16%.

### Preparation of intermediate 141a

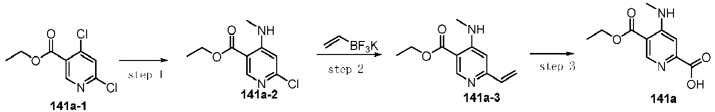

Step 1: Compound **141a-1** (5.0 g, 22.7 mmol) was dissolved in 75 mL ACN, the reaction system was cooled to 0°C, methylamine in ethanol (12.5 mL, 90.8 mmol) was slowly added dropwise to the reaction system, and the reaction continued at 0°C for half an hour after the addition. The reaction system was warmed to room temperature and stirred overnight at room temperature. The reaction solution was concentrated under reduced pressure, and the residue was purified by silica gel column chromatography (eluent: petroleum ether: ethyl acetate = 5:1) to obtain compound **141a-2** (4.7 g, white solid) with a yield of 97%. MS m/z (ESI): 215.0 [M+1].

Step 2: Compound **141a-2** (2.0 g, 9.35 mmol) and potassium vinylfluoroborate (1.5 g, 11.2 mmol) were dissolved in 30 mL 1,4-dioxane and 6 mL water, and tetratriphenylphosphine palladium (1.1 g, 0.935 mmol) and cesium carbonate (4.6 g, 14.0 mmol) were added in sequence. The reaction solution was heated to 100°C under the protection of argon, and reacted for 8 hours. The reaction solution was concentrated under reduced pressure, and the residue was purified by silica gel column chromatography (eluent: petroleum ether: ethyl acetate = 5:1) to obtain compound **141a-3** (1.3 g, yellow oil) with a yield of 67%. MS m/z (ESI): 207.1 [M+1].

Step 3: Compound **141a-3** (1.00 g, 4.85 mmol) was dissolved in 20 mL acetone and 20 mL water, potassium permanganate (2.30 g, 14.6 mmol) was added, the reaction solution was stirred and reacted at room temperature for 3 hours, filtered, and the filtrate was concentrated under reduced pressure to obtain compound **141a** (1.1 g, yellow solid). MS m/z (ESI): 225.1 [M+1].

### Example 1: Preparation of (E)-N-(3-(2-(2-chloro-4-(((2-hydroxyethyl)amino)methyl)-5-methylstyryl) -3-cyanopyridin-4-yl)-2-methylphenyl)-5-(((2-hydroxyethyl)amino)methyl)picolinamide(Z-1)

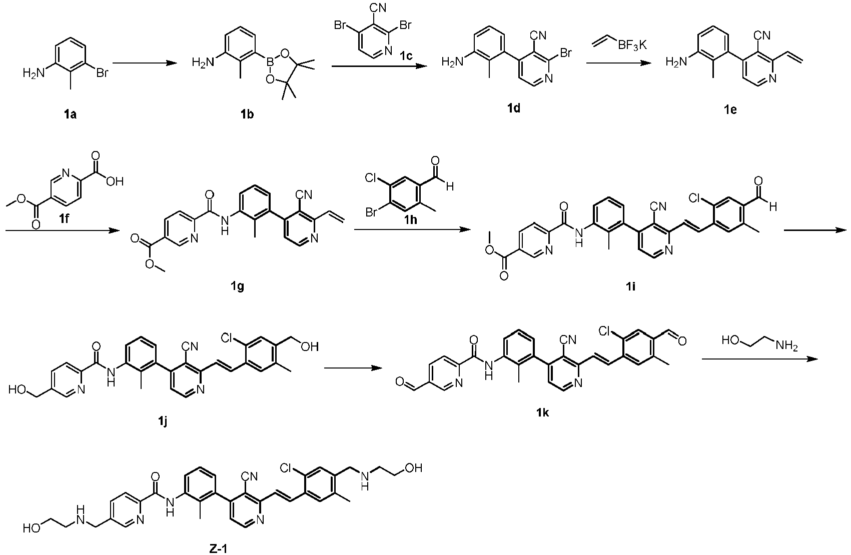

Step 1: Compound 1a (2.53 mmol) was dissolved in 20 mL 1,4-dioxane, bis(pinacolato)diboron(962 mg, 3.79 mmol), potassium acetate(743 mg, 7.58 mmol) and [1,1'-bis(diphenylphosphino)ferrocene]dichloropalladium(II) dichloromethane complex (207 mg, 0.25 mmol) were added in sequence, the reaction solution was replaced with nitrogen three times, heated to 100°C, stirred and reacted for 20 hours and then cooled to room temperature, the reaction solution was filtered through celite, the filtrate was concentrated under reduced pressure, and the residue was purified by silica gel column chromatography (eluent: petroleum ether: ethyl acetate = 80: 20) to obtain compound 1b (520 mg, white solid).

Step 2: 2,4-dibromonicotinonitrile **1c** (2.60g, 10.0 mmol) was dissolved in 45 mL 1,4-dioxane and 15 mL water, compound **1b** (10.0 mmol), [1,1-bis(diphenylphosphino)ferrocene]dichloropalladium(II) dichloromethane complex (733 mg, 1.0 mmol) and sodium carbonate (1.0 g, 10.0 mmol) were added, and the reaction solution was stirred and reacted for 3 hours at 100°C under the protection of argon. The reaction solution was filtered, and the filtrate was concentrated under reduced pressure. The residue was purified by silica gel column chromatography with 10%~75% ethyl acetate in petroleum ether as an eluent to obtain the product 2-bromo-4,4'-bispyridin-3-nitrile **1d** with a yield of 70.3%.

Step 3: Compound **1d** (5.0 mmol) was dissolved in 30 mL 1,4-dioxane and 10 mL water, potassium vinyltrifluoroborate (670 mg, 5.0 mmol), [1,1-bis(diphenylphosphino)ferrocene]dichloropalladium(II) dichloromethane complex (370 mg, 0.5 mmol) and sodium carbonate (530 mg, 5.0 mmol) were added, and the reaction solution was stirred and reacted for 16 hours at 100°C under argon protection. The reaction solution was filtered, and the filtrate was concentrated under reduced pressure. The residue was purified by silica gel column chromatography with 0~25% ethyl acetate in petroleum ether as an eluent to obtain compound **1e**.

Step 4: Compound 5-(methoxycarbonyl)picolinic acid (0.77 g, 4.26 mmol) and 4-(3-amino-2-methylphenyl)-2-vinyl-3-cyanopyridine (1.0 g, 4.26 mmol) were dissolved in 10mL DMF, N,N-diisopropylethylamine (1.1 g, 8.52 mmol) and HATU (1.62 g, 4.26 mmol) were added, after reaction for 16 hours at room temperature, the reaction solution was diluted with 50mL DCM, washed with water (3^{∗}30 ml) and saturated brine (30 ml), dried over anhydrous sodium sulfate, filtered and concentrated. The residue was purified by silica gel column chromatography (eluent: dichloromethane = 100) to obtain compound methyl 6-((3-(3-cyano-2-vinylpyridin-4-yl)-2-methylphenyl)carbamoyl)nicotinate (920 mg, yellow solid) 1g with a yield of 54.4%. MS m/z (ESI): 399.2 [M+1].

Step 5: Compound methyl 6-((3-(3-cyano-2-vinylpyridin-4-yl)-2-methylphenyl)carbamoyl)nicotinate (200 mg, 0.5 mmol), 4-bromo-5-chloro-2-methylbenzaldehyde (118 mg, 0.5 mmol), palladium acetate(12 mg, 0.05 mmol), tris(o-methylphenyl)phosphorus (16 mg, 0.05 mmol) and triethylamine(152 mg, 1.5 mmol) were mixed in 5ml N,N-dimethylacetamide, the mixture was heated to 160 °C under the protection of argon in a microwave reactor, and reacted for 45 minutes. The reactants were diluted with 30 ml ethyl acetate, washed with water (3^{∗}20 ml) and saturated brine (20 ml), dried over anhydrous sodium sulfate, filtered and concentrated. The residue was purified by silica gel column chromatography (eluent: petroleum ether: ethyl acetate = 50: 50) to obtain compound methyl (E)- 6 -((3-(2-(2-chloro-4-formyl-5-methylstyryl)-3-cyanopyridin-4-yl)-2-methylphenyl)carbamoyl) nicotinate 1i (50 mg, yellow solid) with a yield of 18.2%. MS m/z (ESI): 551.2 [M+1].

Step 6: Compound methyl (E)- 6 -((3-(2-(2-chloro-4-formyl-5-methylstyryl)-3-cyanopyridin-4-yl) -2-methylphenyl)carbamoyl)nicotinate **1i** (30 mg, 0.054 mmol) was dissolved in 4ml tetrahydrofuran, 2mg of lithium aluminium hydride was added under ice bath, after reaction for 2 hours, the reaction was quenched with sodium sulfate decahydrate, filtered, and the filter cake was washed with ethyl acetate. The filtrate was concentrated to obtain a crude product (E)-N-(3-(2-(2-chloro-4-(hydroxymethyl)-5-methylstyryl) -3-cyanopyridin-4-yl)-2-methylphenyl)-5- (hydroxymethyl)picolinamide **1j** (35 mg, yellow solid), the crude product does not need to be purified and is directly used in the next reaction. MS m/z (ESI): 525.2[M+1].

Step 7: Compound (E)-N-(3-(2-(2-chloro-4-(hydroxymethyl)-5-methylstyryl)-3-cyanopyridin-4-yl)-2 -methylphenyl)-5-(hydroxymethyl)picolinamide **1j** (35 mg, 0.067 mmol) was dissolved in 5mL dichloromethane, Dess-Martin oxidant (71 mg, 0.168 mmol) was added at room temperature, and the solid was filtered off after 2 hours of reaction. The filtrate was concentrated, and the residue was purified by silica gel column chromatography (eluent: dichloromethane: ethyl acetate = 70: 30) to obtain compound (E)-N-(3-(2-(2-chloro-4-formyl-5- methylstyryl)-3-cyanopyridin-4-yl)-2-methylphenyl)-5-formylpicolinamide lk (25mg, yellow solid) with a yield of 83%. MS m/z (ESI): 521.2[M+1].

Step 8: Compound (E)-N-(3-(2-(2-chloro-4-formyl-5-methylstyryl)-3-cyanopyridin-4-yl)-2-methylphenyl) -5-formylpicolinamide **lk** (25 mg, 0.048 mmol) was dissolved in a mixed solvent of 6mL methanol and 2mL dichloromethane, 2-aminoethanol (7 mg, 0.11 mmol) and a drop of acetic acid were added, the reaction solution was stirred at room temperature for 2 hours, then sodium cyanoborohydride (12 mg, 0.192 mmol) was added, and the reaction solution was reacted overnight The reactants are concentrated and purified by preparative chromatography to obtain the target compound (E)-N-(3-(2-(2-chloro-4 -(((2-hydroxyethyl)amino)methyl) -5-methylstyryl)-3-cyanopyridin-4-yl)-2-methylphenyl)-5-(((2-hydroxyethyl)amino)methyl)picolinamide **Z-1** (1.5 mg, white solid with a yield of 5.2%. MS m/z (ESI): 611.3 [M+1].¹H NMR (400 MHz, DMSO-d6) δ 10.42 (s, 1H), 8.90 (d, *J* = 5.0 Hz, 1H), 8.68 (s, 1H), 8.32 (d, *J* = 15.4 Hz, 1H), 8.13 (d, *J* = 8.4 Hz, 1H), 8.03 (d, *J* = 7.0 Hz, 1H), 7.93 (d, *J =* 7.8 Hz, 1H), 7.77 (s, 1H), 7.60 - 7.50 (m, 1H), 7.49 - 7.32 (m, 2H), 7.18 (d, *J* = 7.4 Hz, 1H), 6.52 (s, 1H), 4.51-4.88 (m, 4H), 3.93 (s, 2H), 3.82 (s, 2H), 3.58 - 3.42 (m, 4H), 2.73 (s, 2H), 2.64 (s, 2H), 2.32 (s, 3H), 2.11 (s, 3H).

### Example 2: Preparation of (S, E)-1-(5-chloro-4-(2-(3-cyano-4-(3-(5-(((2-hydroxyethyl)amino)methyl) picolinamido)-2-methylphenyl)pyridin-2-yl)vinyl)-2-methylbenzyl)piperidine-2-carboxylic acid(Z-2)

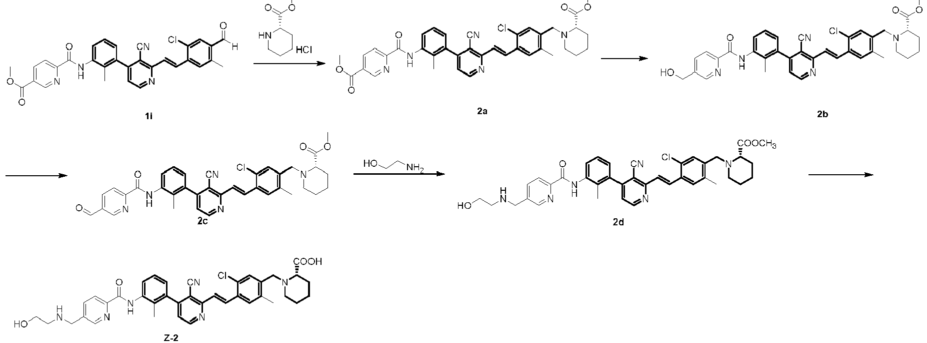

Step 1: Compound methyl (E)-6-((3-(2-(2-chloro-4-formyl-5-methylstyryl)-3-cyanopyridin-4-yl) -2-methylphenyl)carbamoyl)nicotinate **1i** (320 mg, 0.58 mmol) was dissolved in 10mL DMF, methyl (S)-methylpiperidine-2-carboxylate hydrochloride (105 mg, 0.58 mmol) was added, the reaction solution was heated to 60°C and reacted for 1 hour, then sodium cyanoborohydride (73 mg, 1.16 mmol) was added, and the reaction solution was reacted overnight. The reaction solution was diluted with 30mL dichloromethane, washed with water (3^{∗}20 ml) and saturated brine (20 ml), dried over anhydrous sodium sulfate, filtered and concentrated. The residue was purified by silica gel column chromatography (eluent: dichloromethane: ethyl acetate = 70: 30) to obtain compound methyl (S,E)-6-((3-(2-(2-chloro-4-((2-(methoxycarbonyl)piperidin-1-yl)methyl)-5-methylstyryl) -3-cyanopyridin-4-yl)-2-methylphenyl)carbamoyl)nicotinate **2a** (182 mg, yellow solid) with a yield of 46.3%. MS m/z (ESI): 678.3 [M+1].

Step 2: Compound methyl (S,E)-6-((3-(2-(2-chloro-4-((2-(methoxycarbonyl)piperidin-1-yl)methyl) -5-methylstyryl)-3-cyanopyridin-4-yl)-2-methylphenyl)carbamoyl)nicotinate **2a** (247 mg, 0.36 mmol) was dissolved in 10 mL methanol, and sodium borohydride (138 mg, 3.6 mmol) was added in batches. After the addition, the reaction was continued for 2 hours and the reaction solution was poured into water, extracted with dichloromethane (3^{∗}20 ml), the organic phases were combined, washed with saturated brine (30 ml), dried over anhydrous sodium sulfate, filtered and concentrated to obtain the crude product methyl (S,E)-1-(5-chloro-4-(2 -(3-cyano-4-(3-(5-(hydroxymethyl)picolinamido)-2-methylphenyl)pyridin-2-yl)vinyl)-2-methylbenzyl)piperidi ne-2-carboxylate **2b** (220 mg, yellow solid), the crude product does not need to be purified and is used directly in the next reaction. MS m/z (ESI): 650.3 [M+1].

Step 3: Compound methyl (S,E)-1-(5-chloro-4-(2-(3-cyano-4-(3-(5-(hydroxymethyl)picolinamido) -2-methylphenyl)pyridin-2-yl)vinyl)-2-methylbenzyl)piperidine-2-carboxylate **2b** (220 mg, 0.34 mmol) was dissolved in 10mL dichloromethane, and Dess-Martin oxidant (215 mg, 0.51 mmol) was added. After the reaction solution was stirred and reacted at room temperature for two hours, the solid was removed by filtration. The filtrate was concentrated and purified by silica gel column chromatography (eluent: dichloromethane: ethyl acetate = 70: 30) to obtain compound methyl (S, E)-1-(5-chloro-4-(2-(3-cyano-4-(3-(5-formylpicolinamido) -2-methylphenyl)pyridin-2-yl)vinyl)-2-methyl)methylbenzyl)piperidine-2-carboxylate **2c** (145 mg, yellow solid) with a yield of 72.1%. MS m/z (ESI): 648.3 [M+1].

Step 4: Compound methyl (S, E)-1-(5-chloro-4-(2-(3-cyano-4-(3-(5-formylpicolinamido)-2-methylphenyl) pyridin-2-yl)vinyl)-2-methyl) methylbenzyl)piperidine-2-carboxylate **2c** (135 mg, 0.21 mmol) was dissolved in 5mL dichloromethane, and 2-aminoethanol (26 mg, 0.42 mmol) was added. After reacting for two hours at room temperature, sodium cyanoborohydride (40 mg, 0.63 mmol) was added, and reacted overnight. The reaction solution was poured into water, extracted with dichloromethane (3^{∗}10 ml), the organic phases were combined, washed with saturated brine (20 ml), dried over anhydrous sodium sulfate, filtered and concentrated to obtain the crude product methyl (S,E)-1-(5-chloro-4-(2-(3-cyano-4-(3-(5-(((2-hydroxyethyl)amino)methyl)picolinamido) -2-methylphenyl)pyridin-2-yl)vinyl)-2-methylbenzyl)piperidine-2-carboxylate **2d** (165 mg, yellow oil). The crude product does not need to be purified and is used directly in the next reaction. MS m/z (ESI): 693.3 [M+1].

Step 5: Compound methyl (S,E)-1-(5-chloro-4-(2-(3-cyano-4-(3-(5-(((2-hydroxyethyl)amino) methyl)picolinamido)-2-methylphenyl) pyridin-2-yl)vinyl)-2-methylbenzyl)piperidine-2-carboxylate **2d** (165 mg, 0.24 mmol) was dissolved in 5mL methanol, 1mL water and lithium hydroxide monohydrate (40 mg, 0.96 mmol) were added, the reaction solution was heated to 60°C and reacted for 16 hours. The reactants were poured into water, and extracted with dichloromethane (3^{∗}10 ml). The organic phases were combined, washed with saturated brine (20 ml), dried over anhydrous sodium sulfate, filtered and concentrated to obtain the crude product. After purification by preparative chromatography, the target compound (S,E)-1-(5-chloro-4-(2-(3-cyano-4-(3-(5-(((2-hydroxyethyl)amino)methyl)picolinamido)-2-methyl phenyl)pyridin-2-yl)vinyl)-2-methylbenzyl)piperidine-2-carboxylic acid **Z-2** was obtained (6 mg, white solid) with a yield of 3.7%. MS m/z (ESI): 679.3 [M+1].¹H NMR (400 MHz, DMSO-d6) δ 10.40 (s, 1H), 8.90 (d, *J* = 5.0 Hz, 1H), 8.65 (s, 1H), 8.32 (d, *J* = 15.5 Hz, 1H), 8.11 (d, *J* = 7.8 Hz, 1H), 7.98 (t, *J* = 9.2 Hz, 2H), 7.72 (s, 1H), 7.53 (d, *J* = 15.3 Hz, 1H), 7.47 - 7.34 (m, 3H), 7.17 (d, *J* = 7.3 Hz, 1H), 4.66 - 4.33 (m, 2H), 3.83 (s, 2H), 3.73 (d, *J* = 14.2 Hz, 1H), 3.44 (dd, *J* = 11.2, 5.5 Hz, 3H), 3.13 (s, 1H), 2.82 (s, 1H), 2.55 (t, *J* = 5.8 Hz, 2H), 2.31 (s, 3H), 2.20 (s, 1H), 2.12 (s, 3H), 1.73 (s, 2H), 1.44 (s, 4H).

### Example 3: Preparation of (E)-2-(6-(2-(3-cyano-4-(3-(5-(((2-hydroxyethyl)amino)methyl)picolinamido) -2-methylphenyl)pyridin-2-yl)vinyl)-3,4-dihydroisoquinolin-2(1H)-yl)acetic acid (Z-3)

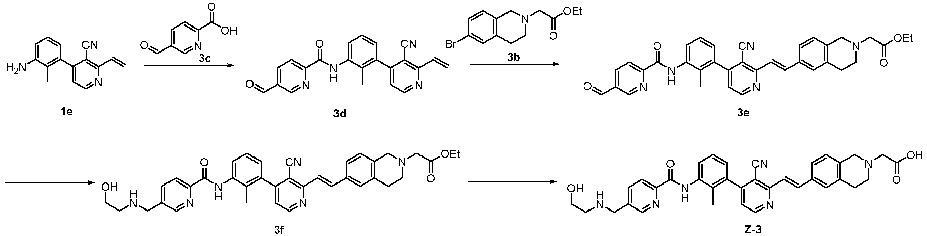

Step 1: 5-formylpicolinic acid **3c** (500mg, 3.3 mmol) was dissolved in 40 ml DMF, and compound **1e** (700mg, 3.3 mmol), TEA (1.0g, 10.0 mmol) and HATU (1.0g, 5.0 mmol) were added in sequence. the reaction solution was stirred and reacted for 1 hour at room temperature, extracted, washed with water, dried, and the residue was purified by column chromatography (eluent: petroleum ether/ethyl acetate = 68%) to obtain compound N-(3-(3-cyano-2-vinylpyridin-4-yl)-2-methylphenyl)-5-formylpicolinamide **3d** (300 mg, pale yellow solid) with a yield of 31%. MS m/z (ESI):368.1 [M+1].

Step 2: N-(3-(3-cyano-2-vinylpyridin-4-yl)-2-methylphenyl)-5-formylpicolinamide **3d** (45 mg, 0.16 mmol), ethyl 2-(6-bromo-3,4-dihydroisoquinolin-2(1H)-yl)acetate **3b** (32 mg, 0.16 mmol) were dissolved in 2 mL N,N-dimethylacetamide, and palladium acetate (1 mg, 0.008 mmol) tri-o-methylphenylphosphine (1 mg, 0.008 mmol) and 0.5 mL triethylamine were added, the reaction solution was replaced with nitrogen three times, heated to 160°C, stirred and reacted in a microwave reactor for 45 minutes, and then cooled to room temperature, concentrated under reduced pressure, and the residue was purified by column chromatography (eluent: petroleum ether/ethyl acetate = 78 %) to obtain compound ethyl (E)-2-(6-(2-(3-cyano-4-(3-(5-formylpicolinamido)-2-methylphenyl)pyridin-2-yl)vinyl)-3,4-dihydroisoquinolin -2(1H)-yl)acetate **3e** (30 mg, light brown solid) with a yield of 35%. MS m/z (ESI):586.2 [M+1].

Step 3: ethyl (E)-2-(6-(2-(3-cyano-4-(3-(5-formylpicolinamido)-2-methylphenyl)pyridin-2-yl)vinyl)-3,4-dihydroisoquinolin-2(1H)-yl)acetate **3e** (30 mg, 0.051 mmol) was dissolved in 10 ml DCM, and ethanolamine (20 mg, 0.3 mmol), sodium cyanoborohydride (5 mg, 0.078 mmol) and acetic acid (0.5ml) were added in sequence. The reaction solution was heated to 45°C, stirred and reacted for 0.5 hours, cooled to room temperature, and the reaction solution was concentrated under reduced pressure to dryness, and used directly in the next step. The target compound ethyl (E)-2-(6-(2-(3-cyano-4-(3-(5-(((2-hydroxyethyl)amino)methyl)picolinamido) -2-methylphenyl)pyridin-2-yl)vinyl)-3,4-dihydroisoquinolin-2(1H)-yl)acetate **3f** (108 mg, reddish brown oil) was obtained with a yield of 90%. MS m/z (ESI):631.3 [M+1].

Step 4: ethyl (E)-2-(6-(2-(3-cyano-4-(3-(5-(((2-hydroxyethyl)amino)methyl)picolinamido)-2-methylphenyl) pyridin-2-yl)vinyl)-3,4-dihydroisoquinolin-2(1H)-yl)acetate **3f** (120 mg, 0.2 mmol) was dissolved in 10 mL methanol and 5 mL water, lithium hydroxide (42 mg, 1 mmol) was added, the reaction solution was heated to 45 °C, stirred and reacted for 1 hour, cooled to room temperature, the reaction solution was concentrated under reduced pressure, and the residue was purified by preparative chromatography to obtain the target compound (E)-2-(6-(2-(3-cyano-4-(3-(5-(((2-hydroxyethyl)amino)methyl)picolinamido)-2-methylphenyl)pyridin-2-yl)vin yl) -3,4-dihydroisoquinolin-2(1H)-yl)acetic acid Z-3 ( 4.5 mg, white solid) with a yield of 20%. MS m/z (ESI):603.3 [M+1].1H NMR (400 MHz, DMSO-d6) δ 10.38(s, 1H), 8.84(s, 1H), 8.65s, 1H), 8.11-7.95(m, 4H), 7.41-7.37(m, 4H), 7.15(d, 1H), 7.07(d, 1H), 4.47(d, 2H), 3.82(s, 2H), 3.68(s, 2H), 3.44(d, 2H), 2.91(s, 2H), 2.78(s, 2H), 2.74(s, 2H), 2.58(d, 2H), 2.11(s, 3H).

### Example 4: Preparation of (E)-N-(3-(3-cyano-2-(2-fluoro-4-(((2-hydroxyethyl)amino)methyl)-5-methylstyryl)pyridin-4-yl)-2-methylphenyl)-5-(((2-hydroxyethyl)amino)methyl)picolinamide (Z-4)

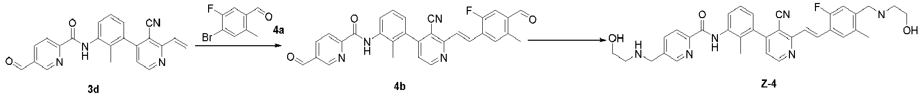

Step 1: N-(3-(3-cyano-2-vinylpyridin-4-yl)-2-methylphenyl)-5-formylpicolinamide **3d** (184 mg, 0.5 mmol), 4-bromo-5-fluoro-2-methylbenzaldehyde **4a** (162 mg, 0.75 mmol) were dissolved in 5 mL N,N-dimethylacetamide, palladium acetate (3 mg, 0.02 mmol) tri-o-methylphenylphosphine (2 mg, 0.02 mmol) and 0.5 mL triethylamine were added, the reaction solution was replaced with nitrogen three times, heated to 160 °C, stirred and reacted in a microwave reactor for 45 minutes, then cooled to room temperature, concentrated under reduced pressure, and the residue was purified by column chromatography (eluent: petroleum ether/ethyl acetate = 63%) to obtain compound (E)-N-(3-(3-cyano-2-(2-fluoro-4-formyl-5-methylstyryl)pyridin -4-yl)-2-methylphenyl)-5-formylpicolinamide **4b** (30 mg, light brown solid) with a yield of 15%. MS m/z (ESI):505.2 [M+1].

Step 2: (E)-N-(3-(3-cyano-2-(2-fluoro-4-formyl-5-methylstyryl)pyridin-4-yl)-2-methylphenyl) -5-formylpicolinamide **4b** (30 mg, 0.068 mmol) was dissolved in 10 ml DCM, ethanolamine (30 mg, 0.4 mmol), sodium cyanoborohydride (5 mg, 0.078 mmol) and acetic acid (0.5ml) were added in sequence, the reaction solution was heated to 45°C, stirred and reacted for 0.5 hours, and cooled to room temperature, the reaction solution was concentrated to dryness under reduced pressure, and the residue was purified by preparative chromatography to obtain the target compound (E)-N-(3-(3-cyano-2-(2-fluoro-4-(((2-hydroxyethyl)amino)methyl) -5-methylstyryl) pyridin-4-yl)-2-methylphenyl)-5-(((2-hydroxyethyl)amino)methyl)picolinamide **Z-4** (4.6 mg, white solid) with a yield of 20%. MS m/z (ESI):595.3 [M+1].1HNMR (400 MHz, DMSO-d6) δ 10.38(s, 1H), 8.84(s, 1H), 8.65s, 1H), 8.20-8.06(m, 3H), 7.95(d, 2H), 7.59(d, 2H), 7.42d, 2H), 7.20(d, 1H), 7.11(d, 1H), 3.88(s, 2H), 3.62(s, 2H), 3.49-3.45(m, 8H), 2.61(s, 2H), 2.59(s, 2H), 2.26(s, 3H), 2.11(s, 3H).

### Example 5: Preparation of (E)-1-((7-chloro-5-(2-(3-cyano-4-(3-(5-(((2-hydroxyethyl)amino)methyl) picolinamido)-2-methylphenyl)pyridin-2-yl)vinyl)benzo[d]oxazol-2-yl)methyl)pyrrolidine-3-carboxylic acid (Z-5)

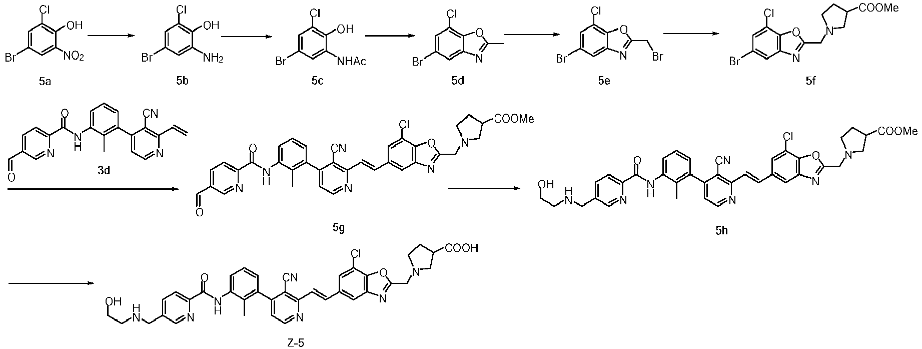

Step 1: 4-bromo-2-chloro-6-nitrophenol **5a** (2.50g, 10.0 mmol) was dissolved in 50 ml EtOH and 20 ml water, iron powder (5.60g, 100.0 mmol) and ammonium chloride (5.60g, 100.0 mmol) were added in sequence, the reaction solution was stirred and reacted for 1.5 hours at 80°C, filtered, concentrated under reduced pressure, extracted, dried and concentrated under reduced pressure, and the residue was purified by column chromatography (eluent: petroleum ether/ethyl acetate = 33%) to obtain compound 2-amino-4-bromo-6-chlorophenol **5b** (1.5g, light yellow solid) with a yield of 66%. MS m/z (ESI):224.1 [M+1].

Step 2: 2-amino-4-bromo-6-chlorophenol **5b** (1.50g, 6.7 mmol) was dissolved in 50 ml DCM, TEA (2.0g, 20.0 mmol) and acetyl chloride (0.8g, 10.0 mmol) were added in sequence, and the reaction solution was stirred and reacted at room temperature for 1.5 hours, washed with water, dried, concentrated under reduced pressure. The residue was purified by column chromatography (eluent: petroleum ether/ethyl acetate = 53%) to obtain compound N-(5-bromo-3-chloro-2-hydroxyphenyl)acetamide **5c** (1.2g, light yellow solid) with a yield of 63%. MS m/z (ESI):266.1 [M+1].

Step 3: N-(5-bromo-3-chloro-2-hydroxyphenyl)acetamide **5c** (1.20g, 5.7 mmol) was dissolved in 50 ml of toluene, p-toluenesulfonic acid (0.2g, 1.2 mmol) was added, the reaction solution was stirred and reacted at 110°C for 12 hours, concentrated under reduced pressure, and the residue was purified by column chromatography (eluent: petroleum ether/ethyl acetate = 30%) to obtain compound 5-bromo-7-chloro-2-methylbenzo[d]oxazole **5d** (0.5g, light yellow solid) with a yield of 49%. MS m/z (ESI):248.1 [M+1].

Step 4: 5-bromo-7-chloro-2-methylbenzo[d]oxazole **5d** (0.5g, 2.0 mmol) was dissolved in 50 ml carbon tetrachloride, NBS (0.36g, 2.0 mmol), AIBN (0.035g, 0.2 mmol) were added. The reaction solution was stirred and reacted at 80°C for 12 hours, concentrated under reduced pressure, and the residue was purified by column chromatography (eluent: petroleum ether/ethyl acetate = 20%) to obtain compound 5-bromo-2-(bromomethyl)-7-chlorobenzo[d]oxazole **5e** (200 mg, pale yellow solid) with a yield of 33%. MS m/z (ESI):328.1 [M+1].

Step 5: Compound 5-bromo-2-(bromomethyl)-7-chlorobenzo[d]oxazole **5e** (200mg, 0.62 mmol) was dissolved in 50 ml DCM, TEA (190 mg, 1.9 mmol) and methyl pyrrole-3-carboxylate hydrochloride (100 mg, 0.62 mmol) were added in sequence, the reaction solution was stirred and reacted at room temperature for 4 hours, washed with water, dried, concentrated under reduced pressure, and the residue was purified by column chromatography (eluent: petroleum ether/ethyl acetate = 53%) to obtain compound methyl 1-((5-bromo-7-chlorobenzo[d]oxazol-2-yl)methyl)pyrrolidine-3-carboxylate **5f (60** mg, pale yellow solid) with a yield of 28%. MS m/z (ESI):375.1 [M+1].

Step 6: N-(3-(3-cyano-2-vinylpyridin-4-yl)-2-methylphenyl)-5-formylpicolinamide **3d** (45 mg, 0.16 mmol), methyl 1-((5-bromo-7-chlorobenzo[d]oxazol-2-yl)methyl)pyrrolidine-3-carboxylate **5f** (60 mg, 0.16 mmol) were dissolved in 3 mL N,N-dimethylacetamide, and palladium acetate (1 mg, 0.008 mmol) tri-o-methylphenylphosphine (1 mg, 0.008 mmol) and 0.5 mL triethylamine were added, the reaction solution was replaced with nitrogen three times, heated to 160°C, stirred and reacted in a microwave reactor for 45 minutes, cooled to room temperature, and concentrated under reduced pressure, and the residue was purified by column chromatography (eluent: petroleum ether/ethyl acetate = 80%) to obtain compound methyl (E)-1-((7-chloro-5-(2-(3-cyano-4-(3-(5-formylpicolinamido)-2-methylphenyl)pyridin-2-yl)vinyl)benzo[d]oxaz ol-2-yl)methyl)pyrrolidine-3-carboxylate **5g** (25 mg, light brown solid) with a yield of 26%. MS m/z (ESI):661.2 [M+1].

Step 7: Methyl (E)-1-((7-chloro-5-(2-(3-cyano-4-(3-(5-formylpicolinamido)-2-methylphenyl)pyridin-2-yl) vinyl)benzo[d]oxazol-2-yl)methyl)pyrrolidine-3-carboxylate **5g** (25 mg, 0.039 mmol) was dissolved in 10 ml DCM, then ethanolamine (20 mg, 0.3 mmol), sodium cyanoborohydride (5 mg, 0.078 mmol) and acetic acid (0.5ml) were added in sequence, and the reaction solution was heated to 45°C, stirred and reacted for 0.5 hours, and cooled to room temperature. The reaction solution was concentrated to dryness under reduced pressure and used directly in the next step. Compound methyl (E)-1-((7-chloro-5-(2-(3-cyano-4-(3-(5-(((2-hydroxyethyl)amino)methyl) picolinamido)-2-methylphenyl) pyridin-2-yl)vinyl)benzo[d]oxazol-2-yl)methyl)pyrrolidine-3-carboxylate **5h** (108 mg, red-brown oil) was obtained with a yield of 90%. MS m/z (ESI):706.3 [M+1].

Step 8: Methyl (E)-1-((7-chloro-5-(2-(3-cyano-4-(3-(5-(((2-hydroxyethyl)amino)methyl)picolinamido)-2- methylphenyl) pyridin-2-yl)vinyl)benzo[d]oxazol-2-yl)methyl)pyrrolidine-3-carboxylate **5h** (120 mg, 0.2 mmol) was dissolved in 10 mL methanol and 5 mL water, lithium hydroxide (42 mg, 1 mmol) was added, and the reaction solution was heated to 45°C, stirred and reacted for 1 hour, and cooled to room temperature. The reaction solution was concentrated under reduced pressure, and the residue was purified by preparative chromatography to obtain the target compound (E)-1-((7-chloro-5-(2-(3-cyano-4-(3-(5-(((2-hydroxyethyl)amino)methyl) picolinamido)-2-methylphenyl)pyridin-2-yl)vinyl)benzo[d]oxazol-2-yl)methyl)pyrrolidine-3-carboxylic acid Z-5 (0.5 mg, white solid) with a yield of 5%. MS m/z (ESI):692.3 [M+1].1H NMR (400 MHz, DMSO-d6) δ 10.38(s, 1H), 8.84(s, 1H), 8.65s, 1H), 8.11-7.95(m, 3H), 7.41-7.37(m, 3H), 7.15(d, 1H), 7.07(d, 1H), 4.47(d, 2H), 3.91(s, 2H), 3.82(s, 2H), 3.44(d, 2H), 3.23(s, 2H), 2.63(s, 2H), 2.56(s, 2H), 2.53(d, 2H), 2.29(s, 1H), 2.11(s, 3H).

### Example 6: Preparation of

### (R)-1-((6-((3-(2-((E)-2-chloro-4-(((R)-3-hydroxypyrrolidin-1-yl)methyl)-5-methylstyryl)-3-cyanopyridin-4-yl)-2-methylphenyl)carbamoyl)pyridin-3-yl)methyl)piperidine-2-carbox ylic acid (Z-6)

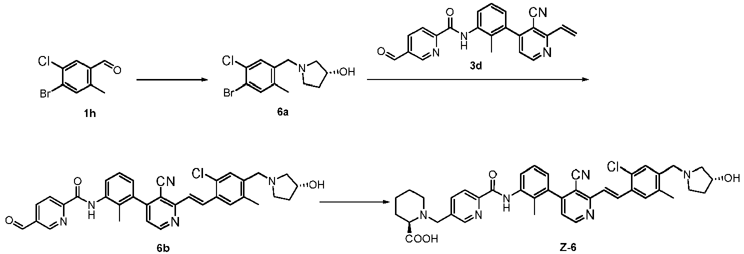

Step 1: Compound 1h (117 mg, 0.5 mmol) and (R)-pyrrolidin-3-ol (45 mg, 0.5 mol) were dissolved in 10 mL methanol, and sodium cyanoborohydride (31 mg, 0.5 mmol) was added. The reaction solution was stirred and reacted under reflux for 2 hours, and concentrated under reduced pressure. The residue was purified by column chromatography (eluent: petroleum ether/ethyl acetate = 68%) to obtain product 6a (100 mg, colorless oil) with a yield of 70.9%. MS m/z (ESI):306.1 [M+1]

Step 2: N-(3-(3-cyano-2-vinylpyridin-4-yl)-2-methylphenyl)-5-formylpicolinamide 3d (36 mg, 0.1 mmol), 6a(45 mg, 0.15 mmol) were dissolved in 2 mL N,N-dimethylacetamide, and palladium acetate (1.1 mg, 0.005 mmol) tri-o-methylphenylphosphine (1.5 mg, 0.005 mmol) and 0.3 mL triethylamine were added. The reaction solution was replaced with nitrogen three times, heat to 160°C, stirred and reacted in a microwave reactor for 45 minutes, then cooled to room temperature, and concentrated under reduced pressure, and the residue was purified by column chromatography (eluent: petroleum ether/ethyl acetate = 78%) to obtain compound 6b (33 mg, light brown solid) with a yield of 55%. MS m/z (ESI):592.2 [M+1]

Step 3: Compound 6b (33 mg, 0.06 mmol) and (R)-piperidine-2-carboxylic acid (18 mg, 0.12 mol) were dissolved in 10 mL methanol, and sodium cyanoborohydride (4 mg, 0.06 mmol) was added. The reaction solution was stirred and reacted under reflux for 2 hours, and concentrated under reduced pressure. The title product Z-6 (6.8 mg, white solid) was obtained by preparative HPLC chromatography with a yield of 17.9%. MS m/z (ESI):705.1 [M+1].1H NMR (400 MHz, DMSO-d6) δ 10.40 (s, 1H), 8.95 (s, 1H), 8.64 (s, 1H), 8.32 (d, 1H), 8.18 (s, 1H), 7.99-7.87 (m, 3H), 7.75 (s, 1H), 7.58 (d, 1H), 7.48 (d, 2H), 7.23 (d, 1H), 6.49 (s, 2H) 4.46 (d, 4H),3.90 (s, 1H), 3.52 (s, 4H), 3.01 (s, 4H), 2.82 (s, 2H) 2.51 (s, 3H), 2.47 (s, 2H), 2.21 (s,3H), 1.75 (d, 2H), 1.48(m, 4H).

### Example 7: Preparation of

### (S)-1-(5-chloro-4-((E)-2-(3-cyano-4-(3-((3-(((R)-3-hydroxypyrrolidin-1-yl) methyl)-1,7-naphthyridin-8-yl)amino)-2-methylphenyl)pyridin-2-yl)vinyl)-2-methylbenzyl)piperidine-2-carboxylic acid (Z-7)

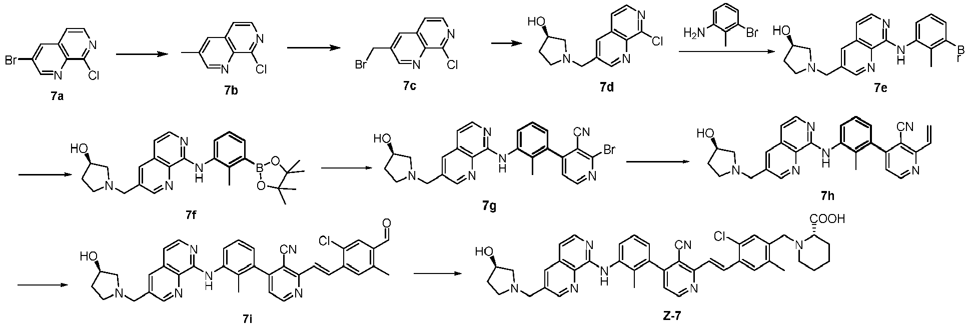

Step 1: 3-bromo-8-chloro-1,7-naphthyridine 7a (5 g, 20.66 mmol) was dissolved in 50 mL 1,4-dioxane, methyl boric acid (3.7 g, 61.98 mmol), potassium carbonate (5.7 g, 41.32 mmol), [1,1-bis(diphenylphosphino)ferrocene]dichloropalladium(II) dichloromethane complex (169 mg, 0.21 mmol) and 5 mL of water were added in sequence, and the reaction solution was replaced with nitrogen three times, heated to 100°C, stirred and reacted for 20 hours. After cooling to room temperature, the reaction solution was filtered through celite, the filtrate was concentrated under reduced pressure, and the residue was purified by silica gel column chromatography (eluent: petroleum ether: ethyl acetate = 95: 5) to obtain 3-methyl-8-chloro-1,7-naphthyridine 7b (2.6 g, yellow solid) with a yield of 71%. MS m/z (ESI): 179.1 [M+1].

Step 2: 3-methyl-8-chloro-1,7-naphthyridine 7b (2.6 g, 14.61 mmol) was dissolved in 50 mL carbon tetrachloride, and N-bromosuccinimide (3.4 g, 18.99 mmol) and azobisisobutyronitrile (280 mg, 1.46 mmol) were added in sequence. The reaction solution was replaced with nitrogen three times, heated to 90°C, stirred and reacted for 3.5 hours and then cooled to room temperature. The reaction solution was filtered through celite, the filtrate was concentrated under reduced pressure, and the residue was purified by silica gel column chromatography (eluent: petroleum ether: ethyl acetate = 70: 30) to obtain 3-bromomethyl-8-chloro-1,7-naphthyridine 7c (1.9 g, white solid). Yield: 51%. MS m/z (ESI): 257.1 [M+1].

Step 3: (R)-pyrrolidin-3-ol hydrochloride (913 mg, 7.42 mmol) was dissolved in 50 mL dichloromethane, N,N-diisopropylethylamine (2.87 g, 22.26 mmol) was added, the mixture was stirred at room temperature for 5 minutes, and then 3-bromomethyl-8-chloro-1,7-naphthyridine 7c (1.9 g, 7.42 mmol) was added. The reaction solution was stirred overnight at room temperature, and concentrated under reduced pressure, and the residue was purified by silica gel column chromatography (eluent: dichloromethane: methanol = 95: 5) to obtain (R)-1-((8-chloro-1,7-naphthyridin-3-yl)methyl)pyrrolidin-3-ol 7d (1.95 g, yellow solid) with a yield of 99%. MS m/z (ESI): 264.1 [M+1].

Step 4: Compound 7d (526 mg, 2.0 mmol), 2-bromo-1-methylaniline (1.9 g, 10.0 mmol), 4M hydrochloric acid in dioxane solution (1.0 mL, 4.0 mmol) and 6 mL ethanol were added into a 20 mL microwave tube. The lid of the microwave tube was closed, and the reaction solution was reacted under microwave at 120°C for 60 minutes. Diisopropylethylamine was added dropwise, the pH was adjusted to 10, and the reaction solution was concentrated under reduced pressure. The the residue was purified by silica gel column chromatography with 0%~10% methanol in dichloromethane to obtain product 7e (680mg, yellow solid) with a yield of 92.6%. MS m/z (ESI):413.1 [M+1].

Step 5: Compound 7e (680mg, 1.6 mmol) was dissolved in 20 mL 1,4-dioxane, bis(pinacolato)diboron (510mg, 2.0 mmol), [1,1-bis(diphenylphosphino)ferrocene]dichloropalladium(II) dichloromethane complex (117 mg, 0.16 mmol) and potassium acetate (400 mg, 4.0 mmol) were added, and the reaction solution was stirred and reacted overnight at 100°C under argon protection. The reaction solution was filtered, the filtrate was concentrated under reduced pressure, and the residue was purified by silica gel column chromatography with 0%~10% methanol in dichloromethane to obtain the product 7f (500 mg, brown solid) with a yield of 75.3%. MS m/z (ESI):461.1 [M+1].

Step 6: Compound 7f (500 mg, 1.1 mmol) was dissolved in 20 mL 1,4-dioxane and 6mL water, 2,4-dibromonicotinonitrile (260 mg, 1.1 mmol), [1,1-bis(diphenylphosphino)ferrocene]dichloropalladium(II) dichloromethane complex (75 mg, 0.11 mmol) and sodium carbonate (230 mg, 2.2 mmol) were added, and the reaction solution was stirred and reacted overnight at 100°C under argon protection. The reaction solution was filtered, the filtrate was concentrated under reduced pressure, and the residue was purified by silica gel column chromatography with 0%~10% methanol in dichloromethane to obtain 7 g (300 mg, light brown solid) with a yield of 60.3%. MS m/z (ESI):515.1 [M+1].

Step 7: Compound 7g (300 mg, 0.6 mmol) was dissolved in 15 mL of 1,4-dioxane and 5 mL of water, potassium vinylfluoroborate (78 mg, 0.6 mmol), [1,1-bis(diphenylphosphino)ferrocene]dichloropalladium(II) dichloromethane complex (45 mg, 0.06 mmol) and sodium carbonate (140 mg, 1.30 mmol) were added, and the reaction solution was stirred and reacted for 16 hours at 100°C under the protection of argon. The reaction solution was filtered, the filtrate was concentrated under reduced pressure, and the residue was purified by silica gel column chromatography with 0%~10% methanol in dichloromethane to obtain the product 7h (103mg, colorless liquid) with a yield of 31.1%. MS m/z (ESI):463.2 [M+1].

Step 8: Compound 7h (50 mg, 0.10 mmol), 4-bromo-5-chloro-2-methylbenzaldehyde (30 mg, 0.15 mmol), palladium acetate (1.2 mg, 0.05 mmol), tri-o-methylphenylphosphine (1.5 mg, 0.05 mmol), 0.3 mL triethylamine and 3 mL dimethylacetamide were added into a 20 mL microwave tube. argon was blown for 1 minute, and the lid of the microwave tube was closed. The reaction solution was reacted under microwave at 160°C for 45 minutes and concentrated under reduced pressure and the residue was purified by silica gel column chromatography with 0%~10% methanol in dichloromethane to obtain the title product 7i (30mg, yellow solid) with a yield of 48.4%. MS m/z (ESI):615.1[M+1].

Step 9: Compound 7i (30 mg, 0.05 mmol) and (R)-piperidine-2-carboxylic acid (18 mg, 0.1 mol) were dissolved in 10 mL methanol, and sodium cyanoborohydride (5 mg, 0.075 mmol) was added. The reaction solution was stirred and reacted under reflux for 2 hours, and concentrated under reduced pressure. The title product Z-7 (5 mg, white solid) was obtained by preparative HPLC chromatography with a yield of 11.9%. MS m/z (ESI):728.1 [M+1].1H NMR (400 MHz, DMSO-d6) δ 9.30 (s, 1H), 8.85 (d, 2H), 8.52 (d, 1H), 8.32 (s, 1H), 8.21 (s, 1H), 8.09 (d, 1H), 7.74 (s, 1H), 7.58-7.34 (m, 3H), 7.21 (s, 1H),7.09 (d, 1H), 4.69 (s, 1H) 4.19 (s, 1H), 3.78-3.68 (m, 2H), 3.45 (m, 3H), 3.31 (m, 3H), 2.78 (d, 1H), 2.74 (d, 1H), 2.65 (d, 1H), 2.31 (s, 3H), 2.24 (s, 3H), 2.01 (d, 1H),1.75 (d, 2H), 1.48(m, 4H).

### Example 10: (R)-1-(4-((E)-2-(3'-((3-(((R)-3-hydroxypyrrolidin-1-yl)mcthyl)-2-(trifluoromcthyl) -1,7-naphthyridin-8-yl)amino)-2,2'-dimcthyl-[1,1'-biphcnyl]-3-yl)vinyl)-2-mcthoxy-5-(trifluoromcthyl) benzyl)pyrrolidine-3-carboxylic acid

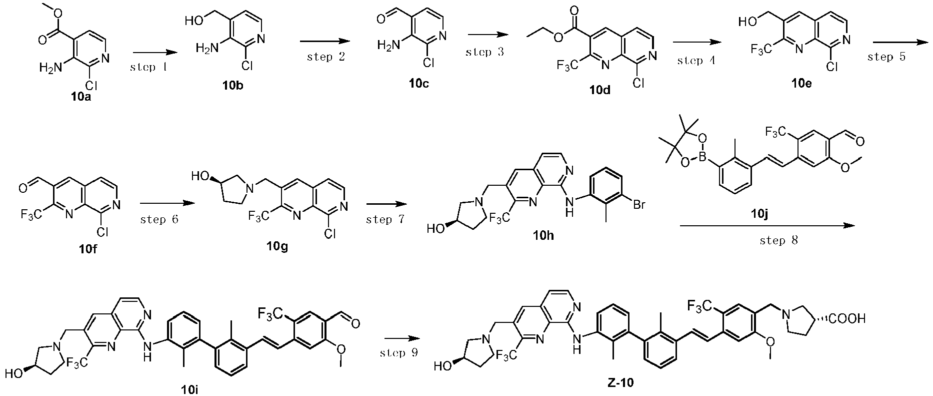

Step 1: Methyl 3-amino-2-chloroisonicotinate **10a** (5g, 26.88 mmol) was dissolved in 100 mL of tetrahydrofuran, the mixture was cooled to 0°C, and lithium aluminum hydride (2.05 g, 53.76 mmol) was added in batches. After stirring at room temperature for 30 minutes, the reaction was quenched with sodium sulfate decahydrate, the reaction solution was filtered and concentrated under reduced pressure to obtain (3-amino-2-chloropyridin-4-yl)methanol **10b** (4.1 g, yellow solid) with a yield of 96%. MS m/z (ESI): 159.3 [M+1].

Step 2: A mixture of compound **10b** (4.1 g, 25.95 mmol), manganese dioxide (11.3 g, 12.75 mmol), 100 mL DCM and 20 mL tetrahydrofuran were stirred overnight at room temperature, filtered, and the filtrate was concentrated under reduced pressure. The residue was purified by silica gel column chromatography to obtain compound 3-amino-2-chloroisonicotinaldehyde **10c** (3.5 g, white solid) with a yield of 86 %. MS m/z (ESI): 157.3 [M+1].

Step 3: A mixture of compound **10c** (2.33 g, 14.94 mmol), ethyl 4,4,4-trifluoro-3-oxobutyrate (3.02 g, 16.43 mmol), piperidine (2.5 g, 29.98 mmol), acetic acid (4.5g, 74.7 mmol) and 100 mL of toluene was heated to 110°C, stirred and reacted for 3 hours, cooled and concentrated under reduced pressure. The residue was purified by silica gel column chromatography to obtain compound ethyl 8-chloro-2-(trifluoromethyl)-1,7-naphthyridine-3- carboxylate **10d** (2.0 g, yellow solid) with a yield of 44 %. MS m/z (ESI): 305.3 [M+1].

Step 4: Compound **10d** (1.8 g, 5.92 mmol) was dissolved in 40 mL DCM, the mixture was cooled to -78 °C under an ice-water bath, diisobutyl aluminum hydride (1.0 M, 11.8 mL, 11.8 mmol) was slowly added dropwise, after the addition, and the reaction solution was stirred and reacted at 0 °C for 0.5 hours. Methanol (5 mL) and 36% hydrochloric acid (5 mL) were added in sequence. The reaction solution was warmed to room temperature, and stirred and reacted for 0.5 hours, extracted twice with ethyl acetate (20 mL), and the combined organic phase was washed with brine (30 mL), dried over anhydrous sodium sulfate, filtered and concentrated under reduced pressure. The residue was purified by silica gel column chromatography to obtain compound (8-chloro-2-(trifluoromethyl)-1,7-naphthyridin-3-yl)methanol **10e** (1.1 g, yellow solid) with a yield of 71%. MS m/z (ESI): 263.1 [M+1].

Step 5: A mixture of compound **10e** ((1.1 g, 4.20 mmol), manganese dioxide (3.6 g, 42.0 mmol), 60 mL DCM and 20 mL tetrahydrofuran was stirred overnight at room temperature, filtered, and the filtrate was concentrated under reduced pressure. The residue was purified by silica gel column chromatography to obtain compound 8-chloro-2-(trifluoromethyl)-1,7-naphthyridine-3-carbaldehyde **10f** (470 mg, yellow solid) with a yield of 43 %. MS m/z (ESI): 261.1 [M+1].

Step 6: Compound **10f** (470 mg, 1.81 mmol) was dissolved in 10 mL methanol, (R)-pyrrolidin-3-ol (189 mg, 2.17 mmol) and sodium cyanoborohydride (342 mg, 5.43 mmol) were added in sequence, and the reaction solution was replaced with nitrogen three times, stirred and reacted at room temperature for 20 hours, the reaction solution was concentrated under reduced pressure, and the residue was purified by silica gel column chromatography to obtain compound **10g** (490 mg, yellow solid) with a yield of 81%. MS m/z (ESI): 332.1 [M+1].

Step 7: A mixture of compound **10g** (480 mg, 1.45 mmol), 3-bromo-2-methylaniline (269 mg, 1.45 mmol), hydrochloric acid (4M, 0.36 mL, 1.45 mmol) and 6 mL isopropanol was heated to 120°C in a microwave reactor, and the reaction solution was stirred and reacted for 1 hour. After cooling, the reaction solution was concentrated under reduced pressure, and the residue was purified by silica gel column chromatography to obtain compound **10h** (230 mg, yellow oil) with a yield of 37%. MS m/z (ESI):481.1 [M+1].

Step 8: Compound **10h** (250 mg, 0.52 mmol) and **10j** (348 mg, 0.78 mmol) were dissolved in 10ml 1,4-dioxane, and [1,1'-bis(diphenylphosphino)ferrocene]dichloropalladium(II) (38 mg, 0.052 mmol), sodium carbonate (110 mg, 1.04 mmol) and 1 ml of water were added in sequence, the reaction solution was heated to 100 °C and reacted for 48 hours under the protection of argon, filtered to remove the solids, the filtrate was concentrated, and the residue was purified by silica gel column chromatography to obtain compound **10i** (50 mg, yellow solid) with a yield of 13%. MS m/z (ESI): 721.1 [M+1].

Step 9: Compound **10i** (50 mg, 0.069 mmol) was dissolved in 10 mL methanol, (R)-pyrrolidine-3-carboxylic acid (24 mg, 0.208 mmol) and sodium cyanoborohydride (22 mg, 0.345 mmol) were added in sequence, and the reaction solution was replaced with nitrogen three times, heated to 80°C, stirred and reacted for 3 hours, and cooled to room temperature. The reaction solution was concentrated under reduced pressure, and the residue was purified by preparative chromatography to obtain compound **Z-10** (21 mg, yellow solid) with a yield of 37%. MS m/z (ESI): 820.4 [M+1]; ¹H NMR (400 MHz, DMSO-d6 ) δ 9.19 (s, 1H), 8.61 (s, 1H), 8.30 (d, J = 8.0 Hz, 1H), 8.15 (d, J = 5.7 Hz, 1H), 7.64 (s, 1H), 7.60 - 7.55 (m, 1H), 7.48 (s, 1H), 7.34 - 7.18 (m, 4H), 7.10 (d, J = 7.7 Hz, 1H), 6.89 (d, J = 6.7 Hz, 1H), 6.51 (s, 1H), 4.77 (s, 2H), 4.23 (s, 2H), 3.92 (d, J = 10.7 Hz, 4H), 3.48 - 3.42 (m, 2H), 2.93 (s, 1H), 2.81 - 2.76 (m, 1H), 2.70 (d, J = 7.6 Hz, 2H), 2.64 (s, 1H), 2.54 (s, 1H), 2.40 (s, 1H), 2.29 (s, 1H), 2.13 (s, 3H), 2.06 - 1.89 (m, 5H), 1.60 (s, 2H), 1.01 (d, J = 6.9 Hz, 1H).

### Example 15: (R)-1-(4-((E)-2-(3'-((5-(((R)-3-hydroxypyrrolidin-1-yl)methyl)-1H-indazole-3-yl)amino) -2,2'-dimethyl-[1,1'-biphenyl]-3-yl)vinyl)-2-methoxy-5-(trifluoromethyl)benzyl)pyrrolidine-3-carboxylic acid

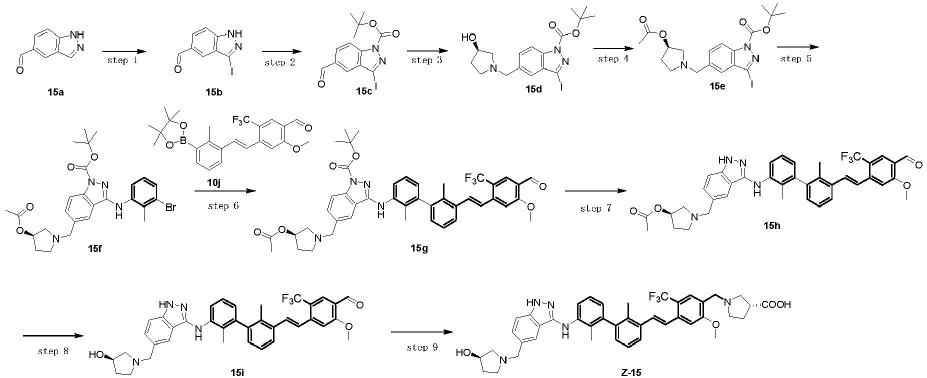

Step 1: A mixture of 1H-indazole-5-carbaldehyde **15a** (5.0 g, 34.25 mmol), sodium hydroxide (4.1 g, 102.7 mmol), iodine (17.4 g, 68.5 mmol) and 10 mL DMF was stirred overnight at room temperature. The reaction solution was concentrated under reduced pressure, and the residue was purified by silica gel column chromatography to obtain compound 3-iodo-1H-indazole-5-carbaldehyde **15b** (9.1 g, yellow solid) with a yield of 97%. MS m/z (ESI): 273.1 [M+1].

Step 2: A mixture of **15b** (9.1 g, 33.45 mmol), di-tert-butyl dicarbonate (14.6 g, 64.69 mmol), TEA (10.2 g, 100.35 mmol), N,N-dimethylpyridin-4-amine (41 mg, 0.334 mmol) and 100 mL DCM was stirred and reacted at room temperature for 5 hours. The reaction solution was concentrated under reduced pressure, and the residue was purified by silica gel column chromatography to obtain compound tert-butyl 5-formyl-3-iodo-1H-indazole-1-carboxylate **15c** (15.2 g, yellow solid) with a yield of 99%. MS m/z (ESI): 373 [M+1].

Step 3: Compound **15c** (1 g, 2.688 mmol) was dissolved in 10 mL methanol, (R)-pyrrolidin-3-ol (702 mg, 8.06 mmol) and sodium cyanoborohydride (508 mg, 8.06 mmol) were added in sequence, and the reaction solution was replaced with nitrogen three times, stirred and reacted at room temperature for 4 hours. The reaction solution was concentrated under reduced pressure, and the residue was purified by silica gel column chromatography to obtain compound **15d** (1g, yellow solid) with a yield of 84%. MS m/z (ESI): 444.1 [M+1].

Step 4: Compound **15d** (700 mg, 1.58 mmol) and TEA (479 mg, 4.74 mmol) were dissolved in 20 mL DCM, and acetyl chloride (370 mg, 4.74 mmol) was added dropwise. The mixture was stirred at room temperature overnight. The reaction solution was concentrated under reduced pressure, and the residue was purified by silica gel column chromatography to obtain compound **15e** (510 mg, yellow solid) with a yield of 89%. MS m/z (ESI): 486.1 [M+1].

Step 5: A mixture of compound **15e** (460 mg, 0.948 mmol), 3-bromo-2-methylaniline (229 mg, 1.23 mmol), tris(dibenzylideneacetone)dipalladium (43 mg, 0.047 mmol), 4,5-bis(diphenylphosphino)-9,9-dimethylxanthene (27 mg, 0.047 mmol), cesium carbonate (618 mg, 1.90 mmol) and 20 mL of toluene was heated to 60°C and reacted for 20 hours, then cooled to room temperature, filtered and concentrated under reduced pressure, and the residue was purified by column chromatography to obtain product **15f** (410 mg, yellow solid) with a yield of 80%. MS m/z (ESI):543.1 [M+1].

Step 6: Compounds **15f** (350 mg, 0.646 mmol) and **10j** (346 mg, 0.775 mmol) were dissolved in 10 ml 1,4-dioxane, and [1,1'-bis(diphenylphosphino)ferrocene]dichloropalladium(II) (24 mg, 0.032 mmol), sodium carbonate (134 mg, 1.29 mmol) and 2 ml of water were added in sequence. The reaction solution was heated to 100 °C under the protection of argon, reacted for 4 hours, filtered to remove the solids, the filtrate was concentrated, and the residue was purified by silica gel column chromatography to obtain compound **15g** (270 mg, yellow solid) with a yield of 53%. MS m/z (ESI): 783.1 [M+1].

Step 7: Compound **15g** (250 mg, 0.32 mmol) was dissolved in 10 mL DCM, 1 mL of trifluoroacetic acid was added, and the reaction solution was stirred and reacted for 3 hours at room temperature, and concentrated under reduced pressure to obtain compound **15 h** (218 mg, yellow oil) with a yield of 99%. MS m/z (ESI):683.3 [M+1].

Step 8: A mixture of compound **15h** (218 mg, 0.32 mmol), lithium hydroxide (153 mg, 6.39 mmol) and 10 mL methanol was stirred at room temperature overnight, and concentrated under reduced pressure, and the residue was purified by silica gel column chromatography to obtain compound **15i** (200 mg, yellow solid) with a yield of 97%. MS m/z (ESI): 641.3 [M+1].

Step 9: Compound **15i** (10 mg, 0.016 mmol) was dissolved in 5 mL methanol, (R)-pyrrolidine-3-carboxylic acid (5 mg, 0.047 mmol) and sodium cyanoborohydride (5 mg, 0.078 mmol) were added in sequence, and the reaction solution was replaced with nitrogen three times, heated to 80°C, stirred and reacted for 3 hours, cooled to room temperature. The reaction solution was concentrated under reduced pressure, and the residue was purified by preparative chromatography to obtain compound **Z-15** (5 mg, white solid) with a yield of 43%. MS m/z (ESI): 740.4 [M+1]; ¹H NMR (400 MHz, DMSO-d6 ) δ 12.04 (s, 1H), 8.14 (s, 1H), 7.63 (s, 1H), 7.59 (d, J = 4.2 Hz, 1H), 7.56 - 7.51 (m, 2H), 7.48 (s, 1H), 7.29 (t, J = 9.5 Hz, 3H), 7.24 (s, 1H), 7.19 (s, 1H), 7.11 - 7.06 (m, 2H), 6.60 (d, J = 6.6 Hz, 1H), 4.66 (s, 2H), 4.14 (s, 2H), 3.93 (s, 3H), 3.59 (d, J = 12.9 Hz, 2H), 2.67 - 2.66 (m, 4H), 2.52 (s, 3H), 2.29 (s, 2H), 2.13 (s, 3H), 1.99 (s, 3H), 1.95 (s, 3H), 1.50 (s, 2H).

**Compound Z-8** can be obtained by referring to the preparation method of steps 8-9 of Example 10, except that compound **10h** is replaced by compound **7e.** Compound **Z-9** can be obtained by referring to the preparation method of steps 8-9 of Example 10, except that compound **10h** is replaced by compound **7e,** and compound **10j** is replaced by compound 17a. Compound **Z-11** can be obtained by referring to the preparation method of steps 8-9 of Example 10, except that compound **10h** is replaced by compound **7e,** and (R)-pyrrolidine-3-carboxylic acid is replaced by (R)-pyrrolidine-2-carboxylic acid. Compound **Z-12** can be obtained by referring to the preparation method of steps 8-9 of Example 10 , except that compound **10h** is replaced by compound **7e,** and compound **10j** is replaced by compound 52a. Compound **Z-13** can be obtained by referring to the preparation method of Example 7, except that 4-bromo-5-chloro-2-methylbenzaldehyde is replaced by 4-bromo-2-methoxy-5-trifluoromethylbenzaldehyde, and (R)-piperidine-2-carboxylic acid is replaced by (R)-pyrrolidine-3-carboxylic acid. Compound **Z-14** can be obtained by referring to the preparation method of steps 8-9 of Example 10, except that compound **10h** is replaced by compound **7e,** and compound **10j** is replaced by compound 49a.

| Example number | structure | MS or ¹H NMR |
|---|---|---|
| 8 | 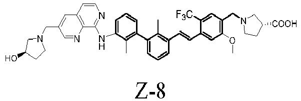 | MS m/z (ESI):752.4 [M+1] 1H NMR (400 MHz, DMSO-d6) δ 9.31 (s, 1H), 8.86 (s, 1H), 8.42 (d, 1H), 8.17 (d, 1H), 8.05 (s, 1H), 7.67-7.59 (m, 3H), 7.51 (s, 1H), 7.38-7.27 (m, 3H), 7.18-7.12 (m, 2H), 6.87 (d, 1H), 4.72 (brs, 1H), 4.25-4.20 (m, 1H), 3.97 (s,3H), 3.86-3.77 (m, 2H), 3.68 - 3.58 (m, 2H), 2.97-2.93 (m, 1H), 2.71 - 2.69 (m, 4H), 2.57-2.53 (m, 2H), 2.47-2.42 (m,1H), 2.36-2.32 (m, 1H), 2.17 (s, 3H), 2.08 (s, 3H), 1.98-1.92 (m, 3H), 1.59-1.56 (m, 1H). |
| 9 | 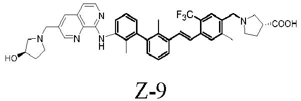 | MS m/z (ESI):736.1 [M+1] ¹H NMR (400 MHz, DMSO-d6) δ 9.31 (s, 1H), 8.86 (d, 1H), 8.42 (s, 1H), 8.19 (s, 2H), 8.06 (d, 1H), 7.87 (s, 1H), 7.63 (s, 2H), 7.59-7.56 (m, 1H), 7.36-7.30 (m, 2H), 7.25 (d, 1H), 7.18 (d, 1H), 7.13 (d, 1H), 6.88 (d, 1H), 6.55 (brs, 1H), 4.22 (s, 1H), 3.83-3.77 (m, 2H), 3.64 - 3.58 (m, 2H), 2.97-2.93 (m, 2H), 2.71 - 2.69 (m, 5H), 2.57-2.53 (m, 1H), 2.48 (s, 3H), 2.36-2.32 (m, 1H), 2.16 (s, 3H), 2.08 (s, 3H), 1.98-1.92 (m, 4H). |
| 11 | 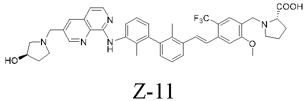 | MS m/z (ESI): 752.3 [M+1] 1H NMR (400 MHz, CD3OD) δ 8.85 (d, J = 4.0Hz, 1H), 8.12 - 8.07 (m, 2H), 7.91 (d, J = 4.0Hz, 1H), 7.79 (s, 1H), 7.65 - 7.55 (m, 2H), 7.52 (s, 1H), 7.33 - 7.27 (m, 3H), 7.15 (d, J = 8.0Hz, 1H), 7.05 (d, J = 8.0Hz, 1H), 6.95 (d, J = 8.0Hz, 1H), 4.59 - 4.46 (m, 2H), 4.40 - 4.36 (m, 2H), 4.09 (s, 3H), 3.92 - 3.82 (m, 3H), 3.60 - 3.55 (m, 1H), 3.22 - 3.15 (m, 2H), 2.84 - 2.80 (m, 2H), 2.60 - 2.54 (m, 2H) , 2.46 - 2.39 (m, 1H), 2.20 - 2.08 (m, 7H) , 1.94 - 1.73 (m, 3H). |
| 12 | 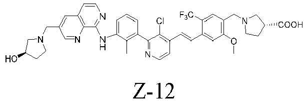 | MS m/z (ESI):773.3 [M+1] 1H NMR (400 MHz, DMSO-d6) δ 9.29 (s, 1H), 8.83 (d, J = 2.0 Hz, 1H), 8.61 (d, J = 5.0 Hz, 1H), 8.38 (d, J = 7.7 Hz, 1H), 8.15 (d, J = 1.8 Hz, 1H), 8.02 (d, J = 5.8 Hz, 1H), 7.80 (d, J = 5.2 Hz, 1H), 7.68 (s, 1H), 7.59 (d, J = 8.9 Hz, 1H), 7.48 (s, 1H), 7.33 (t, J = 7.8 Hz, 1H), 7.15 (d, J = 5.8 Hz, 1H), 6.97 (d, J = 6.7 Hz, 1H), 4.69 (s, 1H), 4.19 (s, 1H), 3.93 (s, 3H), 3.85 - 3.72 (m, 2H), 3.69 - 3.53 (m, 2H), 2.96 - 2.84 (m, 2H), 2.76 - 2.59 (m, 4H), 2.53 (dd, J = 9.7, 5.7 Hz, 1H), 2.39 - 2.28 (m, 2H), 2.07 (s, 3H), 2.01 - 1.89 (m, 3H), 1.56 -1.53 (m, 1H). |
| 13 | 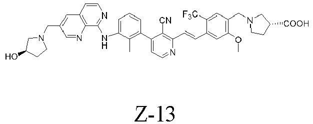 | MS m/z (ESI): 764.4 [M+1] 1H NMR (400 MHz, DMSO-d6) δ 9.35 (s, 1H), 8.93 (d, J = 5.0 Hz, 1H), 8.85 (d, J = 1.9 Hz, 1H), 8.56 - 8.52 (m, 1H), 8.30 (d, J = 15.9 Hz, 1H), 8.17 - 8.16 (m, 2H), 8.04 (d, J = 5.7 Hz, 1H), 7.71 (s, 1H), 7.63- 7.59 (m, 1H), 7.58 - 7.47 (m, 2H), 7.42 -7.38(m, 1H), 7.18 (d, J = 5.7 Hz, 1H), 7.04 (d, J = 7.1 Hz, 1H), 4.27 - 4.11 (m, 1H), 3.94 (s, 3H), 3.87 - 3.71 (m, 2H), 3.71 - 3.57 (m, 4H), 2.91 (s, 1H), 2.75 - 2.59 (m, 4H), 2.53 - 2.50(m, 3H), 2.36- 2.33 (m, 1H), 2.19 (s, 3H), 1.98 - 1.95 (m, 2H), 1.57 - 1.53(m, 1H). |
| 14 | 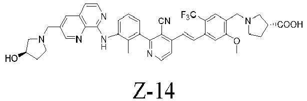 | MS m/z (ESI):764.3 [M+1] 1H NMR (400 MHz, DMSO-d6) δ 9.35 (s, 1H), 8.90 (d, J = 5.4 Hz, 1H), 8.86 (d, J = 1.9 Hz, 1H), 8.47 (d, J = 7.6 Hz, 1H), 8.18 (d, J = 1.7 Hz, 1H), 8.12 (s, 1H), 8.03 (d, J = 5.7 Hz, 1H), 7.96 (d, J = 5.4 Hz, 1H), 7.86 (dd, J = 15.7, 2.1 Hz, 1H), 7.72 (s, 1H), 7.49 (d, J = 15.6 Hz, 2H), 7.38 (t, J = 8.0 Hz, 1H), 7.17 (d, J = 5.8 Hz, 1H), 7.13 (d, J = 6.8 Hz, 1H), 4.24 - 4.17 (m, 1H), 3.94 (s, 3H), 3.83 (d, J = 9.5 Hz, 2H), 3.65 (d, J = 12.1 Hz, 2H), 2.95 - 2.88 (m, 1H), 2.77 - 2.64 (m, 4H), 2.60 - 2.49 (m, 3H), 2.40 (dd, J = 9.7, 3.4 Hz, 1H), 2.16 (s, 3H), 2.04 - 1.91 (m, 3H), 1.56 (dd, J = 8.3, 5.0 Hz, 1H). |

### Example 18:

### (R)-1-((7-cyano-2-(3'-((E)-4-(((R)-3-hydroxypyrrolidin-1-yl)methyl)-5-methyl-2-(trifluoromethyl)styryl)-2,2'-dimethyl-[1,1'-biphenyl]-3-yl)benzo[d]oxazol-5-yl)methyl)pyrrolidine-3-carboxylic acid

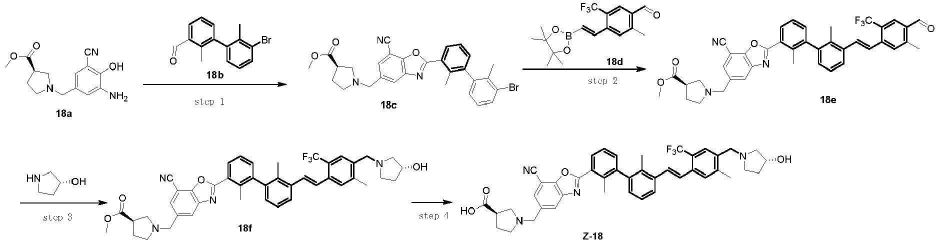

Step 1: Compound **18a** (479 mg, 1.74 mmol) and compound **18b** (540 mg, 1.74 mmol) were dissolved in 20 ml of ethanol, the reaction solution was stirred at room temperature for 2 hours, and then the solvent was distilled off under reduced pressure. The residue was dissolved in 10 ml of DCM and DDQ (395 mg, 1.74 mmol) was added while stirring, and the reaction solution was stirred and reacted at room temperature for 1 hour. The reaction was concentrated, and the residue was purified by silica gel column chromatography (DCM: methanol = 90: 10) to obtain compound **18c** (550 mg, brown oil) with a yield of 32.4%. MS m/z (ESI): 544.0 [M+1].

Step 2: Compound **18c** (545 mg, 1 mmol) was dissolved in 6 ml of 1,4-dioxane, compound **18d** (410 mg, 1 mmol), [1,1'-bis(diphenylphosphino)ferrocene]dichloropalladium(II) ( 73 mg, 0.1 mmol), potassium carbonate (157 mg, 1.14 mmol) and 2 ml of water were added in sequence. The reaction solution was heated to 100 °C under the protection of argon under microwave and reacted for 1 hour, and filtered to remove solids, and the filtrate was concentrated. The residue was purified by silica gel column chromatography (eluent: DCM: methanol = 90: 10) to obtain compound **18e** (320 mg, brown oil) with a yield of 54.4%. MS m/z (ESI): 678.2 [M+1].

Step 3: Compound **18e** (120 mg, 0.17 mmol) was dissolved in 3 ml DMF, and (R)-pyrrolidin-3-ol (15 mg, 0.17 mmol) was added. The reaction solution was heated to 60 °C and reacted for 1 hour, and then sodium cyanoborohydride (12 mg, 0.17 mmol) was added. After the addition, the reaction continued at 60 °C for 1 hour. The reactant was concentrated to obtain compound **18f** (150 mg, yellow solid) with a yield of 45.5%. MS m/z (ESI): 749.3 [M+1].

Step 4: Compound **18f** (150 mg, 0.2 mmol) was dissolved in 10 ml of tetrahydrofuran, and sodium hydroxide (25 mg, 0.4 mmol) aqueous solution (3 ml) was added dropwise. The reaction solution was stirred and reacted at room temperature for 1 hour. The reactant was concentrated and purified by preparative chromatography to obtain compound **Z-18** (18 mg, white solid) with a yield of 24.5%. MS m/z (ESI): 735.0 [M+1]; ¹H NMR (400 MHz, DMSO-d6 ) δ 8.13 (d, *J* = 6.7 Hz, 1H), 8.09 (s, 1H), 7.89 - 7.80 (m, 2H), 7.59 (t, *J* = 9.2 Hz, 2H), 7.52 (t, *J* = 7.7 Hz, 2H), 7.39 (d, *J* = 6.5 Hz, 1H), 7.34 (t, *J* = 7.7 Hz, 1H), 7.22 (d, *J* = 15.6 Hz, 1H), 7.12 (d, *J* = 7.2 Hz, 1H), 4.68 (s, 1H), 4.18 (s, 1H), 3.72 (m, 2H), 3.58 (m, 2H), 2.91 (m, 2H), 2.72 - 2.57 (m, 4H), 2.52 (m, 2H), 2.41 m, 3H), 2.39 (s, 3H), 2.32 (m, 1H), 2.10 (s, 3H), 2.03 - 1.89 (m, 3H), 1.53 (m, 2H).

### Example 21: (R)-1-((7-cyano-2-(3'-((E)-4-(((R)-3-hydroxypyrrolidin -1-yl)methyl)-5-methoxy-2-(trifluoromethyl)styryl)-2,2'-dimethyt-[1,1'-biphenyl] -3-yl)benzo[d]oxazol-5-yl)methyl) pyrrolidine -3-carboxylic acid

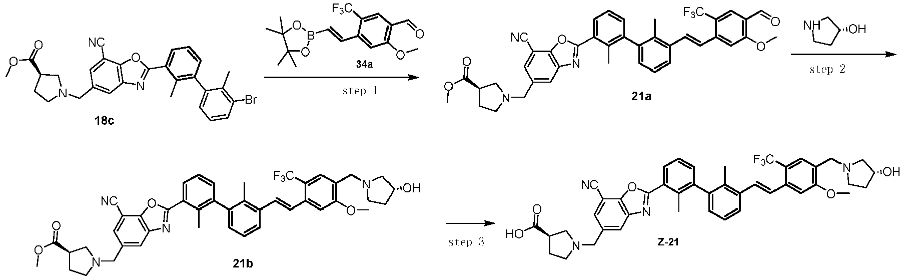

Step 1: Compound **18c** (545 mg, 1 mmol) was dissolved in 6 ml 1,4-dioxane, and compound **34a** (410 mg, 1 mmol), and [1,1'-bis(diphenylphosphino)ferrocene]dichloropalladium(II) (73 mg, 0.1 mmol), potassium carbonate (157 mg, 1.14 mmol) and 2 ml of water were added in sequence. The reaction solution was heated to 100 °C under the protection of argon under microwave, and reacted for 1 hour, the solid was removed by filtration, and the filtrate was concentrated. The residue was purified by silica gel column chromatography (DCM : methanol = 90: 10) to obtain compound **21a** (320 mg, brown oil) with a yield of 54.4%. MS m/z (ESI): 694.2 [M+1].

Step 2: Compound **21a** (120 mg, 0.17 mmol) was dissolved in 3 ml of DMF, and (R)-pyrrolidin-3-ol (30 mg, 0.34 mmol) and sodium cyanoborohydride (12 mg, 0.17 mmol) were added. After the addition, the reaction solution was reacted at 60 °C for 3 hours, and the reactant was concentrated to obtain compound **21b** (150 mg, yellow solid) with a yield of 45.5%. MS m/z (ESI): 764.3 [M+1].

Step 3: Compound **21b** (150 mg, 0.2 mmol) was dissolved in 10 ml of tetrahydrofuran, sodium hydroxide (25 mg, 0.4 mmol) aqueous solution (3 ml) was added dropwise, the reaction solution was stirred and reacted at room temperature for 1 hour, then the reactant was concentrated, and the residue was purified by preparative chromatography to obtain compound **Z-21** (18 mg, white solid) with a yield of 24.5%. MS m/z (ESI):751.4 [M+1]; ¹H NMR (400 MHz, DMSO-*d*₆) δ 8.13 (dd, *J* = 6.1, 1.7 Hz, 1H), 8.08 (d, *J* = 1.2 Hz, 1H), 7.85 (d, *J* = 1.2 Hz, 1H), 7.68 - 7.59 (m, 3H), 7.52 (t, *J* = 7.7 Hz, 1H), 7.48 (s, 1H), 7.38 (d, *J* = 6.6 Hz, 1H), 7.34 (t, *J* = 7.7 Hz, 1H), 7.23 (dd, *J* = 15.8, 2.2 Hz, 1H), 7.12 (d, *J* = 7.1 Hz, 1H), 4.21 - 4.16 (m, 1H), 3.93 (s, 3H), 3.78 - 3.68 (m, 3H), 3.66 - 3.58 (m, 3H), 2.92 (dt, *J* = 15.1, 7.7 Hz, 1H), 2.76 - 2.60 (m, 4H), 2.56 - 2.49 (m, 2H), 2.40 - 2.38 (m, 4H), 2.11 (s, 3H), 2.06 - 1.88 (m, 3H), 1.60 - 1.49 (m, 1H).

### Example 27: (R)-1-((7-carbamoyl-2-(3'-((E)-4-(((R)-3-hydroxypyrrolidin-1-yl)methyl)-5-methoxy-2-(trifluoromethyl)styryl)-2,2'-dimethyl-[1,1'-biphenyl]-3-yl)benzo[d]oxazol-5-yl)methyl)pyrrolidine-3-car boxylic acid

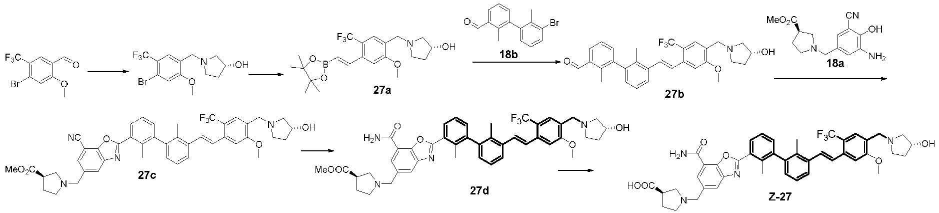

Step 1: 4-bromo-2-methoxy-5-(trifluoromethyl)benzaldehyde (5.0g, 17.8 mmol) was dissolved in 10ml DMF, and (R)-3-pyrrolidinol (2.3g, 26.7 mmol) was added. The reaction solution was heated to 60°C and reacted for 1 hour, and then sodium cyanoborohydride (2.2g, 35.6 mmol) was added. After addition, the reaction was continued for 1 hour at 60 °C. The solid was removed by filtration and the solvent was removed under reduced pressure. The residue was purified by silica gel column chromatography (eluent: DCM: methanol = 90: 10) to obtain (R)-1-(4-bromo-2-methoxy-5-(trifluoromethyl)benzyl)pyrrolidin-3-ol (5.0g, yellow solid) with a yield of 80.6%. MS m/z (ESI): 354.0 [M+1]

Step 2: (R)-1-(4-bromo-2-methoxy-5-(trifluoromethyl)benzyl)pyrrolidin-3-ol (2.1 g, 5.9mmol) was dissolved in 20ml anisole, and tetratriphenylphosphine palladium (681mg, 0.59 mmol), DIEA (1.5g, 11.8 mmol) and vinyl borate pinacol ester (1.5 g, 8.85mmol) were added in sequence. The reaction solution was heated to 150 °C in a microwave reactor under the protection of argon, reacted for 3 hours, and filtered to remove the solids, and the solvent was removed under reduced pressure. The residue was purified by silica gel column chromatography (eluent: ethyl acetate: petroleum ether = 1:3) to obtain compound **27a** (1.1 g, yellow solid) with a yield of 44.0%. MS m/z (ESI): 428.2[M+1].

Step 3: Compound **18b** (442mg, 1.54 mmol) was dissolved in 5ml of 1,4-dioxane, and compound **27a**(550mg**,** 1.28mmol), [1,1'-bis(diphenylphosphino)ferrocene]dichloropalladium(II) (104mg, 0.128mmol), potassium carbonate (353mg, 2.56mmol) and 1ml of water were added in sequence, and the reaction solution was heated to 100 °C in a microwave reactor under the protection of argon, and reacted for 60 minutes. The solid was removed by filtration and the solvent was removed under reduced pressure. The residue was purified by silica gel column chromatography (eluent: petroleum ether: ethyl acetate = 1: 1) to obtain compound **27b** (300mg, brown solid) with a yield of 45.8%. MS m/z (ESI):510.0[M+1].

Step 4: Compound **18a** (244 mg, 0.89 mmol) and compound **27b** (300 mg, 0.59 mmol) were dissolved in 20 ml of tetrahydrofuran, the reaction solution was stirred at room temperature for 2 hours, and then the solvent was distilled off under reduced pressure. The residue was dissolved in 10 ml of DCM and DDQ (134 mg, 0.59 mmol) was added while stirring. The reaction solution was stirred and reacted at room temperature for 1 hour. The reactant was concentrated, and the residue was purified by silica gel column chromatography (eluent: DCM: methanol = 90: 10) to obtain compound **27c** (200 mg, brown solid) with a yield of 44.2%. MS m/z (ESI): 765.4[M+1].

Step 5: Compound **27c** (200mg, 0.26 mmol) was dissolved in 5 ml of ammonia methanol, hydrogen peroxide solution (1ml) was added, the reaction solution was reacted at room temperature for 5 hours, sodium sulfite was added for quenching, the solvent was removed under reduced pressure, and the residue was purified by silica gel column chromatography (eluent: petroleum ether: ethyl acetate = 1: 1) to obtain compound **27d** (60mg, brown solid) with a yield of 30%. MS m/z (ESI):783.3[M+1].

Step 6: Compound **27d** (60mg, 0.076mmol) was dissolved in 5ml methanol and 1ml water, sodium hydroxide (15.2mg, 0.38mmol) was added, the reaction solution was reacted at room temperature for 30 minutes, dilute hydrochloric acid was added for neutralization, the solvent was removed under reduced pressure, the reactant was concentrated and the residue was purified by preparative chromatography to obtain compound **Z-27** (1.8 mg, white solid) with a yield of 3.08%. MS m/z (ESI): 769.3 [M+1].

### Example 34: (R)-1-((7-cyano-2-(3'-((E)-4-((((S)-1-hydroxypropan-2-yl)amino)methyl)-5-methoxy -2-(triftuoromethyt)styryl)-2,2'-dimethyl-[1,1'-biphenyl]-3-yl)benzo[d]oxazol-5-yl)methyl)pyrrolidine-3-carboxylic acid

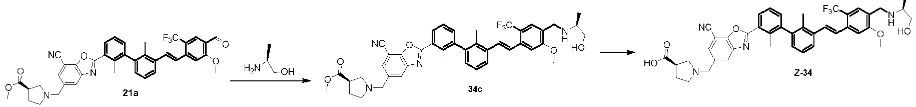

Step 1: Compound **21a** (120 mg, 0.17 mmol) was dissolved in 3 ml DMF, (S)-2-aminopropan-1-ol (30 mg, 0.17 mmol) was added, the reaction solution was heated to 60 °C and reacted for 1 hour, then sodium cyanoborohydride (22 mg, 0.34 mmol) was added. After addition, the reaction was continued at 60 °C for 1 hour. The solid was removed by filtration. The reactant was concentrated and the residue was purified by preparative chromatography to obtain compound **34c** (60 mg, yellow solid) with a yield of 45.5%. MS m/z (ESI): 753.3 [M+1]

Step 2: Compound **34c** (60 mg, 0.08 mmol) was dissolved in 6 ml tetrahydrofuran, sodium hydroxide (30 mg, 0.2 mmol) aqueous solution (2 ml) was added dropwise, the reaction solution was stirred and reacted at room temperature for 1 hour, then concentrated and purified by preparative chromatography to obtain compound **Z-34** (12 mg, white solid) with a yield of 24.5%. MS m/z (ESI): 739.3 [M+1]; ¹H NMR (400 MHz, DMSO-d6 ) δ 8.13 (d, J = 7.4 Hz, 1H), 8.08 (s, 1H), 7.84 (s, 1H), 7.69 (s, 1H), 7.62 (d, J = 8.1 Hz, 1H), 7.60 (s, 1H), 7.52 (t, J = 7.8 Hz, 1H), 7.47 (s, 1H), 7.38 (d, J = 6.7 Hz, 1H), 7.34 (t, J = 7.8 Hz, 1H), 7.23 (d, J = 15.4 Hz, 1H), 7.12 (d, J = 7.1 Hz, 1H), 3.94 (s, 3H), 3.77 - 3.63 (m, 4H), 3.23 (m, 2H), 2.81 (s, 2H), 2.72 - 2.61 (m, 2H), 2.61 - 2.53 (m, 2H), 2.40 (s, 3H), 2.11 (s, 3H), 1.91 (m, 2H), 0.91 (d, J = 6.3 Hz, 3H).

### Example 36: (4-((E)-2-(3'-(7-cyano-5-(((R)-3-hydroxypyrrolidin-1-yl)methyl)benzo[d]oxazol-2-yl) -2,2'-dimethyl-[1,1'-biphenyl]-3-yl)vinyl)-2-methoxy-5-(trifluoromethyl)benzyl)-L-proline

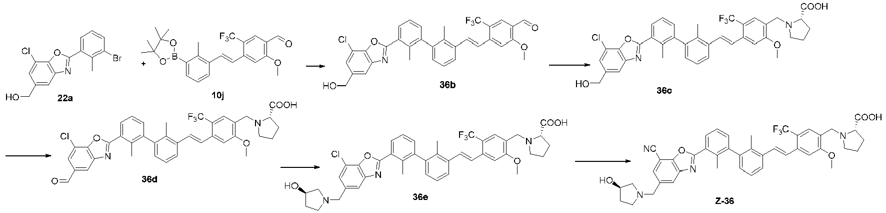

Step 1: Compound **22a** (500mg, 1.42 mmol) was dissolved in 5ml 1,4-dioxane, and compound **10j**(633mg, 1.42mmol), [1,1'-bis(diphenylphosphino)ferrocene]dichloropalladium(II) (115mg, 0.142mmol), potassium carbonate (392mg, 2.84mmol) and 1.5ml of water were added in sequence. The reaction solution was heated to 100 °C under the protection of argon, and reacted for 1 hour. The solids were removed by filtration and the solvent was removed under reduced pressure. The residue was purified by silica gel column chromatography (eluent: DCM: methanol = 20:1) to obtain compound **36b** (294mg, yellow solid) with a yield of 34%. MS m/z (ESI):592.0[M+1].

Step 2: Compound **36b** (140mg, 0.24 mmol) was dissolved in 3ml of DMF, and proline (41.7mg, 0.48 mmol) was added. The reaction solution was heated to 60 °C, and reacted for 1 hour, and sodium cyanoborohydride (15mg, 0.24 mmol) was added. After the addition, the reaction was continued at 60 °C for 1 hour, the solid was removed by filtration, and the solvent was removed under reduced pressure. The residue was purified by silica gel column chromatography (eluent: DCM: methanol = 90: 10) to obtain compound **36c** (116 mg, yellow solid) with a yield of 70 %. MS m/z (ESI): 691.2 [M+1].

Step 3: Compound **36c** (116mg, 0.17 mmol) was dissolved in 5 ml of DCM, and Dess-Martin oxidant (360mg, 0.85 mmol) was added. The reaction solution was reacted at room temperature for 1 hour,quenched with sodium bicarbonate solution, extracted with ethyl acetate (30^{∗}2ml), and dried over anhydrous sodium sulfate. The solid was removed by filtration, and the solvent was removed under reduced pressure. The residue was purified by silica gel column chromatography (eluent: DCM: methanol = 90: 10) to obtain compound **36d** (101 mg, yellow solid) with a yield of 87%. MS m/z (ESI): 689.1[M+1].

Step 4: Compound **36d** (101mg, 0.15 mmol) was dissolved in 3ml DMF, and (R)-3-pyrrolidinol (26.1mg, 0.3 mmol) was added. The reaction solution was heated to 60°C and reacted for 1 hour, and then sodium cyanoborohydride (9.3mg, 0.15 mmol) was added. After the addition, the reaction was continued at 60 °C for 1 hour, the solid was filtered off, the solvent was removed under reduced pressure, and the residue was purified by silica gel column chromatography (eluent: DCM: methanol = 90: 10) to obtain compound **36e** (76mg, yellow solid) with a yield of 68%. MS m/z (ESI): 760.3 [M+1].

Step 5: Compound **36e** (76mg, 0.1 mmol) was dissolved in 2ml of 1,4-dioxane, and potassium ferrocyanide trihyrate (63.3mg, 0.15mmol), methanesulfonato(2-di-t-butylphosphino-2',4',6'-tri-i-propyl-1,1'-biphenyl) (2'-amino-1,1'-biphenyl-2-yl)palladium(II) (7.9mg, 0.01mmol), potassium acetate(19.6mg, 0.2 mmol) and 2 ml of water were added in sequence. The reaction solution was heated to 100 °C under the protection of argon, and reacted for 1 hour. The solid was removed by filtration and the solvent was removed under reduced pressure. The residue was purified by silica gel column chromatography (eluent: DCM: methanol = 20:1) to obtain compound **Z-36** (2.7mg, yellow solid) with a yield of 3.6%. MS m/z (ESI):751.4[M+1].

### Example 41: (S) -1-(4-((E)-2-(3'-(7-cyano-5-((((S)-1-hydroxypropan-2-yl)amino)methyl) benzo[d]oxazol-2-yl)-2,2'-dimethyl-[1,1'-biphenyl]-3-yl)vinyl)-2-methoxy-5-(trifluoromethyl)benzyl) piperidine-2-carboxylic acid

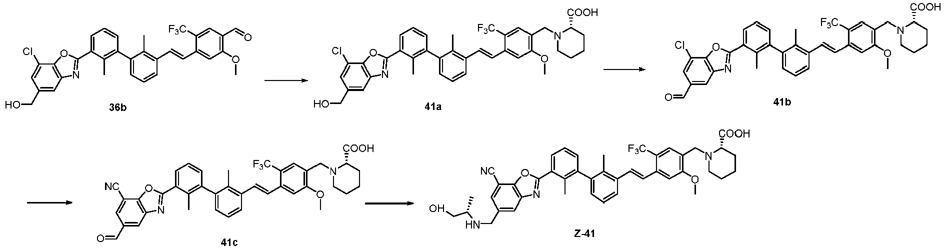

Step 1: Compound **36b** (130mg, 0.22 mmol) was dissolved in 3ml DMF, and L-piperidine-2-carboxylic acid (57mg, 0.22 mmol) was added. The reaction solution was heated to 60 °C and reacted for 1 hour, and then sodium cyanoborohydride (13.6mg, 0.22 mmol) was added. After the addition, the reaction was continued at 60 °C for 1 hour, the solid was removed by filtration, and the solvent was removed under reduced pressure. The residue was purified by silica gel column chromatography (eluent: DCM: methanol = 90: 10) to obtain compound **41a** (112mg, yellow solid) with a yield of 72%. MS m/z (ESI): 705.0 [M+1].

Step 2: Compound **41a** (110mg, 0.16 mmol) was dissolved in 5ml DCM, and Dess-Martin oxidant (340mg, 0.80mmol) was added. The reaction solution was reacted at room temperature for 1 hour, quenched with sodium bicarbonate solution, extracted with ethyl acetate (30^{∗}2ml), and dried over anhydrous sodium sulfate. The solid was removed by filtration, the solvent was removed under reduced pressure, and the residue was purified by silica gel column chromatography (eluent: DCM: methanol = 90: 10) to obtain compound **41b** (95 mg, yellow solid) with a yield of 86%. MS m/z (ESI):703.0M+1].

Step 3: Compound **41b** (95mg, 0.14 mmol) was dissolved in 2ml 1,4-dioxane, and potassium ferrocyanide trihyrate (159mg, 0.20mmol), methanesulfonato(2-di-t-butylphosphino-2',4',6'-tri-i-propyl-1,1'-biphenyl) (2'-amino-1,1'-biphenyl-2-yl)palladium(II) (11mg, 0.014mmol), potassium acetate(27mg,0.28mmol) and 2 ml of water were added in sequence. The reaction solution was heated to 100 °C under the protection of argon, and reacted for 1 hour. The solid was filtered off, and the solvent was removed under reduced pressure. The residue was purified by silica gel column chromatography (eluent: DCM: methanol = 20:1) to obtain compound **41c** (60mg, yellow solid) with a yield of 63.8%. MS m/z (ESI):694.3[M+1].

Step 4: Compound **41c** (60mg, 0.086 mmol) was dissolved in 3ml DMF, and L-aminopropanol (26.1mg, 0.3 mmol) was added. The reaction solution was heated to 60°C and reacted for 1 hour, and then sodium cyanoborohydride (9.3mg, 0.15 mmol) was added. After the addition, the reaction was continued at 60 °C for 1 hour, the solids were removed by filtration, the solvent was removed under reduced pressure, and the reactants were concentrated. The residue was purified by preparative chromatography to obtain compound **Z-41** (3.1 mg, white solid) with a yield of 4.8%. MS m/z (ESI): 753.4 [M+1]; ¹H NMR (400 MHz, DMSO-d6 ) δ 8.39 (s, 1H), 8.14 (s, 1H), 7.91 (s, 1H), 7.75 (s, 1H), 7.64 - 7.58 (m, 2H), 7.52 (t, J = 7.8 Hz, 1H), 7.46 (s, 1H), 7.40 - 7.31 (m, 2H), 7.25 (s, 1H), 7.12 (d, J = 7.1 Hz, 1H), 3.91 (s, 3H), 3.87 (d, J = 13.3 Hz, 2H), 3.82 - 3.72 (m, 2H), 3.60 - 3.58 (m, 2H), 3.25 - 3.05 (m, 4H), 2.86 - 2.85 (m, 1H), 2.57 - 2.55(m, 1H), 2.40 (s, 3H), 2.11 (s, 3H), 1.73 (s, 2H), 1.46 (s, 2H), 1.26 (m, 1H), 0.92 (d, J = 6.3 Hz, 3H).

### Example 53: (R)-1-((8-cyano-2-(3'-((E)-4-(((R)-3-hydroxypyrrolidin-1-yl)methyl)-5-methoxy-2-(trifluoromethyl)styryl)-2,2'-dimethyl-[1,1'-biphenyl]-3-yl)-[1,2,4]triazolo[1,5-a]pyridin-6-yl)methyl) pyrrolidine-3-carboxylic acid

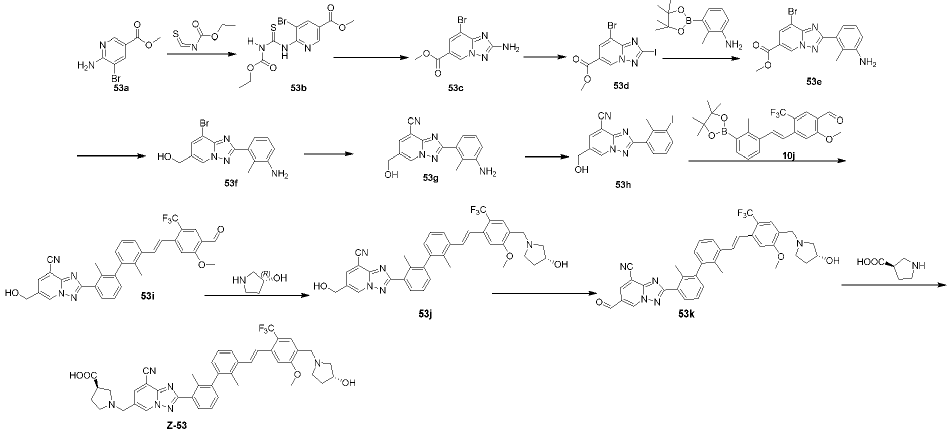

Step 1: Compound methyl 6-amino-5-bromonicotinate **53a** (3.3 g, 14.28 mmol) was dissolved in anhydrous 1,4-dioxane (30 mL), and O-ethyl carbonisothiocyanatidate (4.4 g, 33.84 mmol, 4 mL) was added dropwise under argon protection. The reaction solution was stirred at room temperature for 16 hours. The reaction solution was concentrated to dryness to obtain compound **53b** (4 g, pale yellow solid). MS m/z (ESI): 363.9 [M+1].

Step 2: Compound **53b** (4g, 11.04 mmol) was dissolved in anhydrous methanol (40 mL), and hydroxylamine hydrochloride (3.8 g, 55.2 mmol) and DIEA (4.3 g, 33.12 mmol) were added. The reaction solution was stirred at room temperature for 12 hours under argon protection. A solid was formed in the reaction solution. After the solid was filtered out, the solid was added to anhydrous methanol (30 mL) and heated under reflux for 12 hours. After cooling to room temperature, the solid was filtered and dried to obtain compound **53c** (3.1 g, white solid). MS m/z (ESI):273.0 [M+1].

Step 3: Compound **53c** (1 g, 3.69 mmol) and p-toluenesulfonic acid (4 g, 11.04 mmol) were added to acetonitrile (20 mL), potassium iodide (2.45 g, 14.76 mmol) dissolved in water (8 mL) and sodium nitrite (763 mg, 11.07 mmol) aqueous solution were slowly added, and the reaction solution was stirred at room temperature for 1 hour, and then heated to 40°C and stirred for 12 hours. The reaction solution was cooled and neutralized with saturated sodium bicarbonate, and extracted with ethyl acetate (2^{∗}30 ml). The organic phases were combined, washed with saturated brine (40 ml), dried over anhydrous sodium sulfate, and filtered, and the filtrate was concentrated. The residue was purified by silica gel column chromatography (eluent: DCM: methanol = 95: 5) to obtain compound **53d** (545 mg, white solid) with a yield of 38.9%. MS m/z (ESI):381.9 [M+1].

Step 4: Compound **53d** (800 mg, 3.43 mmol) and 2-methyl-3-(4,4,5,5-tetramethyl-1,3,2-dioxaborane-2-yl)aniline (1.04 g, 4.46 mmol) were dissolved in a mixture of 1,4-dioxane (21 mL) and water (7 mL), and potassium carbonate (947 mg, 6.86 mmol) and [1,1'-bis(diphenylphosphino)ferrocene]palladium dichloride (251mg, 0.343 mmol) were added in sequence at room temperature. The reaction solution was replaced with argon three times and heated to 70 °C, stirred and reacted for 3 hours, cooled to room temperature, and extracted with ethyl acetate (2^{∗}60 ml). The organic phases were combined, washed with water (2^{∗}50 ml) and saturated brine (20 ml), dried over anhydrous sodium sulfate, and filtered. The filtrate was concentrated, and the residue was purified by silica gel column chromatography (eluent: petroleum ether: ethyl acetate = 50: 50) to obtain compound **53e** (450 mg, white solid) with a yield of 31.1%. MS m/z (ESI): 363.0 [M+1].

Step 5: Compound **53e** (500 mg, 1.38 mmol) was dissolved in anhydrous DCM (20 mL), and diisobutyl aluminum hydride (1M, 4.15 mL, 4.15 mmol) was added dropwise at -78 °C under argon protection. The reaction solution was reacted at -78°C for 0.5 hours, and then the reaction was continued at 0°C for 1 hour. After the reaction was quenched with 0.15 mL of water, 0.3 mL of 2M/sodium hydroxide was added and stirred for 5 minutes, then 0.15 mL of water was added, and sodium sulfate was finally added and stirred for 0.5 hours. The reaction solution was filtered with celite, the filtrate was concentrated, and the residue was purified by silica gel column chromatography (eluent: DCM: methanol = 95: 5) to obtain compound **53f** (250 mg, white solid) with a yield of 54.8%. MS m/z (ESI): 333.0 [M+1].

Step 6: Compound **53f** (1.8 g, 5.40 mmol) and zinc cyanide (1.58 g, 13.51 mmol) were dissolved in a mixture of 1,4-dioxane (30 mL) and water (30 mL). and methanesulfonato(2-di-t-butylphosphino-2',4',6'-tri-i-propyl-1,1'-biphenyl)(2'-amino-1,1'-biphenyl-2-yl)palladium(II) potassium carbonate (643 mg, 0.81 mmol) was added at room temperature. The reaction solution was replaced with argon for three times, heated to 90°C, stirred and reacted for 12 hours and then cooled to room temperature. The reaction solution was concentrated, and the residue was purified by silica gel column chromatography (eluent: DCM: methanol = 90: 10) to obtain compound **53g** (1.3 g, brown solid) with a yield of 86%. MS m/z (ESI): 280.0 [M+1].

Step 7: Compound **53g** (50 mg, 0.18 mmol) and p-toluenesulfonic acid (142 mg, 0.82 mmol) were added to acetonitrile (4 mL), and sodium iodide (119 mg, 0.72 mmol) dissolved in water (1.5 mL) and sodium nitrite (37 mg, 0.54 mmol) aqueous solution were slowly added. The reaction solution was stirred at room temperature for 1 hour, then heated to 40°C and stirred for 12 hours. After cooling, the reaction solution was neutralized with saturated sodium bicarbonate and extracted with ethyl acetate (30 ml). The organic phases were combined, washed with saturated brine (10 ml), dried over anhydrous sodium sulfate, and filtered, and the filtrate was concentrated. The residue was purified by silica gel column chromatography (eluent: DCM: methanol = 95: 5) to obtain compound **53h** (50 mg, brown solid) with a yield of 71.6%. MS m/z (ESI):390.9 [M+1].

Step 8: Compounds **53h** (70 mg, 0.18 mmol) and **10j** (78 mg, 0.19 mmol) were dissolved in a mixture of 1,4-dioxane (6 mL) and water (2 mL), and potassium carbonate (62 mg, 0.45 mmol) and [1,1'-bis(diphenylphosphino)ferrocene]palladium dichloride (14 mg, 0.02 mmol) were added in sequence at room temperature. The reaction solution was replaced with argon three times and heated to 100 °C under an oil bath, stirred and reacted for 6 hours, then cooled to room temperature, and concentrated. The residue was purified by silica gel column chromatography (eluent: petroleum ether: ethyl acetate = 30: 70) to obtain compound **53i** (40 mg, brown solid) with a yield of 38.5%. MS m/z (ESI): 583.2 [M+1].

Step 9: Compound **53i** (30 mg, 0.05 mmol) and (R)-pyrrolidin-3-ol (14 mg, 0.15 mmol) were dissolved in methanol (5 mL), and the reaction solution was heated at 60 °C for 10 minutes. Sodium cyanoborohydride (10 mg, 0.15 mmol) was added in batchs, and the reaction solution was reacted at 60 °C for 2 hours, and concentrated. The residue was purified by preparative chromatography (alkaline method) to obtain compound **53j** (20 mg, white solid) with a yield of 59.5%. MS m/z (ESI): 654.3 [M+1].

Step 10: Compound **53j** (20 mg, 0.03 mmol) was dissolved in anhydrous DCM (5 mL), and active manganese dioxide (87 mg, 0.60 mmol) was added at room temperature. The reaction solution was stirred at room temperature for 6 hours, and diluted with DCM. After filtration through celite, the filtrate was concentrated to obtain compound **53k** (11.5 mg, white solid). MS m/z (ESI): 652.3 [M+1].

Step 11: Compound **53k** (10 mg, 0.015 mmol) and (R)-pyrrolidine-3-carboxylic acid (6 mg, 0.050 mmol) were dissolved in methanol (2 mL), and the reaction solution was heated at 60 °C for 10 minutes. Sodium cyanoborohydride (3 mg, 0.05 mmol) was added in batchs, and the reaction solution was reacted at 60 °C for 2 hours, and concentrated. The residue was purified by preparative chromatography (alkaline method) to obtain compound **Z-53** (2.9 mg, white solid) with a yield of 25%. MS m/z (ESI): 751.4 [M+1].

### Example 54: (R)-1-(2-methoxy-4-((E)-2-(3'-(6-methoxy-5-(((((S)-5-oxopyrrolidin-2-yl)methyl)amino) methyl)pyridin-2-yl)-2,2'-dimethyl-[1,1'-biphenyl]-3-yl)vinyl)-5-(trifluoromethyl)benzyl)pyrrolidine-3-ca rboxylic acid

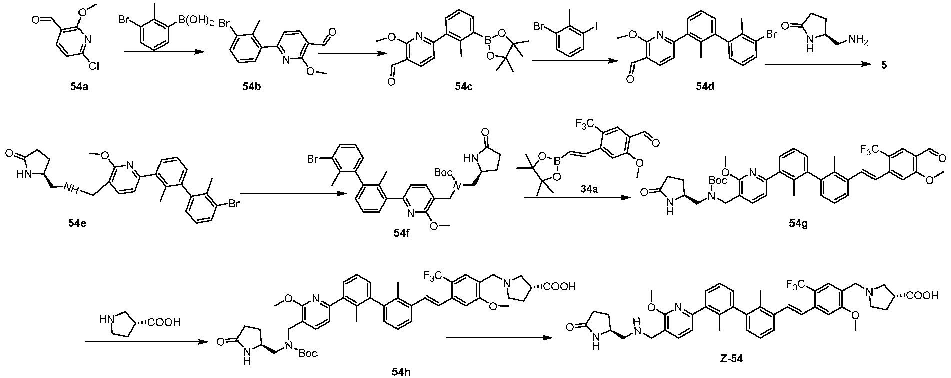

Step 1: Compound 6-chloro-2-methoxynicotinaldehyde **54a** (1.76 g, 10.26 mmol) and (3-bromo-2-methylphenyl)boronic acid (2.0 g, 9.34 mmol) were dissolved in a mixture of 1,4-dioxane (100 mL) and water (10 mL), and potassium carbonate (2.58 g, 20.52 mmol) and tetrakis(triphenylphosphine)palladium (0.54 g, 0.46 mmol) were added in sequence at room temperature. The reaction solution was replaced with argon three times and heated to 95°C, stirred and reacted for 3 hours and then cooled to room temperature, and extracted with ethyl acetate (2^{∗}60 ml). The organic phases were combined, washed with water (2^{∗}50 ml) and saturated brine (30 ml), dried over anhydrous sodium sulfate, and filtered. The filtrate was concentrated, and the residue was purified by silica gel column chromatography (eluent: petroleum ether: ethyl acetate = 85: 15) to obtain compound **54b** (2.5 g, white solid) with a yield of 81%. MS m/z (ESI): 305.9 [M+1].

Step 2: Compound **54b** (2.4 g, 7.84 mmol) and bis(pinacolato)diboron (2.39 g, 9.40 mmol) were dissolved in anhydrous 1,4-dioxane (70 mL), and potassium acetate (2.15 g, 21.95 mmol) and [1,1'-bis(diphenylphosphino)ferrocene]palladium dichloride (0.57 g, 0.78 mmol) were added in sequence at room temperature. The reaction solution was replaced with argon three times, heated to 95 °C, stirred and reacted for 5 hours, cooled to room temperature, and extracted with ethyl acetate (2 ^{∗}60 ml). The organic phases were combined, washed with water (2^{∗}50 ml) and saturated brine (40 ml), dried over anhydrous sodium sulfate, and filtered. The filtrate was concentrated, and the residue was purified by silica gel column chromatography (eluent: petroleum ether: ethyl acetate = 92: 8) to obtain compound **54c** (2.0 g, white solid) with a yield of 72.5%. MS m/z (ESI): 354.1 [M+1].

Step 3: Compound **54c** (2 g, 5.66 mmol) and 1-bromo-3-iodo-2-methylbenzene (2.0 g, 6.79 mmol) were dissolved in a mixture of 1,4-dioxane (60 mL) and water (20 mL), and potassium carbonate (2.58 g, 20.52 mmol) and [1,1'-bis(diphenylphosphino)ferrocene]palladium dichloride (0.62 g, 0.84 mmol) were added in sequence at room temperature. The reaction solution was replaced with argon three time, heated to 80 °C, and stirred and reacted for 5 hours. After cooling to room temperature, the reaction solution was extracted with ethyl acetate (2^{∗}80 ml). The organic phases were combined, washed with saturated brine (30 ml), dried over anhydrous sodium sulfate, and filtered. The filtrate was concentrated, and the residue was purified by silica gel column chromatography (eluent: petroleum ether: ethyl acetate = 90: 10) to obtain compound **54d** (1.6 g, yellow oil) with a yield of 73%. MS m/z (ESI): 396.0 [M+1].

Step 4: Compound **54d** (0.8 g, 2.02 mmol) and (S)-5-(aminomethyl)pyrrolidin-2-one (0.58 g, 5.06 mmol) were dissolved in methanol (40 mL). After stirring for 10 minutes at 60 °C, sodium cyanoborohydride (0.5 g, 8.08 mmol) was added in batches, and the reaction solution was stirred and reacted at 60 °C for 4 hours and then cooled to room temperature. The reaction solution was concentrated, and the residue was purified by silica gel column chromatography (eluent: DCM: methanol = 90: 10) to obtain compound **54e** (0.85 g, brown solid) with a yield of 73%. MS m/z (ESI): 494.1 [M+1].

Step 5: Compound **54e** (130 mg, 0.26 mmol) was dissolved in anhydrous methanol (4 mL), TEA (53 mg, 0.56 mmol) and di-tert-butyl dicarbonate (86 mg, 0.39 mmol) were added. The reaction solution was stirred and reacted at room temperature for 2 hours, and concentrated to give compound **54f** (150 mg, yellow oil). MS m/z (ESI): 594.2 [M+1].

Step 6: Compound **54f** (150 mg, 0.25 mmol) and compound **34a** (135 mg, 0.38 mmol) were dissolved in a mixture of 1,4-dioxane (5 mL) and water (1.5 mL), and potassium carbonate (70 mg, 0.50 mmol) and [1,1'-bis(diphenylphosphino)ferrocene]palladium dichloride (37 mg, 0.05 mmol) were added in sequence at room temperature. The reaction solution was replaced with argon three times, heated to 100 °C under microwave, stirred and reacted for 1 hour, then cooled to room temperature, filtered, and concentrated to obtain compound **54g** (200 mg, yellow oil). MS m/z (ESI): 744.3[M+1].

Step 7: Compound **54g** (117 mg, 0.15 mmol) and (R)-pyrrolidine-3-carboxylic acid (60 mg, 0.50 mmol) were dissolved in methanol (20 mL), and the reaction solution was heated at 60 °C for 10 minutes. Sodium cyanoborohydride (30 mg, 0.5 mmol) was added in batchs, and the reaction solution was reacted at 60 °C for 2 hours, and concentrated. The residue was purified by preparative chromatography (alkaline method) to obtain compound **54h** (56 mg) with a yield of 45%. MS m/z (ESI): 843.4 [M+1].

Step 8: Compound **54h** (100 mg, 0.12 mmol) was dissolved in anhydrous DCM (5 mL), and trifluoroacetic acid (0.3 mL) was added. The reaction solution was stirred overnight at room temperature. After the reaction solution was concentrated, the residue was purified by preparative chromatography (alkaline method) to obtain compound **Z-54** (61 mg, white solid) with a yield of 69.7%. MS m/z (ESI): 743.3 [M+1]; ¹H NMR (400 MHz, DMSO-d6) δ 7.80 (d, J = 7.4 Hz, 1H), 7.73 - 7.56 (m, 4H), 7.50 (s, 1H), 7.43 (dd, J = 7.8, 1.5 Hz, 1H), 7.34 (td, J = 7.6, 5.1 Hz, 2H), 7.24 (dt, J = 15.8, 2.5 Hz, 1H), 7.18 - 7.10 (m, 3H), 3.96 (s, 3H), 3.89 (s, 3H), 3.75 - 3.49 (m, 5H), 2.94 (p, J = 7.4 Hz, 1H), 2.77 - 2.62 (m, 2H), 2.56 -2.53(m, 4H), 2.23 - 1.89 (m, 11H), 1.78 - 1.60 (m, 1H).

### Example 59: (4-((E)-2-(3'-(6-(((R)-3-hydroxypyrrolidin-1-yl)methyl)-1-oxoisoquinolin-2(1H)-yl) -2,2'-dimethyl-[1,1'-biphenyl]-3-yl)vinyl)-2-methoxy-5-(trifluoromethyl)benzyl)-L-proline

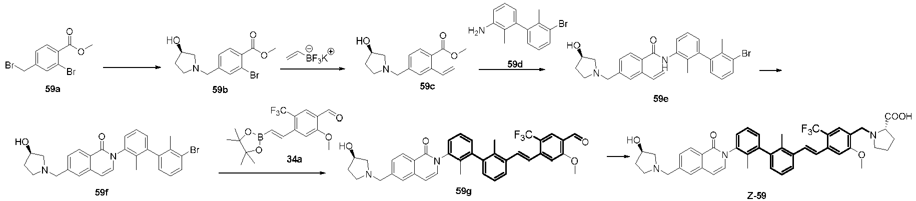

Step 1: Compound methyl 2-bromo-4-bromomethyl benzoate **59a** (2g, 6.6mmol) was dissolved in 5ml of acetonitrile, and (R)-3-pyrrolidinol (861.3mg, 9.9mmol) was added. The reaction solution was reacted at room temperature for 2 hours, filtered to remove solids, extracted with ethyl acetate (30^{∗}3ml), and dried over anhydrous sodium sulfate. The solvent was removed under reduced pressure, and the residue was purified by silica gel column chromatography (eluent: petroleum ether: ethyl acetate = 5:1) to obtain compound **59b** (1.4g, yellow oil) with a yield of 68%. MS m/z (ESI): 314.0 [M+1].

Step 2: Compound **59b** (1.4g, 4.5 mmol) was dissolved in 5ml 1,4-dioxane, and potassium vinyl trifluoroborate (911mg, 6.75mmol), [1,1'-bis(diphenylphosphino)ferrocene]dichloropalladium(II) (367mg, 0.45mmol), potassium carbonate (1.24g, 9.0mmol) and 1ml of water were added in sequence. The reaction solution was heated to 100 °C under the protection of argon, and reacted for 3 hours. The solid was removed by filtration and the solvent was removed under reduced pressure. The residue was purified by silica gel column chromatography (eluent: DCM: methanol = 20:1) to obtain compound **59c** (780mg, yellow solid) with a yield of 69.6%. MS m/z (ESI):262.0[M+1].

Step 3: Compound **59c** (780mg, 3.0mmol) and compound **59d** (825 mg, 3.0mmol) were dissolved in 10ml anhydrous tetrahydrofuran, and potassium tert-butoxide in tetrahydrofuran (1.0 M, 6ml) was added dropwise at -20 °C. After addition, the reaction solution was slowly warmed to room temperature, and stirred and reacted for 30 minutes. The solvent was removed under reduced pressure, and the residue was purified by silica gel column chromatography (eluent: DCM: methanol = 90: 10) to obtain compound **59e** (1.0g, white solid) with a yield of 67%. MS m/z (ESI): 505.0[M+1].

Step 4: Compound **59e** (300mg, 0.6 mmol) was dissolved in 10ml methanol, and bis(acetonitrile)dichloropalladium(II) (15.5mg, 0.06mmol) and copper chloride (8.04mg, 0.06mmol) were added in sequence. The reaction solution was heated to 60 °C and reacted for 8 hours, filtered to remove the solid, and the solvent was removed under reduced pressure. The residue was purified by silica gel column chromatography (eluent: DCM: methanol = 20:1) to obtain compound **59f** (220mg, yellow liquid) with a yield of MS m/z (ESI):503.1[M+1].

Step 5: Compound **59f** (200mg, 0.40 mmol) was dissolved in 3ml 1,4-dioxane, and compound **34a**(171mg, 0.48mmol), [1,1'-bis(diphenylphosphino)ferrocene]dichloropalladium(II) (32.6mg, 0.04mmol), potassium carbonate (110mg, 0.8mmol) and 1ml of water were added in sequence. The reaction solution was heated to 80 °C in a microwave reactor under the protection of argon, and reacted for 1 hour. The solid was removed by filtration and the solvent was removed under reduced pressure. The residue was purified by silica gel column chromatography (eluent: DCM: methanol = 90:10) to obtain compound **59g** (100mg, brown solid) with a yield of. MS m/z (ESI): 653.2[M+1].

Step 6: Compound **59g** (100mg, 0.15 mmol) was dissolved in 3ml of DMF, and L-proline (34.5mg, 0.3 mmol) was added. The reaction solution was heated to 60°C and reacted for 1 hour, then sodium cyanoborohydride (9.3mg, 0.15 mmol) was added. After the addition, the reaction continued at 60 °C for 1 hour, the solid was removed by filtration, and the solvent was removed under reduced pressure. The reactant was concentrated and the residue was purified by preparative chromatography to obtain compound **Z-59** (5.0 mg, yellow solid) with a yield of 5.0%. MS m/z (ESI): 752.4 [M+1]; ¹H NMR (400 MHz, DMSO-d6) δ 8.21 - 8.18 (m, 3H), 7.74 (s, 1H), 7.64 - 7.58 (m, 3H), 7.51 - 7.45 (m, 2H), 7.42 - 7.36 (m, 2H), 7.35 - 7.33 (m, 2H), 7.28 - 7.25 (m, 1H), 7.15 - 7.10 (m, 1H), 6.72 -6.69 (m, 1H), 5.73 (s, 1H), 4.19 (s, 2H), 4.01 (s, 1H), 3.96 (s, 3H), 3.68 (d, J = 7.8 Hz, 4H), 2.70 - 2.58 (m, 5H), 2.32 - 2.30 (m, 1H), 2.16 - 2.13 (m, 2H), 1.97 - 1.96 (m, 2H), 1.72 (s, 3H), 1.54 (s, 1H), 1.20 (s, 3H), 0.83 - 0.80 (m, 1H).

### Example 60: (R)-1-(2-methoxy-4-((E)-2-(3'-(4-methoxy-6-(((S)-5-oxopyrrolidin-2-yl)methyl)-5,6,7,8-tetrahydropyrido[4,3-d]pyrimidin-2-yl)-2,2'-dimethyl-[1,1'-biphenyl]-3-yl)vinyl)-5-(trifluoromethyl)benz yl)pyrrolidine-3-carboxylic acid

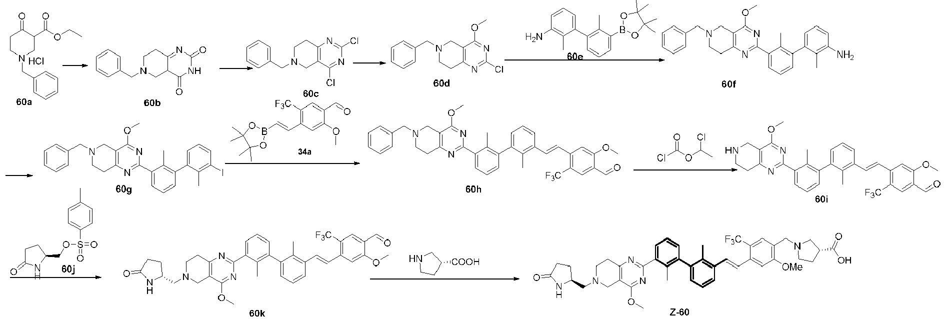

Step 1: Ethyl 1-benzyl-4-oxopiperidine-3-carboxylate hydrochloride **60a** (15 g, 50.37 mmol), urea (15.1 g, 251.86 mmol) and sodium methoxide (13.6 g, 251.86 mmol) were added into 150 mL ethanol, and the reaction solution was refluxed overnight, and concentrated under reduced pressure to obtain compound **60b** (12.3 g, white solid) with a yield of 94%. MS m/z (ESI):258.1 [M+1].

Step 2: Compound **60b** (12.3 g, 47.8 mmol) was dissolved in phosphorus oxychloride (120 ml), and the reaction solution was heated to 90°C, stirred and reacted for 5 hours, cooled to room temperature, and concentrated under reduced pressure. The residue was dissolved in ethyl acetate (150 mL), washed with saturated sodium bicarbonate to neutrality. The residue was purified by column chromatography to obtain compound **60c** (12 g, white solid) with a yield of 85%. MS m/z (ESI):294.0 [M+1].

Step 3: NaH (1.0 g, 25.5 mmol, 60%wt) was added to a solution of methanol (2.18 g, 68 mmol) in tetrahydrofuran (20 mL) in batches at room temperature, and the reaction solution was stirred for half an hour. Then the above solution was added dropwise to a solution of compound **60c**(5 g, 17.0 mmol) in tetrahydrofuran (30 mL), stirred at room temperature for 3 hours, and concentrated under reduced pressure. The residue was purified by column chromatography to obtain compound **60d** (1.0 g, white solid) with a yield of 20%. MS m/z (ESI):290.1 [M+1].

Step 4: Compound **60d** (800 mg, 2.76 mmol), compound **60e** (981 mg, 3.04 mmol), sodium carbonate (877 mg, 8.28 mmol), [1,1'-bis(diphenylphosphino)ferrocene]dichloropalladium(II) (100 mg, 0.14mmol) were dissolved in dioxane (12 mL) and water (3 mL). The reaction mixture was stirred at 80 °C overnight under the protection of nitrogen, cooled to room temperature, and concentrated under reduced pressure. The residue was purified by column chromatography to obtain compound **60f** (1.2 g, white solid) with a yield of 96%. MS m/z (ESI): 451.2 [M+1].

Step 5: Compound **60f** (1.2 g, 2.66 mmol) and p-toluenesulfonic acid monohydrate (1.52 g, 7.99 mmol) were dissolved in 15 mL of a mixed solution of acetonitrile and water (5 mL), and the reaction solution was cooled to 0 °C. NaNO₂( 349 mg, 5.06 mmol) in water (2 mL) was added dropwise, the reaction solution was stirred at 0 °C for half an hour, and KI (1.11 g, 6.66 mmol) in water (3 mL) was added dropwise. The reaction solution was slowly warmed to room temperature and stirred overnight. The reaction solution was diluted with water (20 mL), extracted with ethyl acetate, dried, and concentrated under reduced pressure. The residue was purified by preparative chromatography to obtain compound **60g** (663 mg, white solid) with a yield of 44%. MS m/z (ESI): 562.1 [M+1].

Step 6: Compound **60g** (663 mg, 1.18 mmol), compound **34a,** potassium carbonate (326 mg, 2.36 mmol), and [1,1'-bis(diphenylphosphino)ferrocene]dichloropalladium(II) (43 mg, 0.06 mmol) were dissolved in dioxane (10 mL) and water (3 mL). The reaction solution was replaced with nitrogen three times, heated to 100°C, stirred and reacted overnight, cooled to room temperature, and concentrated under reduced pressure. The residue was purified by preparative chromatography to obtain compound **60h** (750 mg, yellow solid) with a yield of 95%. MS m/z (ESI): 664.3 [M+1].

Step 7: Compound **60h** (650 mg, 0.98 mmol) was dissolved in DCM (10 mL), and 1-chloroethyl carbonochloridate (420 mg, 2.94 mmol) was added. The reaction solution was refluxed for 2 hours, concentrated, and the residue was dissolved in methanol (15 mL), and then reflux for 2 hours. the reaction solution was concentrated, the residue was dissolved in DCM (15 mL), washed with saturated sodium bicarbonate solution (50 mL), dried, concentrated under reduced pressure, and the residue was purified by column chromatography to obtain compound **60i** (470 mg, yellow solid) with a yield of 95%. MS m/z (ESI): 574.2 [M+1].

Step 8: Compound **60i** (200 mg, 0.35 mmol), compound **60j** (141 mg, 0.52 mmol) and potassium carbonate (96 mg, 0.70 mmol) were dissolved in DMF (5 mL), and the reaction solution was heated and stirred at 80 °C for 2 hours. The reaction solution was concentrated under reduced pressure, and the residue was purified by column chromatography to obtain compound **60k** (158 mg, yellow solid) with a yield of 67%. MS m/z (ESI): 671.3 [M+1].

Step 9: A solution of compound **60k** (100 mg, 0.15 mmol) and (R)-pyrrolidine-3-carboxylic acid (26 mg, 0.35 mmol) in methanol was heated to 60 °C, and then sodium cyanoborohydride (56 mg, 0.89 mmol) was added in batches. The reactant was concentrated and the residue was purified by preparative chromatography to obtain compound **Z-60** (33 mg, white solid) with a yield of 29%. MS m/z (ESI): 770.4 [M+1]; ¹H NMR (400 MHz, DMSO-d6) δ 7.69-7.59 (m, 5H), 7.50 (s, 1H), 7.38-7.32 (m, 2H), 7.25 (d, 1H), 7.20 (d, 1H), 7.13 (d, 1H), 3.97 (s, 6H), 3.80-3.77 (m, 1H), 3.61-3.47 (m, 6H), 2.90-2.82 (m, 1H), 2.80-2.75 (m, 4H), 2.73-2.70 (m, 2H), 2.55-2.50 (m, 3H), 2.19-2.13 (m, 8H), 2.10-1.98 (m, 2H), 1.72-1.68 (m, 1H).

### Example 61:(R,E)-1-(5-chloro-4-(2-(3-cyano-4-(3-(5-(dimethylglycyl)-4,5,6,7-tetrahydrothiazolo [5,4-c]pyridin-2-yl)-2-methylphenyl)pyridin-2-yl)vinyl)-2-methylbenzyl)pyrrolidine-3-carboxylic acid

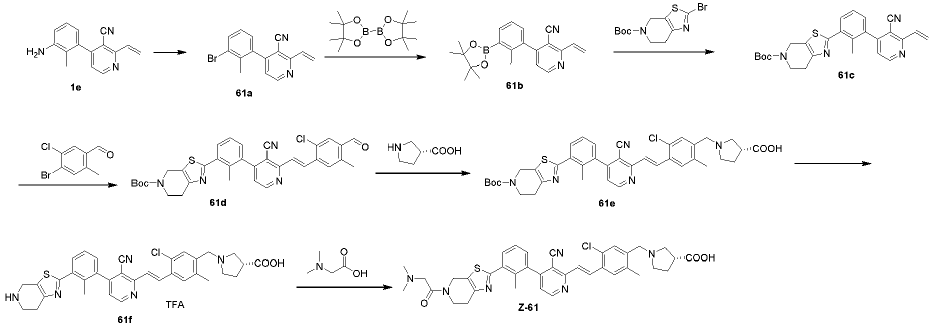

Step 1: Compound **1e** (2.35 g, 10.0 mmol) was dissolved in 50 ml of acetonitrile, and cuprous bromide (2.16 g, 15.0 mmol) was added. The reaction solution was cooled to 0 °C, and isopentyl nitrite (1.76 g, 15.0 mmol) was added dropwise. After the addition, the reaction solution was slowly warmed to room temperature and stirred and reacted overnight. The reaction solution was filtered to remove solids, the filtrate was concentrated, and the residue was purified by silica gel column chromatography (eluent: petroleum ether: ethyl acetate = 90: 10) to obtain compound **61a** (0.85 g, white solid) with a yield of 28.4%. MS m/z (ESI): 298.9 [M+1].

Step 2: Compound **61a** (1.6 g, 5.35 mmol) was dissolved in 30 ml 1,4-dioxane, and bis(pinacolato)diboron (2.72 g, 10.7 mmol), [1,1'-bis(diphenylphosphino)ferrocene]dichloropalladium(II) (395 mg, 0.54 mmol) and potassium acetate (1.05 g, 10.7 mmol) were added. The reaction solution was heated to 100 °C and reacted for 16 hours under the protection of argon, and filtered to remove the solids. The filtrate was concentrated, and the residue was purified by silica gel column chromatography (eluent: petroleum ether: ethyl acetate = 90:10) to obtain compound **61b** (1.02 g, yellow solid) with a yield of 55%. MS m/z (ESI): 347.1 [M+1].

Step 3: Compound **61b** (1.015 g, 2.93 mmol) was dissolved in 10 ml 1,4-dioxane, and tert-butyl 2-bromo-6,7-dihydrothiazolo[5,4-c]pyridine-5(4H)-carboxylate ( 0.94 g, 2.93 mmol), [1,1'-bis(diphenylphosphino)ferrocene]dichloropalladium(II) (107 mg, 0.147 mmol) and potassium carbonate(0.81 g, 5.86 mmol) were added. The reaction solution was heated to 100°C under argon protection and reacted for 16 hours, the solid was removed by filtration, the filtrate was concentrated, and the residue was purified by silica gel column chromatography (eluent: petroleum ether: ethyl acetate = 80:20) to obtain compound **61c** (480 mg, yellow oil) with a yield of 34.3%. MS m/z (ESI): 459.0 [M+1].

Step 4: Compound **61c** (240 mg, 0.52 mmol) was dissolved in 4 ml of N,N-diethylacetamide, 4-bromo-5-chloro-2-methylbenzaldehyde (245 mg, 1.05 mmol), and palladium acetate (12 mg, 0.052 mmol) ), tris(o-methylphenyl)phosphorus (16 mg, 0.052 mmol) and TEA (158 mg, 1.56 mmol) were added in sequence. The reaction solution was heated to 140 °C in a microwave reactor under the protection of argon, reacted for 45 minutes, and filtered to remove the solids. The residue was purified by silica gel column chromatography (eluent: petroleum ether: ethyl acetate = 50:50) to obtain compound **61d** (180 mg, yellow oil) with a yield of 48.4%. MS m/z (ESI): 611.0 [M+1].

Step 5: Compound **61d** (160 mg, 0.26 mmol) was dissolved in 3 ml DMF, and (R)-pyrrolidine-3-carboxylic acid (60 mg, 0.52 mmol) was added. The reaction solution was heated to 60 °C and reacted for 1 hour, and then sodium cyanoborohydride (33 mg, 0.52 mmol) was added. After the addition, the reaction continued at 60 °C for 1 hour, and the solid was removed by filtration. The reactant was concentrated and the residue was purified by preparative chromatography to obtain compound **61e** (26 mg, white solid) with a yield of 14.1%. MS m/z (ESI): 710.0 [M+1].

Step 6: Compound **61e** (26 mg, 0.037 mmol) was dissolved in 2 ml of DCM, 0.5 ml of trifluoroacetic acid was added, and the reaction solution was stirred and reacted at room temperature for 16 hours. The solvent was removed by distillation under reduced pressure to obtain the crude product compound **61f** (25 mg, yellow oil) with a yield of 100%. MS m/z (ESI): 610.0 [M+1].

Step 7: Compound **61f** (25 mg, 0.035 mmol) and TEA (11 mg, 0.105 mmol) were dissolved in 3 ml of DCM, 2-(dimethylamino)acetic acid (11 mg, 0.105 mmol) and HATU (16 mg, 0.042 mmol) were added, and the reaction solution was stirred and reacted at room temperature for 16 hours. The reactant was concentrated and the residue was purified by preparative chromatography to obtain compound **Z-61** (5 mg, white solid) with a yield of 20.8%. MS m/z (ESI): 695.0 [M+1]; ¹H NMR (400 MHz, DMSO-d6) δ 8.90 (d, *J* = 4.8 Hz, 1H), 8.32 (d, *J* = 15.6 Hz, 1H), 7.73 (d, *J* = 8.9 Hz, 2H), 7.56 - 7.36 (m, 5H), 4.90 (s, 1H), 4.76 (s, 1H), 3.86 -3.81(m, 2H), 3.59 - 3.53 (m, 2H), 3.17- 3.14 (m, 2H), 2.90-2.87 (m, 2H), 2.81 (s, 1H), 2.70 - 2.60 (m, 2H), 2.51- 2.50 (m, 2H), 2.30 (s, 3H), 2.27 (s, 3H), 2.18 (s, 3H), 2.15 (s, 3H), 1.98 - 1.90 (m, 2H).

**Compound Z-16** can be obtained by referring to Example 36, except that compound **22a** is replaced by compound **16a,** and proline is replaced by (R)-pyrrolidine-3-carboxylic acid. Compound **Z-17** can be obtained by referring to Example 36, except that compound **22a** is replaced by compound **16a,** and compound **10j** is replaced by compound 17a, and proline is replaced by (R)-pyrrolidine-3-carboxylic acid. Compound **Z-19** can be obtained by referring to Example 36, except that compound **22a** is replaced by compound **16a,** compound **10j** is replaced by compound 19a, and proline is replaced by (R)-pyrrolidine-3-carboxylic acid. Compound **Z-20** can be obtained by referring to Example 36, except that compound **22a** is replaced by compound **16a,** compound **10j** is replaced by compound 20a, and proline is replaced by (R)-pyrrolidine-3-carboxylic acid. Compound **Z-22** can be obtained by referring to the preparation method of compound 36e in Example 36, except that proline is replaced by (R)-pyrrolidine-3-carboxylic acid. Compound **Z-23** can be obtained by referring to Example 18, except that compound **18a** is replaced by compound **23a,** and compound **18d** is replaced by compound **34a.** Compound **Z-24** can be obtained by referring to Example 36, except that proline is replaced by (R)-pyrrolidine-3-carboxylic acid. Compound **Z-25** can be obtained by referring to Example 18, except that compound **18b** is replaced by compound **25a,** and compound **18d** is replaced by compound **34a.** Compound **Z-26** can be obtained by referring to Example 36, except that proline is replaced by L-piperidine-2-carboxylic acid. Compound **Z-28** can be obtained by referring to Example 18, except that compound **18b** is replaced by compound **28a,** and compound **18d** is replaced by compound **34a.** Compound **Z-29** can be obtained by referring to Example 18, except that compound **18d** is replaced by compound **34a,** and (R)-pyrrolidin-3-ol is replaced by (S)-pyrrolidin-3-ol. Compound **Z-30** can be obtained by referring to Example 18, except that compound **18a** is replaced by compound **30a,** compound **18d** is replaced by compound **34a,** and (R)-pyrrolidin-3-ol is replaced by (S)-pyrrolidin-3-ol. Compound **Z-31** can be obtained by referring to Example 18, except that compound **18a** is replaced by compound **31a,** compound **18d** is replaced by compound **34a,** and (R)-pyrrolidin-3-ol is replaced by (S)-pyrrolidin-3-ol. Compound **Z-32** can be obtained by referring to Example 18, except that compound **18a** is replaced by compound **32a,** compound **18d** is replaced by compound **34a,** and (R)-pyrrolidin-3-ol is replaced by (S)-pyrrolidin-3-ol. Compound **Z-33** can be obtained by referring to Example 34, except that (S)-2-aminopropan-1-ol is replaced by 2-aminoethane-1-ol. Compound **Z-35** can be obtained by referring to Example 18, except that compound **18a** is replaced by compound **30a,** and compound **18d** is replaced by compound **34a.** Compound **Z-37** can be obtained by referring to Example 36, except that proline is replaced by (R)-pyrrolidin-3-ol. Compound **Z-38** can be obtained by referring to Example 18, except that compound **18b** is replaced by compound **38a,** and compound **18d** is replaced by compound **34a.** Compound **Z-39** can be obtained by referring to Example 18, except that compound **18a** is replaced by compound **39a,** and compound **18d** is replaced by compound **34a.** Compound **Z-40** can be obtained by referring to Example 41, except that L-aminopropanol is replaced by 2-aminoethane-1-ol. Compound **Z-42** can be obtained by referring to Example 18, except that compound **18b** in step 1 is replaced by compound **42a,** and compound **18d** is replaced by compound **34a.** Compound **Z-43** can be obtained by referring to the preparation method of compound Z-36 in Example 36, except that compound **10j** is replaced by compound 19a, and proline is replaced by (R)-pyrrolidine-3-carboxylic acid. Compound **Z-44** can be obtained by referring to the preparation method of compound 18f in Example 18, except that compound **18a** is replaced by compound **44a,** and compound **18d** is replaced by compound **34a,** and (R)-pyrrolidin-3-ol is replaced by (R)-pyrrolidin-3-ol acetate. Compound **Z-45** can be obtained by referring to the preparation method of compound 18f in Example 18, except that compound **18a** is replaced by compound **44a,** and compound **18d** is replaced by compound **34a.** Compound **Z-46** can be obtained by referring to the preparation method of compound 36e in Example 36, except that compound **10j** is replaced by compound **46a,** and proline is replaced by (R)-pyrrolidine-3-carboxylic acid. Compound **Z-47** can be obtained by referring to Example 36, except that compound **22a** is replaced by compound **16a,** compound **10j** is replaced by compound **46a,** and proline is replaced by (R)-pyrrolidine-3-carboxylic acid. Compound **Z-48** can be obtained by referring to Example 18, except that compound **18a** is replaced by compound **48a,** and compound **18b** is replaced by compound **48b.** Compound **Z-49** can be obtained by referring to Example 36, except that compound **10j** is replaced by compound **49a,** and proline is replaced by (R)-pyrrolidine-3-carboxylic acid. Compound **Z-50** can be obtained by referring to Example 18. **Compound Z-48** can be obtained by referring to Example 18, except that compound **18b** is replaced by compound **48b,** and compound **18d** is replaced by compound **34a.** Compound **Z-51** can be obtained by referring to Example 36, except that compound **10j** is replaced by compound **51a,** and proline is replaced by (R)-pyrrolidine-3-carboxylic acid. Compound **Z-52** can be obtained by referring to Example 36, except that compound **10j** is replaced by compound **52a,** and proline is replaced by (R)-pyrrolidine-3-carboxylic acid. Compound **Z-55** can be obtained by referring to Example 54, except that compound 54a is replaced by compound **55a.** Compound **Z-56** can be obtained by referring to Example 54, except that compound 54a is replaced by 6-chloro-2-methoxy-4-methylnicotine. Compound **Z-57** can be obtained by referring to Example 54, except that compound 54a is replaced by 5-chloro-3-methoxypyrazine-2-carbaldehyde, and (S)-5-(aminomethyl)pyrrolidin-2-one is replaced by (R)-3-pyrrolidinol. Compound **Z-58** can be obtained by referring to Example 54, except that compound 54a is replaced by 5-chloro-3-methoxypyrazine-2-carbaldehyde, and (3-bromo-2-methylphenyl)boronic acid is replaced by (3-bromo-2-chlorophenyl)boronic acid.

| Example number | Structure | MS or ¹H NMR |
|---|---|---|
| 16 | 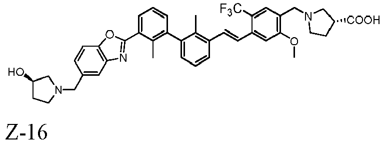 | MS m/z (ESI): 726.1 [M+1] ¹H NMR (400 MHz, DMSO-d6) δ 8.08 (d, *J* = 8.0 Hz, 1H), 7.71 (d, *J* = 10.0 Hz, 2H), 7.61 (dd, *J* = 10.5, 6.1 Hz, 3H), 7.51 - 7.43 (m, 2H), 7.35 (dd, *J* = 18.3, 8.0 Hz, 3H), 7.23 (d, *J* = 15.7 Hz, 1H), 7.12 (d, *J* = 7.4 Hz, 1H), 4.19 (d, *J* = 6.2 Hz, 1H), 3.93 (s, 3H), 3.76 - 3.53 (m, 5H), 2.94 - 2.86 (m, 1H), 2.72 - 2.56 (m, 4H), 2.52 (t, *J* = 6.9 Hz, 2H), 2.43 (s, 1H), 2.39 (s, 3H), 2.34 (dd, *J* = 9.5, 3.7 Hz, 1H), 2.11 (s, 3H), 2.03 - 1.90 (m, 3H), 1.53 (dd, *J* = 8.3, 4.9 Hz, 1H). |
| 17 | 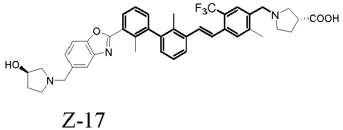 | MS m/z (ESI): 710.1 [M+1] ¹H NMR (400 MHz, DMSO-d6) δ 8.22 (s, 0.5H), 8.08 (d, *J* = 7.9 Hz, 1H), 7.83 (s, 1H), 7.70 (d, *J* = 9.4 Hz, 2H), 7.63 - 7.43 (m, 4H), 7.40 - 7.29 (m, 3H), 7.22 (d, *J* = 16.2 Hz, 1H), 7.11 (d, *J* = 7.5 Hz, 1H), 4.17 (s, 1H), 3.76 - 3.52 (m, 5H), 2.94 - 2.85 (m, 1H), 2.63 (dd, *J* = 20.9, 7.1 Hz, 4H), 2.51 (d, *J* = 6.8 Hz, 2H), 2.44 - 2.28 (m, 8H), 2.08 (d, *J* = 12.8 Hz, 3H), 1.97 (dq, *J* = 21.0, 7.1 Hz, 3H), 1.53 (d, *J* = 3.4 Hz, 1H). |
| 19 | 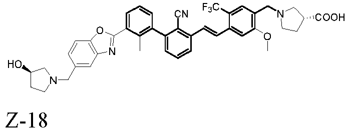 | MS m/z (ESI): 737.1 [M+1] 1H NMR (400 MHz, DMSO-d6) δ 8.18 (dd, J = 7.7, 1.5 Hz, 1H), 7.98 (d, J = 8.2 Hz, 1H), 7.83 (t, J = 7.8 Hz, 1H), 7.77 - 7.59 (m, 4H), 7.58 - 7.42 (m, 5H), 7.38 (d, J = 9.8 Hz, 1H), 4.67 (s, 1H), 4.17 (s, 1H), 3.93 (s, 3H), 3.64 (dt, J = 26.7, 14.0 Hz, 4H), 2.94 - 2.86 (m, 1H), 2.72 - 2.62 (m, 3H), 2.62 - 2.51 (m, 3H), 2.50 (s, 3H), 2.40 (dd, J = 14.2, 8.3 Hz, 1H), 2.31 (dd, J = 9.7, 3.5 Hz, 1H), 1.96 (ddd, J = 20.6, 14.0, 7.0 Hz, 3H), 1.52 (d, J = 3.3 Hz, 1H). |
| 20 | 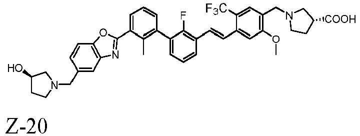 | MS m/z (ESI): 730.1 [M+1] 1H NMR (400 MHz, DMSO-d6) δ 8.21 (s, 2H), 8.12 (dd, J = 7.5, 1.6 Hz, 1H), 7.80 - 7.68 (m, 3H), 7.64 (s, 1H), 7.55 - 7.42 (m, 5H), 7.41 - 7.29 (m, 3H), 4.22 - 4.15 (m, 1H), 3.93 (s, 3H), 3.71 (dd, J = 26.0, 13.1 Hz, 5H), 2.90 (s, 2H), 2.66 (ddd, J = 23.7, 12.5, 7.0 Hz, 5H), 2.52 (dd, J = 11.6, 5.1 Hz, 5H), 2.36 (dd, J = 9.9, 3.4 Hz, 1H), 2.03 - 1.88 (m, 3H), 1.54 (dd, J = 8.2, 4.8 Hz, 1H). |
| 22 | 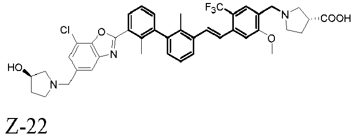 | MS m/z (ESI): 760.0[M+1] ¹H NMR (400 MHz, DMSO-d6) δ 8.10 (d, J = 8.0 Hz, 1H), 7.70 (s, 1H), 7.65 - 7.59 (m, 3H), 7.54 - 7.45 (m, 3H), 7.35 (dd, J = 12.6, 7.3 Hz, 2H), 7.23 (d, J = 15.6 Hz, 1H), 7.12 (d, J = 7.5 Hz, 1H), 4.67 (s, 1H), 4.17 (s, 1H), 3.93 (s, 3H), 3.71 - 3.52 (m, 5H), 2.90 (dd, J = 15.3, 7.4 Hz, 1H), 2.70 - 2.49 (m, 6H), 2.39 (s, 3H), 2.33 (dd, J = 9.6, 3.4 Hz, 1H), 2.11 (s, 3H), 2.03 - 1.89 (m, 3H), 1.53 (d, J = 3.4 Hz, 1H). |
| 23 | 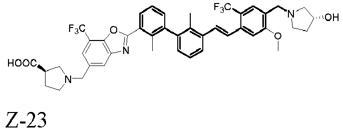 | MS m/z (ESI): 794.0[M+1] 1H NMR (400 MHz, DMSO-d6) δ 8.13 - 8.02 (m, 2H), 7.73 - 7.58 (m, 4H), 7.57 - 7.46 (m, 2H), 7.36 (dd, J = 17.7, 7.9 Hz, 2H), 7.24 (d, J = 15.6 Hz, 1H), 7.13 (d, J = 7.4 Hz, 1H), 4.76 (s, 1H), 4.20 (s, 1H), 3.94 (s, 3H), 3.85 - 3.57 (m, 4H), 2.92 (q, J = 7.6 Hz, 1H), 2.83 - 2.60 (m, 4H), 2.53 (t, J = 7.0 Hz, 2H), 2.40 (s, 3H), 2.34 - 2.19 (m, 1H), 2.12 (s, 3H), 2.07 - 1.76 (m, 4H), 1.56 (s, 1H). |
| 24 | 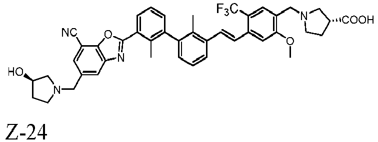 | MS m/z (ESI): 751.0[M+1] ¹H NMR (400 MHz, CD₃OD) δ 8.24 - 8.18 (m, 2H), 7.92 (d, J = 1.4 Hz, 1H), 7.81 (s, 1H), 7.62 (dd, J = 20.6, 12.2 Hz, 2H), 7.53 (dd, J = 16.6, 8.8 Hz, 2H), 7.40 (d, J = 6.5 Hz, 1H), 7.37 - 7.31 (m, 2H), 7.14 (d, J = 7.6 Hz, 1H), 4.37 (dt, J = 25.3, 19.3 Hz, 6H), 4.09 (s, 3H), 4.05 - 3.86 (m, 2H), 3.59 - 3.46 (m, 2H), 3.21 - 3.00 (m, 6H), 2.49 (s, 3H), 2.26 (d, J = 5.6 Hz, 1H), 2.18 (s, 3H), 1.29 (t, J = 7.3 Hz, 1H). |
| 25 | 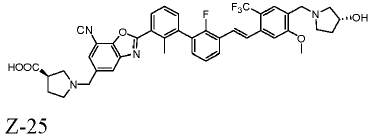 | MS m/z (ESI):755.3 [M+1] ¹H NMR (400 MHz, DMSO-*d*₆) δ 8.17 (dd, J = 7.1, 2.3 Hz, 1H), 8.10 (s, 1H), 7.86 (d, J = 1.5 Hz, 1H), 7.77 (m, 1H), 7.63 (s, 1H), 7.59 - 7.45 (m, 5H), 7.41 - 7.32 (m, 2H), 4.69 (s, 1H), 4.18 (d, J = 7.2 Hz, 1H), 3.94 (s, 3H), 3.73 (d, J = 7.5 Hz, 2H), 3.60 (d, J = 6.5 Hz, 2H), 2.73 - 2.60 (m, 4H), 2.50 (m, 7H), 2.35 (dd, J = 9.7, 3.7 Hz, 1H), 1.96 (m, 3H), 1.53 (m, 1H). |
| 26 | 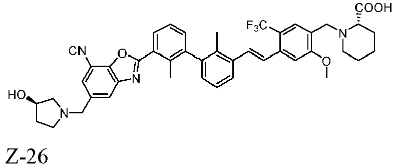 | MS m/z (ESI): 765.4[M+1] |
| 28 | 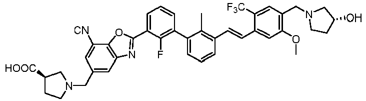 | MS m/z (ESI):755.3 [M+1] ¹H NMR (400 MHz, MD₃OD) δ 8.33 - 8.28 (m, 1H), 8.14 (d, J = 1.5 Hz, 1H), 7.88 (d, J = 1.5 Hz, 1H), 7.72 (s, 1H), 7.65 - 7.56 (m, 3H), 7.53 - 7.46 (m, 2H), 7.38 - 7.30 (m, 2H), 7.25 (d, J = 7.6 Hz, 1H), 4.44 - 4.39 (m, 1H), 4.06 (d, J = 10.1 Hz, 2H), 4.02 (s, 3H), 3.29 (s, 3H), 3.13 - 3.04 (m, 3H), 3.02 - 2.85 (m, 5H), 2.81 (d, J = 14.3 Hz, 1H), 2.17 (m, 4H), 1.83 (m, 2H). |
| | 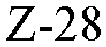 | |
| 29 | 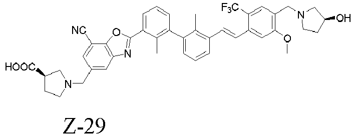 | MS m/z (ESI):751.3 [M+1] ¹H NMR (400 MHz, Methanol-d4) δ 8.23 (d, J = 7.8 Hz, 1H), 8.18 (s, 1H), 7.90 (s, 1H), 7.79 (s, 1H), 7.68 - 7.60 (m, 2H), 7.57 - 7.49 (m, 2H), 7.44 - 7.31 (m, 3H), 7.15 (d, J = 7.5 Hz, 1H), 4.54 (d, J = 27.2 Hz, 2H), 4.37 - 4.13 (m, 3H), 4.07 (s, 3H), 3.54 - 3.43 (m, 1H), 3.24 - 3.00 (m, 10H), 2.50 (s, 3H), 2.19 (s, 6H), 1.96 (d, J = 22.9 Hz, 1H). |
| 30 | 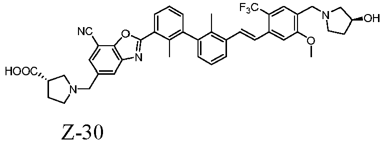 | MS m/z (ESI):376.2 [1/2M+1] ¹H NMR (400 MHz, Methanol-d4) δ 8.26 - 8.14 (m, 2H), 7.90 (s, 1H), 7.79 (s, 1H), 7.68 - 7.59 (m, 2H), 7.56 - 7.47 (m, 2H), 7.45 - 7.31 (m, 3H), 7.15 (d, J = 7.5 Hz, 1H), 4.50 (s, 1H), 4.34 - 4.13 (m, 4H), 4.06 (s, 3H), 3.55 - 3.43 (m, 1H), 3.22 - 2.99 (m, 8H), 2.50 (s, 3H), 2.32 - 2.09 (m, 5H), 1.97 (s, 1H). |
| 31 | 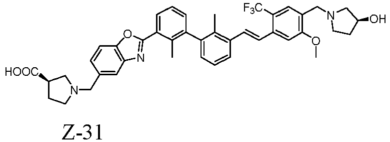 | MS m/z (ESI):726.3 [M+1] |
| 32 | 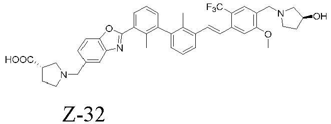 | MS m/z (ESI): 363.7 [1/2M+1] |
| 33 | 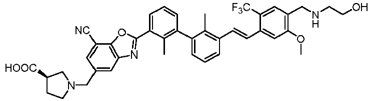 | MS m/z (ESI):725.3 [M+1] ¹H NMR (400 MHz, DMSO-*d*₆) δ 8.42 (s, 1H), 8.13 (d, *J* = 6.8 Hz, 1H), 8.08 (s, 1H), 7.85 (s, 1H), 7.67 (s, 1H), 7.63 (d, *J* = 8.1 Hz, 1H), 7.60 (s, 1H), 7.55 - 7.49 (m, 1H), 7.47 (s, 1H), 7.38 (d, *J* = 7.6 Hz, 1H), 7.34 (t, *J* = 7.6 Hz, 1H), 7.24 (d, *J* = 15.9 Hz, 1H), 7.12 (d, *J* = 7.2 Hz, 1H), 3.94 (s, 3H), 3.79 - 3.63 (m, 4H), 3.53 - 3.42 (m, 3H), 2.85 -2.82 (m, 2H), 2.76 - 2.59 (m, 3H), 2.60 - 2.48 (m, 3H), 2.40 (s, 3H), 2.11 (s, 3H), 2.00 - 1.86 (m, 2H). |
| | 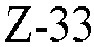 | |
| 35 | 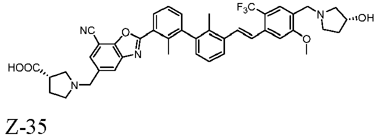 | MS m/z (ESI):751.3 [M+1] |
| 37 | 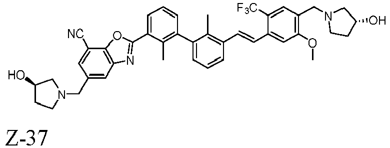 | MS m/z (ESI):362.2[1/2 M +1] ¹H NMR (400 MHz, DMSO-d6) δ 8.15 (d, *J* = 9.6 Hz, 1H), 8.09 (s, 1H), 7.85 (s, 1H), 7.64 - 7.60 (m, 3H), 7.52 (t, *J* = 7.5 Hz, 1H), 7.47 (s, 1H), 7.41 - 7.29 (m, 2H), 7.23 (d, *J* = 16.0 Hz, 1H), 7.11 - 7.08 (m, 1H), 4.69 (s, 2H), 4.18 - 4.17 (m, 2H), 3.93 (s, 3H), 3.76 - 3.69 (m, 2H), 3.66 - 3.54 (m, 2H), 2.68 - 2.60 (m, 4H), 2.46 - 2.41 (m, 2H), 2.40 (s, 3H), 2.34 (d, *J* = 9.6 Hz, 2H), 2.11 (s, 3H), 1.98 - 1.97 (m, 2H), 1.53 - 1.52 (m, 2H). |
| 38 | 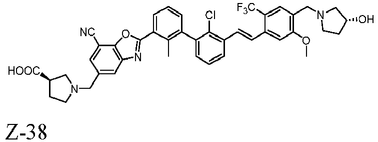 | MS m/z (ESI): 771.3 [M+1] ¹H NMR (400 MHz, DMSO-d6) δ 8.16 (d, *J* = 7.7 Hz, 1H), 8.09 (s, 1H), 7.85 (s, 1H), 7.82 (d, *J* = 7.9 Hz, 1H), 7.56 (ddd, *J* = 21.3, 19.0, 8.1 Hz, 4H), 7.46 - 7.32 (m, 4H), 4.68 (s, 1H), 4.17 (s, 1H), 3.92 (s, 3H), 3.71 (q, *J* = 13.4 Hz, 2H), 3.58 (q, *J* = 14.6 Hz, 2H), 2.93 - 2.85 (m, 1H), 2.64 (tdd, *J* = 22.0, 13.5, 8.3 Hz, 4H), 2.52 (d, *J* = 10.3 Hz, 1H), 2.45 - 2.38 (m, 5H), 2.36 - 2.28 (m, 1H), 1.96 (ddd, *J* = 21.7, 14.6, 7.5 Hz, 3H), 1.52 (d, *J* = 3.7 Hz, 1H). |
| 39 | 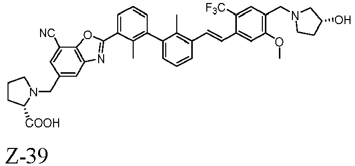 | MS m/z (ESI): 751.3 [M+1] ¹H NMR (400 MHz, DMSO-*d*₆) δ 8.16 - 8.15(m, 1H), 8.12 (d, *J* = 9.9 Hz, 1H), 7.92 (s, 1H), 7.67 - 7.58 (m, 3H), 7.53 (t, *J* = 8.0 Hz, 1H), 7.48 (s, 1H), 7.42 - 7.31 (m, 2H), 7.23 (d, *J* = 15.6 Hz, 1H), 7.13 (d, *J* = 7.5 Hz, 1H), 4.73 (s, 1H), 4.19 (s, 1H), 4.07 (d, *J* = 13.6 Hz, 1H), 3.93 (s, 3H), 3.79 (d, *J* = 14.1 Hz, 1H), 3.68 - 3.55 (m, 2H), 2.97 (s, 2H), 2.76 - 2.54 (m, 3H), 3.52 - 2.48 (m, 1H), 2.46 (s, 3H), 2.40 - 2.28 (m, 1H), 2.11 (s, 3H), 2.04 - 1.86 (m, 2H), 1.90 - 1.64 (m, 3H), 1.56 - 1.53 (m, 1H). |
| 40 | 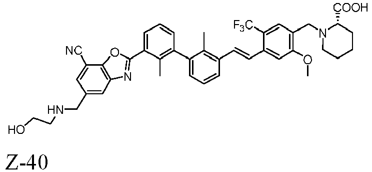 | MS m/z (ESI): 739.3 [M+1]. ¹H NMR (400 MHz, DMSO-*d*₆) δ 8.38 (s, 1H), 8.14 (d, *J* = 6.0 Hz, 2H), 7.89 (s, 1H), 7.75 (s, 1H), 7.66 - 7.56 (m, 2H), 7.52 (t, *J* = 7.7 Hz, 1H), 7.46 (s, 1H), 7.41 - 7.29 (m, 2H), 7.23 (d, *J* = 15.5 Hz, 1H), 7.12 (d, *J* = 7.4 Hz, 1H), 3.91 (s, 3H), 3.85 (s, 2H), 3.76 (d, *J* = 16.4 Hz, 2H), 3.62 - 3.45 (m, 2H), 3.05 (s, 2H), 2.87- 2.85 (m, 2H), 2.73 - 2.70 (m, 1H), 2.53 (t, *J* = 5.8 Hz, 2H), 2.40 (s, 3H), 2.11 (s, 3H), 1.73 (s, 2H), 1.46 (s, 2H), 1.32 - 1.30 m, 1H). |
| 42 | 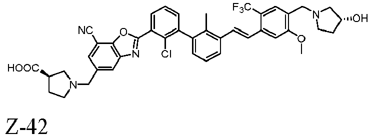 | MS m/z (ESI): 771.3 [M+1] ¹H NMR (400 MHz, DMSO-d6) δ 8.15 (dd, *J* = 9.0, 2.8 Hz, 2H), 7.90 (s, 1H), 7.63 (tt, *J* = 7.3, 4.6 Hz, 5H), 7.47 (s, 1H), 7.36 (t, *J* = 7.7 Hz, 1H), 7.23 (dd, *J* = 15.8, 2.2 Hz, 1H), 7.17 (d, *J* = 7.5 Hz, 1H), 4.68 (s, 1H), 4.20 - 4.13 (m, 1H), 3.93 (s, 3H), 3.73 (dd, *J* = 21.9, 13.4 Hz, 2H), 3.58 (dd, *J* = 21.3, 14.7 Hz, 2H), 2.96 - 2.87 (m, 1H), 2.74 - 2.56 (m, 4H), 2.52 (t, *J* = 7.0 Hz, 2H), 2.44 - 2.38 (m, 1H), 2.33 (dd, *J* = 9.6, 3.6 Hz, 1H), 2.16 (s, 3H), 2.02 - 1.90 (m, 3H), 1.52 (td, *J* = 12.8, 8.0 Hz, 1H). |
| 43 | 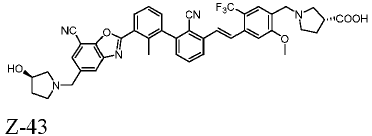 | MS m/z (ESI):762.3[M+1] ¹H NMR (400 MHz, DMSO-d6) δ 8.37 (s, 1H), 8.22 (dd, *J* = 7.5, 1.8 Hz, 1H), 8.11 (s, 1H), 7.98 (d, *J* = 8.0 Hz, 1H), 7.87 - 7.82 (m, 2H), 7.67 (s, 1H), 7.65 - 7.59 (m, 2H), 7.58 - 7.55 (m, 1H), 7.54 (s, 1H), 7.52 - 7.48 (m, 1H), 7.46 (s, 1H), 4.69 (s, 1H), 4.18 - 4.17 (m, 1H), 3.92 (s, 3H), 3.71 (dd, *J* = 25.8, 13.4 Hz, 2H), 3.60 (dd, *J* = 25.5, 14.6 Hz, 2H), 2.92 - 2.80 (m, 1H), 2.75 - 2.55 (m, 4H), 2.55 - 2.48 (m, 5H), 2.43 - 2.37 (m, 1H), 2.33 (dd, *J* = 9.7, 3.5 Hz, 1H), 2.03 - 1.88 (m, 3H), 1.53 - 1.52 (m, 1H). |
| 44 | 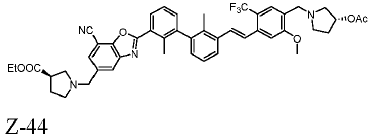 | MS m/z (ESI):821.4 [M+1] ¹H NMR (400 MHz, DMSO-*d*₆) δ 8.14 (d, *J* = 7.0 Hz, 1H), 8.09 (s, 1H), 7.86 (s, 1H), 7.66 - 7.57 (m, 3H), 7.53 (t, *J* = 7.8 Hz, 1H), 7.49 (s, 1H), 7.44-7.36 (m, 1H), 7.34 (d, *J* = 7.6 Hz, 1H), 7.24 (d, *J* = 15.9 Hz, 1H), 7.13 (d, *J* = 7.2 Hz, 1H), 5.08 - 4.97 (m, 1H), 4.04 (q, *J* = 7.1 Hz, 2H), 3.80 (s, 3H), 3.67 - 3.54 (m, 2H), 3.06 - 2.92 (m, 2H), 2.78 - 2.61 (m, 1H), 2.61 - 2.50 (m, 4H), 2.41 (s, 3H), 2.39 - 2.30 (m, 3H), 2.21 - 2.14 (m, 1H), 2.12 (s, 3H), 2.04-2.02 (m, 1H), 1.96 (s, 3H), 1.92 - 1.90 (m, 1H), 1.77 - 1.64 (m, 1H), 1.15 (t, *J* = 7.1 Hz, 3H). |
| 45 | 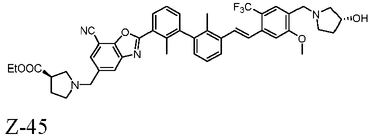 | MS m/z (ESI):779.4 [M+1] |
| 46 | 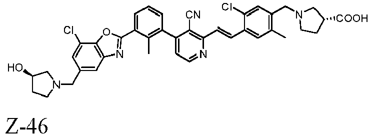 | MS m/z (ESI):722.0 [M+1] ¹H NMR (400 MHz, DMSO-*d*₆) δ 8.93 (d, *J* = 4.9 Hz, 1H), 8.33 (d, *J* = 15.5 Hz, 1H), 8.22 (d, *J* = 5.9 Hz, 1H), 7.73 (d, *J* = 4.3 Hz, 2H), 7.65 - 7.56 (m, 2H), 7.54 (dd, *J* = 10.2, 5.3 Hz, 2H), 7.49 (s, 1H), 7.42 (s, 1H), 4.68 (s, 1H), 4.17 (s, 1H), 3.78 - 3.61 (m, 2H), 3.60 - 3.46 (m, 2H), 2.97 - 2.81 (m, 2H), 2.71 - 2.63 (m, 4H), 2.58 (s, 3H), 2.44 - 2.37 (m, 1H), 2.37 - 2.32 (m, 1H), 2.31 (s, 3H), 2.00 - 1.92 (m, 3H), 1.53 (s, 2H). |
| 47 | 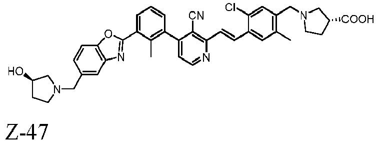 | MS m/z (ESI): 688.0 [M+1] ¹H NMR (400 MHz, DMSO-d6) δ 8.94 (d, *J* = 5.0 Hz, 1H), 8.34 (d, *J* = 15.5 Hz, 1H), 8.22 (dd, *J* = 7.6, 1.4 Hz, 1H), 7.83 - 7.71 (m, 3H), 7.62 - 7.49 (m, 4H), 7.43 (s, 2H), 4.81 (s, 1H), 4.21 (s, 1H), 3.83 (s, 3H), 3.55 (q, *J* = 14.2 Hz, 3H), 2.97 - 2.86 (m, 1H), 2.77 (s, 2H), 2.71 - 2.62 (m, 2H), 2.51 (d, *J* = 8.3 Hz, 5H), 2.31 (s, 3H), 2.06 - 1.89 (m, 3H), 1.58 (s, 1H). |
| 48 | 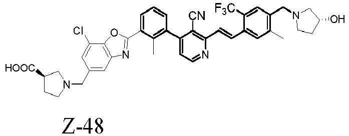 | MS m/z (ESI):756.2 [M+1] ¹H NMR (400 MHz, DMSO-*d*₆) δ 8.96 (d, *J* = 5.0 Hz, 1H), 8.32 (d, *J* = 15.5 Hz, 2H), 8.23 (d, *J* = 7.6 Hz, 1H), 7.87 (s, 1H), 7.73 (s, 1H), 7.68 (s, 1H), 7.62 (d, *J* = 7.3 Hz, 1H), 7.61 - 7.56 (m, 2H), 7.51 (d, *J* = 15.9 Hz, 2H), 4.69 (s, 1H), 4.18 (s, 1H), 3.75 - 3.66 (m, 2H), 3.66 - 3.54 (m, 2H), 2.99 - 2.83 (m, 2H), 2.76 - 2.59 (m, 4H), 2.51 (s, 3H), 2.41 (s, 3H), 2.37 - 2.27 (m, 2H), 2.05 - 1.89 (m, 4H), 1.54 (s, 1H). |
| 49 | 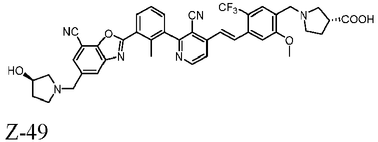 | MS m/z (ESI):763.3 [M+1] ¹H NMR (400 MHz, DMSO-d6) δ 8.94 (d, *J* = 5.4 Hz, 1H), 8.32 - 8.25 (m, 2H), 8.07 - 7.99 (m, 2H), 7.93 - 7.86 (m, 1H), 7.80 (s, 1H), 7.70 (d, *J* = 6.5 Hz, 1H), 7.64 (t, *J* = 7.7 Hz, 1H), 7.57 - 7.47 (m, 2H), 5.20 (s, 1H), 4.33 (s, 1H), 4.25 (s, 2H), 3.97 (s, 3H), 3.91 (s, 2H), 3.04 (s, 6H), 2.83 (s, 3H), 2.53 (s, 3H), 2.04 (d, *J* = 18.1 Hz, 3H), 1.75 (s, 1H). |
| 50 | 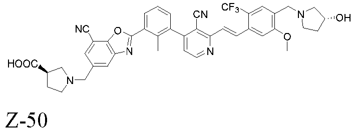 | MS m/z (ESI):763.3 [M+1] ¹H NMR (400 MHz, DMSO-*d*₆) δ 8.96 (d, *J* = 4.9 Hz, 1H), 8.39 - 8.22 (m, 2H), 8.11 (s, 1H), 7.87 (s, 1H), 7.70 - 7.52 (m, 4H), 7.24 - 7.15 (m, 1H), 6.86- 6.85 (m, 1H), 4.69 (s, 1H), 4.18 (s, 1H), 3.77 - 3.67 (m, 2H), 3.68 - 3.53 (m, 2H), 2.93 - 2.89 (m,2H), 2.77 - 2.63 (m, 2H), 2.60 - 2.58 (m, 2H), 2.53 (s, 3H), 2.42 (s, 3H), 2.37 - 2.25 (m, 2H), 2.04 - 1.87 (m, 3H), 1.54 (s, 1H), 1.27 - 1.09 (m, 1H). |
| 51 | 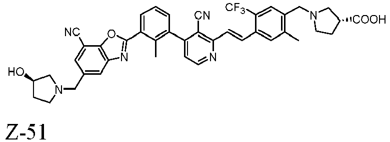 | MS m/z (ESI): 747.1, [M+1] |
| 52 | 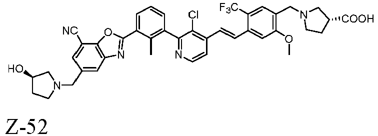 | MS m/z (ESI):772.3 [M+1] ¹H NMR (400 MHz, DMSO-*d*₆) δ 8.64 (d, *J* = 5.0 Hz, 1H), 8.20 (d, *J* = 7.6 Hz, 1H), 8.10 (s, 1H), 7.85 (d, *J* = 6.7 Hz, 2H), 7.69 (s, 1H), 7.67 - 7.45 (m, 5H), 4.68 (s, 1H), 4.17 (s, 1H), 3.93 (s, 3H), 3.78 - 3.67 (m, 2H), 3.66 - 3.53 (m, 2H), 2.90 (s, 2H), 2.72 - 2.56 (m, 3H), 2.54 - 2.51(m,2H), 2.45 - 2.37 (m, 2H), 2.37 - 2.27 (m, 2H), 2.05 - 1.90 (m, 3H), 1.54 (s, 1H), 1.20 (s, 1H). |
| 55 | 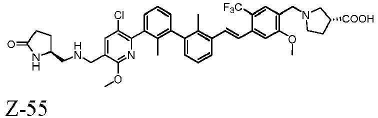 | MS m/z (ESI): 777.3 [M+1] ¹H NMR (400 MHz, DMSO-*d*₆) δ 8.32 (s, 2H), 7.89 (s,1H), 7.72 - 7.51 (m, 3H), 7.46 (s, 1H), 7.38-7.08 (m, 5H), 3.93 (s, 3H), 3.83 (s, 3H), 3.71 - 3.55 (m, 4H), 2.88 (s, 2H), 2.67 - 2.64(m, 3H), 2.29-2.08 (m, 4H), 2.05-1.93 (m, 2H), 1.76 (s, 2H), 1.69 - 1.37 (m, 1H), 1.31 - 1.13 (m, 7H). |
| 56 | 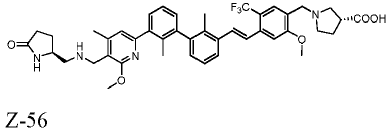 | MS m/z (ESI):757.4 [M+1] |
| 57 | 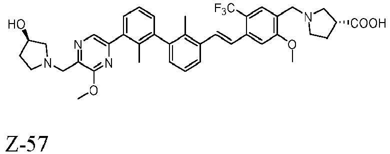 | MS m/z (ESI): 359.2 [1/2M+1] ¹H NMR (400 MHz, CD₃OD) δ 8.26 (s, 1H), 7.77 (s, 1H), 7.64 - 7.52 (m, 3H), 7.46 - 7.44 (m, 1H), 7.38 - 7.29 (m, 3H), 7.21 (*d*, *J* = 8.0Hz, 1H), 7.11 (d, *J* = 8.0Hz, 1H), 4.59 (s, 2H), 4.38 - 4.30 (m, 3H), 4.06 - 3.97 (m, 7H), 3.08 - 2.96 (m, 3H), 2.82 - 2.71 (m, 3H), 2.28 - 2.07 (m, 8H), 1.94 - 1.89 (m, 1H), 1.78 - 1.76 (m, 2H), 1.28 - 1.27 (m, 1H). |
| 58 | 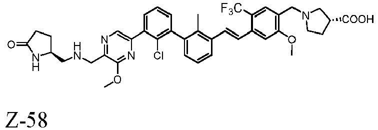 | MS m/z (ESI): 382.7 [1/2M+1] ¹H NMR (400 MHz, CD₃OD) δ 8.26 (s, 1H), 7.79 (s, 1H), 7.66 - 7.62 (m, 1H), 7.58 (*d*, *J* = 8.0Hz, 1H), 7.54 (s, 1H), 7.44 (*d*, *J* = 8.0Hz, 1H), 7.38-7.29 (m, 3H), 7.20 (*d*, *J* = 8.0Hz, 1H), 7.13 (*d*, *J* = 8.0Hz, 1H), 4.42 - 4.40 (m, 2H), 4.48 - 3.97 (m, 7H), 3.89 - 3.84 (m, 1H), 3.54 - 3.50 (m, 1H), 3.42 - 3.35 (m, 2H), 3.15 - 3.03 (m, 2H), 2.84 - 2.73 (m, 2H), 2.35 - 2.18 (m, 8H), 2.07 (s, 3H), 1.87 - 1.82 (m, 2H). |

### Example 62: (3R)-1-(4-((E)-2-(2'-chloro-3'-((5-(((R)-3-hydroxypyrrolidin-1-yl)methyl)-2,3-dihydro-1H-inden-1-yl)oxy)-2-methyl-[1,1'-biphenyl]-3-yl)vinyl)-2-methoxy-5-(trifluoromethyl)benzyl)pyrrolidin e-3-carboxylic acid

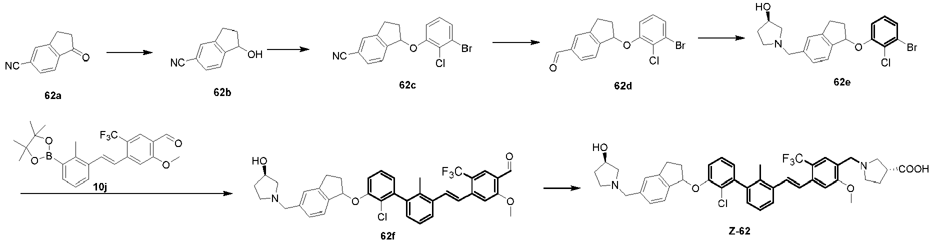

Step 1: 1-oxo-2,3-dihydro-1H-indene-5-carbonitrile **62a** (500 mg, 3.18 mmol) was dissolved in 20 mL methanol, and sodium borohydride (240 mg, 6.36 mmol) was added. The reaction solution was stirred at room temperature for 2 hours. After concentration under reduced pressure, the residue was purified by silica gel column chromatography to obtain compound **62b** (500 mg, white solid) with a yield of 98%. MS m/z (ESI): 160.1 [M+1].

Step 2: **62b** (500 mg, 3.14 mmol), 3-bromo-2-chlorophenol (652 mg, 3.14 mmol), triphenylphosphine (1.65 g, 6.28 mmol) were dissolved in 20 mL tetrahydrofuran, and diisopropyl azodicarboxylate (1.24 mL, 6.28 mmol) was added dropwise. The reaction solution was stirred and reacted at room temperature for 2 hours and then concentrated under reduced pressure, and the residue was purified by silica gel column chromatography to obtain compound **62c** (1.2 g, white solid) with a yield of 99%. MS m/z (ESI): 348.1 [M+1].

Step 3: Compound **62c** (700 mg, 2.02 mmol) was dissolved in 10 mL DCM, the reaction solution was cooled to -78 °C under an ice-water bath, and diisobutyl aluminum hydride (1.0 M, 4.0 mL, 4.0 mmol) was slowly added dropwise. After the addition, the reaction solution was stirred and reacted at 0°C for 0.5 hours. Methanol (5 mL) and 36% hydrochloric acid (5 mL) were added in sequence. The reaction solution was warmed to room temperature, and the reaction continued for 0.5 hours while stirring. The mixture was extracted twice with ethyl acetate (20 mL), and the combined organic phase was washed with brine (30 mL), dried over anhydrous sodium sulfate, filtered, and concentrated under reduced pressure. The residue was purified by silica gel column chromatography to obtain compound **62d** (1.36 g, yellow solid) with a yield of 51%. MS m/z (ESI): 351.1 [M+1].

Step 4: Compound **62d** (340 mg, 0.97 mmol) was dissolved in 10 mL methanol, (R)-pyrrolidin-3-ol (254 mg, 2.91 mmol) and sodium cyanoborohydride (183 mg, 2.91 mmol) were added in sequence, and the reaction solution was replaced with nitrogen three times, stirred and reacted at room temperature for 20 hours,and concentrated under reduced pressure. The residue was purified by silica gel column chromatography to obtain compound **62e** (410 mg, yellow solid) with a yield of 99%. MS m/z (ESI): 422.1 [M+1].

Step 5: Compound **62e** (190 mg, 0.45 mmol) and **10j** (302 mg, 0.677 mmol) were dissolved in 5 ml 1,4-dioxane, and [1,1'-bis(diphenylphosphino)ferrocene]dichloropalladium(II) (37 mg, 0.045 mmol), potassium carbonate (124 mg, 0.90 mmol) and 1 ml of water were added in sequence. The reaction solution was heated to 100 °C and reacted for 4 hours under the protection of argon, and filtered to remove the solids. The filtrate was concentrated, and the residue was purified by silica gel column chromatography to obtain compound **62f** (190 mg, yellow solid) with a yield of 63%. MS m/z (ESI): 662.1 [M+1].

Step 6: Compound **62f** (190 mg, 0.287 mmol) was dissolved in 10 mL methanol, (R)-pyrrolidine-3-carboxylic acid (99 mg, 0.862 mmol) and sodium cyanoborohydride (54 mg, 0.862 mmol) were added in sequence, and the reaction solution was replaced with nitrogen three times, heated to 80°C, stirred and reacted for 2 hours, then cooled to room temperature, and concentrated under reduced pressure. The residue was purified by preparative chromatography to obtain compound **Z-62** (49 mg, white solid) with a yield of 22%. MS m/z (ESI): 761.3 [M+1]; ¹H NMR (400 MHz, DMSO-d6 ) δ 8.19 (s, 1H), 7.64 (d, J = 6.8 Hz, 1H), 7.59 (d, J = 7.6 Hz, 1H), 7.49 (s, 1H), 7.45 - 7.38 (m, 2H), 7.35 - 7.33 (m, 2H), 7.27 (s, 1H), 7.26 - 7.13 (m, 3H), 7.09 (d, J = 7.5 Hz, 1H), 6.91 - 6.87 (m, 1H), 5.96 - 5.92 (m, 1H), 4.19 (s, 1H), 3.95 (d, J = 1.6 Hz, 3H), 3.67 - 3.56 (m, 5H), 3.11 - 3.02 (m, 2H), 2.92 -2.86 (m, 3H), 2.70 - 2.66 (m, 2H), 2.60 - 2.54 (m, 2H), 2.49 - 2.46 (m, 1H), 2.33 (dd, J = 9.5, 3.7 Hz, 1H), 2.12 (d, J = 3.1 Hz, 3H), 2.05 - 1.86 (m, 4H), 1.53 (m, 2H).

### Example 66:(S,E)-1-(5-chloro-4-(2-(3-cyano-4-(3-(1,5-dimethyl-4,5,6,7-tetrahydro-1H-imidazo[4,5-c] pyridine-2-carboxamido)-2-methylphenyl)pyridin-2-yl)vinyl)-2-methylbenzyl)piperidine-2-carboxylic acid

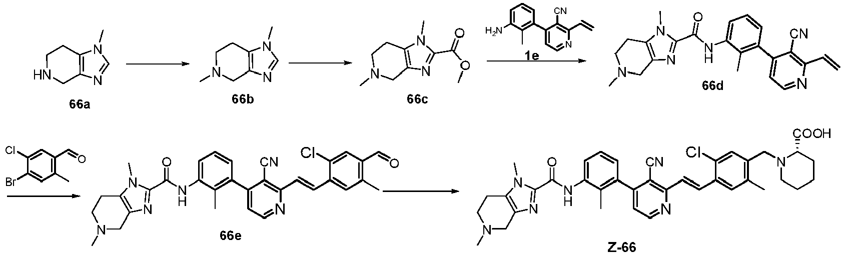

Step 1: 1-methyl-4,5,6,7-tetrahydro-1H-imidazo[4,5-c]pyridine **66a** (1.37g, 10 mmol) was dissolved in 20 ml methanol, and 37% formaldehyde aqueous solution (2.43 g, 30 mmol) and sodium cyanoborohydride (630 mg, 10 mmol) were added in sequence. The reaction solution was stirred and reacted overnight, and concentrated to dryness under reduced pressure, and the residue was purified by silica gel column chromatography with 0%-10% methanol in DCM to obtain compound **66b** (1.3 g, colorless liquid) with a yield of 91.1%. MS m/z (ESI):152.1[M+1].

Step 2: Compound **66b** (1.3g, 8.5 mmol) was dissolved in 30 mL THF, n-butyllithium (7ML, 13 mmol) was added at -78°C under nitrogen atmosphere, and the reaction solution was stirred and reacted for 45 minutes. Methyl chloroformate (1.8g, 17 mmol) was added, and the reaction solution was stirred and reacted for 45 minutes, then warmed to room temperature, and concentrated under reduced pressure. The residue was purified by column chromatography (0%-10% methanol in DCM) to obtain compound **66c** (1.1g, light brown solid) with a yield of 60%. MS m/z (ESI):210.2 [M+1].

Step 3: Compound **66c** (1.1g, 5.3 mmol) and compound **1e** (1g, 5.3 mmol) were dissolved in 30 mL DCM, and IN potassium tert-butoxide (10.6ML, 10.6 mmol) was added at -20°C under nitrogen atmosphere. The reaction solution was stirred and reacted for 45 minutes, and then concentrated under reduced pressure, and the residue was purified by column chromatography (0%-10% methanol in DCM) to obtain compound **66d** (500mg, light brown solid) with a yield of 37%. MS m/z (ESI):413.2 [M+1].

Step 4: Compound **66d** (84mg, 0.2 mmol) and 4-bromo-5-chloro-2-methylbenzaldehyde (94mg, 0.4 mmol) were dissolved in 4 ml DME, and trimethyltriphenylphosphorus (12 mg, 0.04 mmol), palladium acetate (10 mg, 0.04 mmol) and TEA (0.5 ml) were added in sequence. The reaction solution was heated to 150°C, stirred and reacted under microwave for 0.5 hours, cooled to room temperature,and concentrated to dryness under reduced pressure, and the residue was purified by column chromatography (0%-10% methanol in DCM) to obtain compound **66e** (60 mg, reddish brown oil) with a yield of 50%. MS m/z (ESI):565.3 [M+1].

Step 5: Compound **66e** (60 mg, 0.106 mmol) was dissolved in 10 mL methanol, 2-piperidinecarboxylic acid (30 mg, 0.2 mmol) and sodium cyanoborohydride (7 mg, 0.106 mmol) were added, and the reaction solution was heated to 85°C, stirred and reacted for 2 hours. After cooling to room temperature, the reaction solution was concentrated under reduced pressure, and the residue was purified by preparative chromatography to obtain compound **Z-66** (11 mg, white solid) with a yield of 20%. MS m/z (ESI): 678.1 [M+1] ; 1H NMR (400 MHz, DMSO-d6) δ 9.84 (s, 1H), 8.88 (d, J = 5.0 Hz, 1H), 8.31 (d, J = 15.4 Hz, 1H), 7.74 (m, 2H), 7.53 (d, J = 15.3 Hz, 1H), 7.45 (s, 1H), 7.41 (d, J = 5.0 Hz, 1H), 7.35 (t, J = 7.8 Hz, 1H), 7.14 (d, J = 6.8 Hz, 1H), 3.83 (s, 3H), 3.73 (d, J = 14.4 Hz, 1H), 3.43 (d, J = 14.4 Hz, 2H), 3.13 (s, 2H), 2.79 (m, 1H), 2.65 (s, 4H), 2.36 (s, 3H), 2.31 (s, 3H), 2.19 (m, 1H), 2.05 (s, 3H), 1.73 (m, 2H), 1.44 (m, 4H).

### Example 67: 1-(5-chloro-4-((E)-2-(3-cyano-4-(3-(5-(((R)-3-hydroxypyrrolidin-1-yl)methyl)picolinamido) -2-methylphenyl)pyridin-2-yl)vinyl)-2-methylbenzyl)-3-methylpyrrolidine-3-carboxylic acid

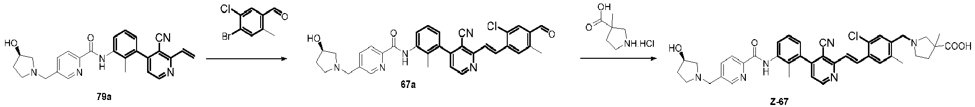

Step 1: Compound **79a** (180 mg, 0.41 mmol) and 4-bromo-5-chloro-2-methylbenzaldehyde (114 mg, 0.49 mmol) were dissolved in N,N-dimethylacetamide (3 mL), and TEA (700 mg, 6.93 mmol), palladium acetate(9 mg, 0.04 mmol) and tris(2-methylphenyl)phosphine(25 mg, 0.08 mmol) were added in sequence. The reaction flask was placed under microwave under the protection of argon, and the reaction solution was heated to 160°C and reacted for 45 minutes. The reaction solution was directly concentrated and the residue was purified by silica gel column (eluent: DCM: methanol = 0-20%) to obtain oily compound **67a** (160 mg, 66%). MS m/z (ESI): 592.2 [M+1].

Step 2: Compound **67a** (100 mg, 0.17 mmol) and 3-methylpyrrolidine-3-carboxylic acid hydrochloride (34 mg, 0.20 mmol) were added to methanol (5 mL), the reaction solution was heated to 60°C and reacted for 1 hour, and then cooled to room temperature and sodium cyanoborohydride (43 mg, 0.68 mmol) was added to the reaction solution. The reaction solution was stirred at room temperature for 2 hours, then saturated ammonium chloride aqueous solution was added dropwise to quench the reaction. The reaction mixture was evaporated to dryness under reduced pressure, and the residue was purified by Pre-HPLC to obtain a white solid product **Z-67** (5.2 mg, 3%). MS m/z (ESI): 705.1 [M+1]; ¹H NMR (400 MHz, DMSO-d6) δ 10.39 (s, 1H), 8.90 (d, *J* = 5.0 Hz, 1H), 8.62 (d, *J* = 1.4 Hz, 1H), 8.32 (d, *J* = 15.5 Hz, 1H), 8.11 (d, *J* = 8.0 Hz, 1H), 7.96 (d, *J* = 8.0 Hz, 2H), 7.72 (s, 1H), 7.53 (d, *J* = 15.4 Hz, 1H), 7.46 - 7.35 (m, 3H), 7.17 (d, *J* = 6.9 Hz, 1H), 4.68 (s, 1H), 4.17 (s, 1H), 3.68 (q, *J* = 13.7 Hz, 2H), 3.54 (q, *J* = 14.1 Hz, 2H), 2.90 (d, *J* = 8.9 Hz, 1H), 2.67 (dd, *J* = 9.5, 6.2 Hz, 1H), 2.58 (dd, *J* = 15.1, 7.7 Hz, 3H), 2.41 (dd, *J* = 14.0, 8.2 Hz, 1H), 2.34 - 2.22 (m, 5H), 2.12 (s, 3H), 2.02 - 1.93 (m, 1H), 1.54 (dd, *J* = 13.0, 7.5 Hz, 2H), 1.22 (s, 3H).

### Example 70: (R,E)-1-(5-chloro-4-(2-(3-cyano-4-(2-methyl-3-(5-(morpholinomethyl) picolinamido) phenyl) pyridin-2-yl)vinyl)-2-methylbenzyl)pyrrolidine-3-carboxylic acid

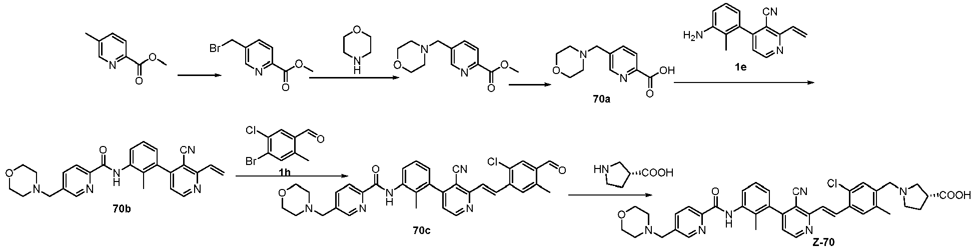

Step 1: Methyl 5-methylpyridine-2-carboxylate (4.25 g, 28.11 mmol), NBS (6.0 g, 33.74 mmol) and AIBN (92 mg, 0.56 mmol) were dissolved in 50 mL CCL₄, and the reaction solution was heated and refluxed overnight, cooled to room temperature, and concentrated under reduced pressure. The residue was purified by column chromatography to obtain the product methyl 5-bromomethylpyridine-2-carboxylate (2.5g, white solid) with a yield of 38%. MS m/z (ESI): 229.9 [M+1].

Step 2: Morpholine (454 mg, 5.22 mmol) and diisopropylethylamine (1.97 g, 15.21 mol) were dissolved in 10 mL DCM. A solution of methyl 5-bromomethylpyridine-2-carboxylate (1.0 g, 4.35 mmol) in DCM (5 mL) was added dropwise within 30 minutes at room temperature, and then continue to stir at room temperature for 1 hour. After concentration under reduced pressure, the residue was purified by column chromatography to obtain the product methyl 5-(morpholinomethyl)pyridine-2-carboxylate (927 mg, white solid) with a yield of 90%. MS m/z (ESI): 237.0 [M+1].

Step 3: Methyl 5-(morpholinomethyl)pyridine-2-carboxylate (927 mg, 3.92 mmol) and lithium hydroxide monohydrate (329 mg, 15.21 mool) were dissolved in 10 mL methanol and 3 mL water, and the reaction solution was stirred overnight at room temperature. The reaction solution was acidified with 1M dilute hydrochloric acid to pH~3, concentrated under reduced pressure and the residue was purified by column chromatography to obtain compound **70a** (850 mg, white solid) with a yield of 97.068%, MS m/z (ESI): 223.0 [M+1].

Step 4: Compound **70a** (400 mg, 1.80 mmol), compound **1e** (423 mg, 1.8 mmol), HATU (820 mg, 2.16 mool) and diisopropylethylamine (348 mg, 2.7 mmol) were dissolved in 5 mL DMF, and the reaction solution was stirred overnight at room temperature. After the reaction solution was concentrated under reduced pressure, the residue was purified by column chromatography to obtain **70b** (485 mg, white solid) with a yield of 61%. MS m/z (ESI): 440.1 [M+1].

Step 5: Compound **70b** (150 mg, 0.34 mmol), 4-bromo-5-chloro-2-methylbenzaldehyde (96 mg, 0.41 mmol), palladium acetate (4 mg, 0.017 mmol) and tris(2-methylphenyl)phosphine (11 mg, 0.034 mmol) were dissolved in 2 mL DMAC and 0.5 mL TEA. After nitrogen replacement, the reaction was carried out in a microwave reactor at 160 °C for 45 min. The reaction solution was concentrated under reduced pressure and the residue was purified by column chromatography to obtain compound **70c** (60 mg, yellow solid) with a yield of 29%. MS m/z (ESI): 592.2 [M+1].

Step 6: Compound **70c** (60 mg, 0.10 mmol) and (R)-pyrrolidine-3-carboxylic acid (35 mg, 0.30 mmol) were dissolved in methanol (2 mL), the reaction solution was heated to 60 °C, and sodium cyanoborohydride (19 mg, 0.30 mmol) was added in batchs. The reactant was concentrated and the residue was purified by preparative chromatography to obtain compound **Z-70** (3.25 mg, white solid) with a yield of 5%. MS m/z (ESI): 691.3 [M+1]; ¹H NMR (400 MHz, DMSO-d6) δ 10.43 (s, 1H), 8.94 (d, 1H), 8.67 (s, 1H), 8.36 (d, 1H), 8.16 (d, 1H), 8.00 (m, 2H), 7.76 (s, 1H), 7.56 (d, 1H), 7.48-7.41 (m, 3H), 7.20 (d, 1H), 3.63-3.57 (m, 8H), 2.90 (m, 1H), 2.73-2.65 (m, 2H), 2.55-2.51 (m, 2H), 2.41-2.39 (m, 4H), 2.34 (s, 3H), 2.15 (s, 3H),1.99-1.96 (m, 2H).

### Example 73: (S, E)-1-(5-chloro-4-(2-(3-cyano-4-(2-methyl-3-(1-methyl-5-(tetrahydro-2H-pyran-4-yl) -4,5,6,7-tetrahydro-1H-imidazo[4,5-c]pyridine-2-carboxamido)phenyl)pyridin-2-yl)vinyl)-2-methylbenzy l) piperidine-2-carboxylic acid

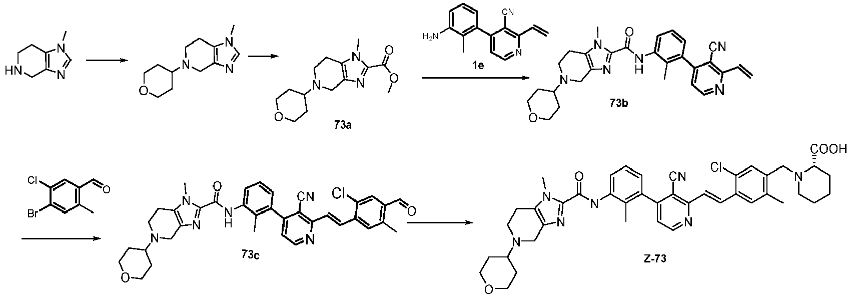

Step 1: 1-methyl-4,5,6,7-tetrahydro-1H-imidazo[4,5-c]pyridine (1.37g, 10 mmol) was dissolved in 20 ml methanol, and dihydro-2H-pyran-4(3H)-one (3.0g, 30 mmol) and sodium cyanoborohydride (630 mg, 10 mmol) were added. The reaction solution was stirred and reacted overnight, and concentrated under reduced pressure to dryness, and the residue was purified by silica gel column chromatography with 0%-10% methanol in DCM to obtain 1-methyl-5-(tetrahydro-2H-pyran-4-yl)-4,5,6,7-tetrahydro-1H-imidazole[4,5-c]pyridine (1.3g, colorless liquid) with ayield of 91.1%. MS m/z (ESI):222.1[M+1].

Step 2: 1-methyl-5-(tetrahydro-2H-pyran-4-yl)-4,5,6,7-tetrahydro-1H-imidazole[4,5-c]pyridine (1.3g, 5.8 mmol) was dissolved in 30 mL THF, n-butyllithium (10ML, 8.7 mmol) was added at -78°C under N₂ protection, and the reaction solution was stirred and reacted for 45 minutes. Methyl chloroformate (1.6g, 14.5 mmol) was added, and the reaction solution was stirred and reacted for 45 minutes, cooled to room temperature, and concentrated under reduced pressure. The residue was purified by column chromatography (eluent: 0%-10% methanol in DCM) to obtain compound **73a** (1.1 g, light brown solid) with a yield of 60%. MS m/z (ESI):280.2 [M+1].

Step 3: Compound **73a** (1.1g, 3.9 mmol) and compound **1e** (0.9g, 3.9 mmol) were dissolved in 30 mL DCM, and IN potassium tert-butoxide (7.8ML, 7.8 mmol) was added at -20°C under N₂ protection. The reaction solution was stirred and reacted for 45 minutes, and concentrated under reduced pressure, and the residue was purified by column chromatography (eluent: 0%-10% methanol in DCM) to obtain compound **73b** (500 mg, light brown solid) with a yield of 37%. MS m/z (ESI):483.2 [M+1].

Step 4: Compound **73b** (96mg, 0.2 mmol) and 4-bromo-5-chloro-2-methylbenzaldehyde (94mg, 0.4 mmol) were dissolved in 4 ml DME, and trimethyltriphenylphosphorus (12 mg, 0.04 mmol), palladium acetate (10 mg, 0.04 mmol) and TEA (0.5 ml) were added in sequence. The reaction solution was heated to 150°C, stirred and reacted under microwave for 0.5 hours, cooled to room temperature, and concentrated to dryness under reduced pressure, and the residue was purified by column chromatography (eluent: 0%-10 % methanol in DCM) to obtain compound **73c** (50 mg, brown oil) with a yield of 40%. MS m/z (ESI):635.3 [M+1].

Step 5: Compound **73c** (50 mg, 0.08 mmol) was dissolved in 10 mL methanol, and L-2-piperidine-carboxylic acid (30 mg, 0.2 mmol) and sodium cyanoborohydride (6 mg, 0.1 mmol) were added. The reaction solution was heated to 85°C, stirred and reacted for 2 hours, cooled to room temperature, and concentrated under reduced pressure, and the residue was purified by preparative chromatography to obtain compound **Z-73** (10 mg, white solid) with a yield of 20%. MS m/z (ESI):748.3 [M+1]; 1H NMR (400 MHz, DMSO-d6) δ 9.81 (s, 1H), 8.88 (d, *J* = 5.0 Hz, 1H), 8.31 (d, *J* = 15.5 Hz, 1H), 7.80 - 7.69 (m, 2H), 7.52 (d, *J* = 15.4 Hz, 1H), 7.45 (s, 1H), 7.41 (d, *J* = 5.0 Hz, 1H), 7.35 (t, *J* = 7.8 Hz, 1H), 7.14 (d, *J* = 7.0 Hz, 1H), 3.87 (d, *J* = 8.9 Hz, 2H), 3.83 (s, 3H), 3.73 (d, *J* = 14.4 Hz, 1H), 3.48 (m, 4H), 3.13 (m, 2H), 2.82 (m, 3H), 2.62 (m, 3H), 2.31 (s, 3H), 2.20 (m, 2H), 2.05 (s, 3H), 1.72 (m, 4H), 1.56 - 1.37 (m, 5H).

### Example 78: (R,E)-2-(5-chloro-4-(2-(3-cyano-4-(3-(5-((3-hydroxypyrrolidin-1-yl)methyl)picolinamido) -2-methylphenyl)pyridin-2-yl)vinyl)-2-methylbenzyl)-2-azaspiro[3.3]heptane-6-carboxylic acid

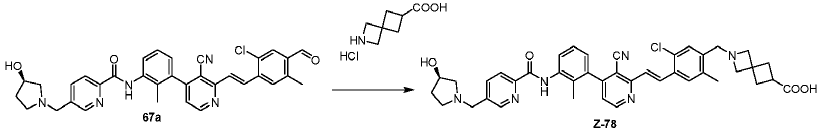

Compound **67a** (100 mg, 0.17 mmol) was dissolved in 3 ml DMF, 2-azaspiro[3.3]heptane-6-carboxylic acid hydrochloride (59 mg, 0.34 mmol) was added, the reaction solution was heated to 60 °C and reacted for 1 hour, and sodium cyanoborohydride (21 mg, 0.34 mmol) was added. After the addition, the reaction continued at 60 °C for 1 hour, the reaction solution was filtered to remove the solids, the filtrate was concentrated and the residue was purified by preparative chromatography to obtain compound **Z-78** (5 mg, white solid) with a yield of 4.4%. MS m/z (ESI): 717.1 [M+1].

### Example 79:(R)-1-(4-((E)-2-(3-cyano-4-(3-(5-(((R)-3-hydroxypyrrolidin-1-yl)methyl)picolinamido) -2-methylphenyl)pyridin-2-yl)vinyl)-5-cyclopropyl-2-methylbenzyl)pyrrolidine-3-carboxylic acid

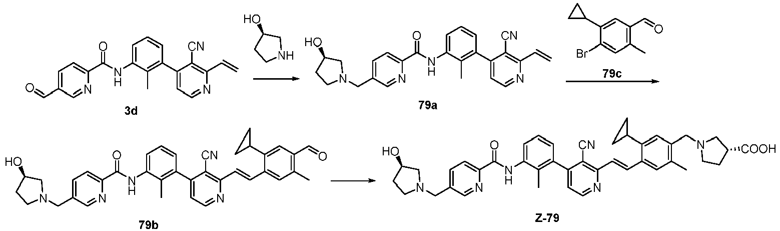

Step 1: Compound **3d** (368 mg, 1 mmol) was dissolved in 20 ml methanol, and (R)-pyrrolidin-3-ol (170 mg, 2.0 mmol) and sodium cyanoborohydride (63 mg, 1 mmol) were added in sequence. The reaction solution was stirred and reacted for 6 hours, and concentrated to dryness under reduced pressure, and the residue was purified by column chromatography (eluent: DCM/MeOH = 0-10%) to obtain compound **79a** (150 mg, white solid) with a yield of 45%. MS m/z (ESI):440.3 [M+1].

Step 2: Compound **79a** (45 mg, 0.1 mmol) and 4-bromo-5-cyclopropyl-2-methylbenzaldehyde **79c** (50 mg, 0.2 mmol) were dissolved in 2 mL DMAC, and palladium acetate (2 mg, 0.01 mmol), tri-o-methylphenylphosphine (3 mg, 0.01 mmol) and 0.3 mL TEA were added. The reaction solution was replaced with nitrogen three times, heated to 160°C, stirred and reacted in a microwave reactor for 45 minutes, cooled to room temperature, and concentrated under reduced pressure, and the residue was purified by column chromatography (eluent: DCM/MeOH = 0-10%) to obtain compound **79b** (18 mg, light brown solid) with a yield of 11%. MS m/z (ESI):598.2 [M+1].

Step 3: Compound **79b** (18 mg, 0.03 mmol) was dissolved in 10 ml methanol, and (R)-pyrrolidine-3-carboxylic acid (30 mg, 0.2 mmol) and sodium cyanoborohydride (6 mg, 0.1 mmol) were added. The reaction solution was heated to 85°C, stirred and reacted for 2 hours, and concentrated to dryness under reduced pressure, and the residue was purified by preparative chromatography to obtain compound **Z-79** (1.3 mg, white solid) with a yield of 8%. MS m/z (ESI): 697.1 [M+1]; ¹H NMR (400 MHz, DMSO-d6) δ 10.39 (s, 1H), 8.87 (d, J = 4.9 Hz, 2H), 8.68 - 8.47 (m, 3H), 8.11 (d, J = 8.0 Hz, 1H), 7.95 (m, 3H), 7.47 (d, J = 6.8 Hz, 1H), 7.44 - 7.27 (m, 3H), 7.17 (d, J = 7.4 Hz, 2H), 7.00 (s, 1H), 4.69 (s, 1H), 4.17 (s, 1H), 3.74 - 3.63 (m, 2H), 3.58 - 3.46 (m, 4H), 2.87 (m, 2H), 2.63 (m, 4H), 2.40 (m, 1H), 2.35 - 2.21 (m, 3H), 2.09 (m, 3H), 2.02-1.85 (m, 3H), 1.53 (s, 2H), 1.20 (s, 1H), 0.96 (d, J = 8.4 Hz, 2H), 0.64 (s, 2H).

### Example 87: 1-(4-((E)-2-(2'-chloro-3'-(5-(((R)-3-hydroxypyrrolidin-1-yl)methyl)picolinamido) -2-methyl- [1,1'-biphenyl]-3-yl)vinyl)-2-methoxy-5-(trifluoromethyl)benzyl)-3-methylpyrrolidine -3-carboxylic acid

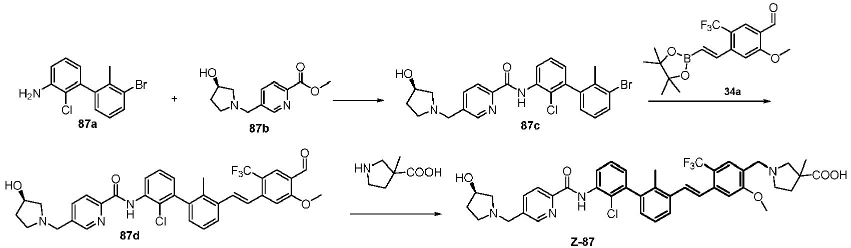

Step 1: Compound **87a** (280 mg, 0.95 mmol) and compound **87b** (224 mg, 0.95 mmol) were dissolved in 10 ml of anhydrous tetrahydrofuran, and a solution of potassium tert-butoxide in tetrahydrofuran (1.0 M, 1.9 ml) was added dropwise at -20 °C. After the addition, the reaction solution was slowly warmed to room temperature, and stirred and reacted for 30 minutes, and the solvent was removed under reduced pressure. The residue was purified by silica gel column chromatography (eluent: petroleum ether: ethyl acetate = 90: 10) to obtain compound **87c** (250mg, white solid) with a yield of 52%. MS m/z (ESI): 500.0 [M+1].

Step 2: Compound **87c** (250 mg, 0.5 mmol) was dissolved in 5 ml of 1,4-dioxane, and compound **34a** (178 mg, 0.5 mmol), [1,1'-bis(diphenylphosphino)ferrocene]dichloropalladium(II) ( 42 mg, 0.05 mmol), potassium carbonate (139 g, 1.01 mmol) and 1 ml of water were added in sequence. The reaction solution was heated to 100 °C in a microwave reactor under the protection of argon, and reacted for 1 hour, the solid was removed by filtration, and the solvent was removed under reduced pressure. The residue was purified by silica gel column chromatography (eluent: petroleum ether: ethyl acetate = 50: 50) to obtain compound **87d** (260 mg, yellow solid) with a yield of 80.0%. MS m/z (ESI): 650.20 [M+1].

Step 3: Compound **87d** (80 mg, 0.12 mmol) was dissolved in 3 ml DMF, and 3-methylpyrrolidine-3-carboxylic acid (40 mg, 0.24 mmol) was added. The reaction solution was heated to 60 °C and reacted for 1 hour, and then sodium cyanoborohydride (15 mg, 0.24 mmol) was added. After the addition, the reaction continued at 60 °C for half an hour, and the solid was removed by filtration. The reactant was concentrated and the residue ws purified by preparative chromatography to obtain compound **Z-87** (6 mg, white solid) with a yield of 5.0%. MS m/z (ESI): 763.0 [M+1]; ¹H NMR (400 MHz, DMSO-d6) δ 10.69 (s, 1H), 8.64 (s, 1H), 8.47 - 8.44 (m, 1H), 8.15 (t, J = 7.1 Hz, 1H), 7.99 (d, J = 9.9 Hz, 1H), 7.66 - 7.59 (m, 3H), 7.48 (t, J = 7.9 Hz, 2H), 7.33 (t, J = 7.6 Hz, 1H), 7.22 (d, J = 15.8 Hz, 1H), 7.14 - 7.08 (m, 2H), 3.93 (s, 3H), 3.74 - 3.66 (m, 2H), 3.63 - 3.55 (m, 3H), 2.94 (d, J = 9.0 Hz, 1H), 2.69 - 2.57 (m, 4H), 2.41 (d, J = 6.2 Hz, 1H), 2.32 - 2.24 (m, 3H), 2.14 (s, 3H),1.98 - 1.94 (m, 1H), 1.54 - 1.53(m, 2H), 1.22 (s, 3H).

### Example 108: (S,E)-1-(4-(2-(2'-chloro-3'-(1,5-dimethyl-4,5,6,7-tetrahydro-1H-imidazo[4,5-c]pyridine-2-carboxamido)-2-methyl-[1,1'-biphenyl]-3-yl)vinyl)-2-methoxy-5-(trifluoromethyl)benzyl)piperidine-2-ca rboxylic acid

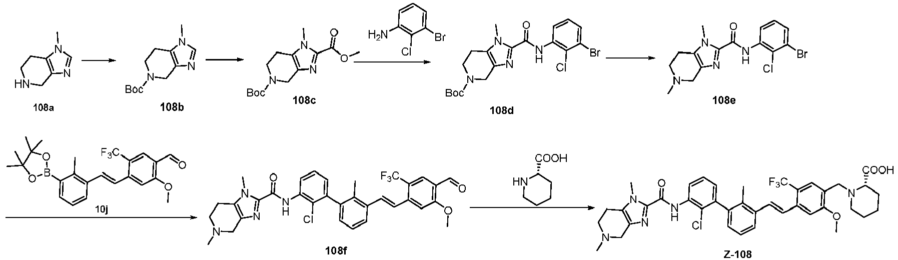

Step 1: Compound **108a** (500 mg, 2.38 mmol) was dissolved in 10 mL DCM, TEA (721 mg, 7.14 mmol) and (Boc)₂O (571 mg, 2.62 mmol) were added in sequence. The reaction solution was stirred and reacted at room temperature for 2 hours. Saturated sodium bicarbonate was added and the reaction solution was extracted three times with DCM. The organic phases were combined, and concentrated under reduced pressure, and the residue was purified by silica gel column chromatography (eluent: DCM: methanol = 15:1) to obtain compound **108b** (450 mg, colorless oil) with a yield of 80%. MS m/z (ESI): 238.0 [M+1].

Step 2: Compound **108b** (400 mg, 1.69 mmol) was dissolved in 20 mL THF, the reaction solution was cooled to -78°C. n-BuLi (1.35 mL, 3.38 mmol) was slowly added and the reaction solution was stirred at -78°C for 0.5 hours. Methyl chloroformate (397 mg, 4.22 mmol) was slowly added, and the reaction solution was warmed to room temperature. Saturated sodium bicarbonate was added, and the reaction solution was extracted three times with DCM. The organic phases were combined, and concentrated under reduced pressure, and the residue was purified by silica gel column chromatography (eluent: ethyl acetate = 100%) to obtain compound **108c** (350 mg, white solid) with a yield of 70%. MS m/z (ESI): 296.2 [M+1].

Step 3: Compound **108c** (330 mg, 1.12 mmol) and compound 3-bromo-2-chloroaniline (277 mg, 1.34 mmol) were dissolved in 20 mL THF, and cooled to -78°C, and a solution of potassium tert-butoxide in tetrahydrofuran (2.2 mL, 2.2 mmol) was added dropwise. After the addition, the reaction solution was slowly warmed to room temperature, water was added to quench the reaction, and the solvent was evaporated under reduced pressure to obtain compound **108d** (600 mg, light yellow solid). MS m/z (ESI): 469.0 [M+1].

Step 4: Compound **108d** (600 mg, 1.12 mmol) was dissolved in 5 mL DCM, 2 mL TFA was added, the mixed system was stirred at room temperature for 2 hours, and the solvent was evaporated under reduced pressure to obtain a brown oil. The oil was dissolved in 15 mL MeOH, 4 mL formaldehyde aqueous solution was added, the reaction system was stirred for 20 min, NaBH₃CN (141 mg, 2.24 mmol) was added. The reaction solution was reacted at room temperature for 8 hours, and concentrated, and the residue was purified by silica gel column chromatography (eluent: DCM: methanol = 15:1) to obtain compound **108e** (150 mg, brown oil) with a yield of 35%. MS m/z (ESI): 383.0 [M+1].

Step 5: Compound **108e** (150 mg, 0.393 mmol) and compound **10j** (350 mg, 0.785 mmol) were dissolved in 10 mL 4-dioxane and 2 mL water, and [1,1'-bis(diphenylphosphino)ferrocene]dichloropalladium(II) (29 mg, 0.0393 mmol) and cesium carbonate (192 mg, 0.590 mmol) were added in sequence. The reaction solution was heated to 100°C and reacted for 45 minutes under the protection of argon, and filtered to remove the solids. The filtrate was concentrated, and the residue was purified by silica gel column chromatography (eluent: DCM: methanol = 15: 1) to obtain compound **108f** (150 mg, yellow oil) with a yield of 61%. MS m/z (ESI): 623.2 [M+1].

Step 6: Compound **108f** (100 mg, 0.161 mmol) was dissolved in 10 mL MeOH, (S)-piperidine-2-carboxylic acid (40 mg, 0.322 mmol) was added, the reaction solution was heated to 60 °C and reacted for 2 hours, then sodium cyanoborohydride (16 mg, 0.244 mmol) was added. After the addition, the reaction continued at 60°C for 0.5 hours. The solid was removed by filtration. The reactant was concentrated and the residue was purified by preparative chromatography to obtain compound **Z-108** (16 mg, white solid) with a yield of 14%. MS m/z (ESI): 736.3 [M+1]; ¹H NMR (400 MHz, CD₃OD) δ 8.42-8.39 (m, 1H), 7.89 (s, 1H), 7.64 - 7.59 (m, 2H), 7.52 (s, 1H),7.40 (t, J = 8.0 Hz, 1H), 7.35 - 7.31 (m, 2H), 7.12 (d, J = 8.0 Hz, 1H), 7.05 (d, J = 8.0Hz, 1H), 4.52 - 4.49 (m, 1H), 4.38 - 4.35 (m, 1H), 4.04 (s, 3H), 3.96 (s, 3H), 3.44 -3.51 (m, 3H), 3.00 - 2.93 (m, 1H), 2.86 - 2.84 (m, 2H), 2.79 - 2.77 (m, 2H), 2.50 (s, 3H), 2.25 -2.18 (m, 4H), 1.81 -1.54 (m, 6H).

### Example 109: (3R)-1-(4-((E)-2-(2,2'-dimethyl-3'-(5-(((2-(S-methylsulfonimidoyl)ethyl)amino)methyl) picolinamido)-[1,1'-biphenyl]-3-yl)vinyl)-2-methoxy-5-(trifluoromethyl)benzyl)pyrrolidine-3-carboxylic acid

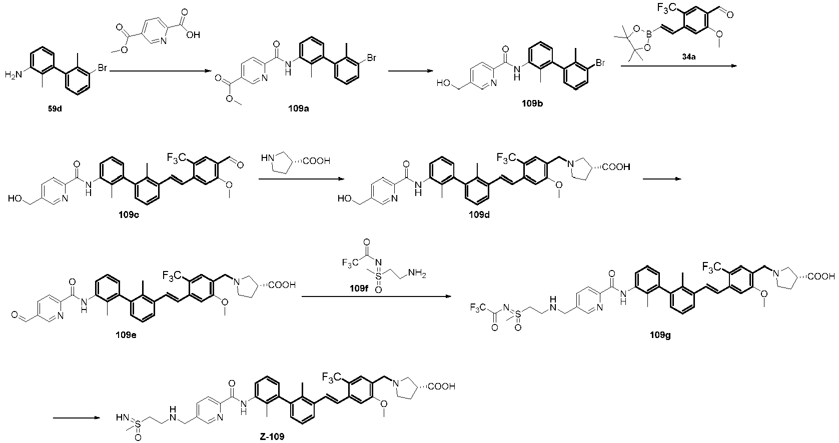

Step 1: Compound **59d** (200 mg, 0.725 mmol) was dissolved in 30 mL DMF, and 5-(methoxycarbonyl)picolinic acid (131 mg, 0.725 mmol), DIEA (187 mg, 1.45 mmol) and HATU (331 mg, 0.870 mmol) were added in sequence. The reaction solution was stirred and reacted at room temperature for 8 hours. Water was added, and the reaction solution was extracted with DCM three times. The organic phases were combined, and concentrated under reduced pressure to obtain a solid. The solid was slurried with methanol to obtain compound **109a** (230 mg, yellow solid) with a yield of 720%. MS m/z (ESI): 439.1 [M+1].

Step 2: Compound **109a** (160 mg, 0.364 mmol) was dissolved in 30 mL THF, and the reaction solution was cooled to -20°C. LiAlH₄ (28 mg, 0.729 mmol) was added, and the reaction solution was warmed to 0°C and stirred for 2 hours. The reaction was quenched with sodium sulfate decahydrate, and the reaction solution was filtered. The filter cake was washed with ethyl acetate, and the filtrate was concentrated to obtain compound **109b** (200 mg, yellow solid), MS m/z (ESI): 411.1 [M+1].

Step 3: Compound **109b** (200 mg, 0.487 mmol) was dissolved in 10 mL of 4-dioxane and 2 mL of water, and compound **34a** (173 mg, 0.487 mmol), [1,1'-bis(diphenylphosphino)ferrocene]dichloropalladium(II) (36 mg, 0.0487 mmol) and potassium carbonate (134 mg, 0.974 mmol) were added in sequence. The reaction solution was heated to 100°C in a microwave reactor under the protection of argon, and reacted for 45 min. The solid was removed by filtration, the filtrate was concentrated, and the residue was purified by preparative chromatography to obtain compound **109c** (90 mg, yellow solid) with a yield of 33%. MS m/z (ESI): 561.2 [M+1].

Step 4: Compound **109c** (80 mg, 0.143 mmol) was dissolved in 10 mL MeOH, (R)-pyrrolidine-3-carboxylic acid (25 mg, 0.215 mmol) and one drop of AcOH were added, the reaction solution was heated to 60 °C and reacted for 0.5 hours, and then NaBH₃CN was added (16 mg, 0.248 mmol). After the addition, the reaction continued at 60 °C for half an hour, the solid was removed by filtration, the reactant was concentrated and the residue was purified by preparative chromatography to obtain compound **109d** (45 mg, yellow solid) with a yield of 48%. MS m/z (ESI): 660.3 [M+1].

Step 5: Compound **109d** (45 mg, 0.0682 mmol) was dissolved in 10 mL ACN, 5 mLTHF and 5 mL DCM, DMP (29 mg, 0.0682 mmol) was added. The reaction solution was reacted at room temperature for 4 hours, and the reactant was concentrated to obtain compound **109e** (60 mg, yellow oil), MS m/z (ESI): 658.0 [M+1].

Step 6: Compound **109e** (50 mg, 0.0761 mmol) and compound **109f** (48 mg, 0.152 mmol) were dissolved in 10 mL MeOH, one drop of AcOH was added, the reaction solution was heated to 60°C and reacted for 0.5 hours, and then NaBH₃CN (10 mg, 0.152 mmol) was added. After the addition, the reaction continued at 60°C for 0.5 hours. The reactants were concentrated to obtain compound **109g** (60 mg, yellow oil). MS m/z (ESI): 860.3 [M+1].

Step 7: Compound **109g** (60 mg, 0.0761 mmol) was dissolved in 10 mL of MeOH, and potassium carbonate (53 mg, 0.381 mmol) was added. The reaction solution was reacted at room temperature for 2 hours, and filtered to remove solids. The reactant was concentrated and the residue was purified by preparative chromatography to obtain compound **Z-109** (5 mg, white solid) with a yield of 8.6%. MS m/z (ESI): 764.3 [1/2M+1]; ¹H NMR (400 MHz, CD₃OD) δ 8.67 (s, 1H), 8.18 (d, *J* = 8.0 Hz, 1H), 8.02 - 7.99 (m, 1H), 7.89 (d, *J* = 8.0 Hz, 1H), 7.77 (s, 1H), 7.63 - 7.56 (m, 2H), 7.52 (s, 1H), 7.34 - 7.28 (m, 3H), 7.11 (d, *J* = 8.0 Hz, 1H), 7.02 (d, *J* = 8.0 Hz, 1H), 4.64 - 4.50 (m, 4H), 4.32 - 4.25 (m, 2H), 4.06 (s, 3H), 3.92 (s, 2H), 3.38 - 3.35 (m, 3H), 3.13 - 3.02 (m, 5H), 2.33 - 2.17 (m, 5H), 2.07 (s, 3H).

### Example 110: (R)-1-(4-((E)-2-(2,2'-dimethyl-3'-(5-(((((S)-5-oxopyrrolidin-2-yl)methyl)amino)methyl) picolinamido)-[1,1'-biphenyl]-3-yl)vinyl)-2-methoxy-5-(trifluoromethyl)benzyl)pyrrolidine-3-carboxylic acid

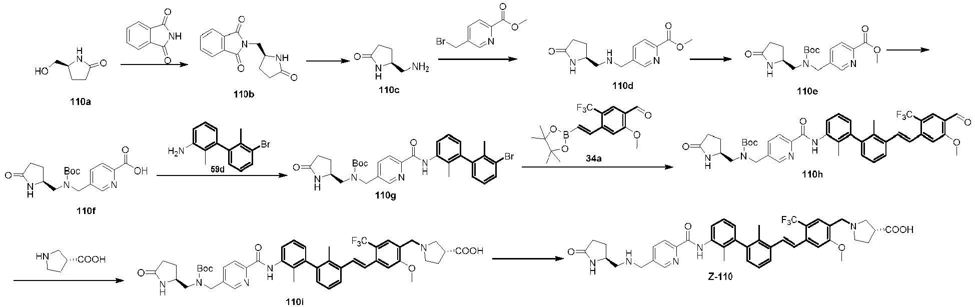

Step 1: Compound L-pyroglutaminol **110a** (2.0 g, 17.4 mmol) and phthalimide (2.81 g, 19.14 mmol) were dissolved in 25 ml of tetrahydrofuran, triphenylphosphine (5.01 g, 19.14 mmol) was added at room temperature, and then diethyl azodicarboxylate (3.33 g, 19.14 mmol) was added. After the addition, the reaction solution was stirred and reacted overnight at room temperature. The reaction solution was diluted with 200 ml of n-hexane and 2 ml of DCM, and a solid was precipitated out, filtered to obtain a white solid. The white solid was pulpedwith 20 ml of ethyl acetate, and the pulp was filtered to obtain 2.2 g of white solid. 20 ml of petroleum ether was added dropwise to the mother liquor with stirring, continued to stir and react for half an hour after the addition, and filtered to obtain 2 g of white solid, which was combined with the previous batch of solid to obtain compound **110b** (4.2 g, white solid) with a yield of 98.6%. MS m/z (ESI): 245.0 [M+1].

Step 2: Compound **110b** (1.5 g, 6.15 mmol) was dissolved in 150 ml of ethanol, and 80% hydrazine hydrate (3.84 g, 61.5 mmol) was added. The reaction solution was heated and refluxed for 4 hours, and solid was precipitated out, filtered to remove the solid. The filtrate was concentrated, and 20 ml of ethanol was added, and stirred overnight at room temperature. The solid was removed by filtration, and the filtrate was concentrated to obtain compound **110c** (750 mg, yellow oil) with a yield of 100%. MS m/z (ESI): 115.0 [M+1].

Step 3: Compound **110c** (0.6 g, 5.22 mmol) and methyl 5-(bromomethyl)picolinate (1.0 g, 4.35 mmol) were dissolved in 20 ml of acetonitrile, potassium carbonate (1.2 g, 8.7 mmol) was added, and the reaction solution was heated to 60 °C and reacted for two hours After cooling to room temperature, the reaction solution was used directly in the next step without treatment. MS m/z (ESI): 264.0 [M+1].

Step 4: Di-tert-butyl dicarbonate (1.9 g, 8.7 mmol) and TEA (0.88 g, 8.7 mmol) were added to the reaction solution in the previous step, the reaction solution was stirred and reacted at room temperature for 16 hours, with removal of most of the solvent. The residue was diluted with 50 ml of DCM, and then washed with 30 ml of water and 30 ml of saturated brine in sequence, dried over anhydrous sodium sulfate, and filtered. The filtrate was concentrated, and the residue was purified by silica gel column chromatography (eluent: DCM: methanol = 90: 10) to obtain compound **110e** (910 mg, brown oil) with a yield of 57.6%. MS m/z (ESI): 364.0 [M+1].

Step 5: Compound **110e** (100 mg, 0.275 mmol) was dissolved in 5 ml methanol, 1 ml of water and lithium hydroxide monohydrate (23 mg, 0.55 mmol) were added, and the reaction solution was stirred and reacted at room temperature for two hours, neutralized with formic acid to neutrality. After purification by preparative chromatography, compound **110f** (42 mg, white solid) was obtained with a yield of 43.8%. MS m/z (ESI): 350.2 [M+1].

Step 6: Compound **110f** (30 mg, 0.086 mmol) was dissolved in 3 ml of DMF, and compound **59d** (24 mg, 0.086 mmol), TEA (17 mg, 0.172 mmol) were added in sequence. The mixture was heated to 50 °C, and then HATU (33 mg, 0.086 mmol) was added. The reaction solution was stirred and reacted at 50 °C for 2 hours, cooled to room temperature, poured into water, and extracted with DCM (3^{∗}20 ml). The organic phases were combined, washed with water (2^{∗}50 ml) and saturated brine (30 ml) in sequence, dried over anhydrous sodium sulfate, and filtered, and the filtrate was concentrated. The residue was purified by silica gel column chromatography (eluent: DCM: methanol = 90: 10) to obtain compound **110g** (52 mg, light yellow oil) with a yield of 74.3%. MS m/z (ESI): 607.2 [M+1].

Step 7: Compound **110g** (450 mg, 0.74 mmol) was dissolved in 10 ml of 1,4-dioxane, and compound **34a** (263 mg, 0.74 mmol), [1,1'-bis(diphenylphosphino)ferrocene]dichloropalladium(II) (54 mg, 0.074 mmol), potassium carbonate (204 mg, 1.48 mmol) and 1 ml of water were added in sequence. The reaction solution was heated to 100 °C and reacted for 5 hours under the protection of argon, filtered to remove the solids, the filtrate was concentrated, and the residue was purified by silica gel column chromatography (eluent: DCM: methanol = 95: 5) to obtain compound **110h** (520 mg, yellow oil) with a yield of 92.9%. MS m/z (ESI): 757.4 [M+1].

Step 8: Compound **110h** (200 mg, 0.26 mmol) was dissolved in 10 ml methanol, and (R)-pyrrolidine-3-carboxylic acid (33 mg, 0.29 mmol) was added. The reaction solution was heated to 60 °C and reacted for 2 hours, and then sodium cyanoborohydride (22 mg, 0.34 mmol) was added. After the addition, the reaction continued at 60 °C for 5 hours. The reaction solution was diluted with 40 ml of DCM, washed with 20 ml of saturated brine, dried over anhydrous sodium sulfate, and filtered, and the filtrate was concentrated and the residue was purified by preparative chromatography to obtain compound **110i** (42 mg, white solid) with a yield of 18.8%. MS m/z (ESI): 856.4 [M+1].

Step 9: Compound **110i** (42 mg, 0.05 mmol) was dissolved in 5 ml of DCM, 1 ml of trifluoroacetic acid was added, and the reaction solution was stirred at room temperature for two hours, and concentrated under reduced pressure. The residue was purified by preparative chromatography to obtain compound **Z-110** (23 mg, white solid) with a yield of 60.5%. MS m/z (ESI): 756.4 [M+1]; ¹H NMR (400 MHz, DMSO-d6) δ 10.31 (s, 1H), 8.65 (s, 1H), 8.11 (d, *J* = 8.1 Hz, 1H), 8.02 - 7.96 (m, 1H), 7.85 (d, *J* = 8.2 Hz, 1H), 7.66 - 7.55 (m, 3H), 7.47 (s, 1H), 7.27 (m, 3H), 7.07 (d, *J* = 7.5 Hz, 1H), 6.95 (d, *J* = 7.3 Hz, 1H), 3.93 (s, 3H), 3.82 (m, 2H), 3.59 (m, 5H), 2.93 - 2.85 (m, 1H), 2.66 (d, *J* = 8.2 Hz, 2H), 2.52 (m, 2H), 1.90-2.41 (m, 1H), 2.15 - 1.89 (m, 10H), 1.70 - 1.59 (m, 1H).

### Example 113: (S, E)-1-(4-(2-(2'-chloro-3'-(5-(2-hydroxyethyl)-1-methyl-4,5,6,7-tetrahydro-1H-imidazo [4,5-c]pyridine-2-carboxamido)-2-methyl-[1,1'-biphenyl]-3-yl)vinyl)-2-methoxy-5-(trifluoromethyl)benz yl) piperidine-2-carboxylic acid

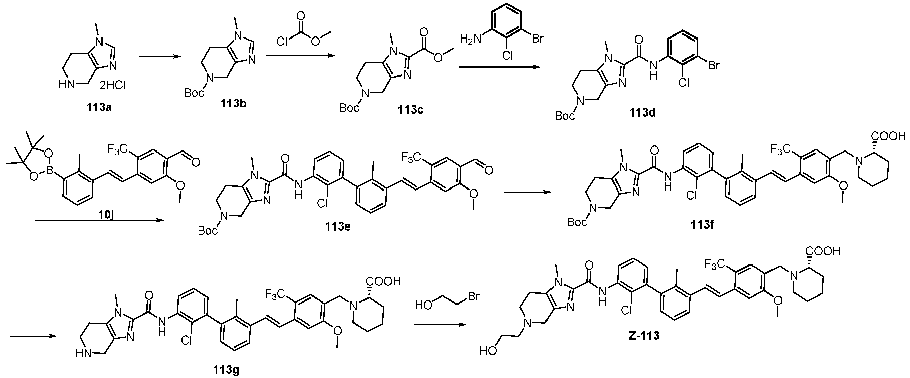

Step 1: Compound **113a** (1g, 4.8 mmol) was dissolved in 10 ml of DCM, and di-tert-butyl dicarbonate (1.56g, 7.1 mmol) and TEA (1.46g, 14.4 mmol) were added in sequence. After the addition, the reaction solution was reacted at room temperature for 1.0 hour. The solid was filtered off, the solvent was removed under reduced pressure, and the residue was purified by silica gel column chromatography (eluent: DCM: methanol = 95: 5) to obtain compound **113b** (1.0 g, yellow solid) with a yield of 88.5%. MS m/z (ESI): 238.2 [M+1].

Step 2: Compound **113b** (1.0g, 4.2 mmol) was dissolved in 20ml anhydrous tetrahydrofuran, and a solution of n-butyllithium in n-hexane (2.5 M, 2.52 ml) was added dropwise at -70°C. After the addition, the reaction solution was stirred at -70°C for one hour, then methyl chloroformate (1.2g, 12.7mmol) was added, and the reaction continued while stirring for 1 hour. 2ml of water was added to quench, and the reaction solution was extracted with ethyl acetate (50^{∗}3ml). The organic phases were dried over anhydrous sodium sulfate, concentrated, and the residue was purified by silica gel column chromatography (eluent: DCM: methanol = 90: 10) to obtain compound **113c** (900mg, white solid) with a yield of 75.0%, MS m/z (ESI): 296.2 [M+1].

Step 3: Compound **113c** (900 mg, 3.05 mmol) and 3-bromo-2-chloroaniline (621 mg, 3.05 mmol) were dissolved in 10 ml anhydrous tetrahydrofuran, and a solution of potassium tert-butoxide in tetrahydrofuran (1.0 M, 6.1 ml) was added dropwise at -20°C. After the addition, the reaction solution was slowly warmed to room temperature, and continued to stir for 30 minutes, the solvent was removed under reduced pressure, and the residue was purified by silica gel column chromatography (eluent: DCM: methanol = 90: 10) to obtain compound **113d** (350mg, white solid) with a yield of 24.5%, MS m/z (ESI): 469.0[M+1].

Step 4: Compound **113d** (350 mg, 0.75 mmol) was dissolved in 6 ml 1,4-dioxane, and compound **10j** (388mg, 0.87mmol), [1,1'-bis(diphenylphosphino)ferrocene]dichloropalladium(II) (47 mg, 0.058 mmol), potassium carbonate (160mg, 1.16 mmol) and 2ml of water were added in sequence. The reaction solution was heated to 120 °C in a microwave reactor under the protection of argon, and reacted for 3 hours, the solid was removed by filtration, the solvent was removed under reduced pressure, and the residue was purified by silica gel column chromatography (eluent: DCM: methanol = 90: 10) to obtain compound **113e** (190 mg, yellow solid) with a yield of 35.7%. MS m/z (ESI): 709.0[M+1].

Step 5: Compound **113e** (170mg, 0.24mmol) was dissolved in 3ml MeOH, L-piperidine-2-carboxylic acid (62 mg, 0.48mmol) was added, the reaction solution was heated to 60°C and reacted for 1 hour, and then sodium cyanoborohydride (7.4 mg, 0.12 mmol) was added. After the addition, the reaction continued at 60 °C for half an hour, the reaction solution was filtered to remove the solid, and the residue was purified by silica gel column chromatography (eluent: DCM: methanol = 90: 10) to obtain compound **113f** (100 mg, white solid) with a yield of 50.6%. MS m/z (ESI): 822.3[M+1].

Step 6: Compound **113f** (90mg, 0.11mmol) was dissolved in 3ml DCM, and a solution of hydrochloric acid in methanol (6M, 2ml) was added. The reaction solution was heated to 30 °C and reacted for 6 hours, and extracted with ethyl acetate (30^{∗}3ml), and the organic phase was dried over anhydrous sodium sulfate and concentrated. The residue was purified by silica gel column chromatography (eluent: DCM: methanol = 90: 10) to obtain compound **113g** (60 mg, white solid) with a yield of 69.0%. MS m/z (ESI): 722.2[M+1].

Step 7: Compound **113g** (60 mg, 0.083 mmol) and bromoethanol (21 mg, 0.166mmol) were dissolved in 5ml anhydrous DMF, potassium carbonate (23mg, 0.166mmol) was added at room temperature, and the reaction solution was stirred at room temperature for 6.0 hours. After filtration, the reactant was concentrated and thre residue was purified by preparative chromatography to obtain compound **Z-113** (1.4mg, white solid) with a yield of 2.2%, MS m/z (ESI): 766.3 [M+1].

### Example 114: (R,E)-1-(4-(2-(3'-(5-((3-(hydroxymethyl)azetidin-1-yl)methyl)-4-methoxypicolinamido) -2,2'-dimethyl-[1,1'-biphenyl]-3-yl)vinyl)-2-methoxy-5-(trifluoromethyl)benzyl)pyrrolidine-3-carboxylic acid

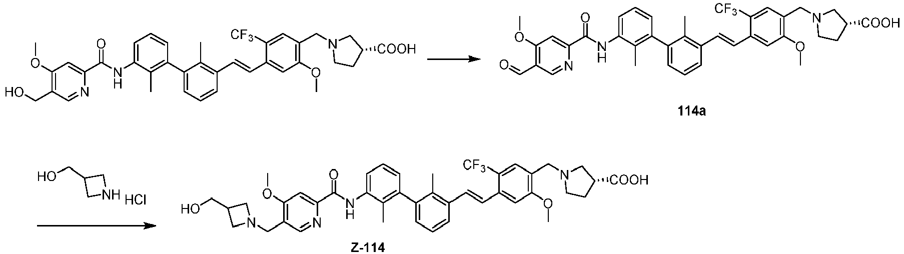

Step 1: Compound (R,E)-1-(4-(2-(3'-(5-(hydroxymethyl)-4-methoxypicolinamido)-2,2'-dimethyl-[1,1'-biphenyl]-3-yl)vinyl)-2-methoxy-5-(trifluoromethyl)benzyl)pyrrolidine-3-carboxylic acid (25 mg, 0.0363 mmol) was dissolved in 5/5ml DCM/acetonitrile, Dess-Martin oxidant (31 mg, 0.0726 mmol) was added at room temperature, and the reaction solution was stirred and reacted at room temperature for half an hour. The reaction was quenched with saturated sodium bicarbonate solution, the reaction solution was extracted with DCM, dried over anhydrous sodium sulfate, filtered to remove solids, and the filtrate was concentrated to give crude compound **114a** (25 mg, yellow solid) with a yield of 100.0 %. MS m/z (ESI): 688.3 [M+1].

Step 2: Compound **114a** (25 mg, 0.0364 mmol) was dissolved in 10 ml MeOH, and azetidin-3-ylmethanol hydrochloride (9 mg, 0.0728 mmol) and a drop of acetic acid were added. The reaction solution was heated to 60 °C and reacted for 1 hour, and sodium cyanoborohydride (5 mg, 0.078 mmol) was added. After the addition, the reaction continued at 60 °C for 1 hour, and the solid was removed by filtration. The reactant was concentrated and purified by preparative chromatography to obtain compound **Z-114** (5.86 mg, white solid) with a yield of 21.24%. MS m/z (ESI): 759.3 [M+1]; ¹H NMR (400 MHz, Methanol-*d₄*) δ 8.39 (s, 1H), 7.91 (d, J = 8.6 Hz, 1H), 7.81 (d, J = 13.2 Hz, 2H), 7.62 (d, J = 15.8 Hz, 1H), 7.56 (d, J = 7.8 Hz, 1H), 7.51 (s, 1H), 7.35 - 7.26 (m, 3H), 7.10 (d, J = 6.9 Hz, 1H), 7.01 (d, J = 7.5 Hz, 1H), 4.57 (s, 1H), 4.46 (d, J = 12.9 Hz, 1H), 4.37 (d, J = 12.9 Hz, 1H), 4.09 (s, 3H), 4.01 (s, 3H), 3.91-3.89 (m, 1H), 3.74 (s, 2H), 3.64-3.62 (m, 3H), 3.48 (t, J = 7.8 Hz, 2H), 3.17-3.16 (m, 3H), 2.69-2.67 (m, 1H), 2.67 - 2.65 (m, 1H), 2.42-2.06 (m, 8H), 1.91 (m, 1H).

### Example 118: (S)-3-hydroxy-4-((4-((E)-2-(3'-(5-(((R)-3-hydroxypyrrolidin-1-yl)methyl)-4-methoxy picolinamido)-2,2'-dimethyl-[1,1'-biphenyl]-3-yl)vinyl)-2-methoxy-5-(trifluoromethyl)benzyl)amino) butanoic acid

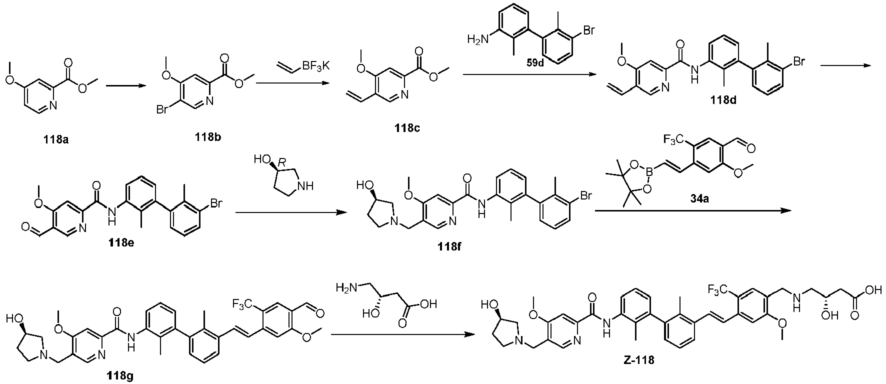

Step 1: Compound **118a** (5.0 g, 29.9 mmol) was dissolved in 38 mL concentrated sulfuric acid, NBS (8.0 g, 44.9 mmol) was added in batches, and the reaction solution was stirred and reacted at room temperature overnight. The reaction solution was added dropwise into saturated sodium bicarbonate aqueous solution, and filtered, and filtrate was extracted three times with DCM. The organic phases were combined, and concentrated under reduced pressure to obtain compound **118b** (6.9 g, white solid) with a yield of 93%. MS m/z (ESI): 246.0 [M+1].

Step 2: Compound **118b** (6.9 g, 28.0 mmol) and potassium vinylfluoroborate (7.5 g, 56.1 mmol) were dissolved in 100 mL 1,4-dioxane and 20 mL water, and [1,1'-bis(diphenylphosphino)ferrocene]dichloropalladium(II) (2.0 g, 2.8 mmol) and potassium carbonate (7.7g, 56.1 mmol) were added in sequence. The reaction solution was heated to 100°C and reacted for 5 hours under argon protection, and water was added to the reaction solution. The reaction solution was extracted three times with DCM, the organic phases were combined, and concentrated under reduced pressure. The residue was purified by silica gel column chromatography (eluent: petroleum ether: ethyl acetate = 1: 1) to obtain compound **118c** (5 g, brown solid) with a yield of 93%. The compound 118c was stored refrigerated under argon atmosphere. MS m/z (ESI): 194.0 [M+1].

Step 3: Compound **118c** (2.00 g, 10.4 mmol) and compound **59d** (2.86 g, 10.4 mmol) were dissolved in 50 mL THF, the reaction solution was cooled to -20°C, and a solution of potassium tert-butoxide in tetrahydrofuran (20.8 mL, 20.8 mmol) was added dropwise. After the addition, the reaction solution was slowly warmed to room temperature, and water was added to the reaction solution. The reaction solution was extracted three times with DCM, the organic phases were combined, and concentrated under reduced pressure to obtain compound **118d** (5.1 g, brown solid). MS m/z (ESI): 437.1 [M+1].

Step 4: Compound **118d** (5.1 g, 10.4 mmol) and potassium osmate (383 mg, 1.04 mmol) were dissolved in 45 mL 1,4-dioxane and 15 mL water, sodium periodate (6.7 g, 31.2 mmol) was added, and the reaction solution was stirred and reacted at room temperature overnight. The reaction was quenched by adding saturated sodium thiosulfate aqueous solution. The reaction solution was extracted three times with DCM, the organic phases were combined and concentrated under reduced pressure, and the residue was purified by silica gel column chromatography (eluent: petroleum ether: ethyl acetate = 1: 1) to obtain compound **118e** (2.3 g, yellow solid) with a yield of 50%. MS m/z (ESI): 439.1 [M+1].

Step 5: Compound **118e** (360 mg, 0.82 mmol) was dissolved in 15 ml of DCM, and (R)-pyrrolidin-3-ol (143 mg, 1.64 mmol) and a drop of acetic acid were added at room temperature. The reaction solution was stirred and reacted at room temperature for 3 hours and then sodium cyanoborohydride (103 mg, 1.64 mmol) was added in batches. The reaction continued for 1 hour at room temperature after the addition, the solid was removed by filtration, the filtrate was concentrated, and the residue was purified by silica gel column chromatography (eluent: DCM: methanol = 95: 5) to obtain compound **118f** (220 mg, light yellow oil) with a yield of 52.6%. MS m/z (ESI): 510.1 [M+1].

Step 6: Compound **118f** (90 mg, 0.176 mmol) was dissolved in 3 ml 1,4-dioxane, and compound **34a** (120 mg, 0.352 mmol), tetrakis(triphenylphosphine)palladium (21 mg, 0.018 mmol), potassium carbonate (49 mg, 0.352 mmol) and 0.6 ml of water were added in sequence. The reaction solution was heated to 120 °C in a microwave reactor under the protection of argon, and reacted for 1 hour. The solid was removed by filtration, the filtrate was concentrated, and the residue was purified by silica gel column chromatography (eluent: DCM: methanol = 90: 10) to obtain compound **118g** (80 mg, yellow oil) with a yield of 70.2%. MS m/z (ESI): 660.3 [M+1].

Step 7: Compound **118g** (150 mg, 0.228 mmol) was dissolved in 10 ml of MeOH, (S)-4-amino-3-hydroxybutanoic acid (27 mg, 0.228 mmol) was added, the reaction solution was heated to 60 °C and reacted for 1 hour, and then sodium cyanoborohydride (15 mg, 0.228 mmol) was added. After the addition, the reaction continued at 60 °C for 1 hour. The solid was removed by filtration. The reactant was concentrated and the residue was purified by preparative chromatography to obtain compound **Z-118** (20.3 mg, white solid) with a yield of 13.8%. MS m/z (ESI):382.2 [1/2M+1]; ¹H NMR (400 MHz, DMSO-d₆) δ 10.33 (s, 1H), 8.45 (s, 1H), 7.70 (d, J = 15.5 Hz, 2H), 7.64 - 7.55 (m, 2H), 7.47 (s, 1H), 7.34 - 7.27 (m, 2H), 7.22 (d, J = 16.1 Hz, 1H), 7.07 (d, J = 7.4 Hz, 1H), 6.95 (d, J = 7.5 Hz, 1H), 4.20 - 4.11 (m, 1H), 3.95- 3.94 (m, 6H), 3.73 (s, 2H), 3.67 - 3.58 (m, 3H), 2.71 - 2.56 (m, 3H), 2.52 (d, J = 5.8 Hz, 2H), 2.44 - 2.40 (m, 1H), 2.40 - 2.31 (m, 2H), 2.22 (dd, J = 15.2, 7.3 Hz, 1H), 2.10 (s, 3H), 2.02 - 1.88 (m, 4H), 1.56 - 1.46 (m, 1H).

### Example 119: (R)-1-(2-(cyanomethoxy)-4-((E)-2-(3'-(5-(((R)-3-hydroxypyrrolidin-1-yl)methyl)-4-methoxypicolinamido)-2,2'-dimethyl-[1,1'-biphenyl]-3-yl)vinyl)-5-(trifluoromethyl)benzyl)pyrrolidine-3-carboxylic acid

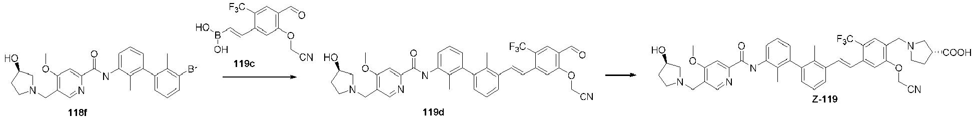

Step 1: Compound **118f** (100 mg, 0.20 mmol) was dissolved in 3 ml of 1,4-dioxane, and compound **119c** (60 mg, 0.20 mmol), [1,1'-bis(diphenylphosphino)ferrocene]dichloropalladium(II) (16 mg, 0.02 mmol), potassium carbonate (55 mg, 0.4 mmol) and 1 ml of water were added in sequence. The reaction solution was heated to 100 °C in a microwave reactor under the protection of argon and reacted for 30 minutes, filtered to remove the solids, the solvent was removed under reduced pressure, and the residue was purified by silica gel column chromatography (eluent: DCM: methanol = 90: 10) to obtain compound **119d** (101 mg, brown solid) with a yield of 73.7%. MS m/z (ESI): 685.3[M+1].

Step 2: Compound **119d** (60 mg, 0.088 mmol) was dissolved in 3 ml DMF, (R)-pyrrolidine-3-carboxylic acid (20 mg, 0.176 mmol) was added, the reaction solution was heated to 60 °C and reacted for 1 hour, and then sodium cyanoborohydride (11 mg, 0.176 mmol) was added. After the addition, the reaction continued at 60 °C for half an hour, and the solid was removed by filtration. The reactant was concentrated and thre residue was purified by preparative chromatography to obtain compound **Z-119** (9.76 mg, white solid) with a yield of 10%. MS m/z (ESI): 784.4 [M+1].

### Example 126: (R,E)-1-(4-(2-(3'-(1,5-dimethyl-4,5,6,7-tetrahydro-1H-imidazo[4,5-c]pyridine-2-carboxamido)-2,2'-dimethyl-[1,1'-biphenyl]-3-yl)vinyl)-2-methoxy-5-(trifluoromethyl)benzyl)pyrrolidine -3-carboxylic acid

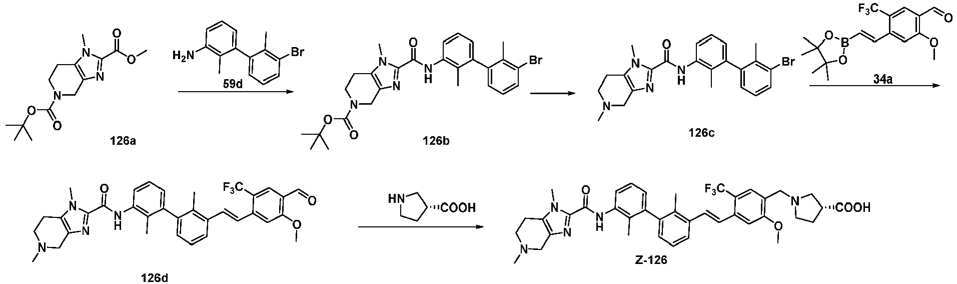

Step 1: Compound **126a** (500mg, 1.69mmol) and compound **59d** (466mg, 1.69mmol) were dissolved in 10 mL of THF, the reaction solution was cooled to -50°C under a dry ice/ethanol system, and a solution of potassium tert-butoxide in tetrahydrofuran (3mL, 3.38mmol) was added dropwise, after the addition, the reaction solution was reacted at -50°C for 1h, quenched by adding saturated ammonium chloride solution, the solvent was evaporated under reduced pressure, and purified by silica gel column chromatography (eluent: petroleum ether: ethyl acetate =3: 1) to obtain compound **126b** (546mg, light yellow liquid) with a yield of 60.03%, MS m/z (ESI): 539.2 [M+1].

Step 2: Compound **126b** (546mg, mmol) was dissolved in 10mL, 5 mL hydrogen chloride (gas)/dioxane solution was added, the mixed system was stirred at room temperature for 3 hours, and the solvent was evaporated under reduced pressure to obtain 708mg of crude light yellow oily liquid. The oily product was dissolved in 10 mL DCM, formaldehyde aqueous solution (242 mg, 3.23 mmol) was added, the reaction system was stirred at room temperature for 2 hours, sodium triacetoxyborohydride (141 mg, 2.24 mmol) was added, the reaction solution was concentrated, and the residue was purified by silica gel column chromatography (eluent: DCM: methanol = 10:1) to obtain compound **126c** (202 mg, light yellow oil) with a yield of 44.10%. MS m/z (ESI):453.1 [M+1].

Step 3: Compound **126c** (100mg, mmol) and **34a** (118mg, 0.332 mmol) were dissolved in 3mL of 1, 4-dioxane and 1 mL of water, and [1,1'-bis(diphenylphosphino)ferrocene]dichloropalladium(II) (16mg, 0.0221mmol) and potassium carbonate (61mg, 0.442mmol) were added in sequence, the reaction solution was heated to 100°C and reacted for 1h under the protection of argon, filtered to remove solids, and the filtrate was concentrated to obtain compound **126d** (200mg, dark red oil). MS m/z (ESI):603.0 [M+1].

Step 4: Compound **126d** (200 mg, 0.332 mmol) was dissolved in 6 mL MeOH, (R)-pyrrolidine-3-carboxylic acid (114 mg, 0.997 mmol) was added, the reaction solution was heated to 80°C and reacted for 1 hour, then sodium cyanoborohydride was added in batches (63 mg, 0.997 mmol), the solid was removed by filtration, and the reactant was concentrated and purified by preparative chromatography to obtain compound **Z-126** (3.6 mg, white solid) with a yield of 1.59%. MS m/z (ESI):702.4 [M+1]; ¹H NMR (400 MHz, DMSO-d6) δ 9.74 (s, 1H), 7.62 - 7.54 (m, 4H), 7.47 (s, 1H), 7.31 - 7.18 (m, 3H), 7.05 (d, *J* = 8.0 Hz, 1H), 6.92 (d, *J* = 8.0 Hz, 1H), 3.93 (s, 3H), 3.83 (s, 3H), 3.64-3.53 (m, 6H), 2.92 - 2.86 (m, 2H), 2.67-2.64 (m, 6H), 2.63 - 2.51 (m, 1H), 2.35 (s, 3H), 2.09(s, 3H), 1.91 (s, 3H).

### Example 129: (E)-1-(4-(2-(2,2'-dimethyl-3'-(7-methyl-5,6,7,8-tetrahydroimidazo[1,2-a]pyrazine-2-carboxamido)-[1,1'-biphenyl]-3-yl)vinyl)-2-methoxy-5-(trifluoromethyl)benzyl)pyrrolidine-3-carboxylic acid

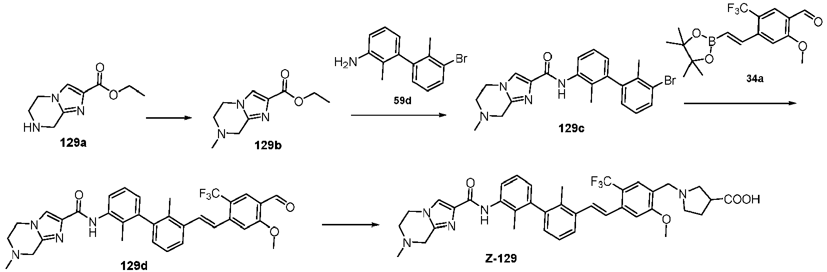

Step 1: Ethyl 5,6,7,8-tetrahydroimidazo[1,2-a]pyrazine-2-carboxylate **129a** (500mg, 2.5mmol) was dissolved in 10ml MeOH, formaldehyde (384 mg, 12.8mmol) was added, and the reaction solution was reacted at room temperature 1 hour, sodium cyanoborohydride (310 mg, 5.0 mmol) was added. After the addition, the reaction was continued at room temperature for half an hour. The solid was removed by filtration. The residue was purified by silica gel column chromatography (eluent: DCM: methanol = 90: 10) to obtain ethyl 7-methyl-5,6,7,8-tetrahydroimidazo[1,2-a]pyrazine-2-carboxylate **129b** (520 mg, white solid) with a yield of 99.2%. MS m/z (ESI): 210.1[M+1].

Step 2: Compound **129b** (520 mg, 2.5 mmol) and compound **59d** (687 mg, 2.5 mmol) were dissolved in 10 ml of anhydrous tetrahydrofuran, and a solution of potassium tert-butoxide in tetrahydrofuran (1.0 M, 5.0 ml) was added dropwise at -20°C. After the addition, the reaction solution was slowly warmed to room temperature, and after the reaction solution was stirred and reacted for 30 minutes, the solvent was removed under reduced pressure, and the residue was purified by silica gel column chromatography (eluent: DCM: methanol = 90: 10) to obtain compound **129c** (600 mg, white solid) with a yield of 54.5%, MS m/z (ESI): 439.1[M+1].

Step 3: Compound **129c** (600 mg, 1.37 mmol) was dissolved in 6 ml 1,4-dioxane, and compound 5 (487 mg, 1.37 mmol), [1,1'-bis(diphenylphosphino)ferrocene]dichloropalladium(II) (112 mg, 0.137 mmol), potassium carbonate (378mg, 2.74 mmol) and 2ml of water were added in sequence, the reaction solution was heated to 100 °C in a microwave reactor under the protection of argon and reacted for 1 hour, the solid was removed by filtration, the solvent was removed under reduced pressure, and the residue was purified by silica gel column chromatography (eluent: DCM: methanol = 90: 10) to obtain **129d** (400 mg, brown solid) with a yield of 49.7%. MS m/z (ESI): 589.0[M+1].

Step 4: Compound **129d** (80 mg, 0.14 mmol) was dissolved in 5 ml of DMF, pyrrolidine-3-carboxylic acid (24 mg, 0.21 mmol) was added, the reaction solution was heated to 60 °C and reacted for 1 hour, then sodium cyanoborohydride (17 mg, 0.28 mmol) was added, After the addition, the reaction was continued for half an hour at 60 °C, and the solid was removed by filtration. The reactant was concentrated and purified by preparative chromatography to obtain compound **Z-129** (9.76 mg, white solid) with a yield of 10%. MS m/z (ESI): 688.3 [M+1]; ¹H NMR (400 MHz, DMSO-d6 ) δ 9.32 (s, 1H), 7.77 (d, J = 6.9 Hz, 1H), 7.71 (s, 1H), 7.62 (d, J = 15.3 Hz, 1H), 7.56 (d, J = 7.8 Hz, 1H), 7.50 (s, 1H), 7.31 - 7.18 (m, 3H), 7.05 (d, J = 6.9 Hz, 1H), 6.88 (d, J = 8.0 Hz, 1H), 6.50 (s, 1H), 4.04 - 4.01 (m, 2H), 3.95 (s, 3H), 3.56 (s, 2H), 2.78 - 2.77 (m, 3H), 2.63 (s, 1H), 2.43 - 2.39 (m, 1H), 2.36 (s, 3H), 2.29 (s, 1H), 2.09 (s, 3H), 2.03 - 1.93 (m, 2H), 1.92 (s, 3H), 1.86-1.54 (m, 2H), 1.12 (s, 1H).

### Example 131:(R)-1-(4-((E)-2-(3'-(5-((1s,4s)-4-hydroxycyclohexyl)-1-methyl-4,5,6,7-tetrahydro-1H-imidazo[4,5-c]pyridine-2-carboxamido)-2,2'-dimethyl-[1,1'-biphenyl]-3-yl)vinyl)-2-methoxy-5-(trifluoro methyl)benzyl)pyrrolidine-3-carboxylic acid

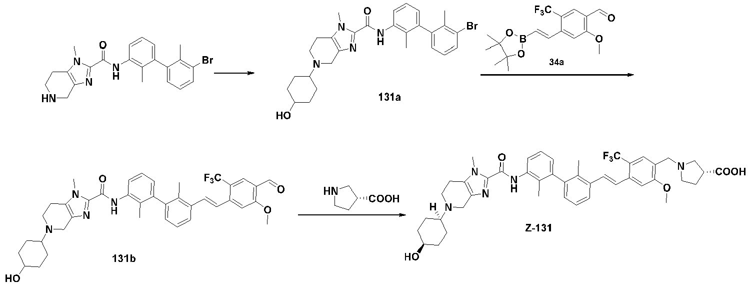

Step 1: Compound N-(3'-bromo-2,2'-dimethyl-[1,1'-biphenyl]-3-yl)-1-methyl-4,5,6,7-tetrahydro-1H-imidazo[4,5-c]pyridine-2-carboxamide (240mg, 0.548mmol) was dissolved in 15mL DCM, 4-hydroxycyclohexan-1-one (187.4 mg, 1.64mmol) was added, sodium triacetoxyborohydride (464.7 mg, 2.192 mmol) was added, the reaction system was stirred at room temperature for 16 hours, the solid was filtered off, the reaction solution was concentrated, and the residue was purified by silica gel column chromatography (eluent: DCM: methanol = 5:1) to obtain compound 131a (266 mg, white solid) with a yield of 90.5%. MS m/z (ESI):537.2 [M+1].

Step 2: Compound 131a (226mg, 0.4216mmol) and (E)-2-methoxy-4-(2-(4,4,5,5-tetramethyl-1,3,2-dioxaborolan-2-yl)vinyl)-5-(trifluoromethyl)benzaldehyde (300.2mg, 0.8432 mmol) were dissolved in 3mL 1, 4-dioxane and 1 mL water, and [1,1'-bis(diphenylphosphino)ferrocene]dichloropalladium(II) (30.8 mg, 0.042mmol) and potassium carbonate (116mg, 0.843mmol) were added in sequence, the reaction solution was heated to 100°C under the protection of argon and reacted for 1h, filtered to remove the solids, and the filtrate was concentrated to obtain crude compound 131b (400mg, brown oil). MS m/z (ESI):687.3 [M+1].

Step 3: The crude compound 131b (400 mg, 0.583 mmol) was dissolved in 15 mL MeOH, (R)-pyrrolidine-3-carboxylic acid (268 mg, 2.33 mmol) was added, the reaction solution was heated to 80°C and reacted for 2 hours, sodium cyanoborohydride (110 mg, 1.749 mmol) was added in batches, filtered to remove the solids, and the reactant was concentrated and purified by preparative chromatography to obtain compound Z-131 (34.63 mg, white solid) with a yield of 7.56%. MS m/z (ESI):786.4 [M+1]; ¹H NMR (400 MHz, DMSO-d6) δ 9.69 (s, 1H), 7.65 - 7.62(m, 2H), 7.58 - 7.55(m, 2H), 7.47 (s, 1H), 7.31 - 7.18 (m, 3H), 7.05 (d, *J* = 8.0 Hz, 1H), 6.91 (d, *J* = 4.0 Hz, 1H), 4.49 (s, 1H), 3.93 (s, 3H), 3.82 (s, 3H), 3.64 - 3.35 (m, 5H), 2.94 - 2.86 (m, 2H), 2.79 - 2.76 (m, 2H), 2.69 - 2.62 (m, 2H), 2.56(m, 2H), 2.53 - 2.51 (m, 2H),2.09 (s, 3H), 1.96 - 1.92 (m, 2H), 1.91 (s, 3H),1.83- 1.81 (m, 2H), 1.75 - 1.72 (m, 2H), 1.36 - 1.28 (m, 2H), 1.18 - 1.09 (m, 2H).

### Example 132:(R)-1-(4-((E)-2-(3'-(5-((1r,4r)-4-hydroxycyclohexyl)-1-methyl-4,5,6,7-tetrahydro-1H-imidazo[4,5-c]pyridine-2-carboxamido)-2,2'-dimethyl-[1,1'-biphenyl]-3-yl)vinyl)-2-methoxy-5-(trifluoro methyl)benzyl)pyrrolidine-3-carboxylic acid

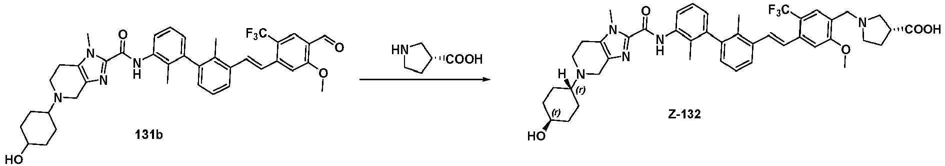

Compound 131b (400 mg, 0.583 mmol) was dissolved in 15 mL MeOH, (R)-pyrrolidine-3-carboxylic acid (268 mg, 2.33 mmol) was added, the reaction solution was heated to 80°C and reacted for 2 hours, sodium cyanoborohydride (110 mg, 1.749 mmol) was added in batches, filtered to remove the solids, and the reactants were concentrated and purified by preparative chromatography to obtain compound Z-132 (22.91 mg, white solid) with a yield of 5.00%. MS m/z (ESI):786.4 [M+1]; ¹H NMR (400 MHz, DMSO-d6) δ 9.71 (s, 1H), 7.64 - 7.62(m, 2H), 7.58 - 7.55(m, 2H), 7.47 (s, 1H), 7.31 - 7.18 (m, 3H), 7.05 (d, *J* = 8.0 Hz, 1H), 6.92 (d, *J* = 8.0 Hz, 1H), 4.27 (s, 1H), 3.93 (s, 3H), 3.82 (s, 3H), 3.69 - 3.53 (m, 5H), 2.94 - 2.86 (m, 2H), 2.80- 2.79 (m, 2H), 2.69 - 2.62 (m, 2H), 2.58(m, 2H), 2.53 - 2.49 (m, 2H),2.09 (s, 3H), 1.96 - 1.94 (m, 2H), 1.91 (s, 3H),1.73-1.63 (m, 4H), 1.46 - 1.35 (m, 4H).

### Example 134: (R,E)-1-(4-(2-(3'-(5-isopropyl-1-methyl-4,5,6,7-tetrahydro-1H-imidazo[4,5-c]pyridine-2-carboxamido)-2,2'-dimethyl-[1,1'-biphenyl]-3-yl)vinyl)-2-methoxy-5-(trifluoromethyl)benzyl)pyrrolidine -3-carboxylic acid

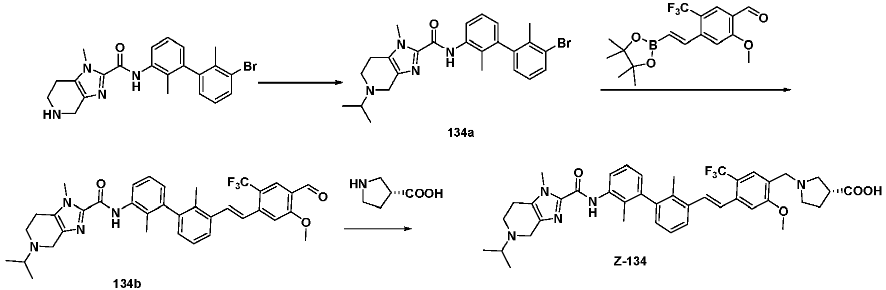

Step 1: Compound N-(3'-bromo-2,2'-dimethyl-[1,1'-biphenyl]-3-yl)-1-methyl-4,5,6,7-tetrahydro-1H-imidazo[4,5-c]pyridine-2-car boxamide (289mg, 0.6598mmol) was dissolved in 20mL 1,2-dichloroethane, acetone (765mg, 13.196mmol) was added, sodium triacetoxyborohydride (559.5 mg, 2.6392 mmol) and 0.2mL acetic acid were added, the reaction system was stirred at room temperature for 6 hours, the reaction solution was concentrated, and the residue was purified by silica gel column chromatography (eluent: DCM: methanol = 9:1) to obtain compound 134a (152 mg, pale yellow oily liquid) with a yield of 48.10%. MS m/z (ESI): 481.2[M+1].

Step 2: Compound 134a(132mg, 0.275mmol) and (E)-2-methoxy-4-(2-(4,4,5,5-tetramethyl-1,3,2-dioxaborolan-2-yl)vinyl)-5-(trifluoromethyl)benzaldehyde (195.8mg, 0.55 mmol) were dissolved in 3mL 1, 4-dioxane and 1 mL water, and [1,1'-bis(diphenylphosphino)ferrocene]dichloropalladium(II) (20.1 mg, 0.0275mmol) and potassium carbonate (75.9mg, 0.55mmol) were added in sequence, the reaction solution was heated to 100°C under argon protection and reacted for 1h, the reaction solution was filtered and concentrated, and the residue was purified by silica gel column chromatography (DCM: methanol =10:1) to obtain compound 134b (153mg, brown oil) with a yield of 88.28%, MS m/z (ESI): 631.3[M+1].

Step 3: Compound 134b (153 mg, 0.2428 mmol) was dissolved in 5 mL MeOH, (R)-pyrrolidine-3-carboxylic acid (83.78 mg, 0.728 mmol) was added, the reaction solution was heated to 80°C and reacted for 1 hour, sodium cyanoborohydride (45.8 mg, 0.728 mmol) was added in batches, filtered to remove the solids, the reactants were concentrated and purified by preparative chromatography to obtain compound Z-134 (51.46 mg, white solid) with a yield of 29.06%. MS m/z (ESI): 365.6[1/2M+1]; ¹H NMR (400 MHz, DMSO-d6) δ 9.68 (s, 1H), 7.67 - 7.55(m, 4H), 7.46 (s, 1H), 7.31 - 7.18 (m, 3H), 7.05 (d, *J* = 8.0 Hz, 1H), 6.92 (d, *J* = 4.0 Hz, 1H), 3.93 (s, 3H), 3.82 (s, 3H), 3.72 (s, 1H),3.64- 3.44 (m, 4H), 2.93 - 2.87 (m, 3H), 2.75 - 2.69 (m, 3H), 2.66- 2.62 (m, 2H), 2.60 - 2.58 (m, 2H), 2.58-2.53(m, 1H), 2.09 (s, 3H), 1.91 (s, 3H),1.56- 1.49(m, 1H), 1.31 - 1.19 (m, 1H), 1.03 - 1.00 (m, 5H).

### Example 137:(R)-1-(4-((E)-2-(3'-(4-cyclopropyl-5-(((R)-3-hydroxypyrrolidin-1-yl)methyl)picolinamido) -2,2'-dimethyl-[1,1'-biphenyl]-3-yl)vinyl)-2-methoxy-5-(trifluoromethyl)benzyl)pyrrolidine-3-carboxylic acid

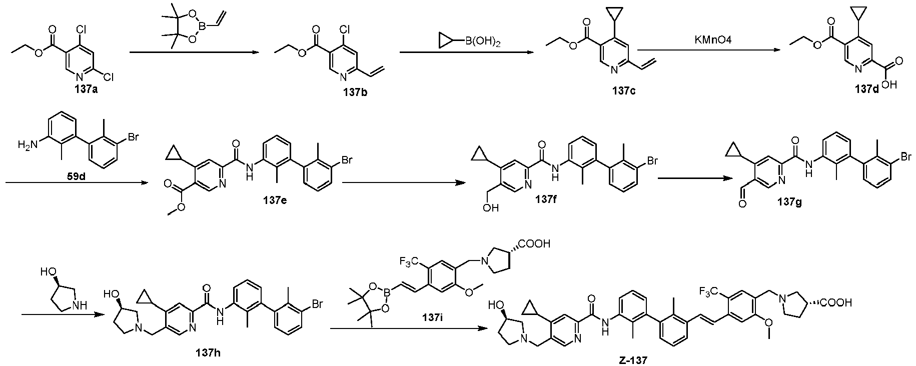

Step 1: Compound **137a** (5 g, 22.72 mmol), 4,4,5,5-tetramethyl-2-vinyl-1,3,2-dioxaborolane (3.85g, 25 mmol), triphenylphosphine (584 mg, 2.23 mmol) and potassium carbonate (6.5 g, 45.44 mmol) were dissolved in a mixture of acetonitrile (100 mL) and ethanol (50 mL), palladium acetate (255 mg, 1.14 mmol) was added at room temperature, the reaction solution was replaced with argon three times and heated to 40 °C, stirred and reacted for 15 hours, then cooled to room temperature, filtered through celite, and the filtrate was washed with ethyl acetate (2^{∗}30 ml), the filtrate was concentrated, and the residue was purified by silica gel column chromatography (eluent: petroleum ether: ethyl acetate = 95: 5) to obtain compound **137b** (5.1g, brown oil). MS m/z (ESI): 212.2 [M+1].

Step 2: Compound **137b** (5 g, 23.6 mmol), cyclopropylboronic acid (6.1 g, 70.8 mmol), Sphos-Pd-G2 catalyst (0.85 g, 1.19 mmol) and potassium phosphate (10 g, 47.2 mmol) were dissolved in toluene (100 mL), the reaction solution was replaced with argon for three times and heated to 110°C, stirred and reacted for 12 hours and then cooled to room temperature, filtered through celite, and the filtrate was washed with ethyl acetate (2^{∗}40 ml), concentrated, and the residue was purified by silica gel column chromatography (eluent: petroleum ether: ethyl acetate = 92: 8) to obtain compound **137c** (2.5 g, brown oil) with a yield of 48.8%. MS m/z (ESI): 218.2 [M+1].

Step 3: Compound **137c** (2.5 g, 11.52 mmol) was dissolved in a mixture of acetone (60 mL) and water (30 mL), potassium permanganate (5.46 g, 34.56 mmol) was added at room temperature, the reaction solution was stirred and reacted at room temperature for 48 hours, then filtered through celite, the filter cake was washed with a mixture of DCM and methanol (10:1, 2^{∗}40 mL), the filtrate was concentrated, and the residue was purified by silica gel column chromatography (eluent: DCM: methanol = 90: 10) to obtain compound **137d**( 1.2 g, white solid) with a yield of 44.4%. MS m/z (ESI): 236.1 [M+1].

Step 4: Compound **137d** (1.2 g, 5.01 mmol) and 59d (1.58 g, 5.76 mmol) were dissolved in tetrahydrofuran (100 mL), HATU (3.81 g, 10.03 mmol) and DIEA (1.62 g, 12.54 mmol) were added in sequence, the reaction solution was stirred at room temperature for 12 hours, DMF (10 mL) was added and the reaction solution was heated to 70 °C and reacted for 1 hour, then cooled to room temperature, diluted with ethyl acetate and water, the aqueous phase was extracted with ethyl acetate (2^{∗}30 mL), the organic phases were combined, and washed with saturated brine (30 mL), dried over anhydrous sodium sulfate, filtered, and the filtrate was concentrated. The residue was purified by silica gel column chromatography (eluent: petroleum ether: ethyl acetate: DCM = 60: 40: 6) to obtain compound **137e** (1.3 g, white solid) with a yield of 52.8%. MS m/z (ESI): 479.1 [M+1].

Step 5: Compound **137e** (1.2 g, 2.44 mmol) was dissolved in tetrahydrofuran (80 mL) and ethanol (40 mL), sodium borohydride (0.37 g, 9.75 mmol) and anhydrous calcium chloride (1.1 g, 9.75 mmol) were added, and the reaction solution was stirred at room temperature for 4 hours, acetic acid was added to quench and the mixture was stirred for 0.5 hours, filtered through celite, and the filter cake was washed with tetrahydrofuran (30 mL) and the filtrate was concentrated to obtain compound 137f (1.2 g, white solid). MS m/z (ESI): 451.1 [M+1].

Step 6: Compound **137f** (0.7 g, 1.55 mmol) was dissolved in tetrahydrofuran (20 mL) and DCM (10 mL), Dess-Martin oxidant (0.98 g, 2.32 mmol) was added, the reaction solution was stirred at room temperature for 12 hours, then saturated sodium bicarbonate was added to quench, the aqueous phase was extracted with DCM (2^{∗}30 ml), the organic phases were combined, washed with saturated brine (30 mL^{∗}2), dried over anhydrous sodium sulfate, filtered, and the filtrate was concentrated to obtain compound **137g** (0.7 g, white solid). MS m/z (ESI): 449.0 [M+1].

Step 7: Compound **137g** (0.7 g, 1.55 mmol) was dissolved in DCM (40 mL), 0.3 mL acetic acid was added, then (R)-pyrrolidin-3-ol (0.34 g, 3.90 mmol) was added, the reaction solution was stirred at room temperature for 5 minutes, then sodium cyanoborohydride (0.24 g, 3.90 mmol) was added in batches, and after stirring for 2 hours at room temperature, the reaction solution was concentrated, and the residue was sequentially purified by silica gel column chromatography (eluent: DCM: methanol = 92: 8) and preparative chromatography (alkaline method) to obtain compound **137h** (0.15 g, white solid) with a yield of 19%. MS m/z (ESI): 520.1 [M+1].

Step 8: Compound **137h** (40 mg, 0.076 mmol) and compound **137i** (42 mg, 0.092 mmol) were dissolved in dioxane (3 mL) and water (1 mL), potassium carbonate (26 mg, 0.19 mmol) was added, and the reaction solution was replaced with argon three times, then tetratriphenylphosphine palladium (9 mg, 0.0076 mmol) was added, the reaction solution was heated to 120 °C under microwave and stirred and reacted for 1 hour, the solid was filtered from the reaction solution, and purified by preparative chromatography (alkaline method) to obtain pure compound **Z-137** (45 mg, white solid ) with a yield of 51%. MS m/z (ESI): 385.2 [1/2M+1].

### Example 138: (R,E)-1-(4-(2-(3'-(1-(1-ethylpiperidin-4-yl)-1H-pyrazole-4-carboxamido)-2,2'-dimethyl-[1,1'-biphenyl]-3-yl)vinyl)-2-methoxy-5-(trifluoromethyl)benzyl)pyrrolidine-3-carboxylic acid

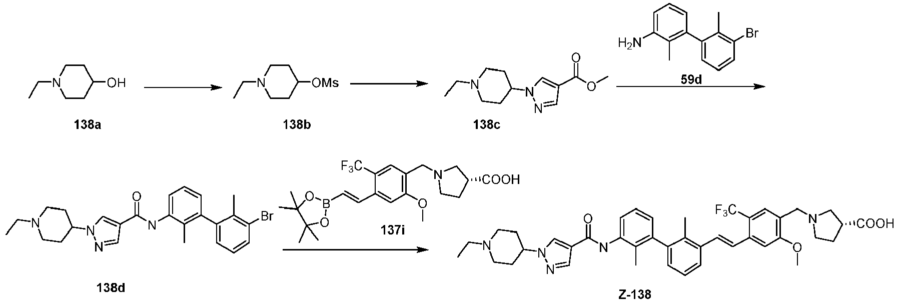

Step 1: 1-ethylpiperidin-4-ol **138a** (2 g, 15.4 mmol) and TEA (6.5 mL, 46.44 mmol) were dissolved in 30 mL DCM, the reaction solution was cooled to 0°C, and methanesulfonyl chloride (1.8 mL, 23.22 mmol) was added dropwise. The reaction solution was stirred and reacted at room temperature for 3 hours, filtered and concentrated under reduced pressure to obtain compound 1-ethylpiperidin-4-yl methanesulfonate **138b** (3.2 g, yellow solid) with a yield of 99%. MS m/z (ESI): 208.1 [M+1].

Step 2: Methyl 1H-pyrazole-4-carboxylate (913 mg, 7.25 mmol) was dissolved in 30 mL DMF, the reaction solution was cooled to 0°C, and sodium hydride (1.3 g, 60%, 21.75 mmol) was added in batches. The mixture was stirred for 15 minutes, 1-ethylpiperidin-4-yl methanesulfonate **138b** was added. The reaction solution was heated to 100°C, stirred and reacted for 20 hours. Cooled to room temperature, concentrated under reduced pressure, and the residue was purified by silica gel column chromatography to obtain compound **138c** (83 mg, yellow solid) with a yield of 5%. MS m/z (ESI): 238.1 [M+1].

Step 3: Compound methyl 1-(1-ethylpiperidin-4-yl)-1H-pyrazole-4-carboxylate **138c** (50 mg, 0.211 mmol) and compound **59d** (58 mg, 0.211 mmol) were dissolved in 5 ml tetrahydrofuran, and the reaction solution was cooled to -70°C, a solution of potassium tert-butoxide in tetrahydrofuran (1M, 0.63 mL, 0.63 mmol) was added dropwise, after the addition, the reaction was continued at 0 °C for 30 minutes, the reaction was quenched by adding water, and extracted with DCM (3^{∗}20ml). The organic phase was combined and washed with saturated brine (30 ml), dried over anhydrous sodium sulfate, filtered, and the filtrate was concentrated. The residue was purified by silica gel column chromatography to obtain compound **138d** (45 mg, light yellow solid) with a yield of 45%. MS m/z (ESI): 481.2 [M+1].

Step 4: A mixture of compound **138d** (20 mg, 0.042 mmol), **137i** (19 mg, 0.042 mmol), tetratriphenylphosphine palladium (5 mg, 0.0042 mmol), potassium carbonate (12 mg, 0.083 mmol), 1,4-dioxane and 0.3 mL water was heated to 120°C in a microwave reactor, stirred and reacted for 1 hour and then cooled to room temperature, filtered, the filtrate was concentrated under reduced pressure, and the residue was purified by preparative chromatography to obtain compound **Z-138** (9 mg, yellow solid) with a yield of 30%. MS m/z (ESI): 365.7 [1/2M+1]; ¹H NMR (400 MHz, DMSO-d6 ) δ 9.55 (s, 1H), 8.36 (s, 1H), 8.02 (s, 1H), 7.65 - 7.58 (m, 3H), 7.50 (s, 1H), 7.36 - 7.20 (m, 4H), 7.08 (d, J = 7.1 Hz, 1H), 6.99 (d, J = 6.4 Hz, 1H), 4.20 -4.15 (m, 1H), 3.96 (s, 3H), 3.67 - 3.56(m, 4H), 2.97 - 2.87 (m, 3H), 2.70 - 2.63 (m, 2H), 2.54 (t, J = 7.0 Hz, 2H), 2.35 (dd, J = 14.3, 7.1 Hz, 2H), 2.12 (s, 3H), 2.06 - 1.88 (m, 9H), 1.01 (t, J = 7.2 Hz, 3H).

**Compound Z-63** can be obtained by referring to Example 3, except that compound 3b is replaced by compound 63a. Compound **Z-64** can be obtained by referring to Example 5, except that 4-bromo-2-chloro-6-nitrophenol is replaced by 4-bromo-5-chloro-2-nitrophenol, and methyl pyrrole-3-carboxylate hydrochloride is replaced by (S)-methyl pyrrole-3-carboxylate hydrochloride, and ethanolamine is replaced by (R)-pyrrolidin-3-ol hydrochloride. Compound **Z-65** can be obtained by referring to Example 87, except that compound 87a is replaced by compound 65a, and compound 34a is replaced by compound 46a, and 3-methylpyrrolidine-3-carboxylic acid is replaced by (R)-pyrrolidine-3-carboxylic acid. Compound **Z-68** can be obtained by referring to Example 79, except that 4-bromo-5-cyclopropyl-2-methylbenzaldehyde is replaced by 4-bromo-5-chloro-2-methylbenzaldehyde. Compound **Z-69** can be obtained by referring to Example 79, except that 4-bromo-5-cyclopropyl-2-methylbenzaldehyde is replaced by 6-bromonicotinaldehyde Compound **Z-71** can be obtained by referring to Example 70, except that (R)-pyrrolidine-3-carboxylic acid is replaced by (S)-piperidine-2-carboxylic acid. Compound **Z-72** can be obtained by referring to Example 79, except that 4-bromo-5-cyclopropyl-2-methylbenzaldehyde is replaced by 4-bromo-2, 5 -dimethylbenzaldehyde. Compound **Z-74** can be obtained by referring to Example 79, except that 4-bromo-5 - cyclopropyl-2-methylbenzaldehyde is replaced by 4-bromo-2-methoxy-5-(trifluoromethyl)benzaldehyde. Compound **Z-75** can be obtained by referring to Example 79, except that 4-bromo-5-cyclopropyl-2-methylbenzaldehyde is replaced by 4-bromo-2,5-dimethoxybenzaldehyde, and (R)-pyrrolidine-3-carboxylic acid is replaced by (S)-piperidine-2-carboxylic acid. Compound Z-76 can be obtained by referring to Example 87, except that compound 87a is replaced by compound 76a, and compound 34a is replaced by compound 46b, and 3-methylpyrrolidine-3-carboxylic acid is replaced by (R)-pyrrolidine-3-carboxylic acid.

**Compound Z-77** can be obtained by referring to the preparation method of compound 109d in Example 109, except that compound 59d is replaced by compound 77a, and compound 34a is replaced by compound 46b. Compound **Z-80** can be obtained by referring to Example 87, except that 3-methylpyrrolidine-3-carboxylic acid is replaced by(R)-pyrrolidine-3-carboxylic acid. Compound **Z-81** can be obtained by referring to Example 87, except that compound 87a is replaced by compound 81a, and compound 34a is replaced by compound 46b, and 3-methylpyrrolidine-3-carboxylic acid is replaced by (S)-piperidine-2-carboxylic acid. Compound **Z-82** can be obtained by referring to Example 87, except that 3-methylpyrrolidine-3-carboxylic acid is replaced by (S)-piperidine-2-carboxylic acid. Compound **Z-88** can be obtained by referring to Example 87, except that compound 87a is replaced by compound 83a, and compound 34a is replaced by compound 46b, and 3-methylpyrrolidine-3-carboxylic acid is replaced by (S)-piperidine-2-carboxylic acid. Compound **Z-84** can be obtained by referring to Example 87, except that compound 87a is replaced by compound 59d, and 3-methylpyrrolidine-3-carboxylic acid is replaced by (R)-pyrrolidine-3-carboxylic acid. Compound **Z-85** can be obtained by referring to Example 87, except that compound 87a is replaced by compound 85a, and 3-methylpyrrolidine-3-carboxylic acid is replaced by (R)-pyrrolidine-3-carboxylic acid. Compound **Z-86** can be obtained by referring to Example 87, except that compound 87a is replaced by compound 59d, and compound 34a is replaced by compound 46b, and 3-methylpyrrolidine-3-carboxylic acid is replaced by (R)-pyrrolidine-3-carboxylic acid. Compound **Z-88** can be obtained by referring to Example 87, except that compound 87a is replaced by compound 88a, and compound 34a is replaced by compound 46b, and 3-methylpyrrolidine-3-carboxylic acid is replaced by (R)-pyrrolidine-3-carboxylic acid. Compound **Z-89** can be obtained by referring to Example 87, except that 3-methylpyrrolidine-3-carboxylic acid is replaced by piperidine-4-carboxylic acid. Compound **Z-90** can be obtained by referring to Example 87, except that compound 87a is replaced by compound 59d, and compound 34a is replaced by compound 18d, and 3-methylpyrrolidine-3-carboxylic acid is replaced by (R)-pyrrolidine-3-carboxylic acid. Compound **Z-91** can be obtained by referring to Example 87, except that compound 87a is replaced by compound 59d, and compound 34a is replaced by compound 91a, and 3-methylpyrrolidine-3-carboxylic acid is replaced by (R)-pyrrolidine-3-carboxylic acid. Compound Z-92 can be obtained by referring to Example 87, except that 3-methylpyrrolidine-3-carboxylic acid is replaced by (R)-tetrahydropyrrole-2-carboxylic acid. Compound **Z-93** can be obtained by referring to Example 87, except that compound 87a is replaced by compound 93a, and 3-methylpyrrolidine-3-carboxylic acid is replaced by (R)-pyrrolidine-3-carboxylic acid. Compound **Z-94** can be obtained by referring to Example 87, except that compound 87a is replaced by compound 94a, and 3-methylpyrrolidine-3-carboxylic acid is replaced by (R)-pyrrolidine-3-carboxylic acid. Compound **Z-95** can be obtained by referring to Example 87, except that compound 87a is replaced by compound 95a, and 3-methylpyrrolidine-3-carboxylic acid is replaced by (R)-pyrrolidine-3-carboxylic acid. Compound **Z-96** can be obtained by referring to Example 87, except that compound 87a is replaced by compound 96a, and 3-methylpyrrolidine-3-carboxylic acid is replaced by (R)-pyrrolidine-3-carboxylic acid. Compound **Z-97** can be obtained by referring to Example 87, except that compound 87a is replaced by compound 59d, and 3-methylpyrrolidine-3-carboxylic acid is replaced by (S)-pyrrolidine-3-carboxylic acid. Compound **Z-98** can be obtained by referring to Example 87, except that compound 87a is replaced by compound 98a, and 3-methylpyrrolidine-3-carboxylic acid is replaced by (R)-pyrrolidine-3-carboxylic acid. Compound **Z-99** can be obtained by referring to Example 87, except that compound 87a is replaced by compound 99a, and 3-methylpyrrolidine-3-carboxylic acid is replaced by (R)-pyrrolidine-3-carboxylic acid. Compound **Z-100** can be obtained by referring to Example 87, except that compound 87a is replaced by compound 100a, and 3-methylpyrrolidine-3-carboxylic acid is replaced by (R)-pyrrolidine-3-carboxylic acid. Compound **Z-101** can be obtained by referring to Example 87, except that compound 87a is replaced by compound 1d, and compound 34a is replaced by compound 18d, and 3-methylpyrrolidine-3-carboxylic acid is replaced by (R)-pyrrolidine-3-carboxylic acid. Compound **Z-102** can be obtained by referring to Example 87, except that compound 87a is replaced by compound 59d, and 3-methylpyrrolidine-3-carboxylic acid is replaced by (S)-piperidine-2-carboxylic acid. Compound **Z-103** can be obtained by referring to Example 87, except that compound 87a is replaced by compound 59d. Compound **Z-104** can be obtained by referring to Example 87, except that compound 87a is replaced by compound 104a, and 3-methylpyrrolidine-3-carboxylic acid is replaced by (R)-pyrrolidine-3-carboxylic acid. Compound **Z-105** can be obtained by referring to Example 87, except that compound 87a is replaced by compound 105a, and 3-methylpyrrolidine-3-carboxylic acid is replaced by (R)-pyrrolidine-3-carboxylic acid. Compound **Z-106** can be obtained by referring to Example 87, except that compound 87a is replaced by compound 59d, and 3-methylpyrrolidine-3-carboxylic acid is replaced by (S)-piperidine-3-carboxylic acid. Compound **Z-107** can be obtained by referring to Example 87, except that 3-methylpyrrolidine-3-carboxylic acid is replaced by (S)-4-amino-3-hydroxybutanoic acid. Compound **Z-111** can be obtained by referring to Example 87, except that compound 87a is replaced by compound 111a, and 3-methylpyrrolidine-3-carboxylic acid is replaced by (R)-pyrrolidine-3-carboxylic acid. Compound **Z-112** can be obtained by referring to Example 118, except that (R)-pyrrolidin-3-ol is replaced by 2-aminoethanol, and (S)-4-amino-3-hydroxybutanoic acid is replaced by (R)-pyrrolidine-2-carboxylic acid. Compound **Z-115** can be obtained by referring to Example 118, except that (S)-4-amino-3-hydroxybutanoic acid is replaced by (R)-pyrrolidine-2-carboxylic acid. Compound **Z-116** can be obtained by referring to Example 109, except that 5-(methoxycarbonyl)picolinic acid is replaced by 6-methoxy-5-(methoxycarbonyl)picolinic acid, and (R)-pyrrolidine-3-carboxylic acid is replaced by (R)-pyrrolidine-2-carboxylic acid, and compound 109f is replaced by (R)-pyrrolidin-3-ol. Compound **Z-117** can be obtained by referring to Example 118, except that (S)-4-amino-3-hydroxybutanoic acid is replaced by (R)-pyrrolidine-3-carboxylic acid. Compound **Z-120** can be obtained by referring to Example 109, except that compound 109f is replaced by (R)-pyrrolidin-3-ol. Compound **Z-121** can be obtained by referring to Example 118, except that (S)-4-amino-3-hydroxybutanoic acid is replaced by 3-methylpyrrolidine-3-carboxylic acid. Compound **Z-122** can be obtained by referring to Example 118, except that (S)-4-amino-3-hydroxybutanoic acid is replaced by piperidine-4-carboxylic acid. Compound **Z-123** can be obtained by referring to Example 109, except that 5-(methoxycarbonyl)picolinic acid is replaced by 4-(methoxycarbonyl)benzoic acid, and compound 109f is replaced by (R)-pyrrolidin-3-ol. Compound **Z-124** can be obtained by referring to Example 118, except that compound 34a is replaced by compound 124a, and (S)-4-amino-3-hydroxybutanoic acid is replaced by (R)-pyrrolidine-3-carboxylic acid. Compound **Z-125** can be obtained by referring to Example 109, except that 5-(methoxycarbonyl)picolinic acid is replaced by 6-methoxy-5-(methoxycarbonyl)picolinic acid, and compound 59d is replaced by compound 94a, and (R)-pyrrolidine-3-carboxylic acid is replaced by (R)-pyrrolidine-2-carboxylic acid, and compound 109f is replaced by (R)-pyrrolidin-3-ol. Compound **Z-127** can be obtained by referring to Example 118, except that compound 34a is replaced by compound 18d, and (S)-4-amino-3-hydroxybutanoic acid is replaced by (R)-pyrrolidine-3-carboxylic acid. Compound **Z-128** can be obtained by referring to Example 118, except that (S)-4-amino-3-hydroxybutanoic acid is replaced by methyl (S)-4-amino-3-hydroxybutyrate. Compound **Z-130** can be obtained by referring to Example 118, except that (S)-4-amino-3-hydroxybutanoic acid is replaced by N-azetidine-3-carboxylic acid. Compound **Z-133** can be obtained by referring to Example 118, except that (S)-4-amino-3-hydroxybutanoic acid is replaced by methyl (R)-pyrrolidine-3-carboxylate. Compound **Z-135** can be obtained by referring to Example 118, except that (S)-4-amino-3-hydroxybutanoic acid is replaced by (R)-pyrrolidin-3-ol. Compound **Z-136** can be obtained by referring to Example 118, except that (R)-pyrrolidin-3-ol is replaced by (R)-pyrrolidine-3-carboxylic acid, and (S)-4-amino-3-hydroxybutanoic acid is replaced by (R)-pyrrolidin-3-ol. Compound **Z-139** can be obtained by referring to Example 118, except that (S)-4-amino-3-hydroxybutanoic acid is replaced by compound **139a.** Compound **Z-140** can be obtained by referring to Example 109, except that 5-(methoxycarbonyl)picolinic acid is replaced by 6-methoxy-5-(methoxycarbonyl)picolinic acid, and compound 59d is replaced by compound 85a, and compound 109f is replaced by (R)-pyrrolidin-3-ol. Compound **Z-141** can be obtained by referring to Example 109, except that 5-(methoxycarbonyl)picolinic acid is replaced by compound **141a,** and compound 109f is replaced by (R)-pyrrolidin-3-ol. Compound **Z-142** can be obtained by referring to Example 21, except that methyl (R)-pyrrolidine-3-carboxylate hydrochloride(raw materials for synthesis of compound 18c) is replaced by methyl 3-morpholinecarboxylate.

| Example number | Structure | MS or ¹H NMR |
|---|---|---|
| 63 | 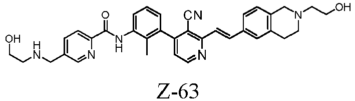 | MS m/z (ESI):589.3 [M+1] |
| 64 | 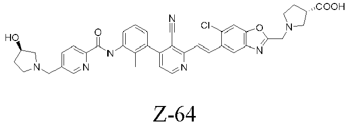 | MS m/z (ESI):718.0 [M+1] |
| 65 | 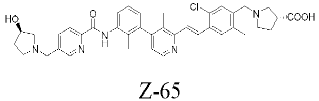 | MS m/z (ESI):680.1 [M+1] |
| | | ¹H NMR (400 MHz, DMSO-d6) δ10.33 (s, 1H), 8.61 (d, *J* = 1.2 Hz, 1H), 8.48 (d, *J* = 4.7 Hz, 1H), 8.10 (dd, *J* = 11.7, 8.8 Hz, 2H), 7.95 (dd, *J* = 8.0, 1.9 Hz, 1H), 7.86 (d, *J* = 8.1 Hz, 1H), 7.81 (s, 1H), 7.56 (d, *J* = 15.4 Hz, 1H), 7.38 - 7.29 (m, 2H), 7.04 (d, *J* = 4.8 Hz, 1H), 6.96 (d, *J* = 7.7 Hz, 1H), 4.17 (s, 1H), 3.72 - 3.63 (m, 2H), 3.52 (q, *J* = 14.2 Hz, 3H), 2.90 (dd, *J* = 14.7, 7.5 Hz, 1H), 2.70 - 2.62 (m, 3H), 2.61 - 2.54 (m, 1H), 2.51 (d, *J* = 6.8 Hz, 2H), 2.40 (dd, *J* = 14.0, 8.1 Hz, 1H), 2.34 - 2.27 (m, 4H), 2.15 (s, 3H), 2.00 - 1.90 (m, 6H), 1.57 - 1.48 (m, 1H). |
| 68 | 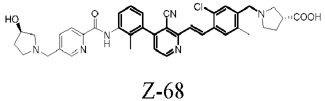 | MS m/z (ESI): 691.0 [M+1] |
| | | ¹H NMR (400 MHz, DMSO-d6) δ 10.39 (s, 1H), 8.90 (d, *J* = 5.2 Hz, 1H), 8.62 (s, 1H), 8.32 (d, *J* = 15.5 Hz, 1H), 8.11 (d, *J* = 7.9 Hz, 1H), 7.96 (d, *J* = 8.0 Hz, 2H), 7.73 (s, 1H), 7.53 (d, *J* = 15.6 Hz, 1H), 7.47 - 7.35 (m, 3H), 7.17 (d, *J* = 7.5 Hz, 1H), 4.67 (s, 1H), 4.18 (s, 1H), 3.68 (dd, *J* = 24.5, 13.9 Hz, 2H), 3.53 (s, 2H), 2.88 (s, 1H), 2.66 (d, *J* = 9.3 Hz, 4H), 2.58 (d, *J* = 6.9 Hz, 1H), 2.50 (s, 2H), 2.40 (s, 1H), 2.31 (s, 3H), 2.11 (s, 3H), 1.94 (s, 3H), 1.54 (s, 1H). |
| 69 | 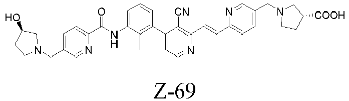 | MS m/z (ESI): 322.7 [1/2M+1] |
| 71 | 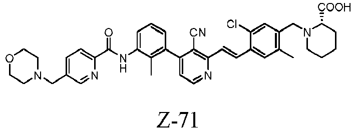 | MS m/z (ESI):705.3 [M+1] |
| | | ¹H NMR (400 MHz, DMSO-d6) δ 10.43 (s, 1H), 8.93 (d, 1H), 8.67 (s, 1H), 8.35 (d, 1H), 8.16 (d, 1H), 8.00 (t, 2H), 7.75 (s, 3H), 7.56 (d, 1H), 7.49-7.41 (m, 3H), 7.21 (d, 1H), 3.78 (d, 1H), 3.63 (s, 2H), 3.60-3.58 (m, 4H), 3.43-3.41 (m, 1H), 3.11 (s, 1H), 2.83 (s, 1H), 2.49-2.46 (m,4H), 2.34 (s, 3H), 2.21-2.15 (m, 1H), 2.14 (s, 3H), 2.00-1.94 (m, 1H), 1.76 (s, 2H), 1.47 (s, 3H). |
| 72 | 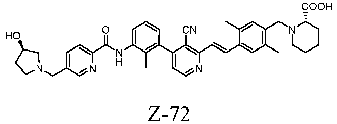 | MS m/z (ESI):685.3 [M+1] |
| | | ¹H NMR (400 MHz, DMSO-d6) δ 10.45 (s, 1H), 8.90 (d, 1H), 8.73 (s, 1H), 8.28 (d, 1H), 8.19 (d, 1H), 8.06 (s, 1H), 7.96 (d, 1H), 7.53 (s, 1H), 7.45-7.40 (m, 3H), 7.22-7.19 (m, 2H), 6.53 (s, 1H), 4.28 (s, 1H), 3.82 (d, 1H), 3.46 (d, 2H), 3.15-3.13 (m, 2H), 2.86-2.84 (m, 2H), 2.41 (s, 3H), 2.34 (s, 3H), 2.26-2.23 (m, 2H), 2.15 (s, 3H), 2.06-2.03 (m, 2H), 1.76 (s, 3H), 1.46-1.23 (m, 5H). |
| 74 | 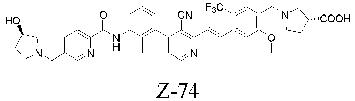 | MS m/z (ESI):741.3 [M+1] |
| | | ¹H NMR (400 MHz, DMSO-d6) δ10.40 (s, 1H), 8.92 (d, J = 4.9 Hz, 1H), 8.62 (s, 1H), 8.29 (d, J = 15.1 Hz, 1H), 8.11 (d, J = 8.0 Hz, 1H), 7.95 (d, J = 8.1 Hz, 2H), 7.70 (s, 1H), 7.60 (d, J = 15.2 Hz, 1H), 7.54 - 7.36 (m, 3H), 7.17 (d, J = 7.3 Hz, 1H), 4.70 (s, 1H), 4.17 (s, 1H), 3.94 (s, 3H), 3.73 - 3.63 (m, 4H), 2.97 - 2.84 (m, 2H), 2.71 - 2.62 (m, 3H), 2.52 (t, J = 7.0 Hz, 2H), 2.40 (d, J = 6.0 Hz, 1H), 2.31 (dd, J = 9.5, 3.6 Hz, 1H), 2.11 (s, 3H), 2.00 - 1.89 (m, 3H), 1.57 - 1.47 (m, 1H). |
| 75 | 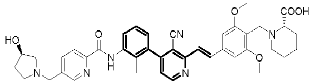 | MS m/z (ESI): 717.1 [M+1] |
| | | ¹H NMR (400 MHz, DMSO-d6) δ 10.40 (s, 1H), 8.87 (d, *J* = 4.8 Hz, 1H), 8.62 (s, 1H), 8.11 (d, *J* = 8.1 Hz, 1H), 8.05 (d, *J* = 15.6 Hz, 1H), 7.95 (d, *J* = 8.0 Hz, 2H), 7.55 (d, *J* = 15.4 Hz, 1H), 7.40 (dd, *J* = 9.7, 6.4 Hz, 2H), 7.17 (d, *J* = 7.2 Hz, 1H), 7.03 (s, 2H), 4.75 - 4.61 (m, 1H), 4.17 (s, 1H), 3.99 (s, 2H), 3.82 (s, 6H), 3.68 (dd, *J* = 24.4, 13.8 Hz, 3H), 3.12 (s, 1H), 3.03 (s, 1H), 2.66 (dd, *J* = 9.5, 6.0 Hz, 1H), 2.62 - 2.55 (m, 1H), 2.43 - 2.36 (m, 1H), 2.31 (dd, *J* = 9.6, 3.7 Hz, 1H),2.11 (s, 3H), 1.97 (dd, *J* = 12.8, 6.9 Hz, 1H), 1.73 (d, *J* = 6.0 Hz, 2H), 1.48 (s, 3H), 1.35 (s, 2H) |
| | 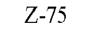 | |
| 76 | 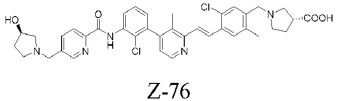 | MS m/z (ESI):700.0 [M+1] |
| | | ¹H NMR (400 MHz, DMSO-d6) δ 10.69 (s, 1H), 8.64 (s, 1H), 8.56 - 8.39 (m, 2H), 8.16 (d, J = 8.0 Hz, 1H), 8.09 (d, J = 15.5 Hz, 1H), 7.99 (d, J = 9.7 Hz, 1H), 7.82 (s, 1H), 7.61 - 7.48 (m, 2H), 7.35 (s, 1H), 7.17 - 7.06 (m, 2H), 4.71 (s, 1H), 4.17 (s, 1H), 3.75 - 3.66 (m, 2H), 3.56 - 3.50 (m, 2H), 2.95 - 2.81 (m, 2H), 2.66 - 2.62 (m, 5H), 2.49 - 2.47 (m, 1H), 2.43 - 2.38 (m, 1H), 2.36 - 2.27 (m, 3H), 2.18 (s, 3H), 2.05 - 1.85 (m, 3H), 1.53 - 1.51 (m, 1H). |
| 77 | 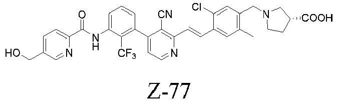 | MS m/z (ESI):676.0 [M+1] |
| | | ¹H NMR (400 MHz, DMSO-*d*₆) δ 10.53 (s, 1H), 8.86 (s, 1H), 8.72 (d, *J* = 5.9 Hz, 1H), 8.29 - 8.08 (m, 4H), 7.82 - 7.66 (m, 3H), 7.45 - 7.36 (m, 2H), 7.34 (d, *J* = 6.1 Hz, 1H), 5.56 (s, 2H), 3.62 - 3.46 (m, 3H), 2.84 (s, 2H), 2.66 - 2.63(m, 2H), 2.30 (s, 3H), 2.02 - 1.83 (m, 2H), 1.19 (s, 1H). |
| 80 | 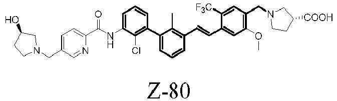 | MS m/z (ESI):749.0 [M+1] |
| | | ¹H NMR (400 MHz, DMSO-*d*₆) δ 10.69 (s, 1H), 8.64 (s, 1H), 8.45 (d, *J* = 8.2 Hz, 1H), 8.16 (d, *J* = 7.8 Hz, 1H), 8.00 (d, *J* = 7.7 Hz, 1H), 7.69 - 7.54 (m, 3H), 7.52 - 7.42 (m, 2H), 7.33 (t, *J* = 7.8 Hz, 1H), 7.22 (d, *J* = 16.2 Hz, 1H), 7.16 - 7.07 (m, 2H), 4.68 (s, 1H), 4.17 (s, 1H), 3.93 (s, 3H), 3.78 - 3.53 (m, 4H), 3.01 - 2.85 (m, 2H), 2.72 - 2.63 (m, 3H), 2.63 - 2.49 (m, 2H), 2.45 - 2.36 (m, 1H), 2.31 (d, *J* = 9.8 Hz, 1H), 2.14 (s, 3H), 2.05 - 1.89 (m, 3H), 1.61 - 1.46 (m, 1H). |
| 81 | 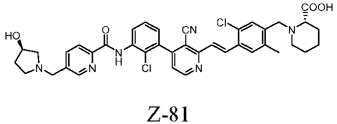 | MS m/z (ESI):725.0 [M+1] |
| 82 | 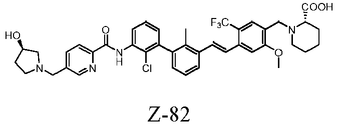 | MS m/z (ESI):763.0 [M+1] |
| | | ¹H NMR (400 MHz, DMSO-*d*₆) δ 10.69 (s, 1H), 8.63 (s, 1H), 8.44 (d, *J* = 8.0 Hz, 1H), 8.16 (d, *J* = 8.0 Hz, 1H), 7.98 (d, *J* = 8.1 Hz, 1H), 7.73 (s, 1H), 7.64 - 7.57 (m, 2H), 7.50 - 7.47 (m, 2H), 7.37 - 7.28 (m, 1H), 7.21 (d, *J* = 14.0 Hz, 1H), 7.15 - 7.05 (m, 2H), 4.69 (s, 1H), 4.17 (s, 1H), 3.92 (s, 3H), 3.81 - 3.58 (m, 4H), 2.88 - 2.84 (m, 2H), 2.70 - 2.53 (m, 2H), 2.43 - 2.37 (m, 1H), 2.30 (d, *J* = 10.4 Hz, 1H), 2.23 (s, 1H), 2.13 (s, 3H), 2.05 - 1.91 (m, 1H), 1.84 - 1.69 (m, 2H), 1.56 - 1.42 (m, 3H), 1.37 (s, 1H). |
| 83 | 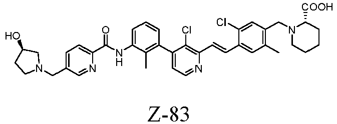 | MS m/z (ESI):714.0 [M+1] |
| 84 | 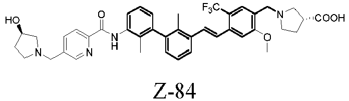 | MS m/z (ESI):729.1[M+1] |
| | | ¹H NMR (400 MHz, DMSO-d6) δ 10.31 (s, 1H), 8.61 (s, 1H), 8.22 (s, 1H), 8.11 (d, *J* = 7.5 Hz, 1H), 7.95 (d, *J* = 7.1 Hz, 1H), 7.83 (d, *J* = 7.8 Hz, 1H), 7.59 (dd, *J* = 16.5, 11.6 Hz, 3H), 7.47 (s, 1H), 7.30 (dd, *J* = 15.2, 7.8 Hz, 2H), 7.22 (d, *J* = 16.5 Hz, 1H), 7.07 (d, *J* = 7.2 Hz, 1H), 6.95 (d, *J* = 6.9 Hz, 1H), 4.17 (s, 1H), 3.93 (s, 3H), 3.73 - 3.60 (m, 4H), 2.91 - 2.89 (m, 1H), 2.67 (s, 3H), 2.59 (d, *J* = 7.3 Hz, 1H), 2.52 - 2.50 (m, 2H), 2.41 (d, *J* = 5.8 Hz, 2H), 2.31 (d, *J* = 9.6 Hz, 2H), 2.11 (s, 3H), 1.98 (s, 3H), 1.94 (d, *J* = 7.9 Hz, 2H), 1.53 - 1.51 (m, 1H). |
| 85 | 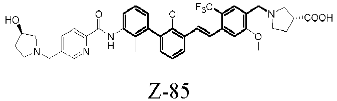 | MS m/z (ESI):749.0 [M+1] |
| | | 1H NMR (400 MHz, DMSO-d6) δ 10.32 (s, 1H), 8.61 (s, 1H), 8.11 (d, J = 7.9 Hz, 1H), 7.95 (d, J = 7.8 Hz, 1H), 7.86 (d, J = 8.0 Hz, 1H), 7.78 (d, J = 7.7 Hz, 1H), 7.69 - 7.56 (m, 2H), 7.52 - 7.22 (m, 6H), 7.00 (d, J = 7.4 Hz, 1H), 4.69 (s, 1H), 4.17 (s, 1H), 3.92 (s, 3H), 3.73 - 3.57 (m, 3H), 2.90 (dd, J = 15.2, 7.4 Hz, 2H), 2.70 - 2.62 (m, 3H), 2.61 - 2.52 (m, 2H), 2.42 - 2.24 (m, 2H), 2.01 (d, J = 8.3 Hz, 3H), 1.99 - 1.88 (m, 3H), 1.53 (d, J = 3.3 Hz, 1H). |
| 86 | 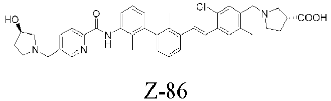 | MS m/z (ESI): 679.1 [M+1] |
| | | ¹H NMR (400 MHz, DMSO-d6) δ 10.30 (s, 1H), 8.61 (s, 1H), 8.27 (s, 0.5H), 8.11 (d, *J* = 8.0 Hz, 1H), 7.95 (dd, *J* = 8.0, 2.0 Hz, 1H), 7.84 (d, *J* = 8.1 Hz, 1H), 7.70 (s, 1H), 7.63 (d, *J* = 7.7 Hz, 1H), 7.50 (d, *J* = 16.0 Hz, 1H), 7.38 - 7.21 (m, 4H), 7.05 (d, *J* = 7.7 Hz, 1H), 6.95 (d, *J* = 7.6 Hz, 1H), 4.22 - 4.14 (m, 1H), 3.68 (dd, *J* = 24.8, 13.7 Hz, 3H), 3.51 (dd, *J* = 19.2, 13.8 Hz, 2H), 2.88 (d, *J* = 7.6 Hz, 1H), 2.71 - 2.55 (m, 4H), 2.52 - 2.47 (m, 2H), 2.41 (dd, *J* = 14.1, 8.3 Hz, 1H), 2.35 - 2.25 (m, 4H), 2.12 (d, *J* = 24.3 Hz, 3H), 2.04 - 1.86 (m, 6H), 1.58 - 1.48 (m, 1H). |
| 88 | 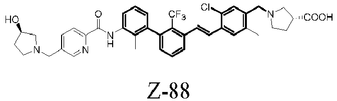 | MS m/z (ESI): 733.0 [M+1] |
| | | ¹H NMR (400 MHz, DMSO-*d*₆) δ 10.31 (s, 1H), 8.64 (s, 1H), 8.19 - 8.08 (m, 1H), 7.98 (d, *J* = 9.1 Hz, 1H), 7.89 - 7.80 (m, 2H), 7.76 - 7.67 (m, 1H), 7.58 (s, 1H), 7.53 - 7.43 (m, 1H), 7.38 (s, 1H), 7.33 - 7.23 (m, 2H), 7.20 (d, *J* = 7.1 Hz, 1H), 6.96 (d, *J* = 7.3 Hz, 1H), 4.80 (s, 1H), 4.20 (s, 1H), 3.77 (s, 1H), 3.55 (s, 2H), 3.13 - 2.99 (m, 2H), 2.97 - 2.82 (m, 2H), 2.78 - 2.61 (m, 3H), 2.58 - 2.50 (m, 1H), 2.30 (s, 4H), 1.97 (s, 3H), 1.97 - 1.87 (m, 3H), 1.56 (s, 2H). |
| 89 | 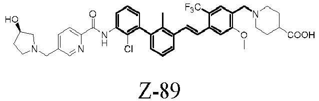 | MS m/z (ESI): 763.0 [M+1] |
| | | ¹H NMR (400 MHz, DMSO-*d*₆) δ 10.69 (s, 1H), 8.64 (s, 1H), 8.45 (d, *J* = 6.8 Hz, 1H), 8.16 (d, *J* = 7.7 Hz, 2H), 7.99 (d, *J* = 8.0 Hz, 1H), 7.67 - 7.56 (m, 2H), 7.54 - 7.44 (m, 2H), 7.33 - 7.32 (m, 1H), 7.21 (d, *J* = 16.2 Hz, 1H), 7.17 - 7.08 (m, 2H), 4.69 (s, 1H), 4.17 (s, 1H), 3.93 (s, 3H), 3.76 - 3.61 (m, 2H), 3.47 (s, 2H), 2.79 - 2.62 (m, 5H), 2.59- 2.55 (m, 1H), 2.45 - 2.38 (m, 1H), 2.34 - 2.31 (m, 1H), 2.14 (s, 3H), 2.10 - 1.99 (m, 2H), 1.99 - 1.90 (m, 1H), 1.76 (d, *J* = 9.2 Hz, 2H), 1.63 - 1.46 (m, 2H). |
| 90 | 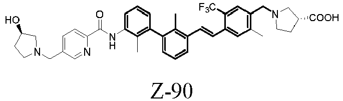 | MS m/z (ESI):713.1 [M+1] |
| | | ¹H NMR (400 MHz, DMSO-d6) δ 10.35 (s, 1H), 8.69 (s, 1H), 8.18-8.13 (m, 1H), 8.04 (s, 1H), 7.87-7.84 (m, 2H), 7.64(s, 1H) 7.61-7.54 (m, 2H), 7.35-7.30 (m, 2H), 7.26-7.22 (m, 1H), 7.09 (d, 1H), 6.99 (d, 1H), 4.83 (s, 1H), 4.25 (s, 1H), 3.83-3.67 (m, 4H), 2.90-2.85 (m, 2H), 2.75-2.71 (m, 4H), 2.61 (s, 3H), 2.42 (s, 3H), 2.13 (s, 3H), 2.02-1.97 (m, 6H), 1.63 (s, 1H). |
| 91 | 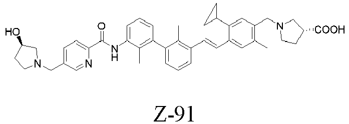 | MS m/z (ESI):685.2 [M+1] |
| 92 | 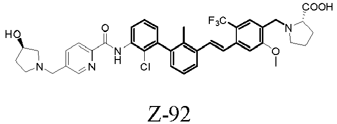 | MS m/z (ESI):749.0 [M+1] |
| | | ¹H NMR (400 MHz, DMSO-*d*₆) δ 10.69 (s, 1H), 8.64 (s, 1H), 8.45 (d, *J* = 8.3 Hz, 1H), 8.16 (d, *J* = 8.1 Hz, 1H), 7.99 (d, *J* = 9.9 Hz, 1H), 7.74 (s, 1H), 7.70 - 7.57 (m,2H), 7.54 - 7.44 (m, 2H), 7.34 (t, *J* = 7.7 Hz, 1H), 7.21 (d, *J* = 14.0 Hz, 1H), 7.12 (dd, *J* = 12.2, 7.6 Hz, 1H), 4.69 (s, 1H), 4.17 (s, 1H), 4.01 (d, *J* = 13.6 Hz, 1H), 3.96 (s, 3H), 3.90 (d, *J* = 14.0 Hz, 1H), 3.76 - 3.60 (m, 2H), 3.16 - 3.04 (m, 1H), 2.83 (s, 1H), 2.70 - 2.64 (m, 1H), 2.64 - 2.54 (m, 2H), 2.44 - 2.40 (m, 1H), 2.31 (dd, *J* = 9.4, 3.9 Hz, 1H), 2.14 (s, 3H), 2.11 - 2.03 (m, 1H), 1.98 (dt, *J* = 13.2, 6.7 Hz, 1H), 1.91 - 1.83 (m, 1H), 1.81 - 1.74 (m, 1H), 1.73 - 1.62 (m, 1H), 1.58 - 1.48 (m, 1H). |
| 93 | 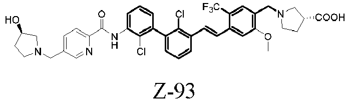 | MS m/z (ESI):769.0 [M+1] |
| | | ¹H NMR (400 MHz, DMSO-*d*₆) δ 10.68 (s, 1H), 8.65 (s, 1H), 8.48 (d, *J* = 7.0 Hz, 1H), 8.17 (d, *J* = 8.3 Hz, 1H), 8.10 (s, 1H), 8.02 (d, *J* = 10.2 Hz, 1H), 7.83 (d, *J* = 7.0 Hz, 1H), 7.68 (s, 1H), 7.59 (d, *J* = 16.0 Hz, 1H), 7.50 (t, *J* = 7.9 Hz, 2H), 7.45 (s, 1H), 7.40 - 7.30 (m, 2H), 7.16 (d, *J* = 7.5 Hz, 1H), 4.74 (s, 1H), 4.19 (s, 1H), 3.93 (s, 3H), 3.84 - 3.55 (m, 4H), 2.94 (s, 2H), 2.79 - 2.52 (m, 5H), 2.29 (s, 1H), 2.06 - 1.86 (m, 4H), 1.56 (s, 1H). |
| 94 | 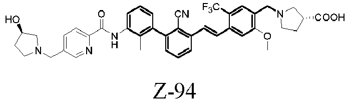 | MS m/z (ESI): 740.1 [M+1] |
| | | ¹H NMR (400 MHz, DMSO-d6) δ 10.36 (s, 1H), 8.62 (s, 1H), 8.11 (d, *J* = 8.0 Hz, 1H), 8.01 - 7.89 (m, 3H), 7.80 (t, *J* = 7.8 Hz, 1H), 7.67 (s, 1H), 7.64 - 7.39 (m, 4H), 7.36 (t, *J* = 7.8 Hz, 1H), 7.12 (d, *J* = 7.2 Hz, 1H), 4.70 (s, 1H), 4.17 (s, 1H), 3.92 (s, 3H), 3.64 (ddd, *J* = 31.8, 25.8, 14.3 Hz, 4H), 2.89 (dd, *J* = 14.6, 7.4 Hz, 1H), 2.71 - 2.49 (m, 6H), 2.41 (dd, *J* = 13.8, 8.2 Hz, 1H), 2.31 (dd, *J* = 9.6, 3.7 Hz, 1H), 2.09 (s, 3H), 1.96 (ddd, J= 21.3, 14.1, 7.1 Hz, 3H), 1.57 - 1.48 (m, 1H). |
| 95 | 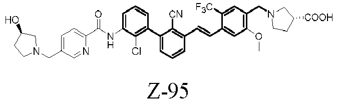 | MS m/z (ESI):760.0 [M+1] |
| 96 | 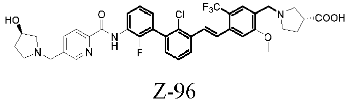 | MS m/z (ESI):377.1 [1/2M+1] |
| | | ¹H NMR (400 MHz, DMSO-d6) δ 10.46 (d, J = 2.1 Hz, 1H), 8.66 (d, J = 1.9 Hz, 1H), 8.24 (t, J = 7.5 Hz, 1H), 8.16 (d, J = 8.0 Hz, 1H), 8.01 (dd, J = 8.0, 2.0 Hz, 1H), 7.86 (d, J = 7.7 Hz, 1H), 7.71 - 7.59 (m, 2H), 7.54 (t, J = 7.7 Hz, 1H), 7.49 - 7.32 (m, 3H), 7.20 (t, J = 6.8 Hz, 1H), 4.73 (s, 1H), 4.21 (tt, J = 6.8, 3.4 Hz, 1H), 3.96 (s, 3H), 3.82 - 3.55 (m, 6H), 3.01 - 2.87 (m, 2H), 2.81 - 2.52 (m, 5H), 2.48 - 2.29 (m, 2H), 2.10 - 1.91 (m, 3H), 1.63-1.50 (m, 1H). |
| 97 | 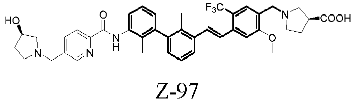 | MS m/z (ESI): 729.1 [M+1] |
| | | ¹H NMR (400 MHz,DMSO-d6) δ 10.32 (s, 1H), 8.64 (s, 1H), 8.11 (d, *J* = 5.6 Hz, 1H), 8.00 (s, 1H), 7.82 (d, *J* = 8.4 Hz, 1H), 7.68 - 7.55 (m, 2H), 7.49 (s, 1H), 7.27 (dt, *J* = 31.3, 12.3 Hz, 3H), 7.08 (d, *J* = 7.5 Hz, 1H), 6.96 (d, *J* = 7.6 Hz, 1H), 6.49 (s, 1H), 4.84 - 4.73 (m, 1H), 4.20 (s, 1H), 3.94 (s, 3H), 3.71 (s, 4H), 2.96 (s, 2H), 2.70 (d, *J* = 47.5 Hz, 6H), 2.11 (s, 3H), 1.98 (s, 6H), 1.57 (s, 1H). |
| 98 | 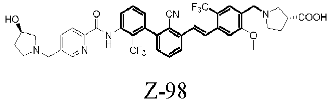 | MS m/z (ESI): 397.6 [1/2M+1] |
| | | ¹H NMR (400 MHz, DMSO-*d*₆) δ 10.69 (s, 1H), 8.63 (s, 1H), 8.31 (s, 2H), 8.23 (d, *J* = 7.6 Hz, 1H), 8.15 (d, *J* = 7.8 Hz, 1H), 8.05 - 7.92 (m, 2H), 7.86 - 7.75 (m, 2H), 7.67 (s, 1H), 7.59 - 7.41 (m, 2H), 7.30 (d, *J* = 7.6 Hz, 1H), 4.17 (s, 1H), 3.92 (s, 3H), 3.78 - 3.55 (m, 6H), 2.75 - 2.63 (m, 3H), 2.63 - 2.55 (m, 1H), 2.57 - 2.48 (m, 2H), 2.42 - 2.38 (m, 1H), 2.35 - 2.28 (m, 2H), 2.04 - 1.89 (m, 2H), 1.54 (s, 1H). |
| 99 | 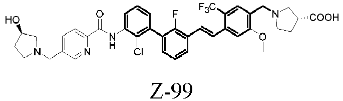 | MS m/z (ESI): 753.0 [M+1] |
| | | ¹H NMR (400 MHz, DMSO-d6) δ 10.75 (s, 1H), 8.69 (s, 1H), 8.51 - 8.49 (m, 1H), 8.22 - 8.20 (m, 1H), 8.05 - 8.03 (m, 1H), 7.84 - 7.80 (m, 1H), 7.68 (s, 1H), 7.58-7.51 (m, 3H), 7.47-7.37 (m, 3H), 7.30 -7.28 (m, 1H), 4.76 (br, 1H), 3.98(br, 1H), 3.78 (s, 3H), 3.75-3.42 (m, 5H), 2.72-2.69 (m, 1H), 2.56-2.52 (m, 4H), 2.48-2.42 (m, 1H), 2.36-2.33 (m, 1H), 2.04-1.92 (m,4H), 1.54-1.52 (m, 2H) |
| 100 | 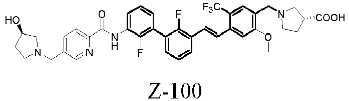 | MS m/z (ESI): 737.3 [M+1] |
| | | ¹H NMR (400 MHz, DMSO-d6) δ 10.50 (s, 1H), 8.67 (s, 1H), 8.19 (t, 1H), 8.12 (d, 1H), 7.97 (dd, 1H), 7.78 (t, 1H), 7.64 (s, 1H), 7.47-7.42 (m, 4H), 7.39-7.33 (m, 2H),7.27 (t, 1H), 4.74 (br, 1H), 4.17(br, 1H), 3.94 (s, 3H), 3.73-3.54 (m, 5H), 2.93-2.87 (m, 1H), 2.69-2.52 (m, 4H), 2.44-2.38 (m, 1H), 2.33-2.29 (m, 1H), 2.04-1.92 (m,4H), 1.54-1.52 (m, 2H) |
| 101 | 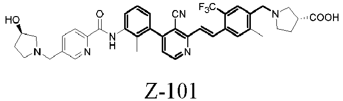 | MS m/z (ESI): 725.1 [M+1] |
| | | ¹H NMR (400 MHz, CD₃OD) δ 8.85 (*d*, *J* = 4.0Hz, 1H), 8.68 (s, 1H), 8.44 - 8.39 (m, 1H), 8.20 - 8.18 (m, 1H), 8.05 - 8.00 (m, 2H), 7.86 (s, 1H), 7.81 (s, 1H), 7.63 - 7.59 (m, 1H), 7.45 - 7.39 (m, 2H), 7.20 - 7.17 (m, 1H), 4.65 - 4.50 (m, 4H), 4.38 - 4.33 (m, 1H), 4.11 (s, 1H), 3.86 - 3.77 (m, 2H), 3.15 - 3.02 (m, 3H), 2.86 - 2.80 (m, 2H), 2.64 - 2.54 (m, 4H), 2.22 - 2.12 (m, 5H), 1.78 - 1.70 (m, 2H). |
| 102 | 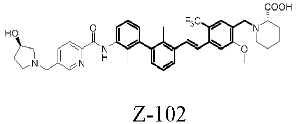 | MS m/z (ESI): 743.1 [M+1] |
| | | ¹H NMR (400 MHz, DMSO-d6) δ 10.31 (s, 1H), 8.61 (s, 1H), 8.11 (d, *J* = 8.0 Hz, 1H), 7.95 (dd, *J* = 8.0, 1.8 Hz, 1H), 7.83 (d, *J* = 8.1 Hz, 1H), 7.73 (s, 1H), 7.59 (dd, *J* = 17.2, 11.9 Hz, 2H), 7.47 (s, 1H), 7.27 (dt, J= 30.6, 11.7 Hz, 3H), 7.07 (d, *J* = 7.4 Hz, 1H), 6.95 (d, *J* = 7.3 Hz, 1H), 4.70 (s, 1H), 4.17 (s, 1H), 3.92 (s, 3H), 3.71 (dt, *J* = 24.9, 14.4 Hz, 4H), 3.16 (s, 1H), 2.90 - 2.82 (m, 1H), 2.67 (dd, *J* = 9.7, 6.1 Hz, 1H), 2.59 (dd, *J* = 15.2, 7.8 Hz, 1H), 2.43 - 2.37 (m, 1H), 2.31 (dd, *J* = 9.5, 3.5 Hz, 1H), 2.26 (s, 1H), 2.11 (s, 3H), 2.04 - 1.91 (m, 4H), 1.75 (d, *J* = 10.4 Hz, 2H), 1.58 - 1.29 (m, 5H). |
| 103 | 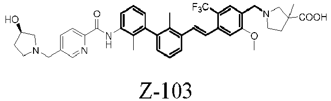 | MS m/z (ESI): 743.1 [M+1] |
| | | ¹H NMR (400 MHz, DMSO-d6) δ 10.31 (s, 1H), 8.61 (d, *J* = 1.4 Hz, 1H), 8.11 (d, *J* = 8.2 Hz, 1H), 7.95 (dd, *J* = 8.0, 2.0 Hz, 1H), 7.84 (d, *J* = 7.7 Hz, 1H), 7.69 - 7.53 (m, 3H), 7.47 (s, 1H), 7.26 (ddd, *J* = 18.0, 15.4, 4.9 Hz, 3H), 7.07 (d, *J* = 6.8 Hz, 1H), 6.95 (d, *J* = 6.6 Hz, 1H), 4.68 (s, 1H), 4.22 - 4.14 (m, 1H), 3.64 (ddd, *J* = 31.6, 23.2, 14.4 Hz, 4H), 2.94 (d, *J* = 9.1 Hz, 1H), 2.71 - 2.52 (m, 4H), 2.41 (td, *J* = 8.1, 5.7 Hz, 1H), 2.34 - 2.19 (m, 3H), 2.11 (s, 3H), 2.03 - 1.91 (m, 4H), 1.59 - 1.47 (m, 2H), 1.22 (s, 3H). |
| 104 | 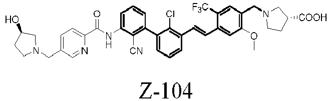 | MS m/z (ESI):760.0[M+1] |
| | | ¹H NMR (400 MHz, DMSO-d6) δ 10.94 (s, 1H), 8.68 (s, 1H), 8.16 (dd, *J* = 8.1, 4.3 Hz, 2H), 8.02 (d, *J* = 7.7 Hz, 1H), 7.88 (d, *J* = 6.7 Hz, 1H), 7.82 (t, *J* = 8.0 Hz, 1H), 7.74 - 7.57 (m, 3H), 7.54 (t, *J* = 7.6 Hz, 1H), 7.48 - 7.36 (m, 3H), 7.33 (d, *J* = 6.9 Hz, 1H), 6.71 (s, 1H), 4.77 (s, 2H), 4.19 (s, 2H), 3.96 - 3.94 (m, 3H), 3.75 - 3.64 (m, 4H), 2.95 - 2.93 (m, 2H), 2.72 - 2.46 (m, 4H), 2.06 - 1.93 (m, 3H), 1.57 - 1.55 (m, 2H). |
| 105 | 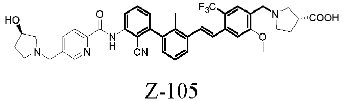 | MS m/z (ESI): 740.3 [M+1] |
| | | ¹H NMR (400 MHz, DMSO-d6) δ 10.92 (s, 1H), 8.65 (s, 1H), 8.16 - 8.11 (m, 2H), 7.99 (d, J = 9.7 Hz, 1H), 7.81 - 7.77 (m, 1H), 7.64 (dd, J = 13.3, 8.8 Hz, 3H), 7.49 (s, 1H), 7.36 (d, J = 7.7 Hz, 1H), 7.24 (dd, J = 15.8, 7.1 Hz, 3H), 4.72 (s, 1H), 4.17 (s, 1H), 3.94 (s, 3H), 3.63 (dd, J = 27.8, 13.5 Hz, 5H), 2.91 (s, 1H), 2.67 - 2.53 (m, 4H), 2.34 - 2.27 (m, 2H), 2.22 (s, 3H), 1.97 (d, J = 19.7 Hz, 4H), 1.52 (s, 2H). |
| 106 | 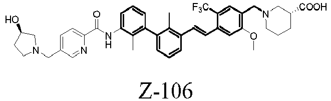 | MS m/z (ESI):743.1 [M+1] |
| | | ¹H NMR (400 MHz, DMSO-*d*₆) δ 10.30 (s, 1H), 8.61 (s, 1H), 8.10 (d, *J* = 8.3 Hz, 1H), 7.94 (d, *J* = 8.0 Hz, 1H), 7.83 (d, *J* = 7.7 Hz, 1H), 7.69 - 7.52 (m, 3H), 7.47 (s, 1H), 7.36 - 7.18 (m, 3H), 7.07 (d, *J* = 7.6 Hz, 1H), 6.96 (t, *J* = 6.8 Hz, 1H), 4.68 (s, 1H), 4.17 (s, 1H), 3.92 (s, 3H), 3.72 - 3.68 (m, 2H), 3.48 (s, 2H), 2.76 (d, *J* = 9.9 Hz, 1H), 2.70 - 2.53 (m, 3H), 2.44 - 2.36 (m, 2H), 2.33 - 2.30 (m, 1H), 2.25 - 2.16 (m, 1H), 2.10 (s, 3H), 1.97 (s, 3H), 1.97 - 1.90 (m, 1H), 1.82 - 1.70 (m, 2H), 1.63 - 1.32 (m, 4H). |
| 107 | 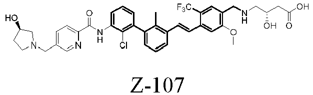 | MS m/z (ESI): 753.0 [M+1] |
| 111 | 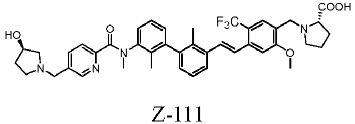 | MS m/z (ESI):743.4 [M+1] |
| | | ¹H NMR (400 MHz, DMSO-*d₆*) δ 8.18 - 8.11 (m, 1H), 7.75 (s, 1H), 7.67 - 7.47 (m, 4H), 7.41 (dd, J = 15.2, 8.0 Hz, 1H), 7.31 - 7.08 (m, 4H), 7.05 (d, J = 7.5 Hz, 1H), 6.89 (d, J = 7.2 Hz, 1H), 4.69 (s, 1H), 4.12 - 4.01 (m, 2H), 3.96 (d, J = 8.9 Hz, 3H), 2.64 (d, J = 7.9 Hz, 2H), 2.52 (m, 1H), 2.48 (s, 6H), 2.42 (m, 1H), 2.33 - 2.09 (m, 4H), 1.84 (m, 2H), 1.69 (m, 2H), 1.48 - 1.38 (m, 1H). |
| 112 | 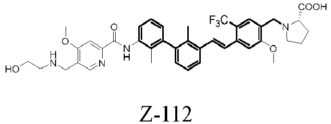 | MS m/z (ESI): 367.1 [1/2M+1] |
| | | ¹H NMR (400 MHz, CD₃OD) δ 8.45 (s, 1H), 7.92 (*d*, *J* = 8.0Hz, 1H), 7.85 (s, 1H), 7.79 (s, 1H), 7.64 - 7.51 (m, 3H), 7.33 - 7.28 (m, 3H), 7.12 -7.10 (m, 1H), 7.02 - 7.01 (m, 1H), 4.57 (s, 1H), 4.47 - 4.44 (m, 1H), 4.38 - 4.35 (m, 1H), 4.08 (s, 3H), 4.03 (s, 3H), 3.90 - 3.86 (m, 3H), 3.67 - 3.65 (m, 2H), 3.59 - 3.54 (m, 1H), 3.17 - 3.13 (m, 1H), 2.74 - 2.71 (m, 2H), 2.45 - 2.40 (m, 1H), 2.16 - 2.06 (m, 7H), 1.94 - 1.87 (m, 2H). |
| 115 | 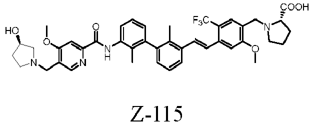 | MS m/z (ESI): 759.3 [M+1] |
| | | ¹H NMR (400 MHz, CD₃OD) δ 8.49 (s, 1H), 7.92 (*d*, *J* = 8.0Hz, 1H), 7.83 (s, 1H), 7.79 (s, 1H), 7.64 - 7.56 (m, 2H), 7.52 (s, 1H), 7.34 - 7.28 (m, 3H), 7.12 - 7.10 (m, 1H), 7.02 - 7.01 (m, 1H), 4.82 - 4.75 (m, 1H), 4.48 - 4.32 (m, 3H), 4.09 (s, 3H), 4.01 (s, 3H), 3.92 - 3.89 (m, 1H), 3.81 - 3.73 (m, 2H), 3.61 - 3.56 (m, 1H), 3.21 - 3.14 (m, 1H), 2.88 - 2.78 (m, 2H), 2.63 - 2.59 (m, 2H), 2.46 - 2.38 (m, 1H) , 2.20 - 2.06 (m, 9H), 2.01 - 1.87 (m, 1H), 1.74 - 1.67 (m, 1H). |
| 116 | 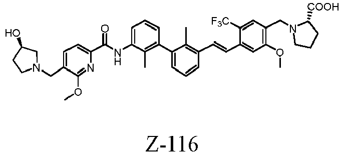 | MS m/z (ESI):759.4[M+1] |
| | | ¹H NMR (400 MHz, DMSO-d6) δ 10.16 (s, 1H), 7.97 (d, *J* = 7.3 Hz, 1H), 7.90 (d, *J* = 7.4 Hz, 1H), 7.80 - 7.70 (m, 2H), 7.65 - 7.50 (m, 2H), 7.50 (s, 1H), 7.31 (t, *J* = 7.8 Hz, 2H), 7.21 (d, *J* = 15.8 Hz, 1H), 7.07 (d, *J* = 7.6 Hz, 1H), 6.95 (d, *J* = 6.7 Hz, 1H), 4.69 (s, 1H), 4.18 (s, 1H), 4.03 - 4.00 (m, 3H), 3.95 (s, 3H), 3.85 (d, *J* = 14.7 Hz, 1H), 3.58 (q, *J* = 15.3 Hz, 2H), 3.09 - 3.08 (m, 2H), 2.69 - 2.67 (m, 1H), 2.62 - 2.61 (m, 1H), 2.53 - 2.52 (m, 1H), 2.48 - 2.46 (m, 2H), 2.36 - 2.33 (m, 2H), 2.12 (s, 3H), 2.03 - 2.02 (m, 3H), 2.00 - 1.98 (m, 1H), 1.87 - 1.86 (m, 1H), 1.70 - 1.66 (m, 2H), 1.53 - 1.52 (m, 1H). |
| 117 | 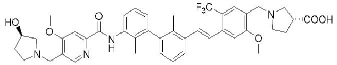 | MS m/z (ESI):380.2 [M/2+1] |
| | 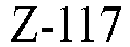 | ¹H NMR (400 MHz, DMSO-*d₆*) δ 10.34 (s, 1H), 8.45 (s, 1H), 7.86 (d, J = 8.0 Hz, 1H), 7.71 (s, 1H), 7.66 - 7.52 (m, 3H), 7.47 (s, 1H), 7.34 - 7.17 (m, 3H), 7.07 (d, J = 7.4 Hz, 1H), 6.95 (d, J = 7.5 Hz, 1H), 4.68 (s, 1H), 4.15 (m, 1H), 3.94 (d, J = 7.3 Hz, 6H), 3.60 (m, 4H), 2.96 - 2.84 (m, 2H), 2.72 - 2.57 (m, 4H), 2.56 - 2.49 (m, 2H), 2.44 - 2.38 (m, 1H), 2.34 (dd, J = 9.6, 3.6 Hz, 1H), 2.10 (s, 3H), 1.98 (s, 3H), 1.93 (m, 2H), 1.51 (m, 1H). |
| 120 | 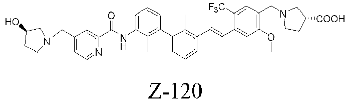 | MS m/z (ESI):729.4 [M+1] |
| 121 | 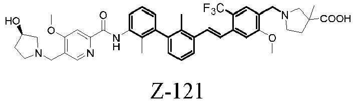 | MS m/z (ESI):387.2 [M/2+1] |
| | | ¹H NMR (400 MHz, DMSO-*d₆*) δ 10.34 (s, 1H), 8.45 (s, 1H), 7.86 (d, J = 8.1 Hz, 1H), 7.71 (s, 1H), 7.65 - 7.55 (m, 3H), 7.47 (s, 1H), 7.33 - 7.26 (m, 2H), 7.23 (s, 1H), 7.07 (d, J = 7.5 Hz, 1H), 6.94 (d, J = 7.5 Hz, 1H), 4.68 (s, 1H), 4.15 (m, 1H), 3.94 (d, J = 7.7 Hz, 6H), 3.61 (dd, J = 14.9, 3.5 Hz, 4H), 2.68 - 2.55 (m, 4H), 2.43 - 2.38 (m, 1H), 2.34 (dd, J = 9.6, 3.6 Hz, 1H), 2.24 (m, 2H), 2.10 (s, 3H), 1.98 (m, 4H), 1.60 - 1.43 (m, 3H), 1.21 (s, 3H). |
| 122 | 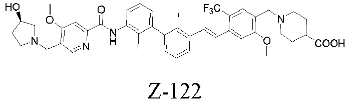 | MS m/z (ESI):387.1 [M/2+1] |
| | | ¹H NMR (400 MHz, DMSO-*d₆*) δ 10.34 (s, 1H), 8.45 (s, 1H), 7.87 (d, J = 8.0 Hz, 1H), 7.71 (s, 1H), 7.65 - 7.53 (m, 3H), 7.47 (s, 1H), 7.34 - 7.26 (m, 2H), 7.21 (dd, J = 15.8, 2.5 Hz, 1H), 7.07 (d, J = 7.5 Hz, 1H), 6.95 (d, J = 7.5 Hz, 1H), 4.67 (s, 1H), 4.15 (s, 1H), 3.93 (d, J = 9.7 Hz, 6H), 3.67 - 3.55 (m, 2H), 3.46 (s, 2H), 2.73 (d, J = 11.3 Hz, 2H), 2.68 - 2.57 (m, 2H), 2.44 - 2.38 (m, 1H), 2.34 (dd, J = 9.6, 3.6 Hz, 1H), 2.10 (s, 4H), 2.07 - 1.90 (m, 6H), 1.75 (d, J = 11.5 Hz, 2H), 1.58 - 1.46 (m, 3H). |
| 123 | 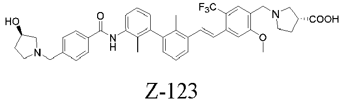 | MS m/z (ESI):728.4 [M+1] |
| 124 | 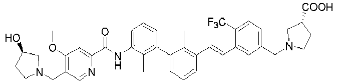 | MS m/z (ESI): 729.4 [M+1] |
| | | ¹H NMR (400 MHz, DMSO-d6) δ 10.34 (s, 1H), 8.45 (s, 1H), 8.28 (s, 2H), 7.91 (s, 1H), 7.87 (d, *J* = 7.7 Hz, 1H), 7.72 (s, 1H), 7.68 (d, *J* = 8.3 Hz, 1H), 7.60 - 7.50 (m, 2H), 7.41 (d, *J* = 7.9 Hz, 1H), 7.34 - 7.20 (m, 3H), 7.07 (d, *J* = 7.2 Hz, 1H), 6.95 (d, *J* = 7.0 Hz, 1H), 4.66 (s, 1H), 4.15 (s, 1H), 3.95 (s, 3H), 3.73 - 3.56 (m, 4H), 2.92 - 2.85 (m, 1H), 2.63 (dt, *J* = 15.4, 8.7 Hz, 4H), 2.52 (d, *J* = 6.7 Hz, 1H), 2.36 (ddd, *J* = 23.3, 15.9, 10.0 Hz, 3H), 2.09 (s, 3H), 2.02 - 1.88 (m, 6H), 1.50 (s, 1H). |
| | 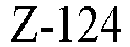 | |
| 125 | 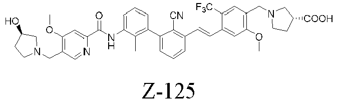 | MS m/z (ESI):770.3 [M+1] |
| 127 | 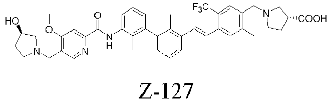 | MS m/z (ESI): 743.4 [M+1] |
| | | ¹H NMR (400 MHz, DMSO-d6) δ 10.35 (s, 1H), 8.49 (s, 1H), 8.10 (s, 1H), 7.84 (d, *J* = 8.8 Hz, 2H), 7.74 (s, 1H), 7.56 (dd, *J* = 20.0, 13.1 Hz, 3H), 7.30 (td, *J* = 7.7, 2.7 Hz, 2H), 7.24 - 7.16 (m, 1H), 7.06 (d, *J* = 7.0 Hz, 1H), 6.95 (d, *J* = 6.8 Hz, 1H), 4.82 (s, 1H), 4.20 (s, 1H), 3.96 (s, 3H), 3.77 (d, *J* = 8.0 Hz, 2H), 3.61 (dd, *J* = 25.4, 14.0 Hz, 2H), 2.96 - 2.87 (m, 1H), 2.78 (m, 2H), 2.67 (d, *J* = 6.9 Hz, 2H), 2.55 (m, 4H), 2.34 (s, 3H), 2.08 (d, *J* = 13.3 Hz, 3H), 2.05 - 1.86 (m, 6H), 1.58 (s, 1H). |
| 128 | 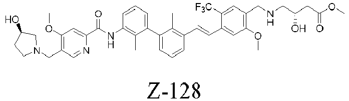 | MS m/z (ESI):389.1 [M/2+1] |
| | | ¹H NMR (400 MHz, DMSO-*d₆*) δ 10.34 (s, 1H), 8.45 (s, 1H), 7.86 (d, J = 8.0 Hz, 1H), 7.78 - 7.50 (m, 4H), 7.46 (s, 1H), 7.36 - 7.14 (m, 3H), 7.07 (d, J = 7.5 Hz, 1H), 6.94 (d, J = 7.5 Hz, 1H), 4.88 (s, 1H), 4.69 (s, 1H), 4.15 (s, 1H), 3.94 (d, J = 4.9 Hz, 6H), 3.78 - 3.56 (m, 4H), 3.54 (s, 3H), 2.70 - 2.57 (m, 2H), 2.52 (m, 3H), 2.42 (m, 1H), 2.31 (m, 2H), 2.04 (m, 7H), 1.51 (m, 1H). |
| 130 | 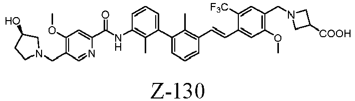 | MS m/z (ESI):373.2 [M/2+1] |
| | | ¹H NMR (400 MHz, DMSO-*d₆*) δ 10.34 (s, 1H), 8.45 (s, 1H), 7.86 (d, J = 8.0 Hz, 1H), 7.71 (s, 1H), 7.63 - 7.51 (m, 3H), 7.46 (s, 1H), 7.30 (m, 2H), 7.20 (dd, J = 15.9, 2.5 Hz, 1H), 7.07 (d, J = 7.4 Hz, 1H), 6.94 (d, J = 7.5 Hz, 1H), 4.68 (s, 1H), 4.18 - 4.13 (m, 1H), 3.94 (d, J = 7.6 Hz, 6H), 3.68 - 3.49 (m, 8H), 2.71 - 2.53 (m, 3H), 2.44 - 2.38 (m, 1H), 2.34 (dd, J = 9.6, 3.6 Hz, 1H), 2.10 (s, 3H), 1.98 (m, 4H), 1.50 (m, 1H). |
| 133 | 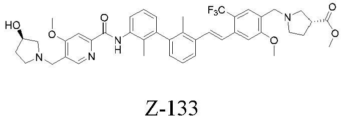 | MS m/z (ESI):387.2 [M/2+1] |
| | | ¹H NMR (400 MHz, DMSO-*d₆*) δ 10.34 (s, 1H), 8.45 (s, 1H), 7.87 (d, J = 8.0 Hz, 1H), 7.71 (s, 1H), 7.67 - 7.51 (m, 3H), 7.47 (s, 1H), 7.34 - 7.17 (m, 3H), 7.07 (d, J = 7.5 Hz, 1H), 6.95 (d, J = 7.5 Hz, 1H), 4.67 (s, 1H), 4.15 (s, 1H), 3.94 (d, J = 7.8 Hz, 6H), 3.73 - 3.41 (m, 7H), 3.05 - 2.97 (m, 1H), 2.74 - 2.48 (m, 6H), 2.45 - 2.23 (m, 3H), 2.10 (s, 3H), 2.04 - 1.89 (m, 5H), 1.55 - 1.46 (m, 1H). |
| 135 | 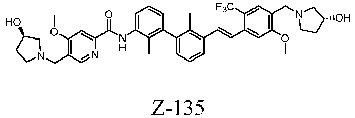 | MS m/z (ESI):731.4 [M+1] |
| 136 | 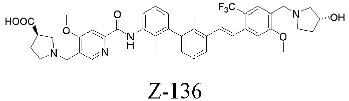 | MS m/z (ESI):759.4[M+1] |
| | | ¹H NMR (400 MHz, DMSO-d6) δ 10.38 (s, 1H), 8.48 (s, 1H), 8.22 (s, 1H), 7.89 (d, *J* = 7.8 Hz, 1H), 7.75 (s, 1H), 7.65 (s, 1H), 7.61 (d, *J* = 11.3 Hz, 1H), 7.50 (s, 1H), 7.40 - 7.28 (m, 2H), 7.25 (d, *J* = 15.6 Hz, 1H), 7.10 (d, *J* = 7.7 Hz, 1H), 6.98 (d, *J* = 7.2 Hz, 1H), 4.71 (s, 1H), 4.21 (s, 1H), 3.99 - 3.96 (m, 5H), 3.74 - 3.57 (m, 4H), 2.94 - 2.92 (m, 1H), 2.71 - 2.55 (m, 5H), 2.50- 2.45 (m, 2H), 2.40 - 2.37 (m, 2H), 2.14 (s, 3H), 2.01 (s, 3H), 2.00 - 1.95 (m, 3H), 1.56 - 1.55 (m, 1H). |
| 139 | 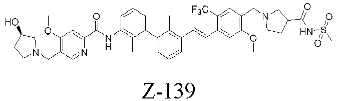 | MS m/z (ESI): 418.7 [1/2M+1]; 1H NMR (400 MHz, DMSO-d6) δ 10.34 (s, 1H), 8.46 (s, 1H), 7.86 (d, J = 8.1 Hz, 1H), 7.71 (d, J = 7.4 Hz, 2H), 7.64 (d, J = 15.8 Hz, 1H), 7.57 (d, J = 7.9 Hz, 1H), 7.51 (s, 1H), 7.30 (q, J = 7.4 Hz, 2H), 7.22 (dd, J = 15.8, 2.5 Hz, 1H), 7.08 (d, J = 7.4 Hz, 1H), 6.95 (d, J = 7.5 Hz, 1H), 4.72 (s, 1H), 4.17 (dt, J = 6.4, 3.4 Hz, 1H), 3.72 (m, 6H), 3.68 (s, 1H), 3.66 -3.62 (m, 3H), 2.97 (s, 3H), 2.90 (t, J = 7.5 Hz, 3H), 2.88 - 2.73 (m, 5H), 2.65 (dd, J = 9.7, 3.3 Hz, 1H), 2.40 (s, 3H), 2.11-1.84 (m, 6H), 1.53 (m, 1H). |
| 140 | 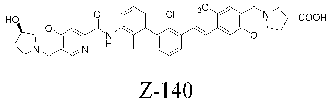 | MS m/z (ESI): 779.3 [M+1] |
| | | ¹H NMR (400 MHz, DMSO-d6) δ 10.34 (s, 1H), 8.45 (s, 1H), 7.89 (d, *J* = 8.1 Hz, 1H), 7.78 (d, *J* = 7.8 Hz, 1H), 7.71 (s, 1H), 7.65 (s, 1H), 7.59 (d, *J* = 15.8 Hz, 1H), 7.47 (t, *J* = 7.6 Hz, 1H), 7.42 (s, 1H), 7.40 - 7.23 (m, 3H), 6.99 (d, *J* = 7.5 Hz, 1H), 4.67 (s, 1H), 4.15 (s, 1H), 3.95 (s, 3H), 3.92 (s, 3H), 3.68 - 3.53 (m, 4H), 2.92 - 2.86 (m, 1H), 2.73 - 2.56 (m, 4H), 2.52 (t, *J* = 6.8 Hz, 2H), 2.44 - 2.38 (m, 1H), 2.34 (dd, *J=* 9.5, 3.6 Hz, 1H), 2.02 (s, 3H), 1.94 (dt, *J* = 12.8, 6.4 Hz, 3H), 1.51 (d, *J* = 3.7 Hz, 1H). |
| 141 | 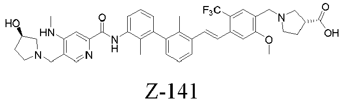 | MS m/z (ESI): 758.4 [M+1] |
| | | ¹H NMR (400 MHz, CD₃OD) δ 8.04 (s, 1H), 7.93 (d, *J* = 8.0Hz, 1H), 7.80 (s, 1H), 7.65 - 7.55 (m, 3H), 7.41 - 7.39 (m, 1H), 7.35 - 7.28 (m, 3H), 7.12 - 7.09 (m, 1H), 7.01 - 6.99 (m, 1H), 4.46 - 4.33 (m, 3H), 4.08 (s, 3H), 3.75 - 3.71 (m, 2H), 3.57 - 3.53 (m, 1H), 3.50 - 3.34 (m, 3H), 3.09 - 3.07 (m, 1H), 2.96 - 2.83 (m, 4H), 2.70 - 2.63 (m, 2H), 2.49 - 2.34 (m, 2H), 2.24 - 2.16 (m, 5H), 2.05 (s, 3H), 1.78 - 1.71 (m, 1H). |
| 142 | 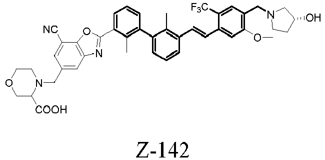 | MS m/z (ESI): 767.3 [M+1] |
| | | ¹H NMR (400 MHz, DMSO-*d₆*) δ 8.16 - 8.14 (m, 2H), 7.89 (s, 1H), 7.66 - 7.59 (m, 2H), 7.56 - 7.50 (m, 1H), 7.48 (s, 1H), 7.41 - 7.32 (m, 2H), 7.24 (dd, *J* = 15.4, 2.0 Hz, 2H), 7.13 (d, *J* = 6.7 Hz, 1H), 4.69 (d, *J* = 4.3 Hz, 1H), 4.17 (s, 1H), 4.01 (d, *J* = 13.9 Hz, 1H), 3.93 (s, 3H), 3.83 - 3.68 (m, 2H), 3.65 - 3.46 (m, 2H), 2.91 (s, 2H), 2.73 - 2.56 (m, 4H), 2.41 (s, 3H), 2.38 - 2.33 (m, 1H), 2.32 - 2.28 (m, 1H), 2.29 - 2.20 (m, 1H), 2.12 (s, 3H), 2.05 - 1.91 (m, 2H), 1.54 (s, 1H). |

### Assay 1 PD-1/PD-L1 Binding Assay

### Experimental Materials:

DMSO is from Sigma, Cat No: D5879; 384-well small volume plate (white) is from Greiner, Cat No: 784075; Anti-PD1 blocking antibody (IC50: 10nM) is from Cisbio, Cat No: 64CUS000C-1a; Anti-PDL1 blocking antibody (IC50: 0.3nM) is from Cisbio, Cat No: 64CUS000C-1b; HTRF PD1 / PD-L1 binding assay kit is from Cisbio, Cat No: 63ADK000CPDPEB.

### Reagent preparation

1. Prepare the compound to be tested at 2× concentration into 1× reaction buffer containing 4% DMSO;
2. Tag1-PD-L1 protein and Tag2-PD1 protein were prepared in 1× reaction buffer at concentrations of 50nM and 10nM, respectively. The final reaction concentration of the experiment was 10nM Tag1-PD-L1 and 2nM Tag1-PD-L1;
3. anti-Tag1-Eu3+ antibody is prepared in 1× concentration detection buffer at a ratio of 1:100;
4. anti-Tag2-XL665 antibody is prepared in 1× concentration detection buffer at a ratio of 1:25; Experimental steps

1. 5µL of compounds to be tested (2×) was added;
2. 2.5ul Tag1-PD-L1 protein and 2.5ul Tag2-PD1 protein were added respectively, and the total reaction system is 10ul, and reacted at 25°C for 15 minutes;
3. 5ul anti-Tag1-Eu3+ antibody and 5ul anti-Tag2-XL665 antibody were added respectively, and reacted at 25°C for 16 hours;
4. The fluorescence microplate reader HTRF reading program was used to read two readings at 665nm and 620nm, the ratio of the two wavelengths was used to judge the activity of the compound, and the IC50 of the compound to be tested is calculated by XLFIT5.0 (IDBS). The test results are shown in Table 1.

**Table 1 PPI-HTRF test results**

| Compound Number | PPI-HTRF (IC₅₀/nM) | Compound Number | PPI-HTRF (IC₅₀/nM) | Compound Number | PPI-HTRF (IC₅₀/nM) |
|---|---|---|---|---|---|
| **Z-1** | 1 | **Z-47** | 1 | **Z-94** | 1 |
| **Z-2** | 1 | **Z-48** | 2 | **Z-95** | <1 |
| **Z-3** | 9 | **Z-49** | 1 | **Z-96** | <1 |
| **Z-4** | <1 | **Z-50** | 1 | **Z-97** | <1 |
| **Z-6** | 2 | **Z-51** | 1 | **Z-98** | 2 |
| **Z-7** | 1 | **Z-52** | 1 | **Z-99** | <1 |
| **Z-8** | 2 | **Z-53** | 1 | **Z-100** | 1 |
| **Z-9** | 1 | **Z-54** | 1 | **Z-101** | 1 |
| **Z-10** | 3 | **Z-55** | 3 | **Z-102** | 1 |
| **Z-11** | 1 | **Z-57** | 1 | **Z-103** | 1 |
| **Z-12** | 1 | **Z-60** | 2 | **Z-104** | 1 |
| **Z-13** | 2 | **Z-61** | 2 | **Z-105** | 1 |
| **Z-14** | <1 | **Z-62** | 3 | **Z-106** | 1 |
| **Z-15** | 3 | **Z-63** | 32 | **Z-107** | 1 |
| **Z-16** | 1 | **Z-64** | 18 | **Z-108** | 2 |
| **Z-17** | 1 | **Z-65** | <1 | **Z-109** | 1 |
| **Z-18** | 1 | **Z-66** | 2 | **Z-110** | 1 |
| **Z-19** | 2 | **Z-67** | 1 | **Z-112** | 1 |
| **Z-20** | 2 | **Z-68** | 1 | **Z-113** | 1 |
| **Z-21** | 1 | **Z-69** | 57 | **Z-114** | 1 |
| **Z-22** | 2 | **Z-70** | 3 | **Z-115** | 1 |
| **Z-23** | 2 | **Z-71** | 5 | **Z-116** | 3 |
| **Z-24** | 1 | **Z-72** | 2 | **Z-117** | 1 |
| **Z-25** | 1 | **Z-73** | 2 | **Z-118** | 1 |
| **Z-26** | 4 | **Z-74** | 1 | **Z-120** | 1 |
| **Z-27** | 1 | **Z-75** | 36 | **Z-121** | 1 |
| **Z-28** | 5 | **Z-76** | 1 | **Z-122** | 1 |
| **Z-29** | 2 | **Z-78** | 13 | **Z-123** | 1 |
| **Z-30** | 2 | **Z-79** | 1 | **Z-124** | 1 |
| **Z-31** | 2 | **Z-80** | 1 | **Z-125** | <1 |
| **Z-32** | 2 | **Z-81** | 1 | **Z-126** | 1 |
| **Z-33** | 1 | **Z-82** | 1 | **Z-127** | 1 |
| **Z-34** | 2 | **Z-83** | 1 | **Z-128** | <1 |
| **Z-35** | 2 | **Z-84** | 1 | **Z-129** | 1 |
| **Z-36** | 2 | **Z-85** | 1 | **Z-130** | 1 |
| **Z-37** | 1 | **Z-86** | 1 | **Z-131** | 1 |
| **Z-38** | 1 | **Z-87** | 1 | **Z-132** | 1 |
| **Z-39** | 1 | **Z-88** | 3 | **Z-133** | 2 |
| **Z-40** | 1 | **Z-89** | 1 | **Z-134** | 1 |
| **Z-41** | 2 | **Z-90** | 1 | **Z-135** | ≤1 |
| **Z-42** | 1 | **Z-91** | 1 | **Z-136** | 5 |
| **Z-43** | 1 | **Z-92** | 1 | **Z-140** | ≤1 |
| **Z-46** | 1 | **Z-93** | 2 | **Z-141** | ≤1 |

As can be seen from Table 1, the exemplary compound of the present disclosure has a good inhibitory activity on PPI-HTRF.

### Assay 2 Jurkat T(PD1 Luc-)/CHOK1(PDL-1) cell experiment

Experimental Materials:
F-12K medium, Gibco, CAS: 21127-030, Cat Number 2120443;
G418, Sangon Biotech #A100859-0001, Cat Number G108BA0003, 5ml of deionized water being added into each bottle, store at 4°C.

### Experimental steps

CHO/PD-L1-OKT3 cells (constructed by referring to Promega's PD-1/PD-L1 Blockade Bioassays system) cultured in F-12K medium (containing 10% FBS, 800ug/ml G418, 400ug/ml D Hygromycin B) were planted into a 96-well plate at 2×10⁴ per well, and incubated for 24 hours at 37°C and 5% CO2. part of the medium was removed, and the test compound dissolved in DMSO with a final concentration of 1uM was added, and DMSO at the same concentration was added into the control cells. Jurkat/PD1-NFAT-Luc cells (constructed by referring to Promega's PD-1/PD-L1 Blockade Bioassays system) were added at 5X10⁵ per well at the same time, and cultured for 6 hours. The corresponding luciferase signal was detected by Bio-Glo^{™} (Promega, G7940) reagent. The EC50 of each compound to be tested was calculated through the non-linear regression of XLfit software. The experiment results are shown in Table 2.

**Table 2 cell experiment results**

| Compoun d Number | Jurkat T (PD1 Luc-)/CHOK1(PDL-1 )(EC₅₀/nM) | Compoun d Number | Jurkat T (PD1 Luc-)/CHOK1(PDL-1 )(EC₅₀/nM) | Compoun d Number | Jurkat T (PD1 Luc-)/CHOK1(PDL-1 )(EC₅₀/nM) |
|---|---|---|---|---|---|
| **Z-1** | 162 | **Z-53** | 28 | **Z-103** | 19 |
| **Z-2** | 79 | **Z-54** | 149 | **Z-105** | 14 |
| **Z-8** | 47 | **Z-57** | 66 | **Z-106** | 20 |
| **Z-9** | 48 | **Z-58** | 78 | **Z-107** | 16 |
| **Z-11** | 137 | **Z-60** | 79 | **Z-108** | 57 |
| **Z-12** | 153 | **Z-74** | 51 | **Z-109** | 121 |
| **Z-14** | 208 | **Z-76** | 361 | **Z-110** | 153 |
| **Z-18** | 142 | **Z-80** | 36 | **Z-112** | 25 |
| **Z-21** | 5 | **Z-81** | 44 | **Z-113** | 66 |
| **Z-24** | 41 | **Z-82** | 29 | **Z-114** | 54 |
| **Z-26** | 39 | **Z-83** | 113 | **Z-115** | 31 |
| **Z-27** | 176 | **Z-84** | 25 | **Z-117** | 13 |
| **Z-29** | 109 | **Z-85** | 34 | **Z-118** | 13 |
| **Z-30** | 104 | **Z-86** | 38 | **Z-120** | 15 |
| **Z-33** | 74 | **Z-87** | 26 | **Z-121** | 31 |
| **Z-34** | 79 | **Z-89** | 16 | **Z-122** | 18 |
| **Z-35** | 29 | **Z-90** | 24 | **Z-124** | 109 |
| **Z-36** | 50 | **Z-91** | 147 | **Z-125** | 24 |
| **Z-38** | 83 | **Z-92** | 6 | **Z-128** | 39 |
| **Z-40** | 108 | **Z-93** | 8 | **Z-131** | 124 |
| **Z-41** | 109 | **Z-94** | 10 | **Z-132** | 168 |
| **Z-42** | 64 | **Z-95** | 4 | **Z-133** | 31 |
| **Z-43** | 72 | **Z-96** | 10 | **Z-134** | 91 |
| **Z-45** | 70 | **Z-97** | 2 | **Z-135** | 44 |
| **Z-48** | 70 | **Z-98** | 174 | **Z-139** | 7 |
| **Z-49** | 78 | **Z-99** | 35 | **Z-140** | 7 |
| **Z-51** | 56 | **Z-101** | 122 | **Z-141** | 74 |
| **Z-52** | 153 | **Z-102** | 9 | | |

All publications mentioned herein are incorporated by reference as if each individual document was cited as a reference, as in the present application. It should also be understood that, after reading the above teachings of the present disclosure, those skilled in the art can make various changes or modifications, equivalents of which falls in the scope of claims as defined in the appended claims.

## Claims

1. A compound represented by formula (I), or a stereoisomer, pharmaceutically acceptable salt, solvate or prodrug thereof: 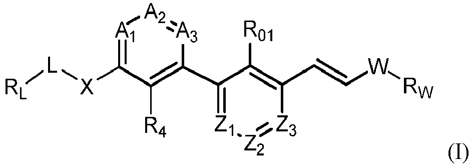 wherein,
L, W are each independently C₆₋₁₀ aryl, C₅₋₁₄ heteroaryl or 3- to 16-membered saturated or unsaturated heterocycle; the C₆₋₁₀ aryl, C₅₋₁₄ heteroaryl, 3- to 16-membered saturated or unsaturated heterocycle are unsubstituted or substituted with 1, 2 or 3 substituents independently selected from R_{P};
A₁ is N or CR₁; A₂ is N or CR₂; A₃ is N or CR₃; A₁, A₂, A₃ are not N at the same time;
Z₁ is N or CR₀₂; Z₂ is N or CR₀₃; Z₃ is N or CR₀₄; Z₁, Z₂, Z₃ are not N at the same time;
R₁, R₂, R₃, R₀₂, R₀₃, R₀₄ are each independently hydrogen or -X₁-(CR₁₁R₁₂)ₙ₁-X₂-(CR₂₁R₂₂)ₙ₂-R_{A};
X is a bond, O, S, NR₅, -C(O)NR₅-, -NR₅C(O)-, -SO₂NR₅-, -NR₅SO₂-, -NR₅C(O)NR₅-, -NR₅C(O)O-, -CH=CH-, -C≡C-, -(CR₆R₇)_{q}-, -(CR₆R₇)_{q}-O-, -O-(CR₆R₇)_{q}-, C₃₋₆ cycloalkyl or 3- to 6-membered saturated monoheterocycle; the C₃₋₆ cycloalkyl, 3- to 6-membered saturated monoheterocycle are optionally substituted with 1, 2 or 3 groups independently selected from halogen, C₁₋₃ alkyl, C₁₋₃ alkoxy, halogenated C₁₋₃ alkyl, or halogenated C₁₋₃ alkoxy;
X₁, X₂ are each independently a bond, O, S, NR₈, -C(O)-, -C(O)NR₈-, -NR₈C(O)-, -SO₂NR₈-, -NR₈SO₂-, - NR₈C(O)NR₈-, -NR₈C(O)O-, -CH=CH-, -C≡C- , -(CR₉R₁₀)ᵣ-, -(CR₉R₁₀)ᵣ-O-, -O-(CR₉R₁₀)ᵣ-, C₃₋₆ cycloalkyl or 3- to 6-membered saturated monoheterocycle; the C₃₋₆ cycloalkyl, 3- to 6-membered saturated monoheterocycle are optionally substituted with 1, 2 or 3 groups independently selected from halogen, C₁₋₃ alkyl, C₁₋₃ alkoxy, halogenated C₁₋₃ alkyl, or halogenated C₁₋₃ alkoxy;
n1, n2 are each independently 0, 1, 2, 3, or 4;
q, r are each independently 1, 2, 3 or 4;
R₅, R₆, R₇, R₈, R₉, R₁₀ are each independently hydrogen, cyano, acetyl, hydroxyl, carboxyl, nitro, halogen, substituted or unsubstituted C₁₋₁₀ alkyl, substituted or unsubstituted C₁₋₁₀ alkoxy, halogenated C₁₋₁₀ alkyl, halogenated C₁₋₁₀ alkoxy, halogenated C₁₋₁₀ alkylthio, NRₐ₀R_{b0}, substituted or unsubstituted C₃₋₁₀ cycloalkyl, substituted or unsubstituted C₆₋₁₀ aryl, substituted or unsubstituted C₅₋₁₄ heteroaryl, or substituted or unsubstituted 3- to 16-membered saturated or unsaturated heterocycle; wherein Rₐ₀, R_{b0} are each independently hydrogen, C₁₋₃ alkyl or halogenated C₁₋₃ alkyl;
R₄, R₀₁, R₁₁, R₁₂, R₂₁, R₂₂ are each independently hydrogen, cyano, acetyl, hydroxyl, carboxyl, nitro, halogen, substituted or unsubstituted C₁₋₁₀ alkyl, substituted or unsubstituted C₁₋₁₀ alkoxy, halogenated C₁₋₁₀ alkyl, halogenated C₁₋₁₀ alkoxy, halogenated C₁₋₁₀ alkylthio, NRₐ₀R_{b0}, substituted or unsubstituted C₃₋₁₀ cycloalkyl, substituted or unsubstituted C₆₋₁₀ aryl, substituted or unsubstituted C₅₋₁₄ heteroaryl, or substituted or unsubstituted 3- to 16-membered saturated or unsaturated heterocycle; wherein Rₐ₀, R_{b0} are each independently hydrogen, C₁₋₃ alkyl or halogenated C₁₋₃ alkyl;
the "substituted" means that 1, 2, 3, 4 or 5 hydrogen atoms in the group are each independently replaced by R_{P};
R_{A}, R_{P} are each independently cyano, nitro, halogen, C₁₋₁₀ alkyl, C₃₋₁₀ cycloalkyl, C₂₋₁₀ alkenyl, C₂₋₁₀ alkynyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle, -OR^{a}, -SR^{a}, -C(O)R^{b}, -OC(O)R^{b}, -S(O)R^{b}, -S(O)₂R^{b}, -NR^{d}C(O)R^{b}, -NR^{d}S(O)R^{b}, -NR^{d}S(O)₂R^{b}, -NHOR^{c}, -C(O)OR^{c}, -NR^{d}C(O)OR^{c}, -C(O)NR^{d}R^{e}, -OC(O)NR^{d}R^{e}, -NR^{d}R^{e}, -NR^{d}C(O)NR^{d}R^{e}, -C(=NR^{d})R^{f}, -C(=NR^{d})NR^{d}R^{e}, -NR^{d}C(=NR^{d})NR^{d}R^{e}, -S(O)NR^{d}R^{e} or -S(O)₂NR^{d}R^{e}; wherein the C₁₋₁₀ alkyl, C₃₋₁₀ cycloalkyl, C₂₋₁₀ alkenyl, C₂₋₁₀ alkynyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle are unsubstituted or substituted with 1, 2, 3, 4 or 5 groups independently selected from -X₃-(CR₃₁R₃₂)ₘ₁-X₄-(CR₄₁R₄₂)ₘ₂-R_{B};
R_{L} is hydrogen, cyano, nitro, halogen, halogenated C₁₋₁₀ alkylthio, C₁₋₁₀ alkyl, C₃₋₁₀ cycloalkyl, C₂₋₁₀ alkenyl, C₂₋₁₀ alkynyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle, -C₁₋₄ alkyl-NR^{g}R^{h}, -halogenated C₁₋₄ alkyl- NR^{g}R^{h}, -C₃₋₆ cycloalkyl-NR^{g}R^{h}, -C₂₋₄ alkenyl-NR^{g}R^{h}, -C₂₋₄ alkynyl-NR^{g}R^{h}, -NR^{g}R^{h}, -NHNR^{g}R^{h}, -C(O)NR^{g}R^{h}, -NHC(O)NR^{g}R^{h}, -S(O)₂NR^{g}R^{h}, -OR^{a}, -SR^{b}, -C(O)R^{b}, -OC(O)R^{b}, -NR^{d}C(O)R^{b}, -NR^{d}S(O)R^{b}, -NR^{d}S(O)₂R^{b}, -S(O)R^{b}, -S(O)₂R^{b}, -NHOR^{c}, -C(O)OR^{c}, -NR^{d}C(O)OR^{c}, -OC(O)NR^{d}R^{e}, -C(=NR^{d})R^{f}, -C(=NR^{d})NR^{d}R^{e}, -NR^{d}C(=NR^{d})NR^{d}R^{e} or -S(O)NR^{d}R^{e}; wherein the C₁₋₁₀ alkyl, C₃₋₁₀ cycloalkyl, C₂₋₁₀ alkenyl, C₂₋₁₀ alkynyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl-3- to 16-membered saturated or unsaturated heterocycle are unsubstituted or substituted with 1, 2, 3, 4 or 5 groups independently selected from -X₃-(CR₃₁R₃₂)ₘ₁-X₄-(CR₄₁R₄₂)ₘ₂-R_{B};
wherein R^{a}, R^{b}, R^{c}, R^{d}, R^{e}, R^{f} are each independently hydrogen, halogen, cyano, nitro, amino, acetyl, hydroxyl, -C₁₋₄ alkyl-carboxyl, -NHC₁₋₄ alkyl, -N(C₁₋₄ alkyl)₂, -S(O)₂NRₐ₀R_{b0}, C₁₋₁₀ alkyl, C₃₋₁₀ cycloalkyl, C₁₋₁₀ alkoxy, C₂₋₁₀ alkenyl, C₂₋₁₀ alkynyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle; wherein Rₐ₀, R_{b0} are each independently hydrogen, C₁₋₃ alkyl or halogenated C₁₋₃ alkyl; the C₁₋₁₀ alkyl, C₃₋₁₀ cycloalkyl, C₁₋₁₀ alkoxy, C₂₋₁₀ alkenyl, C₂₋₁₀ alkynyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₁₀ cycloalkyl, -C₁₋₄ alkyl- 3- to 16-membered saturated or unsaturated heterocycle are unsubstituted or substituted with 1, 2, 3, 4 or 5 groups independently selected from -X₃-(CR₃₁R₃₂)ₘ₁-X₄-(CR₄₁R₄₂)ₘ₂-R_{B};
R^{g}, R^{h} are each independently hydrogen, C₁₋₆ alkyl, halogenated C₁₋₆ alkyl, C₃₋₆ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -C(O)R^{g1}, -C(O)OR^{g1}, -C(O)NR^{g1}R^{g2} or -SO₂NR^{g1}R^{g2}, wherein the C₁₋₆ alkyl, halogenated C₁₋₆ alkyl, C₃₋₆ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 Rⁱ¹;
or R^{g}, R^{h} and a nitrogen atom attached thereto together form a 4- to 11-membered saturated or unsaturated heterocycle, wherein the 4- to 11-membered saturated or unsaturated heterocycle are optionally substituted with 1, 2 or 3 Rⁱ²;
R^{g1} and R^{g2} are each independently hydrogen, C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, or -C₁₋₄ alkyl- 3- to 16-membered saturated or unsaturated heterocycle, wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 Rⁱ³;
Rⁱ¹, Rⁱ², Rⁱ³ are each independently cyano, halogen, C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -OR^{j1}, -SR^{j1}, , -NHOR^{j1}, -C(O)R^{j1}, -C(O)NR^{j1}R^{j2}, -C(O)OR^{j1}, -OC(O)R^{j1}, -OC(O)NR^{j1}R^{j2}, -NR^{j1}R^{j2}, -NR^{j1}C(O)R^{j2}, -NR^{j1}C(O)OR^{j2}, -NR^{j1}C(O)NR^{j2}R^{j3}, -C(=NR^{j1})R^{j2}, -C(=NR^{j1})NR^{j2}R^{j3}, -NR^{j1}C(=NR^{j2})NR^{j3}R^{j4}, -NR^{j1}S(O)R^{j2}, -NR^{j1}S(O)₂R^{j2}, -S(O)R^{j1}, -S(O)NR^{j1}R^{j2}, -S(O)₂R^{j1} or -S(O)₂NR^{j2}R^{j3}; wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, or -C₁₋₄ alkyl- 3- to 16-membered saturated or unsaturated heterocycle are optionally substituted with 1, 2 or 3 R^{k1};
R^{j1}, R^{j2}, R^{j3}, R^{j4} are each independently hydrogen, C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy or halogenated C₁₋₆ alkoxy, wherein the C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy, halogenated C₁₋₆ alkoxy are optionally substituted with 1, 2 or 3 R^{k1};
X₃, X₄ are each independently a bond, O, S, NR₁₃, -C(O)NR₁₃-, -NR₁₃C(O)-, -SONR₁₃-, -NR₁₃SO₂-, -NR₁₃C(O)NR₁₃-, -NR₁₃C(O)O-, -CH=CH-, -C≡C-, -(CR₁₄R₁₅)ₚ-, -(CR₁₄R₁₅)ₚ-O-, -O-(CR₁₄R₁₅)ₚ-, C₃₋₆ cycloalkyl or 3- to 6-membered saturated monoheterocycle; the C₃₋₆ cycloalkyl, 3- to 6-membered saturated monoheterocycle are optionally substituted with 1, 2 or 3 groups independently selected from halogen, C₁₋₃ alkyl, C₁₋₃ alkoxy, halogenated C₁₋₃ alkyl, or halogenated C₁₋₃ alkoxy;
m1, m2 are each independently 0, 1, 2, 3 or 4;
p is 1, 2, 3 or 4;
R₁₃, R₁₄, R₁₅ are each independently hydrogen or C₁₋₁₀ alkyl; the C₁₋₁₀ alkyl is unsubstituted or substituted with 1, 2 or 3 substituents independently selected from the following group: cyano, acetyl, hydroxyl, carboxyl, nitro, halogen, C₁₋₁₀ alkyl, C₁₋₁₀ alkoxy, halogenated C₁₋₁₀ alkyl, halogenated C₁₋₁₀ alkoxy, halogenated C₁₋₁₀ alkylthio, NRₐ₀R_{b0}, C₃₋₁₀ cycloalkyl, C₆₋₁₀ aryl, C₅₋₁₄ heteroaryl, 3- to 16-membered saturated or unsaturated heterocycle; wherein the C₁₋₁₀ alkyl, C₁₋₁₀ alkoxy, C₆₋₁₀ aryl, C₅₋₁₄ heteroaryl, C₃₋₁₀ cycloalkyl, 3- to 16-membered saturated or unsaturated heterocycle are unsubstituted or substituted with 1, 2 or 3 substituents each independently selected from acetyl, hydroxyl, cyano, carboxyl, nitro, halogen, C₁₋₆ alkyl, halogenated C₁₋₆ alkyl, C₁₋₆ alkoxy, halogenated C₁₋₆ alkoxy, C₃₋₆ cycloalkyl, NRₐ₀R_{b0}, -CONRₐ₀R_{b0}, or -SO₂NRₐ₀R_{b0}; Rₐ₀, R_{b0} are each independently hydrogen, C₁₋₃ alkyl or halogenated C₁₋₃ alkyl;
R₃₁, R₃₂, R₄₁, R₄₂ are each independently hydrogen, cyano, acetyl, hydroxyl, carboxyl, nitro, halogen, C₁₋₁₀ alkyl, C₁₋₁₀ alkoxy, halogenated C₁₋₁₀ alkyl, halogenated C₁₋₁₀ alkoxy, halogenated C₁₋₁₀ alkylthio, NRₐ₀R_{b0}, C₃₋₁₀ cycloalkyl, C₆₋₁₀ aryl, C₅₋₁₄ heteroaryl, or 3- to 16-membered saturated or unsaturated heterocycle; the C₁₋₁₀ alkyl, C₁₋₁₀ alkoxy, C₆₋₁₀ aryl, C₅₋₁₄ heteroaryl, C₃₋₁₀ cycloalkyl, 3- to 16-membered saturated or unsaturated heterocycle are unsubstituted or substituted with 1, 2 or 3 substituents each independently selected from acetyl, hydroxyl, cyano, carboxyl, nitro, halogen, C₁₋₆ alkyl, halogenated C₁₋₆ alkyl, C₁₋₆ alkoxy, halogenated C₁₋₆ alkoxy, C₃₋₆ cycloalkyl, NRₐ₀R_{b0}, -CONRₐ₀R_{b0}, or -SO₂NRₐ₀R_{b0}; Rₐ₀, R_{b0} are each independently hydrogen, C₁₋₃ alkyl or halogenated C₁₋₃ alkyl;
R_{B} is cyano, acetyl, hydroxyl, carboxyl, nitro, halogen, C₁₋₁₀ alkyl, C₁₋₁₀ alkoxy, halogenated C₁₋₁₀ alkyl, halogenated C₁₋₁₀ alkoxy, halogenated C₁₋₁₀ alkylthio, NRₐ₀R_{b0}, -CONRₐ₀R_{b0}, -SO₂NRₐ₀R_{b0}, -SO₂C₁₋₁₀ alkyl, C₆₋₁₀ aryl, C₅₋₁₄ heteroaryl, C₃₋₁₀ cycloalkyl, or 3- to 16-membered saturated or unsaturated heterocycle, wherein the C₆₋₁₀ aryl, C₅₋₁₄ heteroaryl, C₃₋₁₀ cycloalkyl, 3- to 16-membered saturated or unsaturated heterocycle are unsubstituted or substituted with 1, 2 or 3 substituents each independently selected from acetyl, hydroxyl, cyano, carboxyl, nitro, halogen, C₁₋₆ alkyl, halogenated C₁₋₆ alkyl, C₁₋₆ alkoxy, halogenated C₁₋₆ alkoxy, C₃₋₆ cycloalkyl, NRₐ₀R_{b0}, -CONRₐ₀R_{b0}, or -SO₂NRₐ₀R_{b0}; Rₐ₀, R_{b0} are each independently hydrogen, C₁₋₃ alkyl, or halogenated C₁₋₃ alkyl.
R_{W} is hydrogen, cyano, halogen, hydroxyl, carboxyl, amino, C₁₋₄ alkyl, C₁₋₄ alkoxy, halogenated C₁₋₄ alkyl, halogenated C₁₋₄ alkoxy, -NH-C₁₋₄ alkyl, -N(C₁₋₄ alkyl)₂, or -(CR^{m1}R^{m2})ᵥ-NR^{m3}R^{m4}; wherein the C₁₋₄ alkyl, C₁₋₄ alkoxy are optionally substituted with 1 or 2 cyano, halogen, hydroxyl, carboxyl, amino, C₁₋₄ alkyl, C₁₋₄ alkoxy, halogenated C₁₋₄ alkyl, halogenated C₁₋₄ alkoxy, -NHC₁₋₄ alkyl or -N(C₁₋₄ alkyl)₂;
v is 0, 1, 2 or 3;
R^{m1}, R^{m2} are each independently hydrogen, cyano, halogen, hydroxyl, carboxyl, amino, C₁₋₄ alkyl, C₁₋₄ alkoxy, halogenated C₁₋₄ alkyl, halogenated C₁₋₄ alkoxy, -NH-C₁₋₄ alkyl, -N(C₁₋₄ alkyl)₂, C₃₋₆ cycloalkyl, phenyl, 5- or 6-membered monocyclic heteroaryl or 4- to 6-membered saturated or unsaturated heterocycle, wherein the C₁₋₄ alkyl, C₁₋₄ alkoxy, halogenated C₁₋₄ alkyl, halogenated C₁₋₄ alkoxy, -NH-C₁₋₄ alkyl, -N(C₁₋₄ alkyl)₂, C₃₋₆ cycloalkyl, phenyl, 5- or 6-membered monocyclic heteroaryl, 4- to 6-membered saturated or unsaturated heterocycle are optionally substituted with 1, 2 or 3 R^{k2};
or R^{m1}, R^{m2} and a carbon atom attached thereto together form a 3-, 4-, 5- or 6-membered cycloalkyl ring or 4-, 5-, 6- or 7-membered saturated or unsaturated heterocycle, wherein the cycloalkyl ring, saturated or unsaturated heterocycle are optionally substituted with 1 or 2 R^{k2};
or R^{m1} and R^{m3} are connected to form a 4-, 5-, 6- or 7-membered saturated or unsaturated heterocycle, wherein the saturated or unsaturated heterocycle contains 1 or 2 heteroatoms independently selected from N, O or S, and the saturated or unsaturated heterocycle are optionally substituted with 1 or 2 R^{k2};
R^{m3}, R^{m4} are each independently hydrogen, C₁₋₆ alkyl, halogenated C₁₋₆ alkyl, C₃₋₆ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -C(O)Rⁿ¹, -C(O)ORⁿ¹, -C(O)NRⁿ¹Rⁿ², or SO₂NRⁿ¹Rⁿ², wherein the C₁₋₆ alkyl, halogenated C₁₋₆ alkyl, C₃₋₆ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl-3- to 16-membered saturated or unsaturated heterocycle are optionally substituted with 1, 2 or 3 R^{o1};
or R^{m3}, R^{m4} and a nitrogen atom attached thereto together form a 4-, 5-, 6-, 7-, 8-, 9-, 10- or 11-membered saturated or unsaturated heterocycle, wherein the saturated or unsaturated heterocycle is optionally substituted with 1, 2 or 3 R^{o2};
Rⁿ¹, Rⁿ² are each independently hydrogen, C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, or -C₁₋₄ alkyl- 3- to 16-membered saturated or unsaturated heterocycle, wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl-3- to 16-membered saturated or unsaturated heterocycle are optionally substituted with 1, 2 or 3 R^{o3};
R^{o1}, R^{o2}, R^{o3} are each independently cyano, halogen, C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl-3- to 16-membered saturated or unsaturated heterocycle, -OR^{p1}, -SR^{p1}, -NHOR^{p1}, -C(O)R^{p1}, -C(O)NR^{p1}R^{p2}, -C(O)OR^{p1}, -OC(O)R^{p1}, -OC(O)NR^{p1}R^{p2}, -NR^{p1}R^{p2}, -NR^{p1}C(O)R^{p2}, -NR^{p1}C(O)OR^{p2}, -NR^{p1}C(O)NR^{p2}R^{p3}, -C(=NR^{p1})R^{p2}, -C(=NR^{p1})NR^{p2}R^{p3}, -NR^{p1}C(=NR^{p2})NR^{p3}R^{p4}, -NR^{p1}S(O)R^{p2}, -NR^{p1}S(O)₂R^{p2}, -S(O)R^{p1}, -S(O)NR^{p1}R^{p2}, -S(O)₂R^{p1} or -S(O)₂NR^{p2}R^{p3}; wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 R^{k2};
R^{p1}, R^{p2}, R^{p3}, R^{p4} are each independently hydrogen, C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy or halogenated C₁₋₆ alkoxy, wherein the C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy, halogenated C₁₋₆ alkoxy are optionally substituted with 1, 2 or 3 R^{k2}; and
R^{k1}, R^{k2} are each independently cyano, halogen, hydroxyl, carboxyl, amino, C₁₋₆ alkyl, -NH-C₁₋₆ alkyl, -N(C₁₋₆ alkyl)₂, C₁₋₆ alkoxy, halogenated C₁₋₆ alkyl, halogenated C₁₋₆ alkoxy, C₃₋₆ cycloalkyl, phenyl, 5- or 6-membered monocyclic heteroaryl or 4- to 6-membered saturated or unsaturated heterocycle, wherein the C₁₋₆ alkyl, -NH-C₁₋₆ alkyl, -N(C₁₋₆ alkyl)₂, C₁₋₆ alkoxy, halogenated C₁₋₆ alkyl, halogenated C₁₋₆ alkoxy, C₃₋₆ cycloalkyl, phenyl, 5- or 6-membered monocyclic heteroaryl, 4- to 6-membered saturated or unsaturated heterocycle are optionally substituted with 1, 2 or 3 groups independently selected from cyano, halogen, hydroxyl, carboxyl, amino, C₁₋₄ alkyl, C₁₋₄ alkoxy, halogenated C₁₋₄ alkyl, halogenated C₁₋₄ alkoxy, C₃₋₆ cycloalkyl, phenyl, 5- or 6-membered monocyclic heteroaryl, 4- to 6-membered saturated or unsaturated heterocycle.

2. The compound of claim 1, or a stereoisomer, pharmaceutically acceptable salt, solvate or prodrug thereof, wherein L, W are each independently phenyl, 5- or 6-membered monocyclic heteroaryl, 8- to 10-membered bicyclic heteroaryl, 11- to 14-membered tricyclic heteroaryl, 3- to 6-membered saturated or unsaturated monoheterocycle, 7- to 11-membered monospiro heterocycle, 10- to 16-membered bispiro heterocycle, 7- to 10-membered bicyclic fused heterocycle, or 7- to 10-membered bridged heterocycle, or are structures shown in formula A, formula B, formula C or formula D: 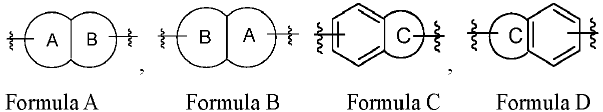 wherein ring A is 5- or 6-membered monocyclic heteroaryl ring, and ring B , ring C are each independently 5- to 6-membered saturated or unsaturated monoheterocycle or 5- to 6-membered saturated or unsaturated monocyclic ring; the phenyl, 5- or 6-membered monocyclic heteroaryl, 8- to 10-membered bicyclic heteroaryl, 11- to 14-membered tricyclic heteroaryl, 3- to 6-membered saturated or unsaturated monoheterocycle, 7- to 11-membered monospiro heterocycle, 10- to 16-membered bispiro heterocycle, 7- to 10-membered bicyclic fused heterocycle, 7- to 10-membered bridged heterocycle, the structures shown in formula A, formula B, formula C, and formula D are unsubstituted or substituted with 1, 2 or 3 substituents independently selected from Rp.

3. The compound of claim 2, or a stereoisomer, pharmaceutically acceptable salt, solvate or prodrug thereof, wherein the 5- or 6-membered monocyclic heteroaryl in L and W is selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

4. The compound of claim 2, or a stereoisomer, pharmaceutically acceptable salt, solvate or prodrug thereof, wherein the 8- to 10-membered bicyclic heteroaryl in L and W is a 9- or 10-membered bicyclic heteroaryl formed by fusing a benzene ring with a 5- or 6-membered monocyclic heteroaryl, or is an 8- to 10-membered bicyclic heteroaryl formed by fusing a 5- or 6-membered monocyclic heteroaryl with a 5- or 6-membered monocyclic heteroaryl; wherein the 5- or 6-membered monocyclic heteroaryl is selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

5. The compound of claim 2, or a stereoisomer, pharmaceutically acceptable salt, solvate or prodrug thereof, wherein the 8- to 10-membered bicyclic heteroaryl in L, W is selected from: benzoxazole, benzoisoxazole, benzimidazole, benzothiazole, benzisothiazole, benzotriazole, benzofuran, benzothiophene, indole, indazole, isoindole, quinoline, isoquinoline, quinazoline, quinoxaline, cinnoline, pyridoxazole, pyridoisoxazole, pyridoimidazole, pyridothiazole, pyridoisothiazole, pyridotriazole, pyridofuran, pyridothiophene, pyridopyrrole, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, or 1,5-naphthyridine.

6. The compound of claim 2, or a stereoisomer, pharmaceutically acceptable salt, solvate or prodrug thereof, wherein the ring A in formula A and formula B is selected from: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

7. The compound of claim 2, or a stereoisomer, pharmaceutically acceptable salt, solvate or prodrug thereof, wherein when the ring B in formula A and formula B , and the ring C in formula C and formula D are 5- to 6-membered saturated or unsaturated monoheterocycle, they are each independently selected from: oxazolidine, pyrrolidine-2-one, pyrrolidine-2,5-dione, 1,3-dioxolane, dihydrofuran-2(3H)-one, dihydrofuran-2,5-dione, piperidine-2-one, piperidine-2,6-dione, tetrahydro-2H-pyran-2-one, imidazolidine, tetrahydrofuran, tetrahydrothiophene, tetrahydropyrrole, 1,3-dioxolane-2-one, oxazolidine-2-one, imidazolidine-2-one, piperidine, piperazine, piperazine-2-one, morpholine, morpholine-3-one, morpholine-2-one, thiomorpholine-3-one-1,1-dioxide, thiomorpholine, thiomorpholine-1,1-dioxide, or tetrahydropyran.

8. The compound of claim 2, or a stereoisomer, pharmaceutically acceptable salt, solvate or prodrug thereof, wherein when the ring B in formula A and formula B, and the ring C in formula C and formula D are 5- to 6-membered saturated or unsaturated monocyclic ring, they are each independently selected from: cyclopentyl, cyclopentenyl, cyclohexyl, cyclohexenyl, cyclohexadienyl, cyclopentanone, cyclopentane-1,3-dione, cyclohexanone, or cyclohexane-1,3-dione.

9. The compound of claim 2, or a stereoisomer, pharmaceutically acceptable salt, solvate or prodrug thereof, wherein the 8- to 10-membered bicyclic heteroaryl ring in L and W is selected from: 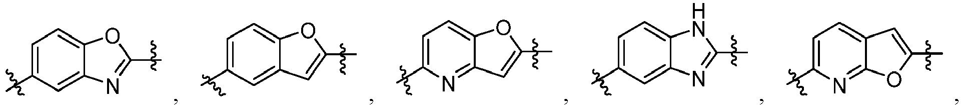 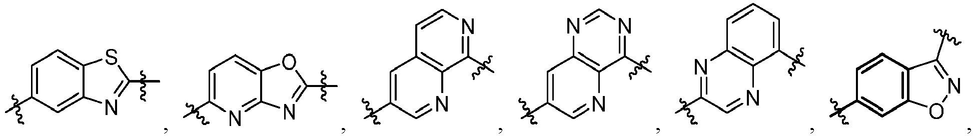 or

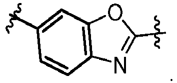

10. The compound of claim 2, or a stereoisomer, pharmaceutically acceptable salt, solvate or prodrug thereof, wherein the formula A, formula B, formula C, and formula D are independently selected from the following structures:

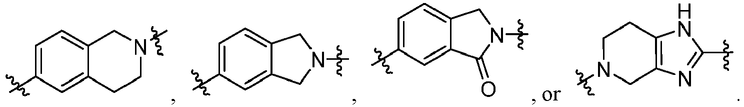

11. The compound of claim 1 or 2, or a stereoisomer, pharmaceutically acceptable salt, solvate or prodrug thereof, wherein L and W are each independently selected from the following rings: 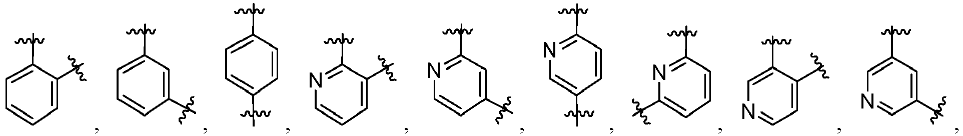 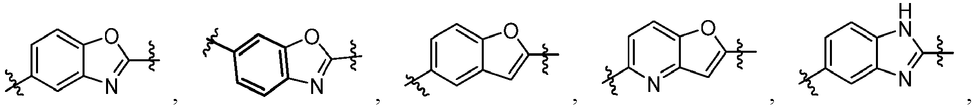 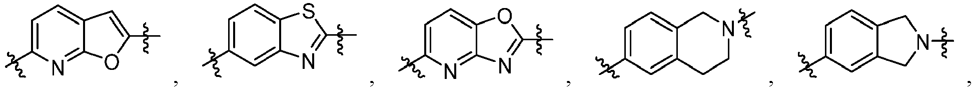 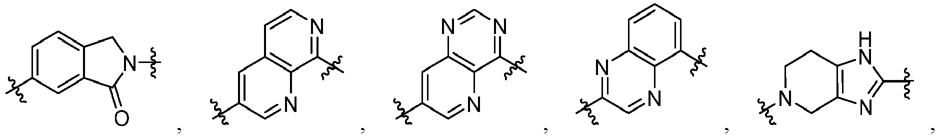 or 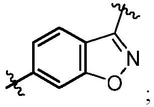 and the ring is unsubstituted or substituted with 1, 2 or 3 substituents independently selected from Rp.

12. The compound of claim 1, or a stereoisomer, pharmaceutically acceptable salt, solvate or prodrug thereof, wherein R_{L} is hydrogen or -Y₀-NR^{g}R^{h}; wherein Y₀ is a bond, NH, C₁₋₄ alkyl, halogenated C₁₋₄ alkyl, C₃₋₆ cycloalkyl, -NHC(O) -, -SO₂-, -C(O)-, -CH=CH- or -C≡C-;
R^{g}, R^{h} are each independently hydrogen, C₁₋₆ alkyl, halogenated C₁₋₆ alkyl, C₃₋₆ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -C(O)R^{g1}, -C(O)OR^{g1}, -C(O)NR^{g1}R^{g2} or -SO₂NR^{g1}R^{g2}, wherein the C₁₋₆ alkyl, halogenated C₁₋₆ alkyl, C₃₋₆ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 Rⁱ¹;
or R^{g}, R^{h} and the nitrogen atom attached thereto together form a 4- to 11-membered saturated or unsaturated heterocycle, wherein the 4- to 11-membered saturated or unsaturated heterocycle are optionally substituted with 1, 2 or 3 Rⁱ²;
R^{g1} and R^{g2} are each independently hydrogen, C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, or -C₁₋₄ alkyl- 3- to 16-membered saturated or unsaturated heterocycle, wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 Rⁱ³;
Rⁱ¹, Rⁱ², Rⁱ³ are each independently cyano, halogen, C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle, -OR^{j1}, -SR^{j1}, , -NHOR^{j1}, -C(O)R^{j1}, -C(O)NR^{j1}R^{j2}, -C(O)OR^{j1}, -OC(O)R^{j1}, -OC(O)NR^{j1}R^{j2}, -NR^{j1}R^{j2}, -NR^{j1}C(O)R^{j2}, -NR^{j1}C(O)OR^{j2}, -NR^{j1}C(O)NR^{j2}R^{j3}, -C(=NR^{j1})R^{j2}, -C(=NR^{j1})NR^{j2}R^{j3}, -NR^{j1}C(=NR^{j2})NR^{j3}R^{j4}, -NR^{j1}S(O)R^{j2}, -NR^{j1}S(O)₂R^{j2}, -S(O)R^{j1}, -S(O)NR^{j1}R^{j2}, -S(O)₂R^{j1} or -S(O)₂NR^{j2}R^{j3}; wherein the C₁₋₆ alkyl, C₂₋₆ alkenyl, C₂₋₆ alkynyl, C₁₋₆ alkoxy, halogenated C₁₋₆ alkyl, halogenated C₁₋₆ alkoxy, C₃₋₁₀ cycloalkyl, C₆₋₁₀ aryl, C₅₋₁₄ heteroaryl, 3- to 16-membered saturated or unsaturated heterocycle, -C₁₋₄ alkyl-C₆₋₁₀ aryl, -C₁₋₄ alkyl-C₅₋₁₄ heteroaryl, -C₁₋₄ alkyl-C₃₋₆ cycloalkyl, -C₁₋₄ alkyl- 3- to 16-membered saturated or unsaturated heterocycle are optionally substituted with 1, 2 or 3 R^{k1};
R^{j1}, R^{j2}, R^{j3}, R^{j4} are each independently hydrogen, C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy or halogenated C₁₋₆ alkoxy, wherein the C₁₋₄ alkyl, C₁₋₄ alkoxy, C₃₋₆ cycloalkyl, C₆₋₁₀ aryl, 5- or 6-membered monocyclic heteroaryl, 4- to 7-membered saturated or unsaturated heterocycle, C₂₋₄ alkenyl, C₂₋₄ alkynyl, halogenated C₁₋₆ alkyl, C₁₋₆ alkoxy, halogenated C₁₋₆ alkoxy are optionally substituted with 1, 2 or 3 R^{k1}; and
R^{k1} is cyano, halogen, hydroxyl, carboxyl, amino, C₁₋₆ alkyl, -NH-C₁₋₆ alkyl, -N(C₁₋₆ alkyl)₂, C₁₋₆ alkoxy, halogenated C₁₋₆ alkyl, halogenated C₁₋₆ alkoxy, C₃₋₆ cycloalkyl, phenyl, 5- or 6-membered monocyclic heteroaryl or 4- to 6-membered saturated or unsaturated heterocycle, wherein the C₁₋₆ alkyl, -NH-C₁₋₆ alkyl, -N(C₁₋₆ alkyl)₂, C₁₋₆ alkoxy, halogenated C₁₋₆ alkyl, halogenated C₁₋₆ alkoxy, C₃₋₆ cycloalkyl, phenyl, 5- or 6-membered monocyclic heteroaryl, 4- to 6-membered saturated or unsaturated heterocycle are optionally substituted with 1, 2 or 3 groups independently selected from cyano, halogen, hydroxyl, carboxyl, amino, C₁₋₄ alkyl, C₁₋₄ alkoxy, halogenated C₁₋₄ alkyl, halogenated C₁₋₄ alkoxy, C₃₋₆ cycloalkyl, phenyl, 5- or 6-membered monocyclic heteroaryl, or 4- to 6-membered saturated or unsaturated heterocycle.

13. The compound of claim 12, or a stereoisomer, pharmaceutically acceptable salt, solvate or prodrug thereof, wherein Y₀ is a bond, NH, CH₂, CH₂CH₂, halogenated C₁₋₃ alkyl, C₃₋₆ cycloalkyl, -NHC(O)-, -SO₂-, -C(O)-, -CH=CH- or -C≡C-; the C₃₋₆ cycloalkyl is cyclopropyl, cyclobutyl, cyclopentyl or cyclohexyl.

14. The compound of claim 1, or a stereoisomer, pharmaceutically acceptable salt, solvate or prodrug thereof, wherein R_{L} is -Y₀-NR^{g}R^{h}; wherein Y₀ is a bond, NH, CH₂, CH₂CH₂, halogenated C₁₋₃ alkyl, C₃₋₆ cycloalkyl, -NHC(O)-, -SO₂-, -C(O)-, -CH=CH- or -C≡C-; wherein the C₃₋₆ cycloalkyl is cyclopropyl, cyclobutyl, cyclopentyl or cyclohexyl;
R^{g}, R^{h} and the nitrogen atom attached thereto together form a 4- to 6-membered saturated monoheterocycle, wherein the 4- to 6-membered saturated monoheterocycle are optionally substituted with 1, 2 or 3 Rⁱ²;
Rⁱ² is cyano, halogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -CH₂-phenyl, -CH₂-5- or 6-membered monocyclic heteroaryl, -CH₂-C₃₋₆ cycloalkyl, -CH₂-3- to 6-membered saturated monoheterocycle, -OR^{j1}, -SR^{j1}, -NHOR^{j1}, -C(O)R^{j1}, -C(O)NR^{j1}R^{j2}, -C(O)OR ^{j1}, -OC(O)R^{j1}, -OC(O)NR^{j1}R^{j2}, -NR^{j1}R^{j2}, -NR^{j1}C(O)R^{j2}, -NR^{j1}C(O)OR^{j2}, -NR^{j1}C(O)NR^{j2}R^{j3}, -C(=NR^{j1})R^{j2}, -C(=NR^{j1})NR^{j2}R^{j3}, -NR^{j1}C(=NR^{j2})NR^{j3}R^{j4}, -NR^{j1}S(O)R^{j2}, -NR^{j1}S(O)₂R^{j2}, -S(O)R^{j1}, -S(O)NR^{j1}R^{j2}, -S(O)₂R^{j1} or -S(O)₂NR^{j2}R^{j3}; wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -CH₂-phenyl, -CH₂-5- or 6-membered monocyclic heteroaryl, -CH₂-C₃₋₆ cycloalkyl, -CH₂-3- to 6-membered saturated monoheterocycle are optionally substituted with 1, 2 or 3 R^{k1};
R^{j1}, R^{j2}, R^{j3}, R^{j4} are each independently hydrogen, C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy or halogenated C₁₋₃ alkoxy, wherein the C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy, halogenated C₁₋₃ alkoxy are optionally substituted with 1, 2 or 3 R^{k1}; and
R^{k1} is cyano, halogen, hydroxyl, carboxyl, amino, C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle, wherein the C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle are optionally substituted with 1, 2 or 3 groups independently selected from cyano, halogen, hydroxyl, carboxyl, amino, C₁₋₃ alkyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, or 4- to 6-membered saturated monoheterocycle.

15. The compound of claim 1, or a stereoisomer, pharmaceutically acceptable salt, solvate or prodrug thereof, wherein -NR^{g}R^{h} is selected from:

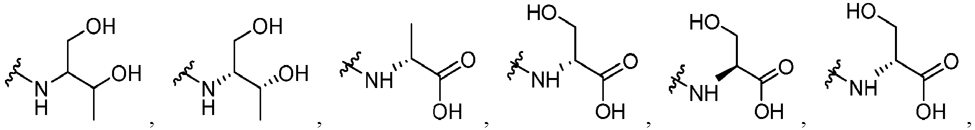

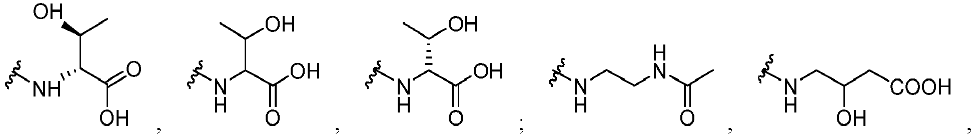

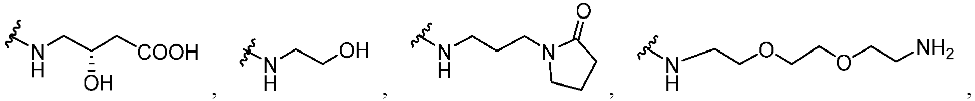

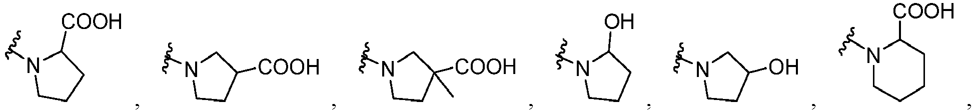

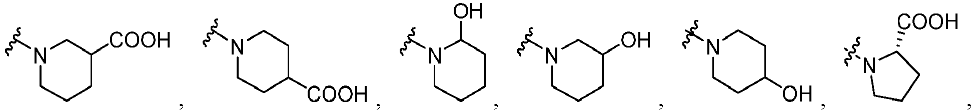

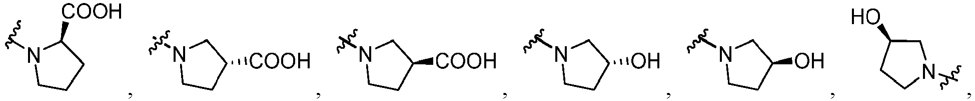

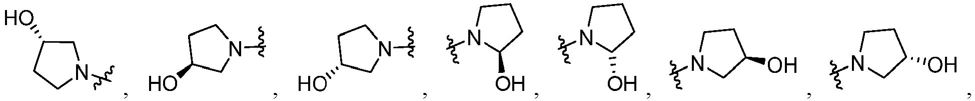

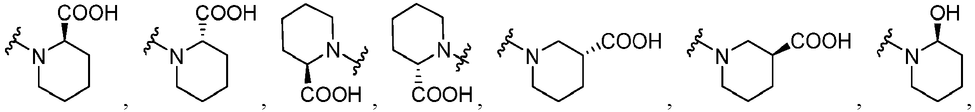

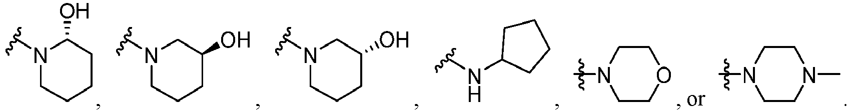

16. The compound of claim 1, or a stereoisomer, pharmaceutically acceptable salt, solvate or prodrug thereof, wherein R_{W} is hydrogen, cyano, halogen, hydroxyl, carboxyl, amino, C₁₋₃ alkyl, C₁₋₃ alkoxy, halogenated C₁₋₃ alkyl, halogenated C₁₋₃ alkoxy, -NH-C₁₋₃ alkyl, -N(C₁₋₃ alkyl)₂, or -(CR^{m1}R^{m2})ᵥ-NR^{m3}R^{m4}; wherein the C₁₋₃ alkyl, C₁₋₃ alkoxy are optionally substituted with 1 or 2 cyano, halogen, hydroxyl, carboxyl, amino, C₁₋₃ alkyl, C₁₋₃ alkoxy, halogenated C₁₋₃ alkyl, halogenated C₁₋₃ alkoxy, -NH C₁₋₃ alkyl or -N (C₁₋₃ alkyl)₂;
v is 0, 1, 2 or 3;
R^{m1}, R^{m2} are each independently hydrogen, cyano, halogen, hydroxyl, carboxyl, amino, C₁₋₃ alkyl, C₁₋₃ alkoxy, halogenated C₁₋₃ alkyl, halogenated C₁₋₃ alkoxy, -NH-C₁₋₃ alkyl, -N(C₁₋₃ alkyl)₂, C₃₋₆ cycloalkyl, phenyl, 5- or 6-membered monocyclic heteroaryl or 4- to 6-membered saturated monoheterocycle, wherein the C₁₋₃ alkyl, C₁₋₃ alkoxy, halogenated C₁₋₃ alkyl, halogenated C₁₋₃ alkoxy, -NH-C₁₋₃ alkyl, -N(C₁₋₃ alkyl)₂, C₃₋₆ cycloalkyl, phenyl, 5- or 6-membered monocyclic heteroaryl, 4- to 6-membered saturated monoheterocycle are optionally substituted with 1, 2 or 3 R^{k2};
or R^{m1}, R^{m2} and the carbon atom attached thereto together form a 3-, 4-, 5- or 6-membered cycloalkyl ring or 4-, 5- or 6-membered saturated monoheterocycle, wherein the cycloalkyl ring, saturated monoheterocycle are optionally substituted with 1 or 2 R^{k2};
or R^{m1} and R^{m3} are connected to form a 4-, 5-, 6- or 7-membered saturated monoheterocycle, wherein the saturated monoheterocycle contains 1 or 2 heteroatoms independently selected from N, O or S, and the saturated monoheterocycle are optionally substituted with 1 or 2 R^{k2};
R^{m3}, R^{m4} are each independently hydrogen, C₁₋₃ alkyl, halogenated C₁₋₃ alkyl, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, -C(O)Rⁿ¹, -C(O)ORⁿ¹, -C(O)NRⁿ¹Rⁿ², or SO₂NRⁿ¹Rⁿ², wherein the C₁₋₃ alkyl, halogenated C₁₋₃ alkyl, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 R^{o1};
or R^{m3}, R^{m4} and the nitrogen atom attached thereto together form a 4-, 5-, 6-, 7-, 8-, 9-, 10- or 11-membered saturated or unsaturated heterocycle, wherein the saturated or unsaturated heterocycle is optionally substituted with 1, 2 or 3 R^{o2};
Rⁿ¹, Rⁿ² are each independently hydrogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, or -C₁₋₂ alkyl-3- to 6-membered saturated monoheterocycle, wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 R^{o3};
R^{o1}, R^{o2}, R^{o3} are each independently cyano, halogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, -OR^{p1}, -SR^{p1}, , -NHOR^{p1}, -C(O)R^{p1}, -C(O)NR^{p1}R^{p2}, -C(O)OR^{p1}, -OC(O)R^{p1}, -OC(O)NR^{p1}R^{p2}, -NR^{p1}R^{p2}, -NR^{p1}C(O)R^{p2}, -NR^{p1}C(O)OR^{p2}, -NR^{p1}C(O)NR^{p2}R^{p3}, -C(=NR^{p1})R^{p2}, -C(=NR^{p1})NR^{p2}R^{p3}, -NR^{p1}C(=NR^{p2})NR^{p3}R^{p4}, -NR^{p1}S(O)R^{p2}, -NR^{p1}S(O)₂R^{p2}, -S(O)R^{p1}, -S(O)NR^{p1}R^{p2}, -S(O)₂R^{p1} or -S(O)₂NR^{p2}R^{p3}; wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 R^{k2};
R^{p1}, R^{p2}, R^{p3}, R^{p4} are each independently hydrogen, C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy or halogenated C₁₋₃ alkoxy, wherein the C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy, halogenated C₁₋₃ alkoxy are optionally substituted with 1, 2 or 3 R^{k2}; and
R^{k2} is cyano, halogen, hydroxyl, carboxyl, amino, C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle, wherein the C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle are optionally substituted with 1, 2 or 3 groups independently selected from cyano, halogen, hydroxyl, carboxyl, amino, C₁₋₃ alkyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, or 4- to 6-membered saturated monoheterocycle.

17. The compound of claim 1, or a stereoisomer, pharmaceutically acceptable salt, solvate or prodrug thereof, wherein R_{W} is hydrogen or -(CR^{m1}R^{m2})ᵥ-NR^{m3}R^{m4};
wherein v is 0, 1, 2 or 3;
R^{m1}, R^{m2} are each independently hydrogen, cyano, halogen, hydroxyl, carboxyl, amino, C₁₋₃ alkyl, C₁₋₃ alkoxy, halogenated C₁₋₃ alkyl, halogenated C₁₋₃ alkoxy, -NH-C₁₋₃ alkyl, or -N(C₁₋₃ alkyl)₂;
R^{m3}, R^{m4} and the nitrogen atom attached thereto together form a 4-, 5- or 6-membered saturated monoheterocycle, wherein the saturated monoheterocycle are optionally substituted with 1, 2 or 3 R^{o2};
R^{o2} is cyano, halogen, C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle, -OR^{p1}, -SR^{p1}, -NHOR^{p1}, -C(O)R^{p1}, -C(O)NR^{p1}R^{p2}, -C(O)OR^{p1}, -OC(O)R^{p1}, -OC(O)NR^{p1}R^{p2}, -NR^{p1}R^{p2}, -NR^{p1}C(O)R^{p2}, -NR^{p1}C(O)OR^{p2}, -NR^{p1}C(O)NR^{p2}R^{p3}, -C(=NR^{p1})R^{p2}, -C(=NR^{p1})NR^{p2}R^{p3}, -NR^{p1}C(=NR^{p2})NR^{p3}R^{p4}, -NR^{p1}S(O)R^{p2}, -NR^{p1}S(O)₂R^{p2}, -S(O)R^{p1}, -S(O)NR^{p1}R^{p2}, -S(O)₂R^{p1} or -S(O)₂NR^{p2}R^{p3}; wherein the C₁₋₃ alkyl, C₂₋₄ alkenyl, C₂₋₄ alkynyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, 3- to 6-membered saturated monoheterocycle, -C₁₋₂ alkyl-phenyl, -C₁₋₂ alkyl-5- or 6-membered monocyclic heteroaryl, -C₁₋₂ alkyl-C₃₋₆ cycloalkyl, -C₁₋₂ alkyl-3- to 6-membered saturated monoheterocycle are optionally substituted with 1, 2 or 3 R^{k2};
R^{p1}, R^{p2}, R^{p3}, R^{p4} are each independently hydrogen, C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy or halogenated C₁₋₃ alkoxy, wherein the C₁₋₃ alkyl, C₁₋₃ alkoxy, C₃₋₆ cycloalkyl, phenyl, 5- or 6-membered monocyclic heteroaryl, 4- to 6-membered saturated monoheterocycle, C₂₋₄ alkenyl, C₂₋₄ alkynyl, halogenated C₁₋₃ alkyl, C₁₋₃ alkoxy, halogenated C₁₋₃ alkoxy are optionally substituted with 1, 2 or 3 R^{k2}; and
R^{k2} is cyano, halogen, hydroxyl, carboxyl, amino, C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle, wherein the C₁₋₃ alkyl, -NH-C₁₋₃ alkyl, -N(C₁₋₃ alkyl)₂, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl or 4- to 6-membered saturated monoheterocycle is optionally substituted with 1, 2 or 3 groups independently selected from cyano, halogen, hydroxyl, carboxyl, amino, C₁₋₃ alkyl, C₁₋₃ alkoxy, halogenated C₁₋₃ alkyl, halogenated C₁₋₃ alkoxy, C₃₋₆ cycloalkyl, phenyl, 5- or 6-membered monocyclic heteroaryl, or 4- to 6-membered saturated monoheterocycle.

18. The compound of claim 1, or a stereoisomer, pharmaceutically acceptable salt, solvate or prodrug thereof, wherein -NR^{m3}R^{m4} is selected from:

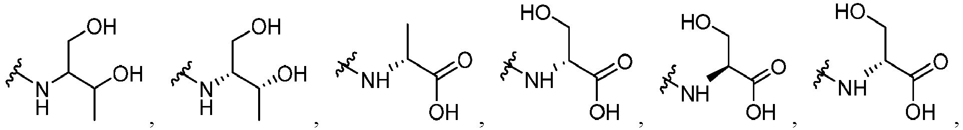

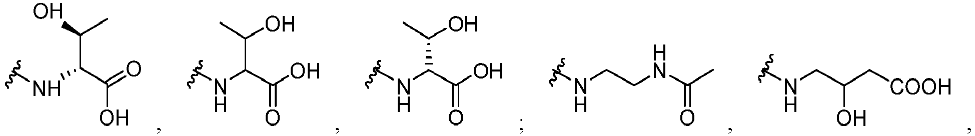

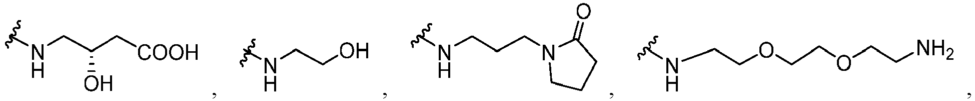

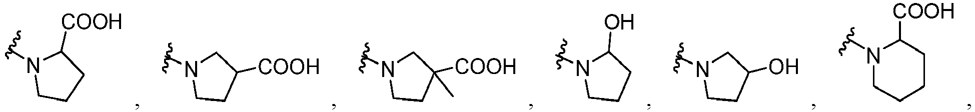

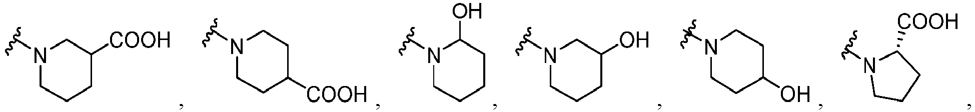

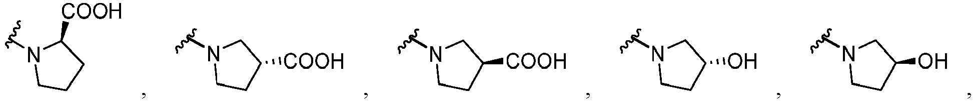

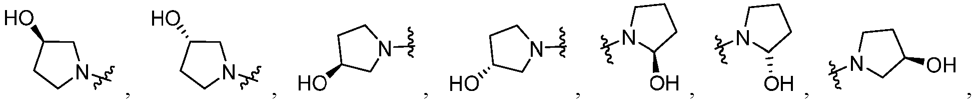

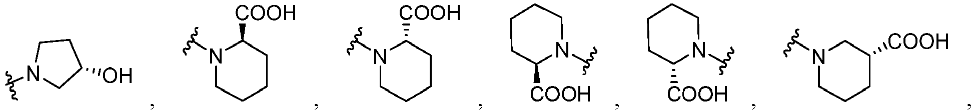

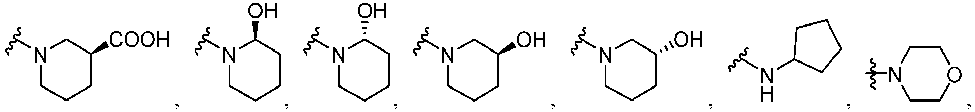

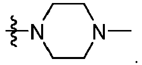

19. The compound of claim 1, or a stereoisomer, pharmaceutically acceptable salt, solvate or prodrug thereof, wherein X is a bond, NH, -C(O)NH- or - NHC(O)-.

20. The compound of claim 1, or a stereoisomer, pharmaceutically acceptable salt, solvate or prodrug thereof, wherein A₁ is CR₁; A₂ is CR₂; and A₃ is CR₃.

21. The compound of claim 1, or a stereoisomer, pharmaceutically acceptable salt, solvate or prodrug thereof, wherein Z₁ is CR₀₂; Z₂ is CR₀₃; and Z₃ is N.

22. The compound of claim 1, or a stereoisomer, pharmaceutically acceptable salt, solvate or prodrug thereof, wherein Z₁ is CR₀₂; Z₂ is CR₀₃; and Z₃ is CR₀₄.

23. The compound of claim 1, or a stereoisomer, pharmaceutically acceptable salt, solvate or prodrug thereof, wherein Z₁ is N; Z₂ is CR₀₃; and Z₃ is CR₀₄.

24. The compound of claim 1, or a stereoisomer, pharmaceutically acceptable salt, solvate or prodrug thereof, wherein R_{A}, Rp are each independently hydrogen, cyano, acetyl, hydroxyl, carboxyl, nitro, halogen, C₁₋₃ alkyl, C₁₋₃ alkoxy, halogenated C₁₋₃ alkyl, halogenated C₁₋₃ alkoxy, halogenated C₁₋₃ alkylthio, NRₐ₀R_{b0}, -CONRₐ₀R_{b0}, -SO₂NRₐ₀R_{b0}, -SO₂C₁₋₃ alkyl, phenyl, 5- or 6-membered monocyclic heteroaryl, C₃₋₆ cycloalkyl, or 3- to 6-membered saturated monoheterocycle, wherein the phenyl, 5- or 6-membered monocyclic heteroaryl, C₃₋₆ cycloalkyl, 3- to 6-membered saturated monoheterocycle are unsubstituted or substituted with 1, 2 or 3 substituents each independently selected from acetyl, hydroxyl, cyano, carboxyl, nitro, halogen, C₁-₃ alkyl, halogenated C₁₋₃ alkyl, C₁₋₃ alkoxy, halogenated C₁₋₃ alkoxy, C₃₋₆ cycloalkyl, NRₐ₀R_{b0}, -CONRₐ₀R_{b0}, or -SO₂NRₐ₀R_{b0}; and wherein Rₐ₀, R_{b0} are each independently hydrogen, C₁₋₃ alkyl or halogenated C₁₋₃ alkyl.

25. The compound of claim 1, or a stereoisomer, pharmaceutically acceptable salt, solvate or prodrug thereof, wherein R₁, R₂, R₃, R₄ are each independently hydrogen, hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, halogenated C₁₋₃ alkylthio, -NRₐ₀R_{b0}, C₃₋₆ cycloalkyl, -CONRₐ₀R_{b0}, -SO₂NRₐ₀R_{b0}; and wherein Rₐ₀, R_{b0} are each independently hydrogen or C₁₋₃ alkyl.

26. The compound of claim 1, or a stereoisomer, pharmaceutically acceptable salt, solvate or prodrug thereof, wherein R₁, R₂, R₃ are hydrogen; R₄ is hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, halogenated C₁₋₃ alkylthio, -NRₐ₀R_{b0}, C₃₋₆ cycloalkyl, -CONRₐ₀R_{b0}, or -SO₂NRₐ₀R_{b0}; and wherein Rₐ₀, R_{b0} are each independently hydrogen or C₁₋₃ alkyl.

27. The compound of claim 1, or a stereoisomer, pharmaceutically acceptable salt, solvate or prodrug thereof, wherein R₀₁, R₀₂, R₀₃ are each independently hydrogen, hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, halogenated C₁₋₃ alkylthio, -NRₐ₀R_{b0}, C₃₋₆ cycloalkyl, -CONRₐ₀R_{b0}, or -SO₂NRₐ₀R_{b0}; and wherein Rₐ₀, R_{b0} are each independently hydrogen or C₁₋₃ alkyl.

28. The compound of claim 1, or a stereoisomer, pharmaceutically acceptable salt, solvate or prodrug thereof, wherein R₁, R₂, R₃, R₀₂, R₀₃ are hydrogen; and R₀₁, R₄ are each independently F, Cl, cyano, C₁₋₃ alkyl, C₁₋₃ alkoxy, halogenated C₁₋₃ alkyl or halogenated C₁₋₃ alkoxy.

29. The compound of claim 1, or a stereoisomer, pharmaceutically acceptable salt, solvate or prodrug thereof, wherein L, W are each independently substituted or unsubstituted phenyl or pyridyl; the "substituted" means that 1, 2 or 3 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, C₁₋₃ alkoxy, halogenated C₁₋₃ alkylthio, -NRₐ₀R_{b0}, C₃₋₆ cycloalkyl, -CONRₐ₀R_{b0}, or -SO₂NRₐ₀R_{b0}; and wherein Rₐ₀, R_{b0} are each independently hydrogen or C₁₋₃ alkyl.

30. The compound of claim 1, or a stereoisomer, pharmaceutically acceptable salt, solvate or prodrug thereof, wherein L, W are each independently substituted or unsubstituted 8- to 10-membered bicyclic heteroaryl ring ; the "substituted" means that 1, 2 or 3 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, C₁₋₃ alkoxy, halogenated C₁₋₃ alkylthio, -NRₐ₀R_{b0}, C₃₋₆ cycloalkyl, -CONRₐ₀R_{b0}, or -SO₂NRₐ₀R_{b0}; and wherein Rₐ₀, R_{b0} are each independently hydrogen or C₁₋₃ alkyl.

31. The compound of claim 1, or a stereoisomer, pharmaceutically acceptable salt, solvate or prodrug thereof, wherein L is substituted or unsubstituted phenyl or pyridyl; W is substituted or unsubstituted 8- to 10-membered bicyclic heteroaryl ring; and the "substituted" means that 1, 2 or 3 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, C₁₋₃ alkoxy, halogenated C₁₋₃ alkylthio, -NRₐ₀R_{b0}, C₃₋₆ cycloalkyl, -CONRₐ₀R_{b0}, or -SO₂NRₐ₀R_{b0}; and wherein Rₐ₀, R_{b0} are each independently hydrogen or C₁₋₃ alkyl.

32. The compound of claim 1, or a stereoisomer, pharmaceutically acceptable salt, solvate or prodrug thereof, wherein L is substituted or unsubstituted 8- to 10-membered bicyclic heteroaryl ring ; W is substituted or unsubstituted phenyl or pyridyl; and the "substituted" means that 1, 2 or 3 hydrogen atoms in the group are substituted by substituents each independently selected from the following group: hydroxyl, cyano, nitro, acetyl, carboxyl, F, Cl, hydroxymethyl, hydroxyethyl, cyanomethyl, cyanoethyl, C₁₋₃ alkyl, halogenated C₁₋₃ alkyl, halogenated C₁₋₃ alkoxy, C₁₋₃ alkoxy, halogenated C₁₋₃ alkylthio, -NRₐ₀R_{b0}, C₃₋₆ cycloalkyl, -CONRₐ₀R_{b0}, or -SO₂NRₐ₀R_{b0}; and wherein Rₐ₀, R_{b0} are each independently hydrogen or C₁₋₃ alkyl.

33. The compound of claim 1, or a stereoisomer, pharmaceutically acceptable salt, solvate or prodrug thereof, wherein the compound is selected from Table A.

34. The compound of claim 1, or a stereoisomer, pharmaceutically acceptable salt, solvate or prodrug thereof, wherein the compound is selected from Table B.

35. The compound of claim 2, or a stereoisomer, pharmaceutically acceptable salt, solvate or prodrug thereof, wherein the 8- to 10-membered bicyclic heteroaryl ring in L and W is selected from:

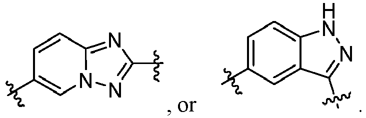

36. The compound of claim 2, or a stereoisomer, pharmaceutically acceptable salt, solvate or prodrug thereof, wherein the formula A, formula B, formula C, and formula D are independently selected from the following structures:

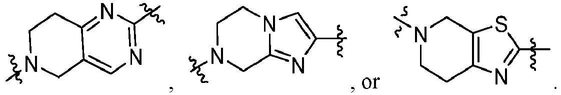

37. The compound of claim 1 or 2, or a stereoisomer, pharmaceutically acceptable salt, solvate or prodrug thereof, wherein L and W are each independently selected from the following rings: 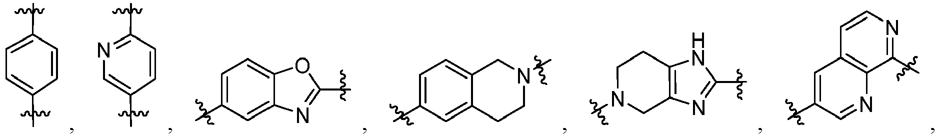 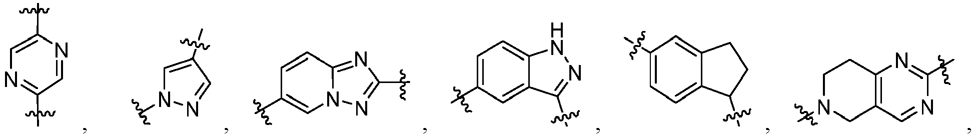 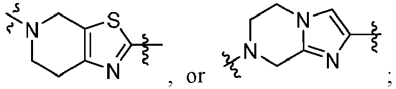 and the ring is unsubstituted or substituted with 1, 2 or 3 substituents independently selected from Rp.

38. The compound of claim 1, or a stereoisomer, pharmaceutically acceptable salt, solvate or prodrug thereof, wherein -NR^{g}R^{h} is selected from:

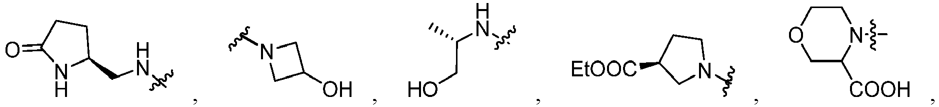

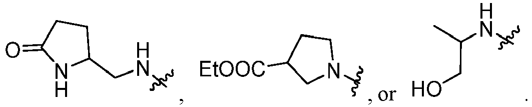

39. The compound of claim 1, or a stereoisomer, pharmaceutically acceptable salt, solvate or prodrug thereof, wherein -NR^{m3}R^{m4} is selected from:

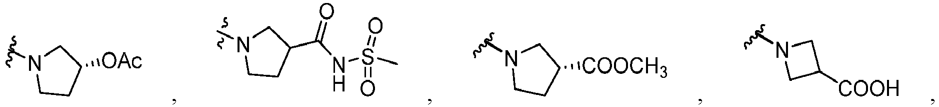

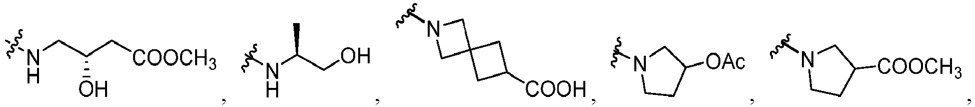

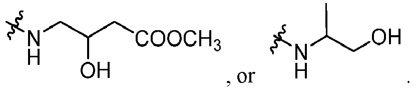

40. A pharmaceutical composition, comprising: the compound of any one of claims 1 to 39, or a stereoisomer, pharmaceutically acceptable salt, solvate or prodrug thereof; and a pharmaceutically acceptable carrier.

41. A pharmaceutical composition, comprising: the compound of any one of claims 1 to 39, or a stereoisomer, pharmaceutically acceptable salt, solvate or prodrug thereof; and at least one other agent, wherein the other agent is an anticancer agent, a chemotherapeutic agent, or an antiproliferative compound.

42. Use of the compound of any one of claims 1 to 39, or a stereoisomer, pharmaceutically acceptable salt, solvate or prodrug thereof; or the pharmaceutical composition of claim 40 in the preparation of a drug, and the drug is for treating a cancer or infectious disease.

43. The use of claim 42, wherein the cancer is selected from: bone cancer, cancer of the head or neck, pancreatic cancer, skin cancer, cutaneous or intraocular malignant melanoma, uterine cancer, ovarian cancer, rectal cancer, cancer of the anal region, stomach cancer, testicular cancer, uterine cancer, fallopian tube cancer, endometrial cancer, cervical cancer, vaginal cancer, vulval cancer, Hodgkin's Disease, non-Hodgkin's lymphoma, esophagus cancer, small intestine cancer, cancer of the endocrine system, thyroid gland cancer, parathyroid gland cancer, adrenal gland cancer, soft tissue sarcoma, urethral cancer, penis cancer, chronic or acute leukemias including acute myeloid leukemia, chronic myeloid leukemia, acute lymphocytic leukemia, chronic lymphocytic leukemia, childhood solid tumours, lymphocytic lymphoma, bladder cancer, kidney or ureter cancer, renal pelvis cancer, central nervous system (CNS) neoplasm, primary CNS lymphoma, tumour angiogenesis, spinal axis tumour, brain stem glioma, pituitary adenoma, Kaposi's sarcoma, epidermoid cancer, squamous cell cancer, T-cell lymphoma, environmentally induced cancers including those induced by asbestos, and combinations of said cancers.

44. The use of claim 42, wherein the infectious disease is a bacterial infectious disease, a viral infectious disease or a fungal infectious disease.
